# EUROPEAN PATENT SPECIFICATION

(11) **EP 3 322 733 B1**
(45) Date of publication and mention of the grant of the patent: **15.09.2021**
(21) Application number: 16751633.5
(22) Date of filing: 13.07.2016
(51) Int. Cl.: C07K 16/28, A61K 39/395, A61P 35/00

(54) **HIDE1 COMPOSITIONS AND METHODS**
HIDE1-ZUSAMMENSETZUNGEN UND VERFAHREN
COMPOSITIONS DE HIDE1 ET MÉTHODES ASSOCIÉES

(30) Priority: 13.07.2015 US 201562191775 P; 13.07.2015 US 201562191804 P
(43) Date of publication of application: 23.05.2018
(60) Divisional application: 21188908.4
(73) Proprietor: Compugen Ltd., 5885849 Holon (IL)
(72) Inventor: DICKEN, Yosef, 5885849 Holon (IL); DASSA, Liat, 5885849 Holon (IL); NOVIK, Amit, 5885849 Holon (IL); TOPORIK, Amir, 5885849 Holon (IL); COJOCARU, Gad, S., 5885849 Holon (IL); KLIGER, Yossef, 5885849 Holon (IL); BENITA, Yair, 5885849 Holon (IL); LEVY, Ofer, 5885849 Holon (IL); VAKNIN, Ilan, 5885849 Holon (IL); MACHLENKIN, Arthur, 5885849 Holon (IL); HECHT, Iris, 5885849 Holon (IL); KIM, Jungmin, 5885849 Holon (IL); POW, Andrew, 5885849 Holon (IL); DRAKE, Andrew, W., 5885849 Holon (IL)
(74) Representative: Fuchs Patentanwälte Partnerschaft mbB
(86) International application number: PCT/IB2016/001079
(87) International publication number: WO 2017/009712

(56) References cited:
- EP-A1- 1 712 563
- WO-A2-01/09162
- WO-A2-02/066600
- WO-A2-2004/080148

## Description

### BACKGROUND OF THE INVENTION

Naive T cells must receive two independent signals from antigen-presenting cells (APC) in order to become productively activated. The first, Signal 1, is antigen-specific and occurs when T cell antigen receptors encounter the appropriate antigen-MHC complex on the APC. The fate of the immune response is determined by a second, antigen-independent signal (Signal 2) which is delivered through a T cell costimulatory molecule that engages its APC-expressed ligand. This second signal could be either stimulatory (positive costimulation) or inhibitory (negative costimulation or coinhibition). In the absence of a costimulatory signal, or in the presence of a coinhibitory signal, T-cell activation is impaired or aborted, which may lead to a state of antigen-specific unresponsiveness (known as T-cell anergy), or may result in T-cell apoptotic death.

Costimulatory molecule pairs usually consist of ligands expressed on APCs and their cognate receptors expressed on T cells. The prototype ligand/receptor pairs of costimulatory molecules are B7/CD28 and CD40/CD40L. The B7 family consists of structurally related, cell-surface protein ligands, which may provide stimulatory or inhibitory input to an immune response. Members of the B7 family are structurally related, with the extracellular domain containing at least one variable or constant immunoglobulin domain.

Both positive and negative costimulatory signals play critical roles in the regulation of cell-mediated immune responses, and molecules that mediate these signals have proven to be effective targets for immunomodulation. Based on this knowledge, several therapeutic approaches that involve targeting of costimulatory molecules have been developed, and were shown to be useful for prevention and treatment of cancer by turning on, or preventing the turning off, of immune responses in cancer patients and for prevention and treatment of autoimmune diseases and inflammatory diseases, as well as rejection of allogenic transplantation, each by turning off uncontrolled immune responses, or by induction of "off signal" by negative costimulation (or coinhibition) in subjects with these pathological conditions.

Manipulation of the signals delivered by B7 ligands has shown potential in the treatment of autoimmunity, inflammatory diseases, and transplant rejection. Therapeutic strategies include blocking of costimulation using monoclonal antibodies to the ligand or to the receptor of a costimulatory pair, or using soluble fusion proteins composed of the costimulatory receptor that may bind and block its appropriate ligand. Another approach is induction of co-inhibition using soluble fusion protein of an inhibitory ligand. These approaches rely, at least partially, on the eventual deletion of auto- or allo-reactive T cells (which are responsible for the pathogenic processes in autoimmune diseases or transplantation, respectively), presumably because in the absence of costimulation (which induces cell survival genes) T cells become highly susceptible to induction of apoptosis. Thus, novel agents that are capable of modulating costimulatory signals, without compromising the immune system's ability to defend against pathogens, are highly advantageous for treatment and prevention of such pathological conditions.

Costimulatory pathways play an important role in tumor development. Interestingly, tumors have been shown to evade immune destruction by impeding T cell activation through inhibition of co-stimulatory factors in the B7-CD28 and TNF families, as well as by affecting regulatory T cells, which inhibit anti-tumor T cell responses (see Wang (2006), "Immune Suppression by Tumor Specific CD4+ Regulatory T cells" in Cancer. Semin. Cancer. Biol. 16:73-79; Greenwald, et al. (2005), "The B7 Family Revisited", Ann. Rev. Immunol. 23:515-48; Watts (2005) "TNF/TNFR Family Members in Co-stimulation of T Cell Responses", Ann. Rev. Immunol. 23:23-68; Sadum, et al. (2007), "Immune Signatures of Murine and Human Cancers Reveal Unique Mechanisms of Tumor Escape and New Targets for Cancer Immunotherapy", Clin. Canc. Res. 13(13): 4016-4025). Such tumor expressed co-stimulatory molecules have become attractive cancer biomarkers and may serve as tumor-associated antigens (TAAs). Furthermore, costimulatory pathways have been identified as immunologic checkpoints that attenuate T cell dependent immune responses, both at the level of initiation and effector function within tumor metastases. As engineered cancer vaccines continue to improve, it is becoming clear that such immunologic checkpoints are a major barrier to the vaccines' ability to induce therapeutic anti-tumor responses. In that regard, costimulatory molecules can serve as adjuvants for active (vaccination) and passive (antibody-mediated) cancer immunotherapy, providing strategies to thwart immune tolerance and stimulate the immune system.

In addition, such agents could be of use in other types of cancer immunotherapy, such as adoptive immunotherapy, in which tumor-specific T cell populations are expanded and directed to attack and kill tumor cells. Agents capable of augmenting such anti-tumor response have great therapeutic potential and may be of value in the attempt to overcome the obstacles to tumor immunotherapy. Recently, novel agents that modulate several costimulatory pathways were indeed introduced to the clinic as cancer immunotherapy.

Regulating costimulation using agonists and/or antagonists to various costimulatory proteins has been extensively studied as a strategy for treating autoimmune diseases, graft rejection, allergy and cancer. This field has been clinically pioneered by CTLA4-Ig (Abatacept, Orencia®) which is approved for treatment of RA, mutated CTLA4-Ig (Belatacept, Nulojix®) for prevention of acute kidney transplant rejection and by the anti-CTLA4 antibody (Ipilimumab, Yervoy®), recently approved for the treatment of melanoma. Other costimulation regulators are currently in advanced stages of clinical development including anti-PD-1 antibody (BMS-936558) which is in development for treatment of Non-Small Cell Lung cancer and other cancers. Furthermore, such agents are also in clinical development for viral infections, for example the anti PD-1 Ab, MDX-1106, which is being tested for treatment of hepatitis C, and the anti-CTLA-4 Ab CP-675,206 (tremelimumab) which is in a clinical trial in hepatitis C virus-infected patients with hepatocellular carcinoma.

In addition recently researchers have developed compounds which target regulatory T cells (iTregs) for use in immunotherapy. With respect thereto it is known that inducible regulatory T cells (iTregs) are commonly seen in many tumors, and form the major subset of immune suppressor cells in the tumor tissue and moreover represent a major tumor resistance mechanism from immune surveillance. Accordingly, iTregs are therefore viewed as important cellular targets for cancer therapy.

Multiple immune-checkpoint receptors, such as CTLA4, PD-1, TIM3 and LAG3, are expressed at high levels on the surface of iTregs and directly promote Treg cell-mediated suppression of effector immune responses. Therefore, some immune-checkpoint antibodies may, in addition to increasing CTL immunity, further block the immunosuppressive activity of iTregs and thereby enhance anti-tumor immunity. For example, CTLA4 blockade by ipilimumab both enhances effector T cell activity, and inhibits Treg immunosuppressive activity.

B cells play a critical role in recognition of foreign antigens and they produce the antibodies necessary to provide protection against various types of infectious agents. T cell help to B cells is a pivotal process of adaptive immune responses. Follicular helper T (Tfh) cells are a subset of CD4⁺ T cells specialized in B cell help (reviewed by Crotty, Annu. Rev. Immunol. 29: 621-663, 2011). Tfh cells express the B cell homing chemokine receptor, CXCR5, which drives Tfh cell migration into B cell follicles within lymph nodes in a CXCL13-dependent manner. The requirement of Tfh cells for B cell help and T celldependent antibody responses indicates that this cell type is of great importance for protective immunity against various types of infectious agents, as well as for rational vaccine design. In addition, regulatory B cells (Bregs) have a role in impairing effective clearance of tumors. The mechanisms for Bregs effects in cancer are not well understood, however one proposed mechanism is through inhibition of cytotoxic CD8⁺ T cells.

NK cells are effector lymphocytes of the innate immune system that are known to be involved in killing of pathological or diseased cells such as cancer and infected cells and pathogens. Natural killer cells have the capacity to kill cellular targets and produce cytokines without prior specific sensitization. NK cells are unique, as they have the ability to recognize stressed cells in the absence of antibodies and MHC, allowing for a much faster immune reaction. This role is especially important because harmful cells that are missing MHC 1 markers cannot be detected and destroyed by other immune cells, such as T cells.

Induction of immune tolerance has long been considered the "holy grail" for autoimmune disease therapy. The immune system has the reciprocal tasks to protect the host against invading pathogens, but simultaneously to prevent damage resulting from unwanted reactions to self-antigens. The latter part is known as immune tolerance and performed by a complex set of interactive and complementary pathways, which regulate immune responses. T cells have the ability to react to a variety of antigens, both self and nonself. Therefore, there are many mechanisms that exist naturally to eliminate potentially self-reactive responses - this is known as natural tolerance. The main mechanism for eliminating potential autoreactive T cells occurs in the thymus and is known as central tolerance. Some potentially autoreactive T cells escape central tolerance and, therefore, peripheral tolerance mechanisms also exist. Despite these mechanisms, some self-reactive T cells may 'escape' and be present in the repertoire; it is believed that their activation may lead to autoimmune disease.

Studies on therapeutic tolerance have attempted to induce and amplify potent physiological mechanisms of tolerance in order to eliminate or neutralize self-reactive T cells and prevent or treat autoimmune diseases. One way to induce tolerance is by manipulation of the interaction between costimulatory ligands and receptors on antigen presenting cells (APCs) and lymphocytes.

CTLA-4 is the most extensively studied costimulatory molecule which downregulates immune responses. The attributes of immunosuppressive qualities and capacity to induce tolerance have made its recognition as a potential immuno-therapeutic agent for autoimmune mediated inflammatory disorders. Abatacept (commercial name: Orencia) is a fusion protein composed of the ECD (extracellular domain) of CTLA-4 fused to the Fc fragment of hIgG1. Abatacept is believed to induce costimulation blockade, which has been approved for treating patients with rheumatoid arthritis, by effectively interfering with the inflammatory cascade.

Induction of disease control with the current therapies, followed by progressive withdrawal in parallel with re-establishing immune tolerance, may be an attractive approach in the future of autoimmune therapies. Furthermore, due to their immune specificity, in the absence of global immunosuppression, such therapies should be safe for chronic use.

Certain mechanisms are known to be widespread in various autoimmune diseases. T helper type 1 (Th1) cells are induced by IL-12 and produce IFN-γ; while T helper type 2 (Th2) cells secrete IL-4, IL-5 and IL-13. Th1 cells can mediate proinflammatory or cell-mediated immune responses, whereas Th2 cells mainly promote certain types of humoral immunity. Some immune related diseases, such as autoimmune reactions, inflammation, chronic infection and sepsis, are characterized by a dysregulation of the pro- versus antiinflammatory tendencies of the immune system, as well as an imbalance in the Th1 versus Th2 cytokine balance. During inflammation, induction of a shift in the balance from Th1 to Th2 protects the organism from systemic 'overshooting' with Th1/pro-inflammatory cytokines, by reducing the inflammatory tendencies of the immune system. Immunomodulatory therapies that are associated with a Th1 to Th2 immune shift have protective effects in Th1-mediated autoimmune diseases, such as multiple sclerosis and rheumatoid arthritis. For example, Laquinimod, which has demonstrated efficacy in animal models of several autoimmune diseases including MS, shows immunomodulatory effects through Thl/Th2 shift, and does not lead to immunosuppression. Glatiramer acetate (Copaxone®) also induces Th1/Th2 shift with decreased secretion of proinflammatory cytokines, and increased secretion of antiinflammatory cytokines. Furthermore, glatiramer acetate -specific Th2 cells are able to migrate across the blood-brain barrier and cause in situ bystander suppression of autoaggressive Th1 T cells.

Certain immune cells and immune cell signal transduction pathways are promising targets for new agents for treating immune disorders. For example Th1, Th17, Th2 and regulatory T cells (Tregs) play important roles in modulating autoimmunity and inflammation. Mounting evidence from numerous studies shows the importance of these immune cells in disorders such as rheumatoid arthritis, inflammatory bowel disease, multiple sclerosis, psoriasis, lupus erythematosus, type 1 diabetes and uveitis. Most existing therapies target only one pathway at a time.

Accordingly, there is a need for additional agonists and antagonists of immune checkpoint pathways.

EP 1 712 563 A1 describes that activated lymphocytes may be used for treatment of cancer. An anti-HIDE1 antibody is used for obtaining such activated lymphocytes. However, the antibody is not used for the activation step itself. Rather, it is used for removing HIDE1-expressing monocytes from a blood sample so that lymphocytes are enriched for a subsequent activation step.

### BRIEF SUMMARY OF THE INVENTION

The present invention provides for an anti-HIDE1 antibody for use in treating cancer in a patient, the use comprising administering the anti-HIDE1 antibody to said patient, wherein said cancer is treated, and wherein the antibody stimulates the immune system by inhibiting the action of HIDE1.

According to at least some embodiments, the cancer is treated by activating cytotoxic T cells (CTLs) of the patient, wherein a subset of the CTLs of said patient are activated.

According to at least some embodiments, the cancer is treated by activating NK cells of the patient, wherein a subset of the NK cells of said patient are activated.

According to at least some embodiments, the cancer is treated by activating γδ T cells of the patient, wherein a subset of the γδ T cells of said patient are activated.

According to at least some embodiments, the cancer is treated by activating Th1 cells of the patient, wherein a subset of the Th1 cells of said patient are activated.

According to at least some embodiments, the cancer is treated by decreasing or eliminating cell number and/or activity of at least one of regulatory T cells (Tregs) in the patient.

According to at least some embodiments, the cancer is treated by increasing interferon-y production and/or pro-inflammatory cytokine secretion in the patient.

According to at least some embodiments, the cancer is treated by modulating myeloid cell polarization in the patient.

According to at least some embodiments, the cancer is treated by modulating myeloid cell shifting toward a pro-inflammatory response in the patient.

According to at least some embodiments, the cancer is treated by shifting myeloid from M2 toward M1 phenotype in the patient.

According to at least some embodiments, the cancer is treated by modulating myeloid cell in the TME to support anti-cancer immune response in the patient.

According to at least some embodiments, the cancer is treated by restricting the pro-tumorigenic effects of the myeloid cells in the tumor microenvironment in the patient.

According to at least some embodiments, the cancer is treated by eliciting one or more of the following effects on immunity in the patient, wherein said effect is selected from the group consisting of: i) increases immune response, (ii) increases T cell activity, (iii) increases activation of αβ and/or γδ T cells, (iv) increases cytotoxic T cell activity, (v) increases NK and/or NKT cell activity, (vi) alleviates αβ and/or γδ T-cell suppression, (vii) increases pro-inflammatory cytokine secretion, (viii) increases IL-2 secretion; (ix) increases interferon-y production, (x) increases Th1 response, (xi) decrease Th2 response, (xii) decreases or eliminates cell number and/or activity of at least one of regulatory T cells (Tregs), myeloid derived suppressor cells (MDSCs), iMCs, mesenchymal stromal cells, TIE2-expressing monocytes, (xiii) reduces regulatory cell activity, and/or the activity of one or more of myeloid derived suppressor cells (MDSCs), iMCs, mesenchymal stromal cells, TIE2-expressing monocytes, (xiv) decreases or eliminates M2 macrophages, (xv) reduces M2 macrophage pro-tumorigenic activity, (xvi) decreases or eliminates N2 neutrophils, (xvii) reduces N2 neutrophils pro-tumorigenic activity, (xviii) reduces inhibition of T cell activation, (xix) reduces inhibition of CTL activation, (xx) reduces inhibition of NK and/or NKT cell activation, (xxi) reverses αβ and/or γδ T cell exhaustion, (xxii) increases αβ and/or γδ T cell response, (xxiii) increases activity of cytotoxic cells, (xxiv) stimulates antigen-specific memory responses, (xxv) elicits apoptosis or lysis of cancer cells, (xxvi) stimulates cytotoxic or cytostatic effect on cancer cells, (xxvii) induces direct killing of cancer cells, (xxviii) increases Th17 activity and (xxix) modulating myeloid cell polarization, (xxx) modulating myeloid cell shifting toward a pro-inflammatory response, (xxxi) shifting myeloid from M2 toward M1 phenotype, (xxxii) modulating myeloid cell in the TME to support anti-cancer immune response, (xxxiii) restricting the pro-tumorigenic effects of the myeloid cells in the TME, (xxxiv) enhancing myeloid and lymphoid infiltration into the tumor cite thereby shifting the tumor into more immunogenic, (xxxv) induces complement dependent cytotoxicity and/or antibody dependent cell-mediated cytotoxicity.

According to at least some embodiments, the cancer is treated by depleting myeloid cells or other circulating tumor cells expressing HIDE1 from a patient or patient sample, said method comprising: i) contacting said patient or said patient sample with an anti-HIDE1 antibody, wherein said anti-HIDE1 antibody binds to HIDE1 expressing cells, ii) identifying cells to which said anti-HIDE1 antibody has bound, and iii) removing said cells in step ii) from said patient or said patient sample.

In some embodiments, the cancer is selected from the group consisting of Acute Myeloid Leukemia, Acute Myeloid Leukemia Induction Failure, Acute Lymphoblastic Leukemia, Diffuse Large B-cell Lymphoma, Malignant Lymphoma, Non-Hodgkin Lymphoma, Diffuse Large B-Cell Lymphoma, Glioblastoma multiforme, Mesothelioma, Thymoma, Testicular Germ Cell Tumors, Kidney renal clear cell carcinoma, Sarcoma, Brain Lower Grade Glioma, Chronic Lymphocytic Leukemia, Non-Hodgkin Lymphoma - Follicular Lymphoma, Uterine Carcinosarcoma, Pediatric Brain Tumors, Lung adenocarcinoma, Cervical squamous cell carcinoma, endocervical adenocarcinoma, Pancreatic adenocarcinoma, Skin Cutaneous Melanoma, Kidney renal papillary cell carcinoma, Liver hepatocellular carcinoma; Bladder Urothelial Carcinoma, Colon adenocarcinoma, Head and Neck squamous cell carcinoma, Lung squamous cell carcinoma, Rectum adenocarcinoma, and Stomach adenocarcinoma. In some embodiments, the cancer is a cancer having high immune infiltrate of myeloid cells expressing HIDE1.

In some embodiments, the anti-HIDE1 antibody is selected from the group consisting of CPA.12.001 human IgG4, CPA.12.002 human IgG4, CPA.12.003 human IgG4, CPA. 12.004 human IgG4, CPA. 12.005 human IgG4, CPA.12.006 human IgG4, CPA.12.007 human IgG4, CPA.12.008 human IgG4, CPA.12.009 human IgG4, CPA. 12.011 human IgG4, CPA.12.012 human IgG4, CPA. 12.013 human IgG4, CPA. 12.014 human IgG4, and CPA. 12.015 human IgG4. In some embodiments, the anti-HIDE1 antibody is selected from the group consisting of CPA.12.006-H4, CPA.12.007-H4, and CPA.12.0012-H4.

In some embodiments, the anti-HIDE1 antibody is selected from the group consisting of CPA.12.001, CPA.12.002, CPA.12.003, CPA.12.004, CPA.12.005, CPA.12.006, CPA. 12.007, CPA. 12.008, CPA.12.009, CPA.12.011, CPA.12.012, CPA.12.013, CPA.12.014, and CPA.12.015. In some embodiments, the anti-HIDE1 antibody is selected from the group consisting of CPA.12.006, CPA. 12.007, and CPA. 12.0012.

According to at least some embodiments, the present invention also provides for an anti-HIDE1 antigen-binding domain comprising: a) a heavy chain variable domain comprising a vhCDR1, vhCDR2, and vhCDR3 from an anti-HIDE1 antibody; and b) a light chain variable domain comprising a vlCDR1, vlCDR2, and vlCDR3 from said anti-HIDE1 antibody; wherein said anti-HIDE1 antibody is selected from the group consisting of CPA. 12.001 human IgG4, CPA. 12.002 human IgG4, CPA.12.003 human IgG4, CPA.12.004 human IgG4, CPA.12.005 human IgG4, CPA. 12.006 human IgG4, CPA. 12.007 human IgG4, CPA. 12.008 human IgG4, CPA. 12.009 human IgG4, CPA.12.011 human IgG4, CPA.12.012 human IgG4, CPA. 12.013 human IgG4, CPA. 12.014 human IgG4, and CPA.12.015 human IgG4.

According to at least some embodiments, the present invention also provides for an anti-HIDE1 antigen-binding domain comprising: a) a heavy chain variable domain comprising a vhCDR1, vhCDR2, and vhCDR3 from an anti-HIDE1 antibody; and b) a light chain variable domain comprising a vlCDR1, vlCDR2, and vlCDR3 from said anti-HIDE1 antibody; wherein said anti-HIDE1 antibody is selected from the group consisting of CPA.12.001, CPA. 12.002, CPA.12.003, CPA.12.004, CPA.12.005, CPA. 12.006, CPA. 12.007, CPA. 12.008, CPA.12.009, CPA.12.011, CPA.12.012, CPA.12.013, CPA. 12.014, and CPA.12.015.

In some embodiments, the anti-HIDE1 antigen binding domain of the antibody is a single chain Fv (scFv), wherein said heavy chain variable domain and said light chain variable domain are covalently attached via a scFv linker.

According to at least some embodiments, the present invention also provides for an anti-HIDE1 antibody comprising: a) a heavy chain variable domain comprising a vhCDR1, vhCDR2, and vhCDR3 from an anti-HIDE1 antibody; and b) a light chain variable domain comprising a vlCDR1, vlCDR2, and vlCDR3 from said anti-HIDE1 antibody; wherein said anti-HIDE1 antibody is selected from the group consisting of CPA.12.001 human IgG4, CPA. 12.002 human IgG4, CPA. 12.003 human IgG4, CPA.12.004 human IgG4, CPA.12.005 human IgG4, CPA.12.006 human IgG4, CPA. 12.007 human IgG4, CPA. 12.008 human IgG4, CPA.12.009 human IgG4, CPA.12.011 human IgG4, CPA.12.012 human IgG4, CPA.12.013 human IgG4, CPA.12.014 human IgG4, and CPA.12.015 human IgG4.

According to at least some embodiments, the present invention also provides for an anti-HIDE1 antibody comprising: a) a heavy chain variable domain comprising a vhCDR1, vhCDR2, and vhCDR3 from an anti-HIDE1 antibody; and b) a light chain variable domain comprising a vlCDR1, vlCDR2, and vlCDR3 from said anti-HIDE1 antibody; wherein said anti-HIDE1 antibody is selected from the group consisting of CPA.12.001, CPA. 12.002, CPA. 12.003, CPA. 12.004, CPA.12.005, CPA.12.006, CPA. 12.007, CPA. 12.008, CPA. 12.009, CPA. 12.011, CPA.12.012, CPA.12.013, CPA.12.014, and CPA.12.015.

According to at least some embodiments, the present invention also provides for an anti-HIDE1 antibody that competes for binding with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of CPA.12.001 human IgG4, CPA.12.002 human IgG4, CPA.12.003 human IgG4, CPA.12.004 human IgG4, CPA. 12.005 human IgG4, CPA. 12.006 human IgG4, CPA.12.007 human IgG4, CPA.12.008 human IgG4, CPA. 12.009 human IgG4, CPA. 12.011 human IgG4, CPA.12.012 human IgG4, CPA.12.013 human IgG4, CPA.12.014 human IgG4, and CPA.12.015 human IgG4.

According to at least some embodiments, the present invention also provides for an anti-HIDE1 antibody that competes for binding with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of CPA.12.001, CPA.12.002, CPA.12.003, CPA.12.004, CPA.12.005, CPA. 12.006, CPA. 12.007, CPA. 12.008, CPA.12.009, CPA.12.011, CPA.12.012, CPA.12.013, CPA.12.014, and CPA.12.015.

According to at least some embodiments, the present invention also provides for an anti-HIDE1 antibody that competes for binding with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of AB-506, AB-507, AB-508, AB-509, and AB-510.

According to at least some embodiments, the present invention also provides for an anti-HIDE1 antibody that competes for binding with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of 33B4, 36C1, and 39A7.

According to at least some embodiments, the present invention also provides for an anti-HIDE1 antibody that competes for functional activity with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of CPA. 12.001 human IgG4, CPA. 12.002 human IgG4, CPA. 12.003 human IgG4, CPA.12.004 human IgG4, CPA.12.005 human IgG4, CPA. 12.006 human IgG4, CPA. 12.007 human IgG4, CPA. 12.008 human IgG4, CPA.12.009 human IgG4, CPA.12.011 human IgG4, CPA.12.012 human IgG4, CPA. 12.013 human IgG4, CPA. 12.014 human IgG4, and CPA. 12.015 human IgG4.

According to at least some embodiments, the present invention also provides for an anti-HIDE1 antibody that competes for functional activity with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of CPA.12.001, CPA.12.002, CPA.12.003, CPA.12.004, CPA.12.005, CPA. 12.006, CPA. 12.007, CPA.12.008, CPA.12.009, CPA. 12.011, CPA. 12.012, CPA. 12.013, CPA. 12.014, and CPA. 12.015.

According to at least some embodiments, the present invention also provides for an anti-HIDE1 antibody that competes for functional activity with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of AB-506, AB-507, AB-508, AB-509, and AB-510.

According to at least some embodiments, the present invention also provides for an anti-HIDE1 antibody that competes for functional activity with an antibody comprising: a) a heavy chain variable domain comprising a vhCDR1, vhCDR2, and vhCDR3 from an anti-HIDE1 antibody; and b) a light chain variable domain comprising a vlCDR1, a vlCDR2 and vlCDR3, from said anti-HIDE1 antibody; wherein said anti-HIDE1 antibody is selected from the group consisting of 33B4, 36C1, and 39A7.

In some embodiments, in the composition comprising an anti-HIDE1 antibody comprise an antibody selected from the group consisting of CPA. 12.001 human IgG4, CPA. 12.002 human IgG4, CPA. 12.003 human IgG4, CPA.12.004 human IgG4, CPA.12.005 human IgG4, CPA.12.006 human IgG4, CPA.12.007 human IgG4, CPA. 12.008 human IgG4, CPA. 12.009 human IgG4, CPA. 12.011 human IgG4, CPA.12.012 human IgG4, CPA.12.013 human IgG4, CPA.12.014 human IgG4, and CPA.12.015 human IgG4. In some embodiments, in the composition comprising an anti-HIDE1 antibody, the antibody is selected from the group consisting CPA.12.006-H4, CPA.12.007-H4, and CPA. 12.0012-H4.

In some embodiments, in the composition comprising an anti-HIDE1 antibody comprise an antibody selected from the group consisting of CPA. 12.001, CPA.12.002, CPA. 12.003, CPA. 12.004, CPA.12.005, CPA.12.006, CPA. 12.007, CPA. 12.008, CPA. 12.009, CPA.12.011, CPA.12.012, CPA.12.013, CPA.12.014, and CPA.12.015. In some embodiments, in the composition comprising an anti-HIDE1 antibody, the antibody is selected from the group consisting CPA.12.006-H4, CPA.12.007-H4, and CPA. 12.0012-H4.

In some embodiments, the treatment is an increase in immune response. In some embodiments, the treatment is an increase in activation of αβ and/or γδ T cells. In some embodiments, the treatment is an increase in cytotoxic T cell activity. In some embodiments, the treatment is an increase in natural killer (NK) and/or NKT cell activity. In some embodiments, the treatment is an increase in αβ and/or γδ T-cell activity. In some embodiments, the treatment is an increase in pro-inflammatory cytokine secretion. In some embodiments, the treatment is increase in IL-2 secretion. In some embodiments, the treatment is an increase in interferon-y production. In some embodiments, the treatment is an increase in Th1 response. In some embodiments, the treatment is a decrease in the cell number and/or activity of regulatory T cells. In some embodiments, the treatment decreases cell number and/or activity of at least one or more cells selected from the group consisting of regulatory T cells (Tregs), myeloid derived suppressor cells (MDSCs), iMCs, mesenchymal stromal cells, and TIE2-expressing monocytes. In some embodiments, the treatment is decreases the cell activity, and/or the activity of one or more cells selected from the group consisting of myeloid derived suppressor cells (MDSCs), iMCs, mesenchymal stromal cells, and TIE2-expressing monocytes. In some embodiments, the said treatment is a decrease in M2 macrophages. In some embodiments, the treatment is a decrease in M2 macrophage activity. In some embodiments, the treatment is a decrease in N2 neutrophils. In some embodiments, the treatment is a decrease in N2 neutrophils activity. In some embodiments, the treatment is a decrease in inhibition of T cell activation. In some embodiments, the treatment is a decrease in inhibition of CTL activation. In some embodiments, the treatment is a decrease in inhibition of NK cell activation. In some embodiments, the treatment is a decrease in αβ and/or γδ T cell exhaustion. In some embodiments, the treatment is an increase in αβ and/or γδ T cell response. In some embodiments, the treatment is an increase in activity of cytotoxic cells. In some embodiments, the treatment is an induction of antigen-specific memory responses. In some embodiments, the treatment induces apoptosis or lysis of cells. In some embodiments, the treatment is an increase in cytotoxic or cytostatic effect on cells. In some embodiments, the treatment induces direct killing of cells. In some embodiments, the treatment is an increase in Th17 activity. In some embodiments, the treatment induces complement dependent cytotoxicity and/or antibody dependent cell-mediated cytotoxicity.

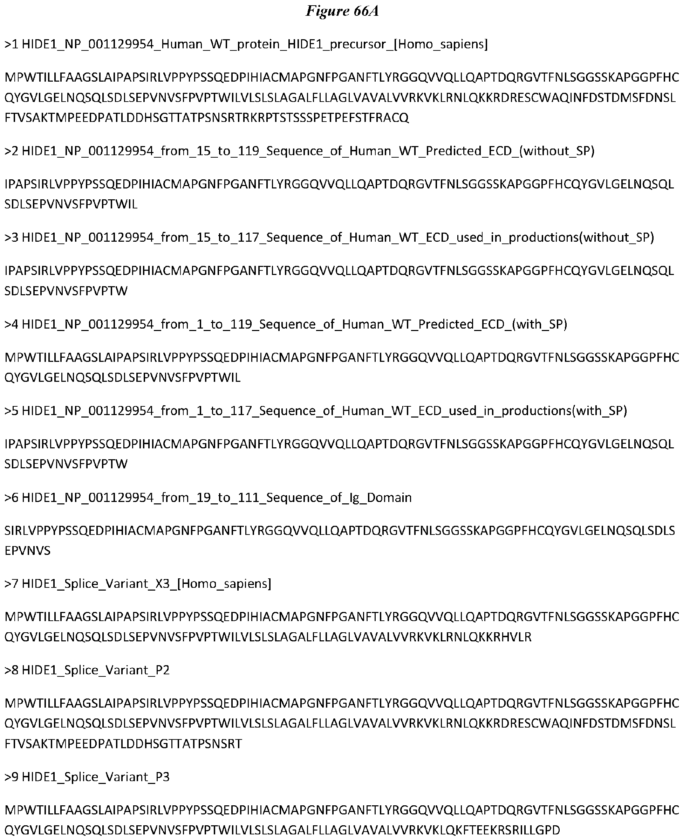

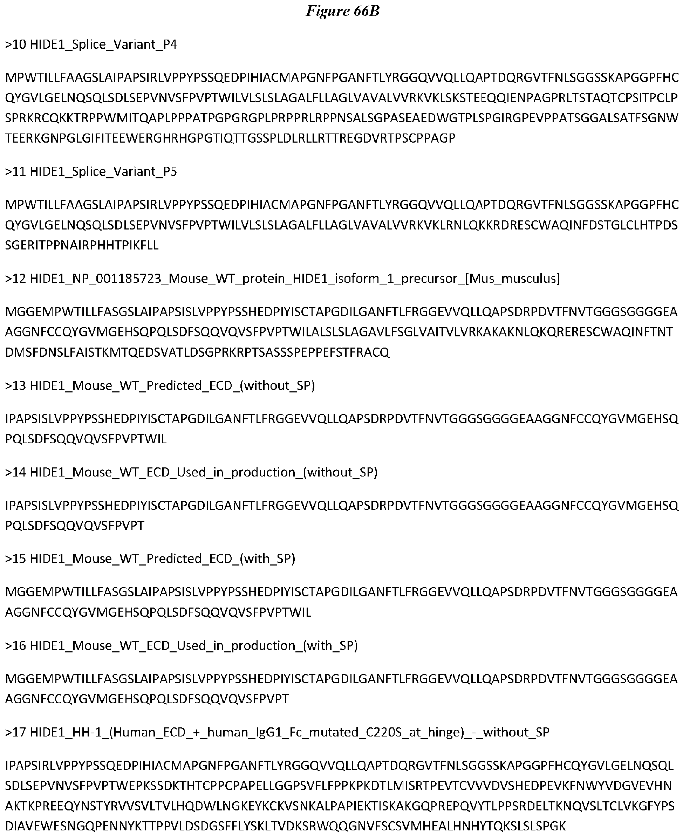

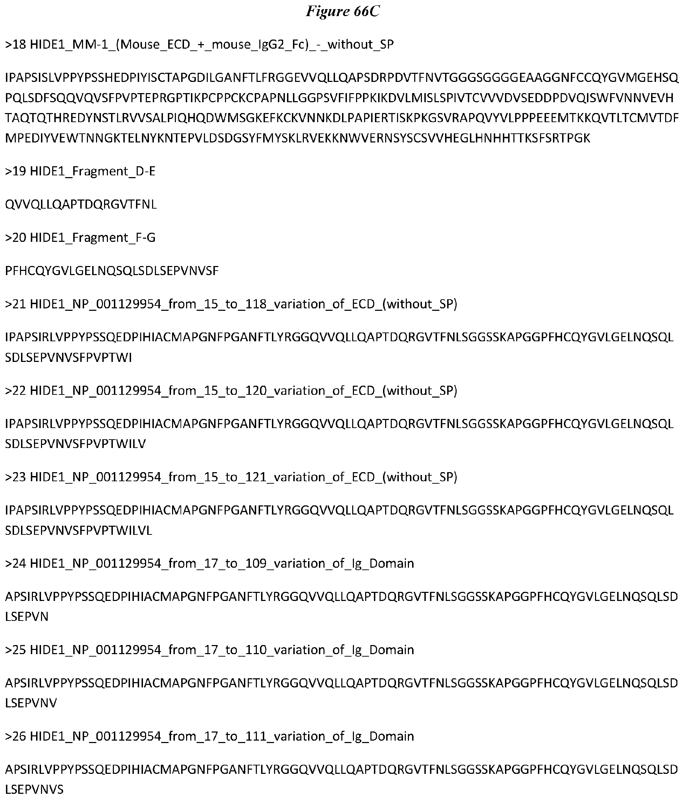

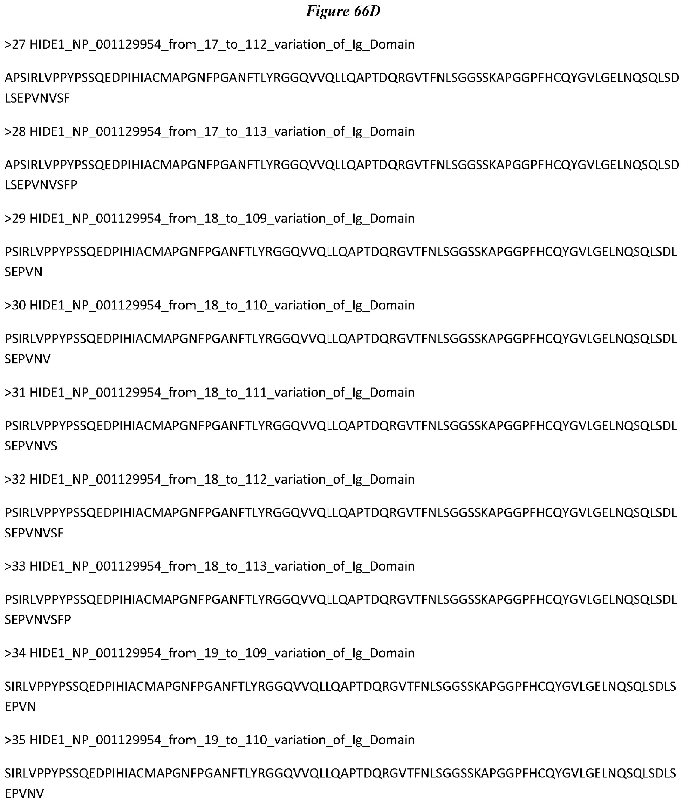

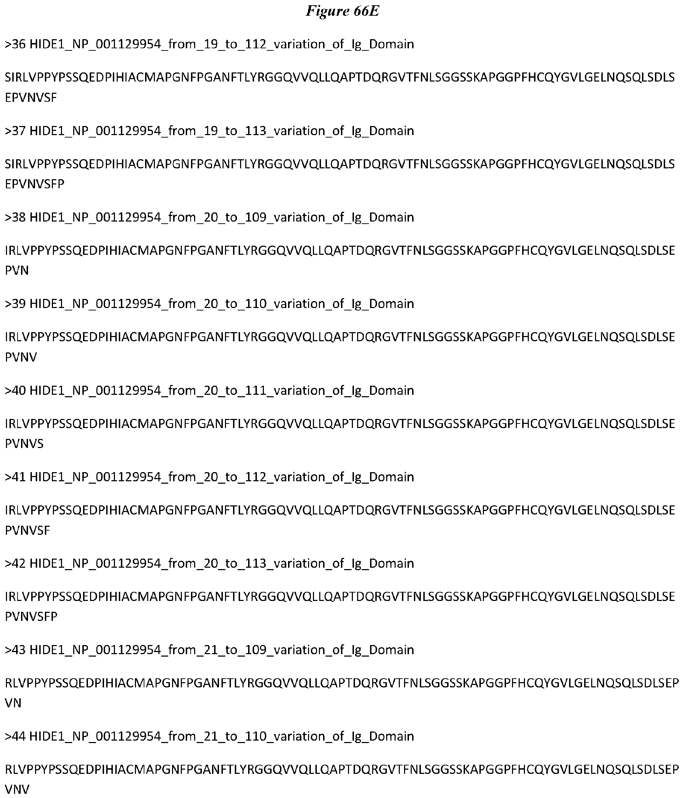

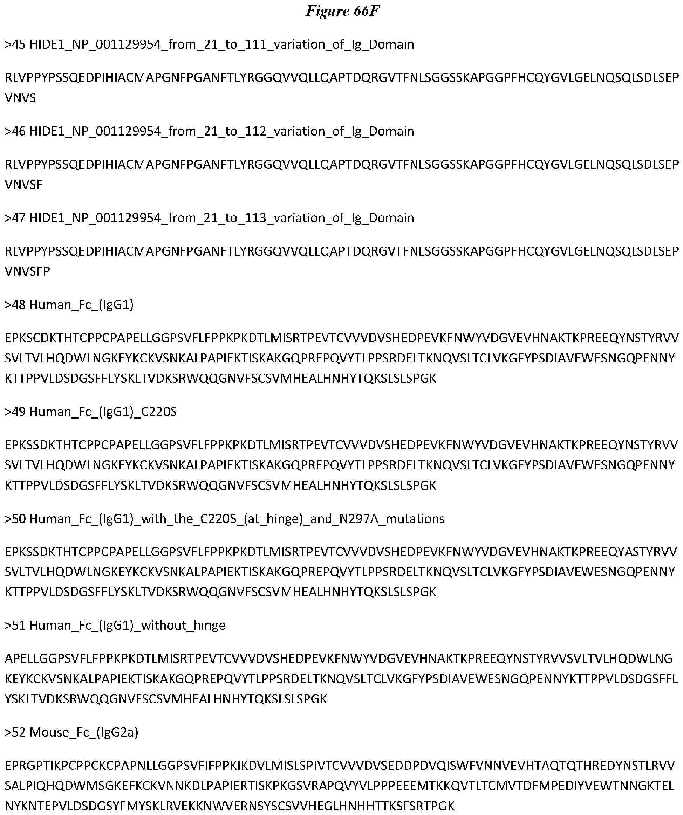

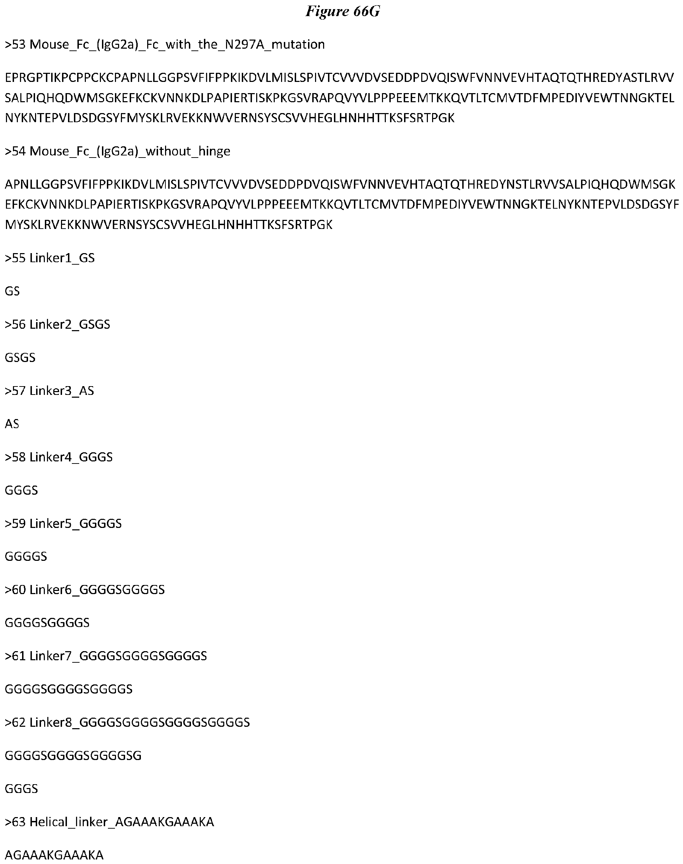

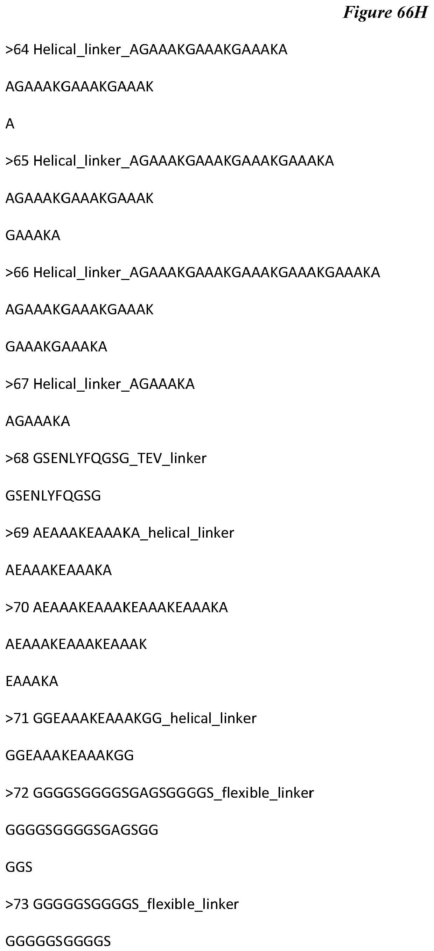

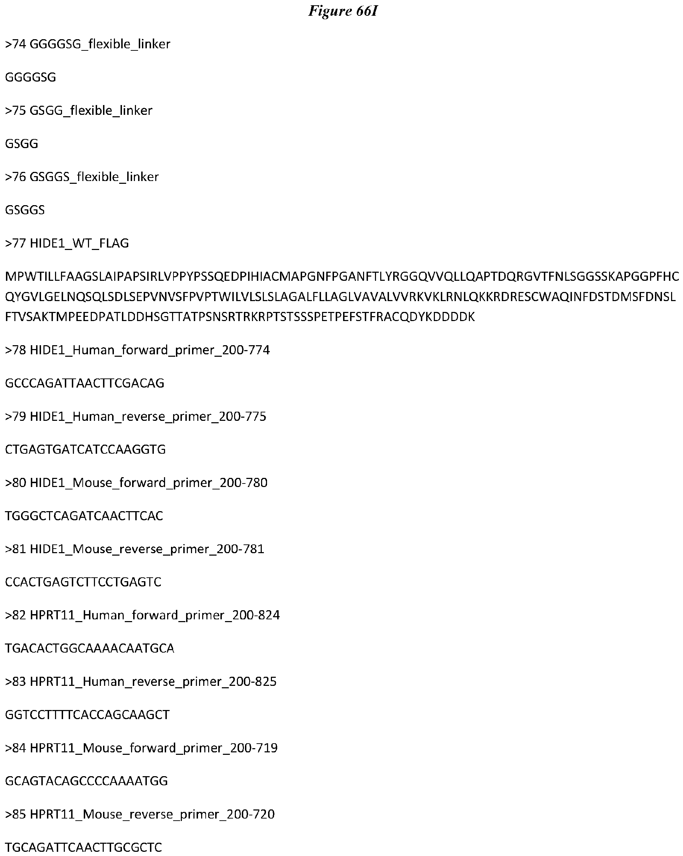

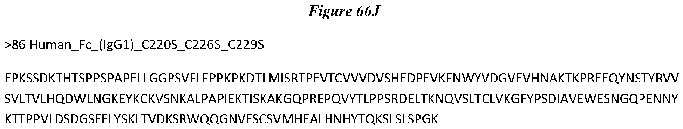

According to at least some embodiments, the anti-HIDE1 antibody binds to an isolated HIDE1 polypeptide consisting of a HIDE1 polypeptide ECD domain having at least 95% identity to the ECD domain of an amino acid sequence selected from the group consisting of the sequences depicted in Figure 66. In some embodiments of the composition, the isolated HIDE1 polypeptide has at least 99% identity to an amino acid sequence selected from the group consisting of the sequences depicted in Figure 66. In some embodiments of the composition, the isolated HIDE1 polypeptide is selected from the group consisting of the sequences depicted in Figure 66.

### BRIEF DESCRIPTION OF THE DRAWINGS

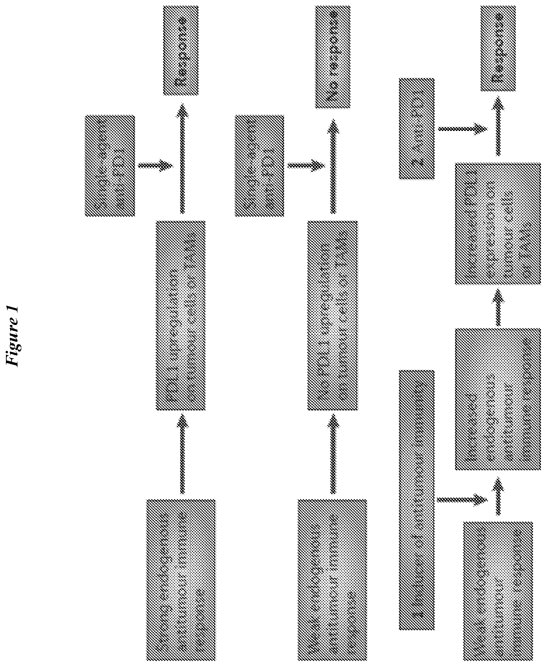

**Figure 1****:** Schematic presentation of elevation of endogenous expression of the immune checkpoint ligand (PDL-1) by induction of anti-tumor immunity.

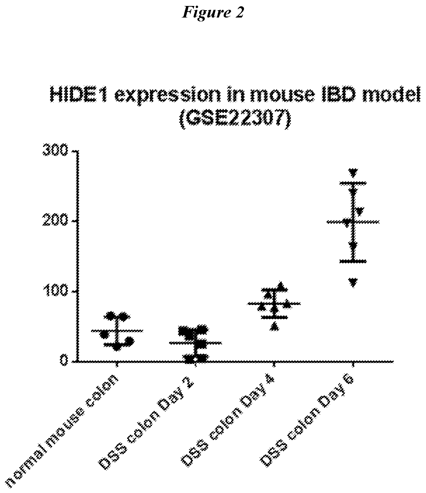

**Figure** 2: Shows induction of HIDE1 expression in DSS model of IBD.

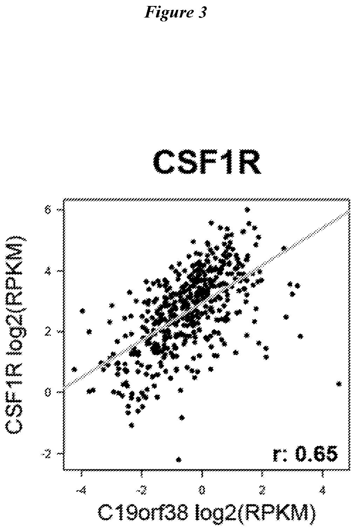

**Figure 3****:** Correlation of HIDE1 to CSF1R in human colorectal cancer (TCGA data).

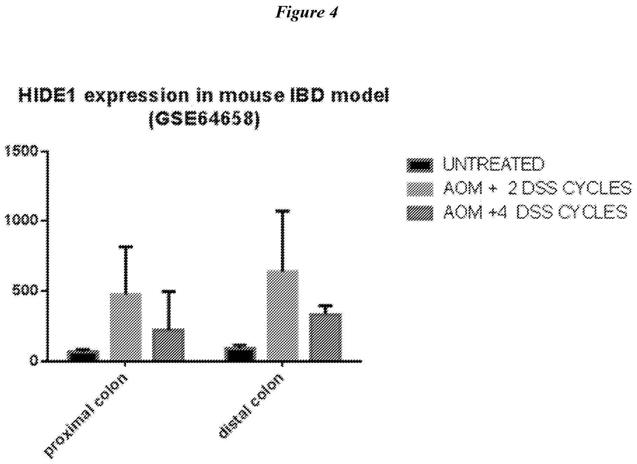

**Figure 4****:** Shows induction of HIDE1 expression in DSS + AOM model of IBD.

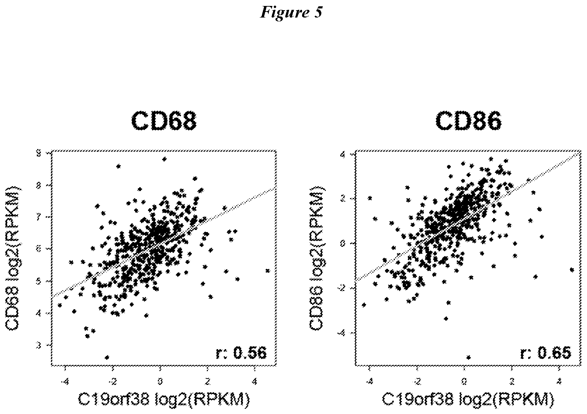

**Figure 5****:** Correlation of HIDE1 to CD86 and CD68 in human colorectal cancer (TCGA data).

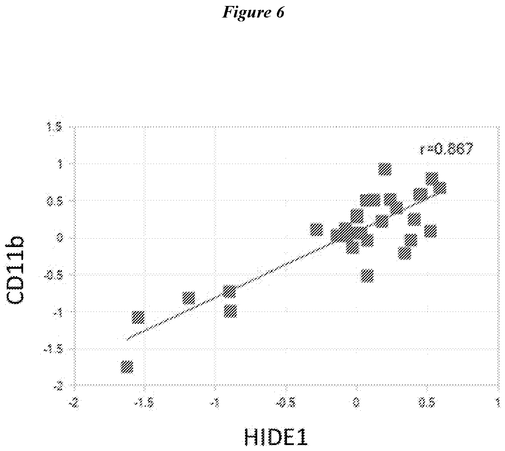

**Figure 6****:** Presents correlation of HIDE1 and CD11b in multiple autoimmune patient's derived samples.

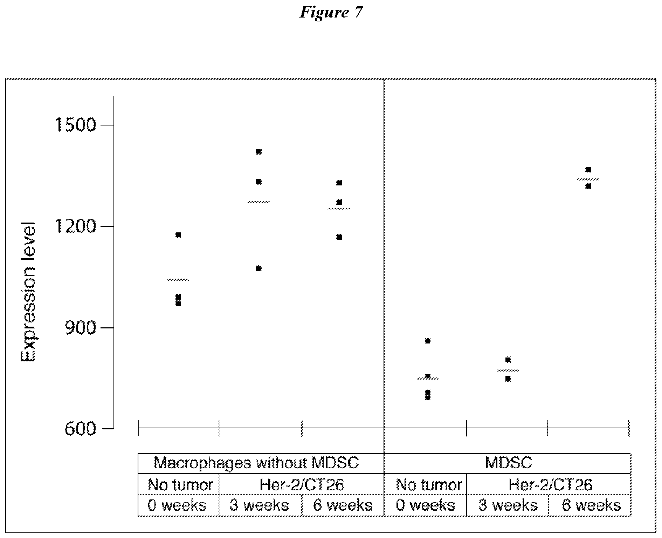

**Figure 7****:** Expression of HIDE1 in both monocytic and granulocytic MDSCs derived from mouse tumor model. Mean expression values for each group are shown as gray horizontal lines.

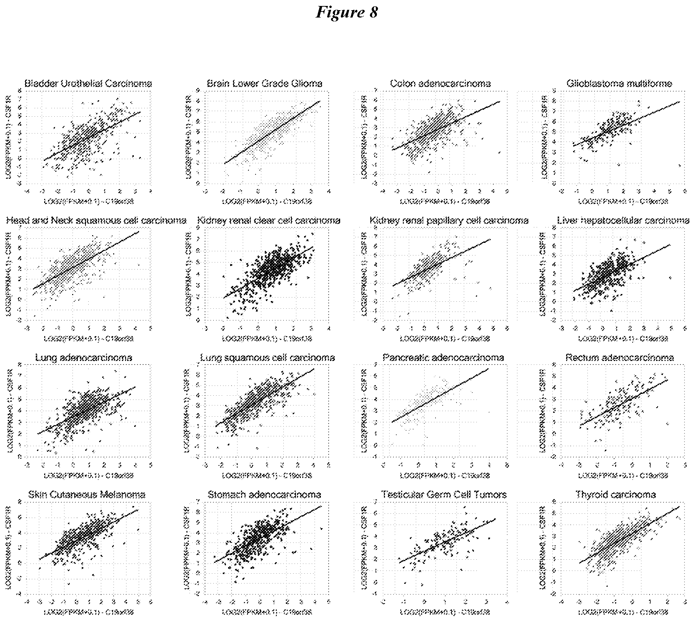

**Figure 8****:** Presents cancers with a strong myeloid infiltration, based on the CSF1R expression profile.

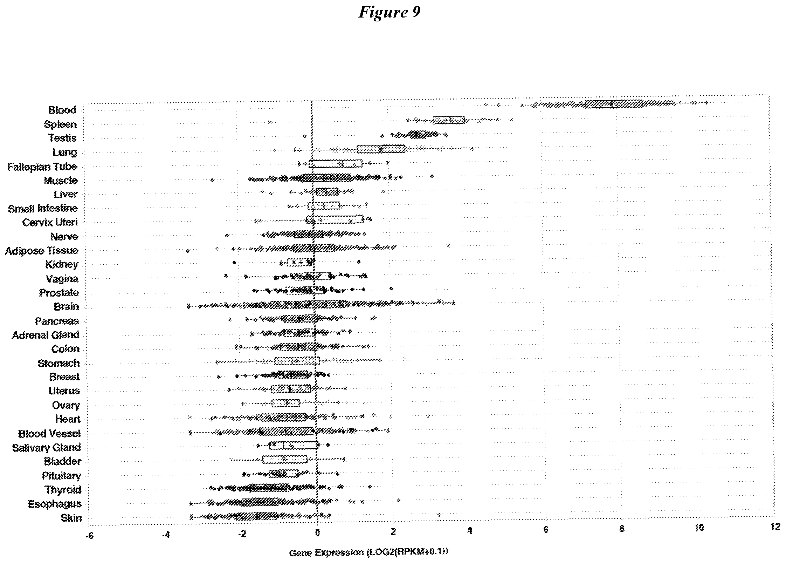

**Figure 9****:** HIDE1 expression in normal tissues (GTEx data).

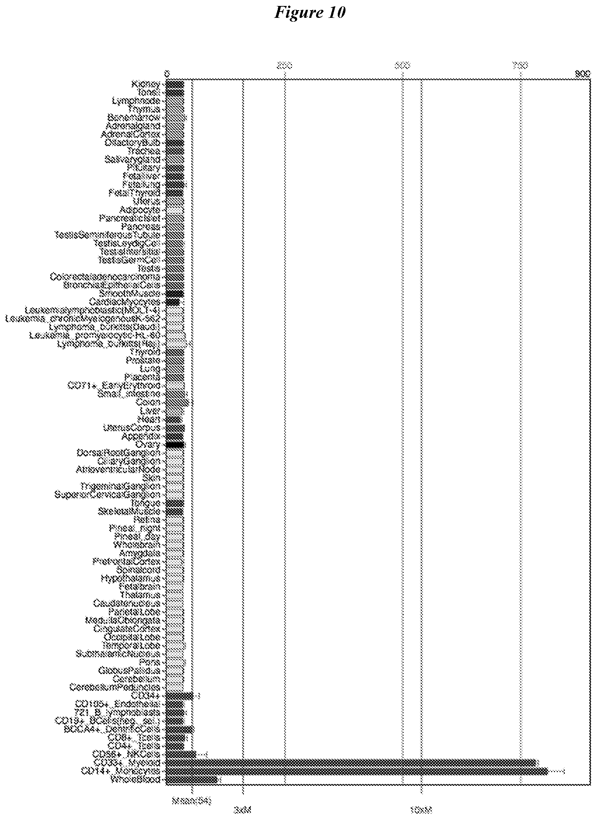

**Figure 10****:** HIDE1 expression pattern in BioGPS.

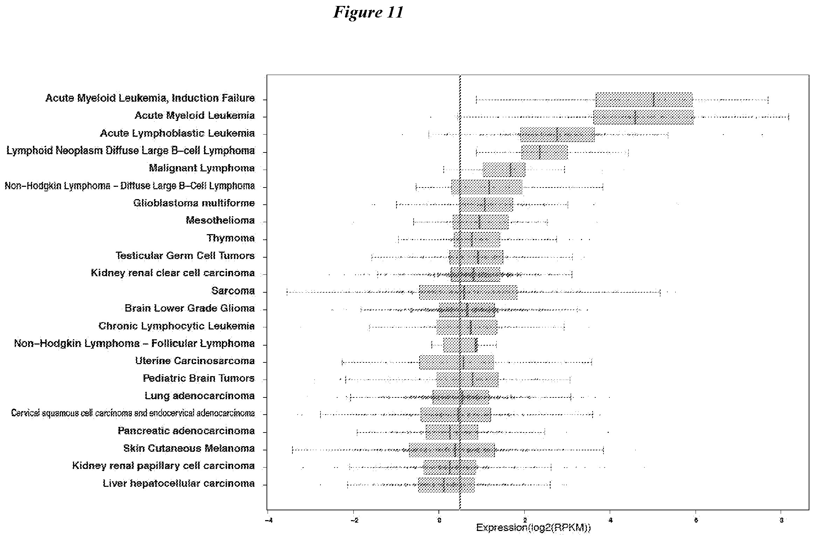

**Figure 11****:** HTDE1 expression in cancer.

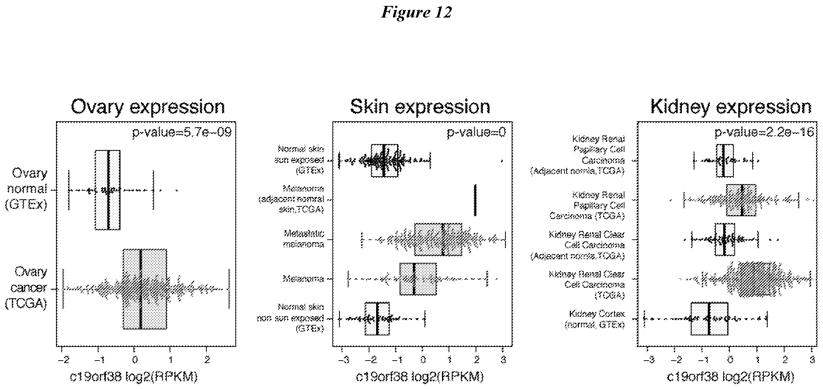

**Figure 12****:** HIDE1 is upregulated in several tumor types. Examples from ovary cancer, melanoma and kidney cancers are shown.

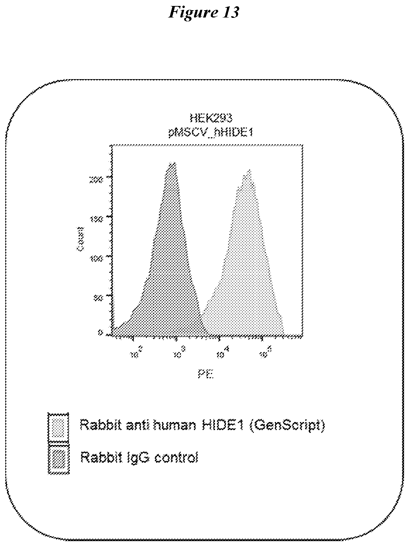

**Figure 13****:** FACS analysis of ectopically expressed HEK293 cells expressing human HIDE1 Flag pMSCV vector HEK293 cells expressing the human HIDE1 Flag were analyzed by FACS using rabbit anti Human HIDE1 (GenScript, light blue line). Rabbit IgG (Jackson, pink line) was used as isotype control. Detection was carried out using donkey anti-rabbit PE-conjugated secondary antibody and analysis by FACS.

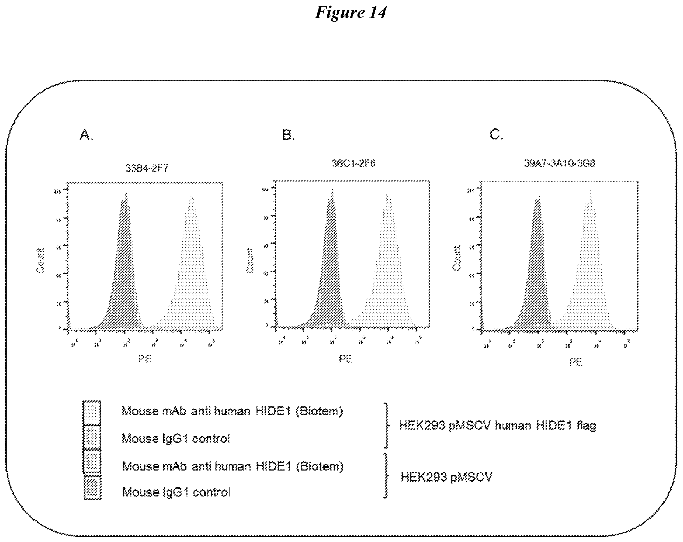

**Figure 14****:** FACS analysis of ectopically expressed HEK293 cells expressing human HIDE1 Flag pMSCV vector using anti human HIDE1 mAbs. HEK293 cells expressing the human HIDE1 Flag or HEK293 pMSCV empty vector were analyzed by FACS using mouse anti Human HIDE1 mAbs (Biotem, A-C, green line or orange line respectively) or using mouse IgG isotype control (light blue or pink line respectively). Detection was carried out using goat anti mouse-PE-conjugated secondary Ab.

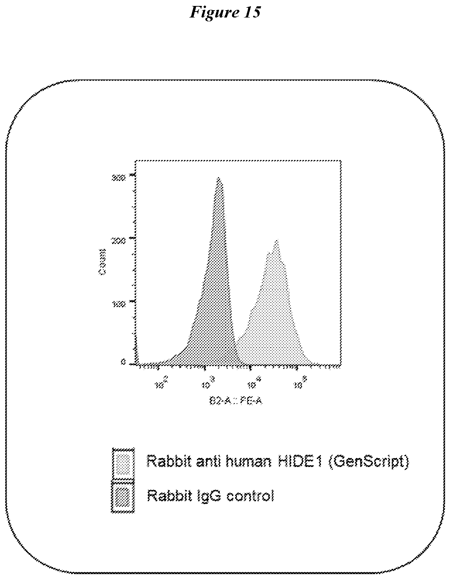

**Figure 15****:** FACS analysis of ectopically expressed SK-MEL-5 cells expressing human HIDE1 Flag pMSCV vector SK-MEL-5 cells expressing the human HIDE1 Flag were analyzed by FACS using Rabbit anti Human HIDE1 (GenScript, light blue line). Rabbit IgG (Jackson, pink line) was used as isotype control. Detection was carried out using donkey anti-rabbit PE-conjugated secondary antibody and analysis by FACS .

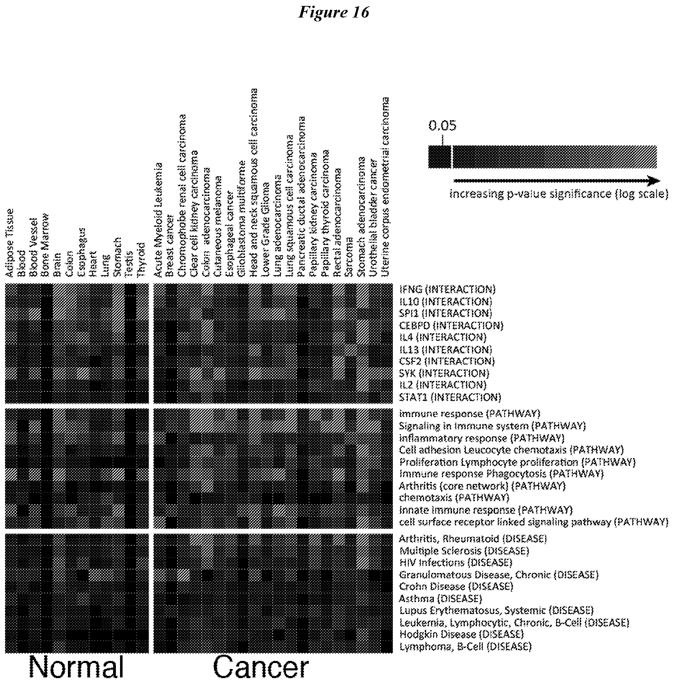

**Figure 16****:** Top 10 most enriched interactions, pathways and diseases for genes highly correlated with HIDE1 in a variety of cancers. Gradient black to gray scale reflects -log10(p-values). Rows are ranked order by the sum of each row.

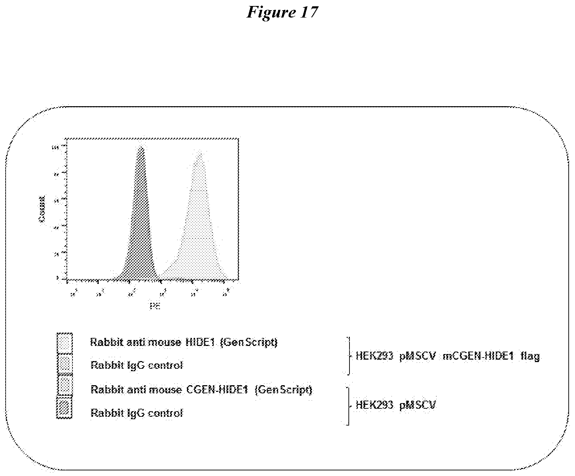

**Figure 17****:** FACS analysis of ectopically expressed HEK293 cells expressing mouse HIDE1 Flag pMSCV vector. HEK293 cells over expressing the mouse HIDE1 Flag or HEK293 cells transduced with an empty vector were analyzed by FACS using Rabbit polyclonal anti mouse HIDE1 (GenScript #488536_13). Rabbit IgG (Jackson) was used as an isotype control. Detection was carried out using Donkey Anti-Rabbit PE-conjugated secondary Ab.

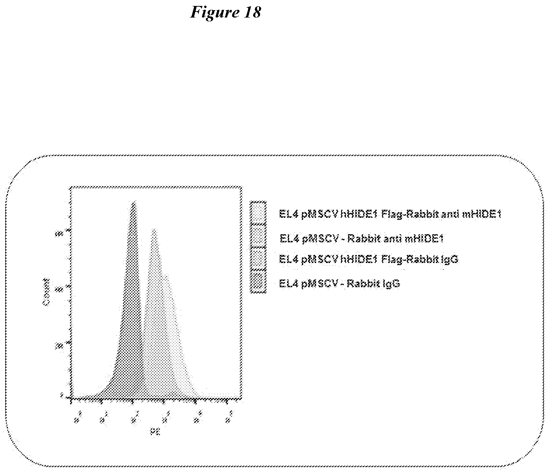

**Figure 18****:** FACS analysis of EL4 cells ectopically expressing mouse HIDE1 Flag pMSCV vector. EL4 cells over expressing the mouse HIDE1 Flag or EL4 cells transduced with an empty vector were analyzed by FACS using Rabbit (Rb) polyclonal anti mouse HIDE1 (GenScript #488536_13). Rabbit IgG (Jackson) was used as an isotype control. Detection was carried out using Donkey Anti-Rabbit PE-conjugated secondary Ab.

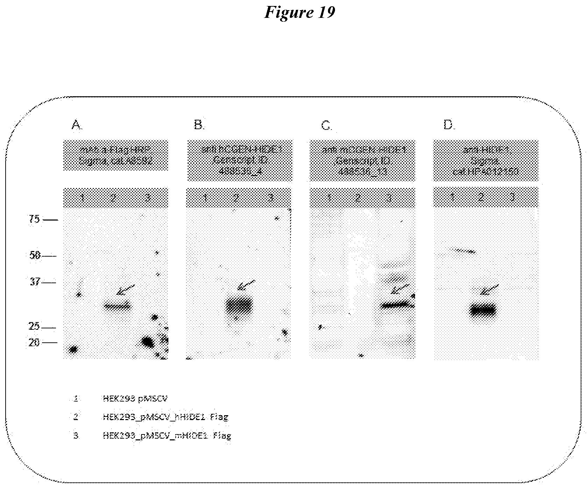

**Figure 19****:** WB analysis using anti Human or mouse HIDE1 pAbs on HEK293 cells expressing human or mouse HIDE1 protein. Whole cells extracts of HEK293 cells expressing the human HIDE1 flag (lane 2), HEK293 cells expressing the mouse HIDE1 flag (lane 3) or HEK293 transfected with an empty vector (lanes 1) were analyzed using anti Flag antibody (A), a commercial antibody anti human HIDE1 (Sigma, D), pAb anti human-HIDE1 (GenScript, B) or with pAb anti mouse-HIDE1 (GenScript, C). Detection was carried out using goat anti rabbit-HRP (except anti flag which is already conjugated to HRP).

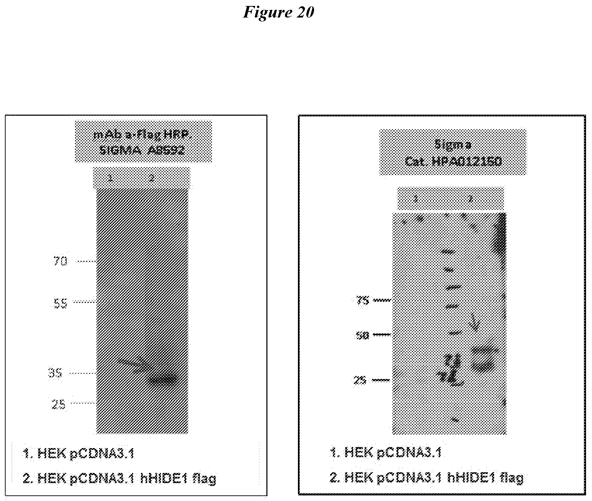

**Figure 20****:** presents WB analysis of ectopically expressed human HIDE1 Flag pCDNA3.1 vector. Whole cell extracts of HEK293 cell pools, transfected with expression construct encoding human HIDE1-flag (lane 2) or with empty vector (lane 1) were analyzed by WB using an anti-flag antibody (left panel) or anti-HIDE1 antibodies (right panel).

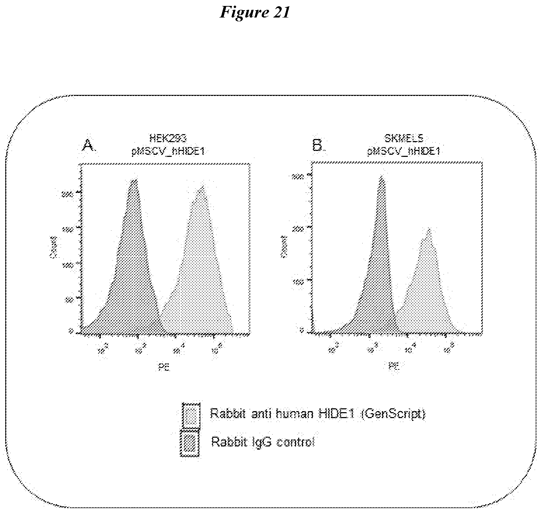

**Figure 21****:** FACS analysis using anti Human HTDE1 pAbs on HEK293 or SKMEL5 cells expressing Human HIDE1 protein. HEK293 cells expressing the human HTDE1 Flag (A) or SKMEL-5 expressing the human HIDE1 Flag (B) were analyzed by FACS using Rabbit anti Human HIDE1 (GenScript, light blue line). Rabbit IgG (Jackson, pink line) was used as isotype control. Detection was carried out using donkey anti-rabbit PE-conjugated secondary antibody and analysis by FACS.

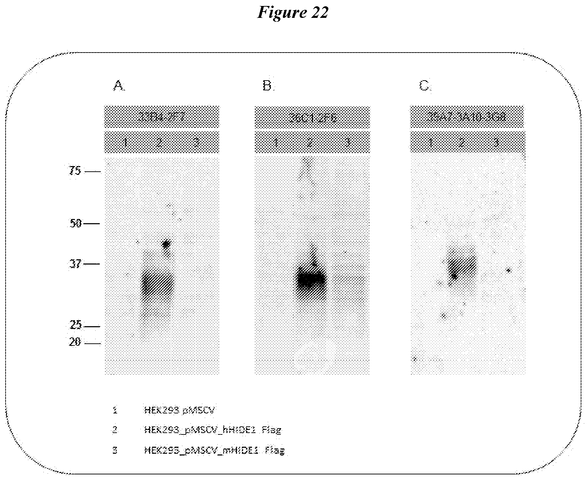

**Figure 22****:** WB analysis using anti Human HTDE1 mAbs on HEK293 cells expressing human or mouse HIDE1 protein. Whole cells extracts of HEK293 cells expressing the human HIDE1 flag (lanes 2), HEK293 cells expressing the mouse HIDE1 flag (lanes 3) or HEK293 transfected with an empty vector (lanes 1) were analyzed by WB using mAbs anti human HIDE1 (Biotem): 33B4-2F7(A), 36C1-2F6 (B) or 39A7-3A10-3G8 (C). Detection was carried out using Goat Anti Mouse-HRP (Anti flag is conjugated to HRP).

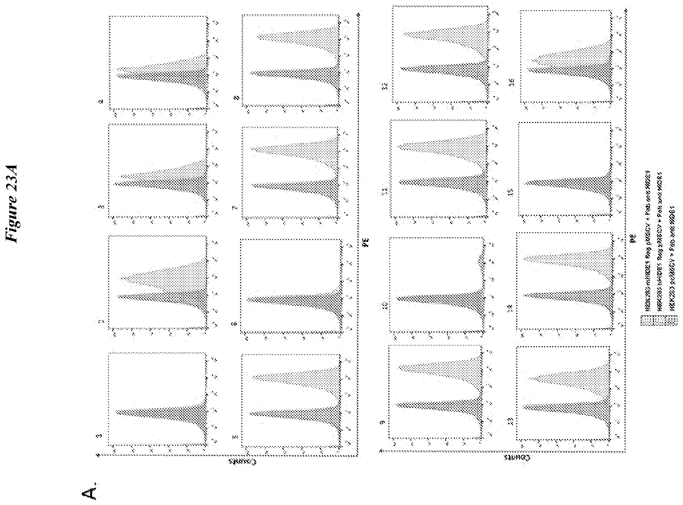

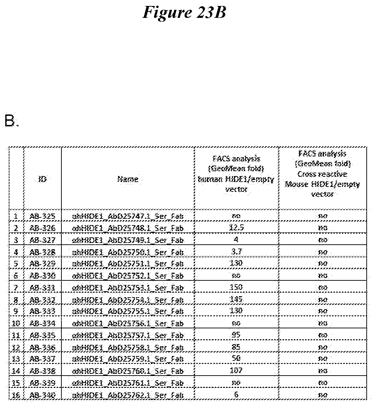

**Figure 23A** **&** **23B****:** FACS analysis using anti human HIDE1 Fab's on HEK293 cells over-expressing human HIDE1 Flag protein. (A)HEK293 cells over-expressing the human HIDE1 Flag (blue line) or HEK293 cells over-expressing the mouse HIDE1 Flag (orange line) or HEK293 transduced with empty vector (red line) were analyzed by FACS using Serotec anti-Human HIDE1 Fab's (1-16). Detection was carried out using Goat Anti Human IgG F(ab')2-PE secondary Ab. (B) Summary table of Serotec anti-human HIDE1 Fab's 1-16 FACS analysis, the Geo mean ratio of HEK293 cells over-expressing the human HIDE1 Flag/ HEK293 transduced with empty vector. And the cross reactive validation, the Geo-mean ratio of HEK293 cells over-expressing the mouse HIDE1 Flag/ HEK293 transduced with empty vector.

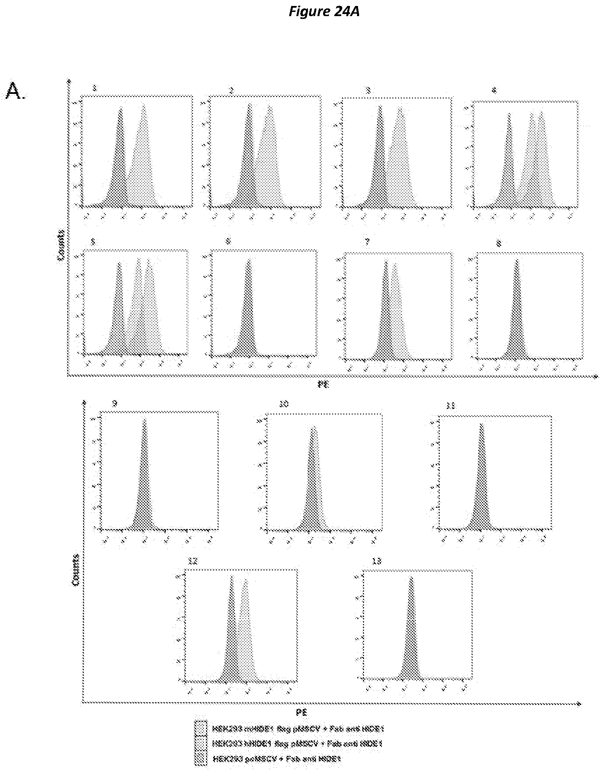

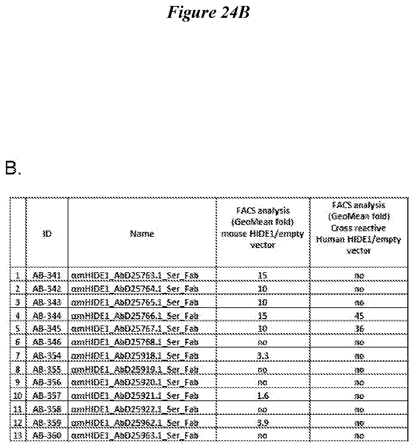

**Figure 24A** **&** **24B****:** FACS analysis using anti-mouse HIDE1 Fab's on HEK293 cells over-expressing mouse HIDE1 Flag protein. (A)HEK293 cells over-expressing the mouse HIDE1 Flag (orange line) or HEK293 cells over-expressing the human HIDE1 Flag (blue line) or HEK293 transduced with empty vector (red line) were analyzed by FACS using Serotec anti-mouse HIDE1 Fab's (1-13). Detection was carried out using Goat Anti Human IgG F(ab')2-PE secondary Ab. (B) Summary table of Serotec anti-mouse HIDE1 Fab's 1-13 FACS analysis, the Geo mean ratio of HEK293 cells over-expressing the mouse HIDE1 Flag/ HEK293 transduced with empty vector. And the cross reactive validation, the Geo mean ratio of HEK293 cells over-expressing the human HIDE1 Flag/ HEK293 transduced with empty vector.

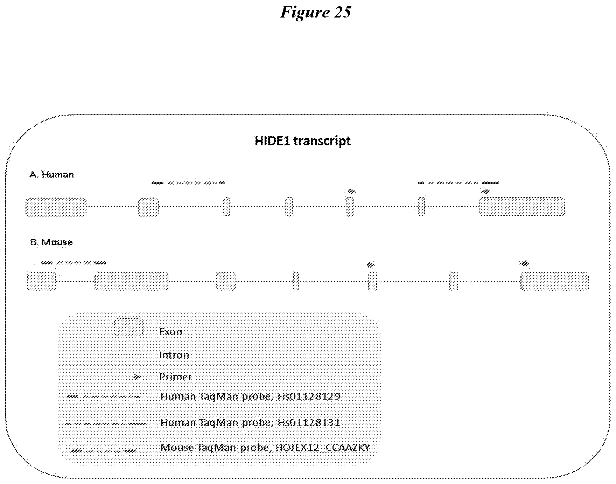

**Figure 25****:** Schematic representation of the exon structure of Human (A) and Mouse (B) HIDE1 in the mRNA transcript (Introns are not represented in a real proportion). Primers that were used for the qRT-PCR are represented as a red arrows. TaqMan probes are indicated by different colors.

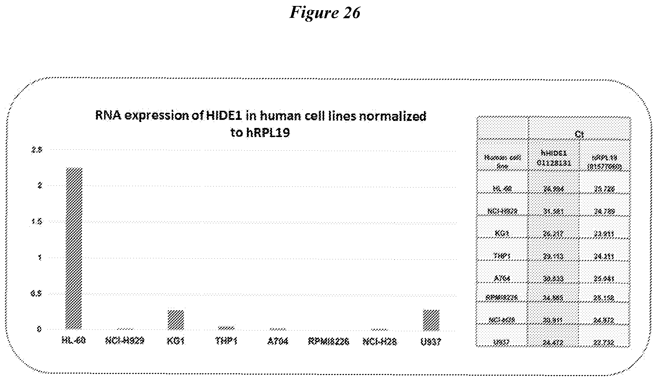

**Figure 26****:** Transcript expression of human HIDE1 in various Human cancer cell lines. Verification of the human transcript in several cell lines was performed by qRT-PCR using TaqMan probes. Column diagram represents data observed using TaqMan probe Hs01128131_m1. Ct values are detailed in the table. Analysis indicating high transcript in HL-60 KG-1 and U937 And lower levels in THP1, A704 and NCI-H28 cell lines.

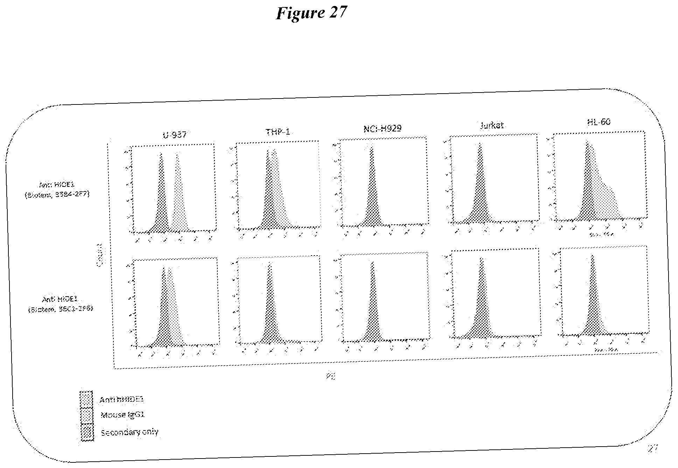

**Figure 27****:** Membrane expression of human HIDE1 protein in various human cancer cell lines. Human cell lines were stained with monoclonal anti-HIDE1 Abs BIOTEM 33B4-2F7 (Orange, upper panel) or 36C1-2F6 (Orange, lower panel) or with IgG1 isotype control antibody (light blue). Following cell washing, PE-Goat anti-mouse secondary conjugated Ab was added. Cells that were stained with the secondary antibody only are represented by a red color. Binding was evaluated by FACS. Histograms represent living cells gated using viability stain 450 (BD Bioscience).

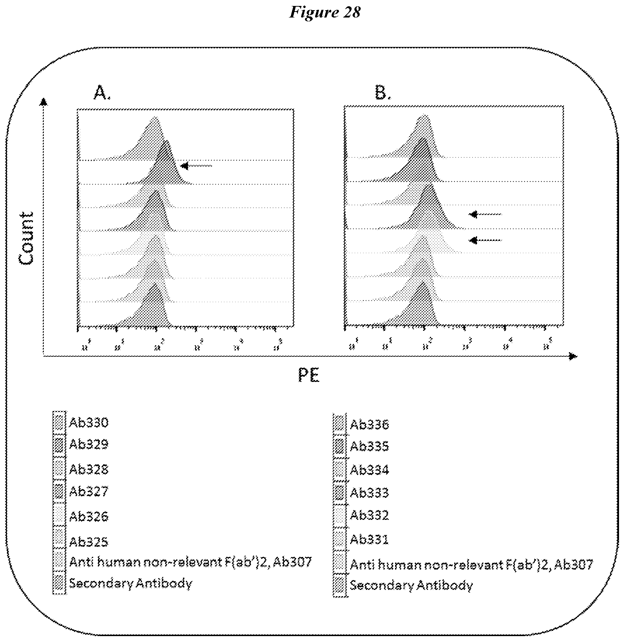

**Figure 28****:** Membrane expression of human HTDE1 protein in U937 human cancer cell line. U937 cell line was stained with various anti-human HIDE1 F(ab')2, Serotec. Only 3 F(ab')2 showed specific membrane staining of U937 cell line as shown by the black arrows in the Figure: Ab3295 (A. Purple line,), AbD333 (B. Olive line) and with Ab332 (B. Green line). Following cell washing, PE-Goat anti-F(ab')2 secondary conjugated Ab was added. Cells that were stained with the secondary antibody only are represented by a red color. Binding was evaluated by FACS. Histograms represent living cells gated using viability stain 450 (BD Bioscience).

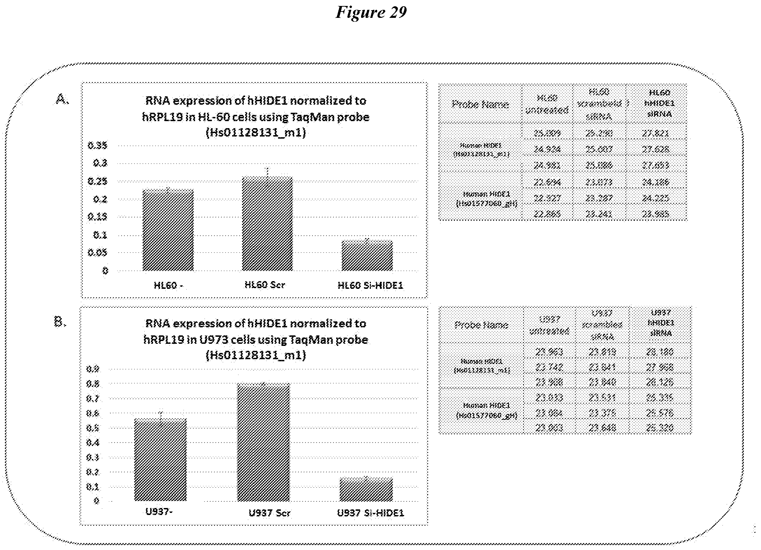

**Figure 29****:** qRT-PCR analysis of human HTDE1 transcript in HL-60 and U937 cell lines transfected with HIDE1 siRNA. HL-60 (A) or U973 (B) human cancer cell lines, untreated, transfected with human HIDE1 siRNA or with scrambled siRNA were analyzed by qRT-PCR using human HIDE1 TaqMan probe # Hs01128131_m1. Ct values are detailed in the table. Standard deviation of technical triplicates of the PCR reaction are indicated.

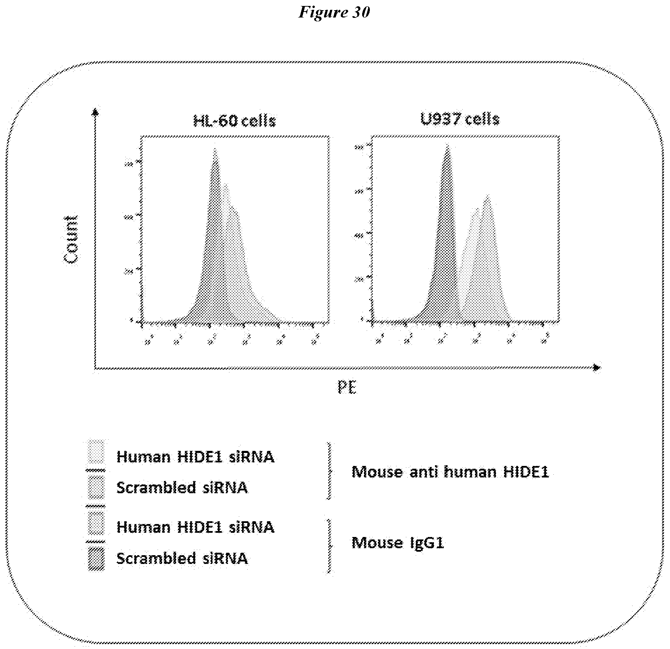

**Figure 30****:** Membrane expression of human HIDE1 protein in HL-60 and U937 human cell lines transfected with human HIDE1 siRNA. HL-60 or U937 Cells transfected with Human HIDE1 siRNA were stained with monoclonal anti-HIDE1 Abs BIOTEM, 33B4-2F7 (green) or with IgG1 isotype control antibody (blue). Cells transfected with Scrambled siRNA were stained with the same anti-HIDE1 (orange) or isotype control (red). Following cell washing, PE-Goat anti-mouse secondary conjugated Ab was added.

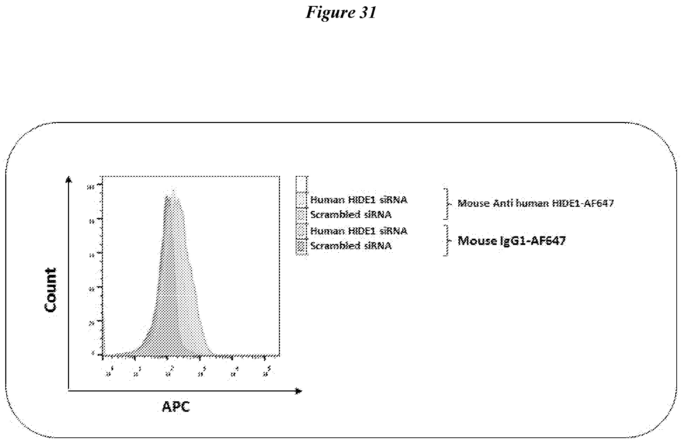

**Figure 31****:** Membrane expression of human HTDE1 protein in THP1 human cell line transfected with human HTDE1 siRNA. THP1 cells transfected with Human HTDE1 siRNA were stained with monoclonal anti-HIDE1 Abs BIOTEM (conjugated to AF647), Ab-263 (green) or with IgG1 isotype control-AF647 antibody (blue). Cells transfected with Scrambled siRNA were stained with the same anti-HIDE1 (orange) or isotype control (red).

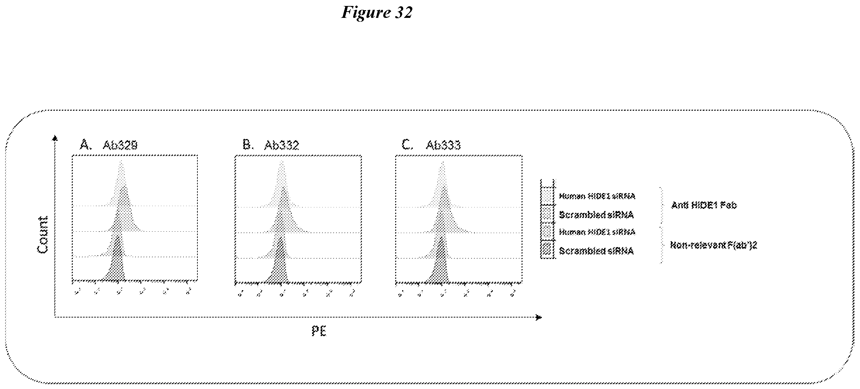

**Figure 32****:** Membrane expression of human HIDE1 protein in U937 human cell line transfected with human HTDE1 siRNA. U937 cells transfected with Human HTDE1 siRNA were stained with 3 different anti-HIDE1 F(ab')2, Serotec (A. Ab329, B. Ab332 and C. Ab333- green line), or with non-relevant F(ab')2, Ab 307 (blue). Cells transfected with Scrambled siRNA were stained with the same anti-HIDE1 (orange) or isotype control (red).

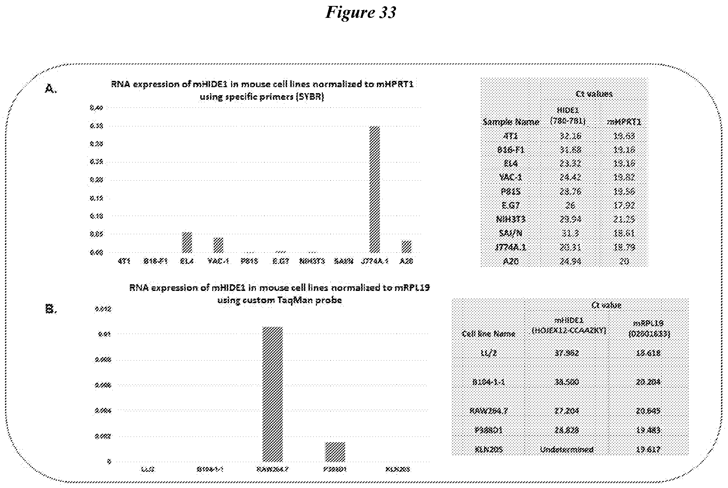

**Figure 33****:** Transcript expression of mouse HIDE1 in various mouse cell lines. Verification of the mouse transcript in several cell lines was performed by qRT-PCR using specific primers (A) or TaqMan probe (B). Ct values are indicated in the tables. Analysis indicating relatively high transcript level in J774A. 1 EL4, YAC-1, A20 as well as in RAW264.7, and P388D1 cell lines

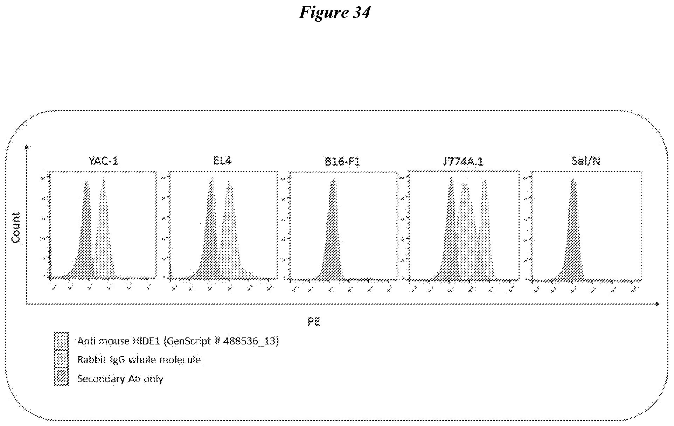

**Figure 34****:** Membrane expression of mouse HIDE1 protein in various mouse cell lines. Mouse cell lines were stained with rabbit anti-HIDE1 pAb (Genescript, ID 488536_13) (Orange) or with rabbit IgG isotype control (light blue). Following cell washing, PE-donkey anti-rabbit secondary conjugated Ab was added. Cells that were stained with the secondary antibody only are represented by a red color. Binding was evaluated by FACS. Histograms represent living cells gated using viability stain 450 (BD Bioscience).

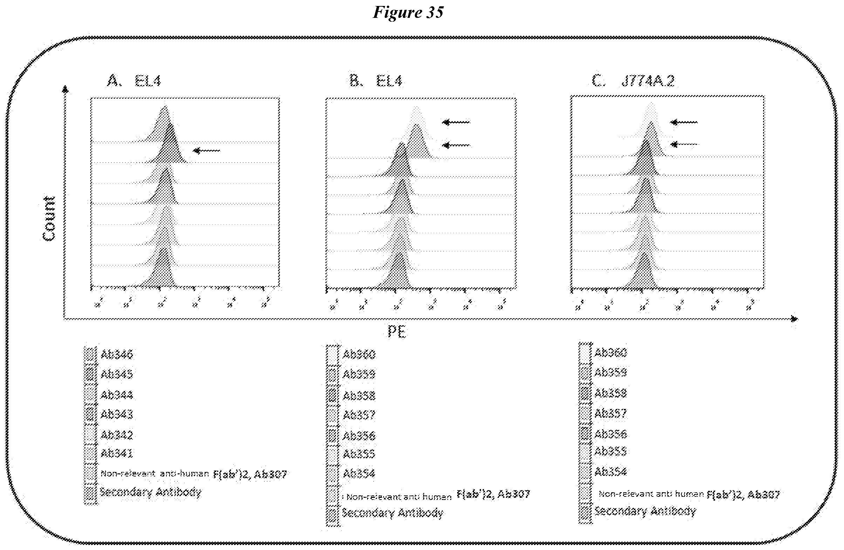

**Figure 35****:** Membrane expression of mouse HTDE1 protein in EL4 and J774A.2 mouse cancer cell lines. EL4 cell line was stained with various anti-mouse HIDE1 F(ab')2, Serotec. Only 3 F(ab')2 showed specific membrane staining of EL4 cell line (indicated with the black arrows in the figure): Ab345 (A. Purple line), Ab360 and Ab359 (B. yellow and blue lines respectively), whereas only 2 F(ab')2 showed specific staining of J774A.2 cell line (indicated by the black arrows in the figure): Ab360 and Ab359 (C. yellow and blue lines respectively) only. Following cell washing, PE-Goat anti-F(ab')2 secondary conjugated Ab was added. Cells that were stained with the secondary antibody only are represented by a red color. Binding was evaluated by FACS. Histograms represent living cells gated using viability stain 450 (BD Bioscience).

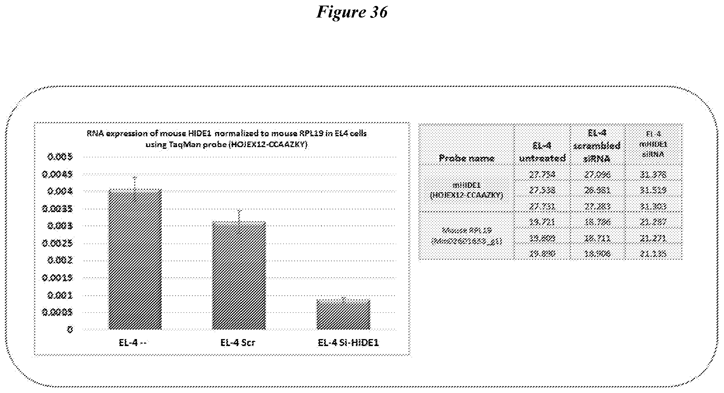

**Figure 36****:** qRT-PCR analysis of mouse HIDE1 transcript in EL4 mouse cell line EL4 cells: untreated (left column), transfected with mouse HIDE1 siRNA (right column) or with scrambled siRNA (middle column) were analyzed by qRT-PCR using mouse HIDE1 TaqMan custom probe # HOJEX12_CCAAZKY. Ct values are indicated in the table. Standard deviation of technical triplicates of the PCR reaction are indicated are indicated.

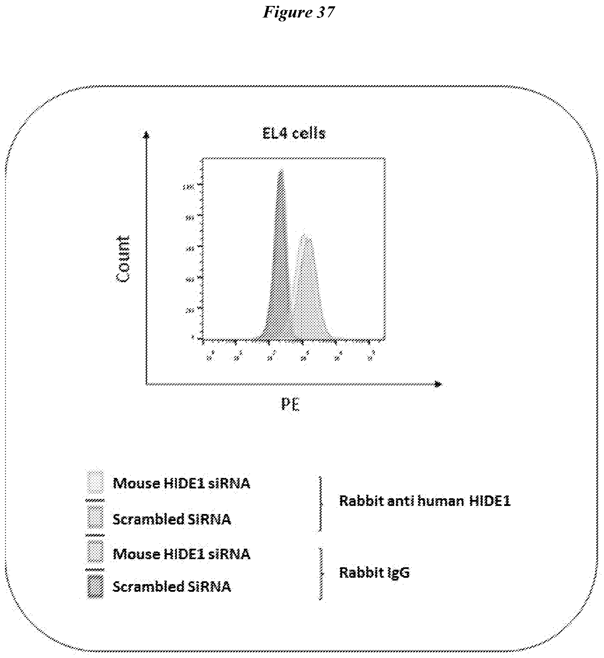

**Figure 37****:** Membrane expression of mouse HIDE1 protein in EL4 mouse cell line transfected with mouse HIDE1 siRNA. EL4 mouse cell line transfected with mouse HIDE1 siRNA was stained (48 hours post transfection) with rabbit anti-HIDE1 pAbs (Genscript, ID. 488536_13, green) or with Rabbit IgG isotype control antibody (blue). Cells transfected with Scrambled siRNA were stained with the same anti-HIDE1 (orange) or isotype control (red). Following cell washing, PE-Donkey anti-Rabbit secondary conjugated Ab was added.

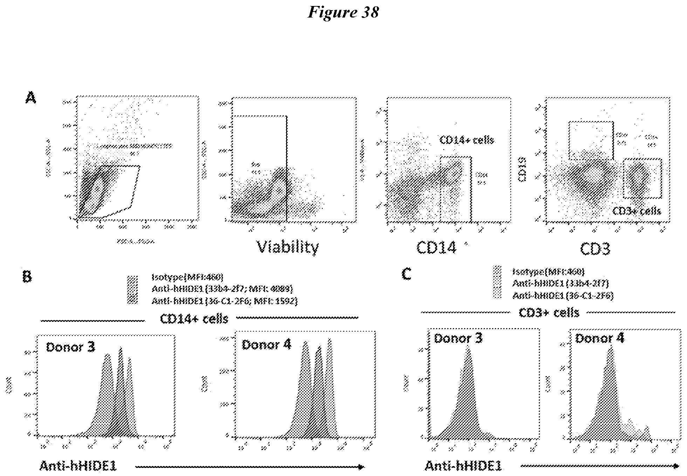

**Figure 38****:** HIDE1 Expression profile on fresh PBMCs. Freshly thawed PBMCs from 2 donors were thawed and stained with viability dye, washed and pre-blocked with Fc blocking solution followed by surface staining with surface markers in the presence of 2 anti-hHIDE1 antibodies or isotype control as described in materials and methods. HIDE1 surface expression levels were analyzed by flow cytometry. (A) Gating strategy for flow cytometry analysis gating on CD14+ and CD3+ cells is shown (B) Histograms plots represent the staining of anti-hHIDE1 Abs gating on CD14+ cells from 2 donors. (C) Histograms plots represent the staining of anti-hHIDE1 Abs gating on CD3+ cells. Values represent geometric mean fluorescent intensity (gMFI).

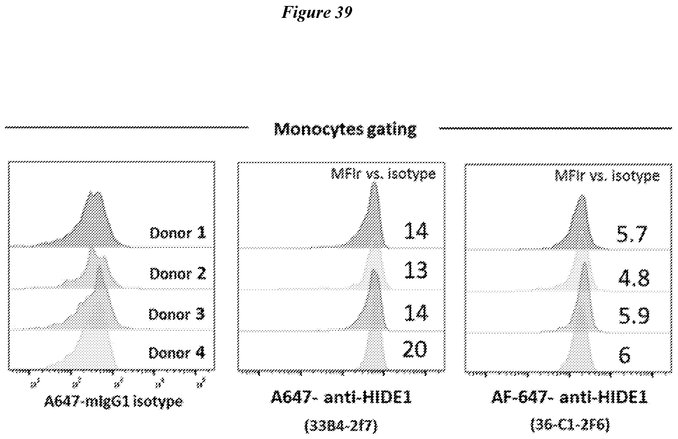

**Figure 39****:** HTDE1 expression profile on fresh PBMCs. PBMCs from 4 donors were pre-blocked with human Ig Fc fragments (200µg/ml) in addition to Fc-blocking solution (Biolegend) to avoid non-specific binding via Fc receptors. Cells were then stained with anti-hHIDE1 antibodies or isotype control as described in materials and analyzed by flow cytometry. Histograms plots represent the staining of anti-hHIDE1 Abs gating on Monocytes (FSC-A, SSC-A). Values represent geometric mean fluorescent intensity ratio (MFIr) compare to isotype control.

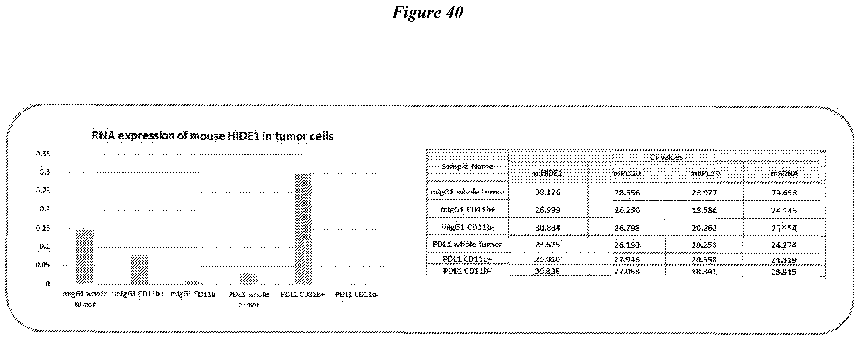

**Figure 40****:** Transcript expression of mouse HIDE1 in subpopulation cells (CD11b+, CD11b-) and in whole tumor cells. Verification of the mouse transcript was performed by qRT-PCR using TaqMan probe. Column diagram represents data observed using TaqMan probe HOJEX12-CCAAZKY. Ct values are detailed in the table. Analysis indicating high transcript in CD11b+ subpopulation, and lower levels whole tumor cells and in CD11b-subpopulation.

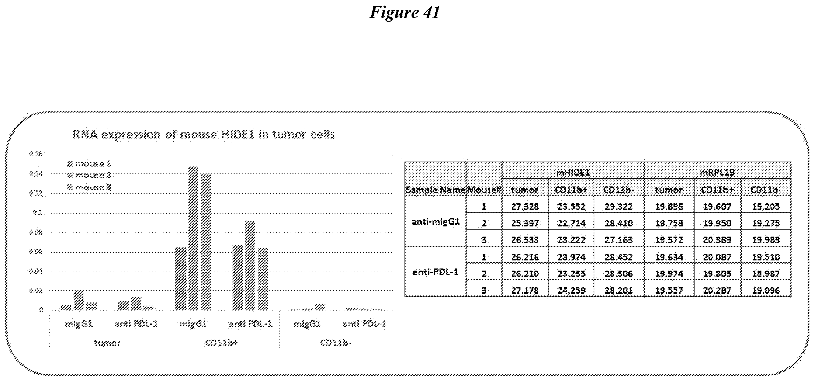

**Figure 41****:** Transcript expression of mouse HIDE1 in subpopulation cells (A. CD11b+, CD11b-) and in whole tumor cells. Verification of the mouse transcript was performed by qRT-PCR using TaqMan probe. Column diagram represents data observed using TaqMan probe HOJEX12-CCAAZKY. Ct values are detailed in the table. Analysis indicating high transcript in CD11b+ subpopulation, and lower levels whole tumor cells and in CD11b- subpopulation.

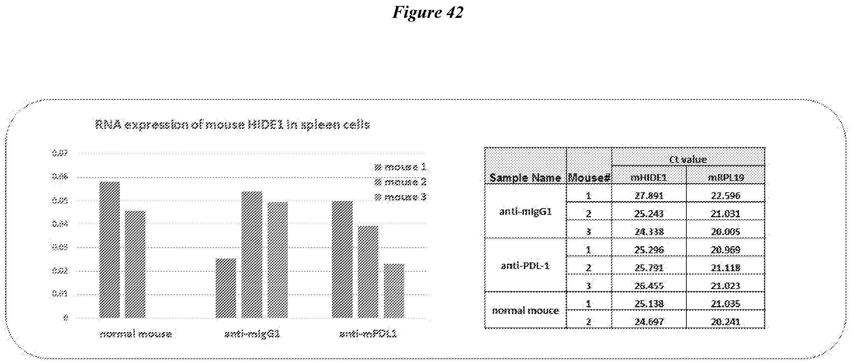

**Figure 42****:** Transcript expression of mouse HIDE1 in spleen cells. Verification of the mouse transcript was performed by qRT-PCR using TaqMan probe. Column diagram represents data observed using TaqMan probe HOJEX12-CCAAZKY. Ct values are detailed in the table. Analysis indicating high transcript in spleen cells.

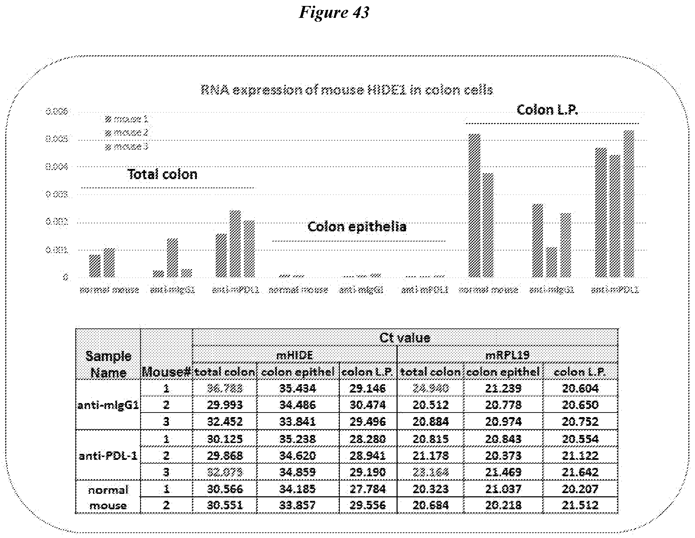

**Figure 43****:** Transcript expression of mouse HIDE1 in spleen cells. Verification of the mouse transcript was performed by qRT-PCR using TaqMan probe. Column diagram represents data observed using TaqMan probe HOJEX12-CCAAZKY. Ct values are detailed in the table. Analysis indicating high transcript in colon lamima propia and total colon cells compared to colon epithelia cells.

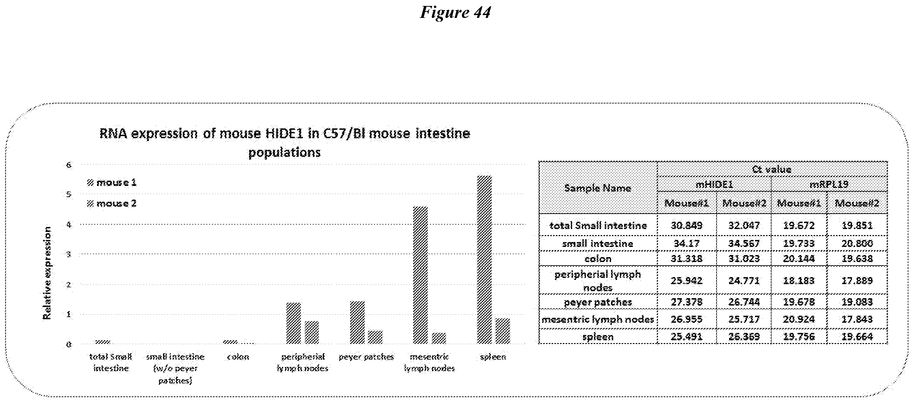

**Figure 44****:** Transcript expression of mouse HIDE1 in intestine cell populations. Verification of the mouse transcript was performed by qRT-PCR using TaqMan probe. Column diagram represents data observed using TaqMan probe HOJEX12-CCAAZKY. Ct values are detailed in the table. Analysis indicating high transcript in lymph nodes, spleen and peyer patches population, and lower levels in colon and small intestine cells population.

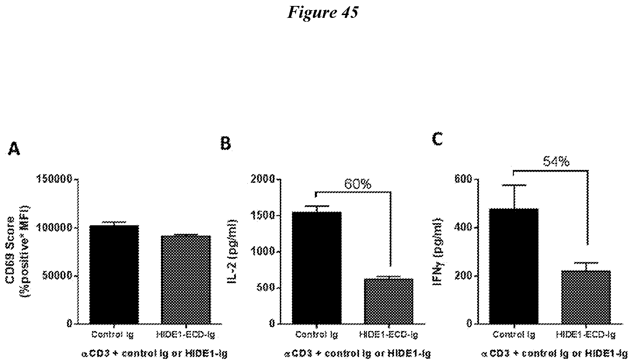

**Figure 45****:** Effect of various HIDE1-ECD-Ig (SEQ ID NO: 18) on mouse CD4 T cell activation. Plates were coated with anti-CD3 mAb (2µg/mL) in the presence of 10µg/ml HIDE1-Ig (batch #195) ybar is the mean of duplicate cultures, the error bars indicating the standard deviation. **(****Figures 45B-45C****)** Culture supernatants were collected at 48 h post-stimulation and mouse IL-2 and IFNγ levels were analyzed by ELISA. Results are shown as Mean ± Standard errors of duplicate samples. One experiment out of two performed is presented.

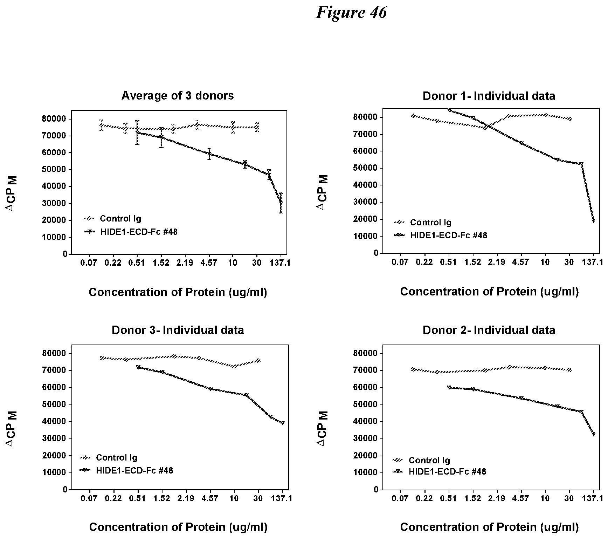

**Figure 46****:** Demonstrates inhibition of human T cell proliferation induced by anti-CD3 and anti-CD28 in the presence of irradiated autologous PBMCs by human HIDE1 ECD-Ig (SEQ ID NO: 17). **Figure 46A** shows averages of three donors tested. **Figures 46B-46D** show the individual data of each donor. The control Ig is Synagis.

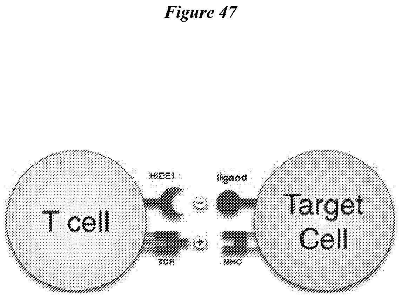

**Figure 47****:** Schematic illustration of the experimental system.

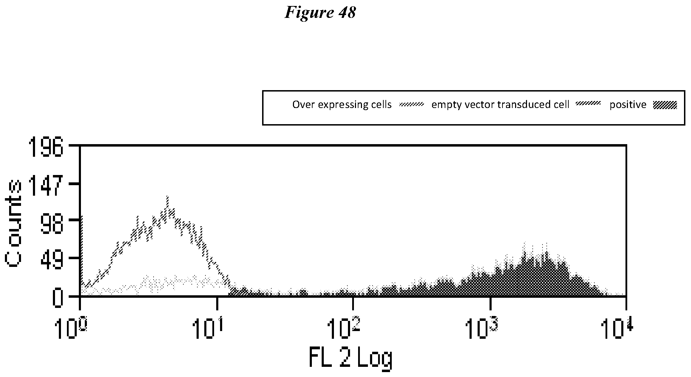

**Figure 48****:** FACS analysis on HIDE1 transduced PBLs using an anti-FLAG antibody with intra-cellular staining. The percent of cells staining positive (relative to empty vector transduced) for the protein is provided

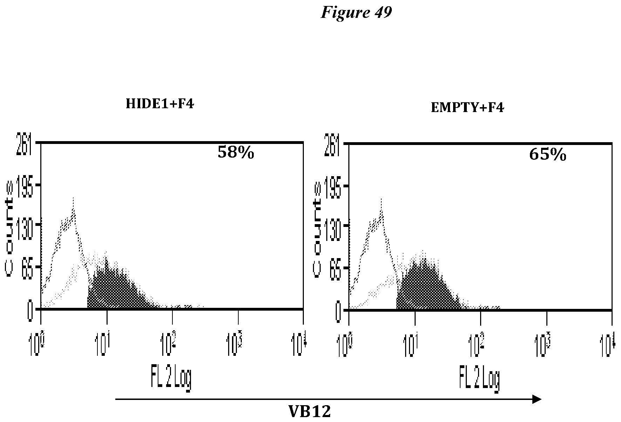

**Figure 49****:** FACS analysis performed on TCR transduced stimulated PBLs using a specific monoclonal antibody that recognizes the extra-cellular domain of the betachain from the transduced specific TCR. The percentage of cells staining positive is provided

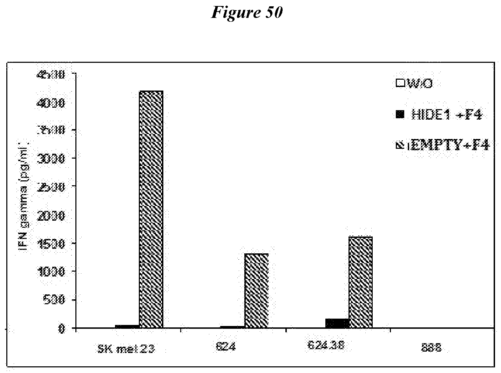

**Figure 50****:** Expression of HIDE1 on the F4 expressing PBLs causes a reduction of IFNγ secretion upon co-culture with SK-MEL23, MEL-624 and MEL-624.38 in comparison to expression of an empty vector

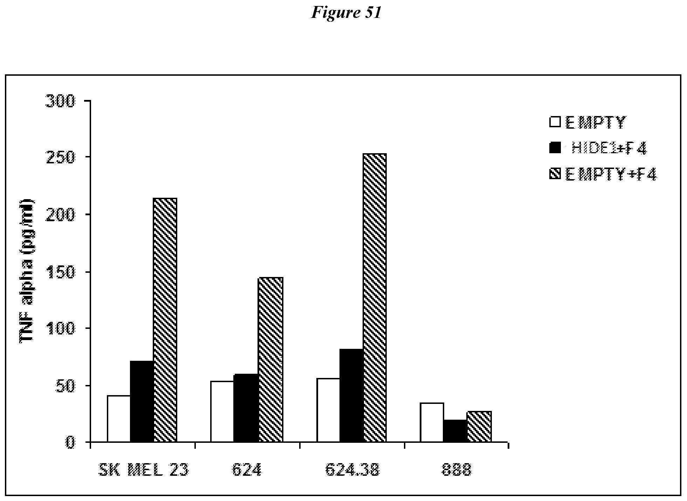

**Figure 51****:** Expression of HIDE1 on F4 expressing PBLs causes a reduction of secretion upon co-culture with SK-MEL23, MEL-624 and MEL-624.38.

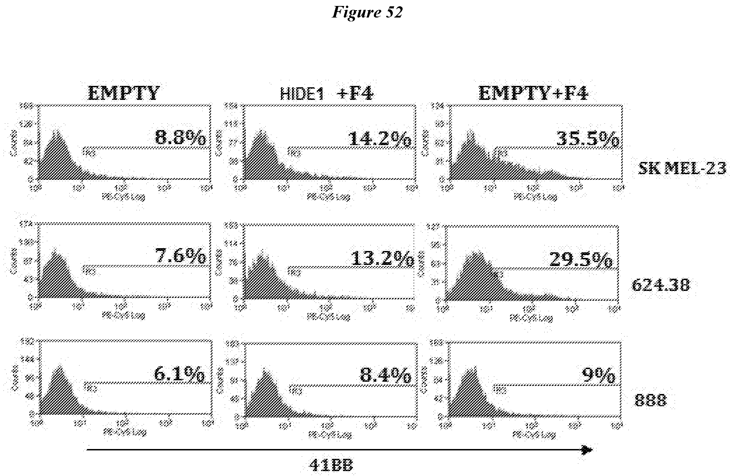

**Figure 52****:** Expression of HIDE1 on F4 expressing PBLs causes a reduction in the expression of CD137 (4-1BB) upon co-culture with SK-MEL23, MEL-624 and MEL-624.38.

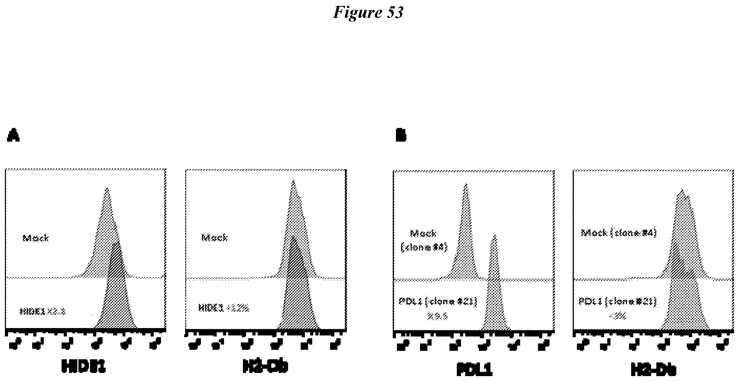

**Figure 53****:** HTDE1 expression and H2Db expression on EL4 cells. (A) Mock cells and HIDE1 transuded EL4 cells (0.5x10⁵ per sample) were stained with rabbit anti-HIDE1 (Genscript, 10µg/ml) followed by PE donkey anti-rabbit IgG and analyzed by flow cytometry. **(B)** Mock and PDL1 transduced EL4 clone cells were stained with APC-anti-PDL1 and PE-anti-H2Db antibodies.

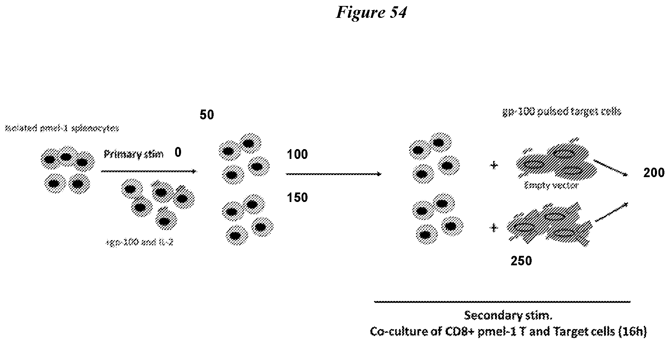

**Figure 54****:** Schematic illustration of pmel-1 experimental system. Primary stimulation: Spleen cells were cultured in the presence of 1µg/ml of gp100-peptide and 25ng/ml of IL-2 for 4 days. CD8+ cells were then isolated and cultured in the presence of 25ng/ml of IL-2 for overnight rest. Secondary stimulation: pmel-1 cultures were washed and co-cultured overnight with gp-100 pulsed EL4 cells as described in material and methods. Surface CD137 and CD25 expression levels on gated pmel-1 CD8+ T cells were examined by flow cytometry. Cytokines secretion levels was tested by Th1/Th2/Th17 cytokine CBA kit.

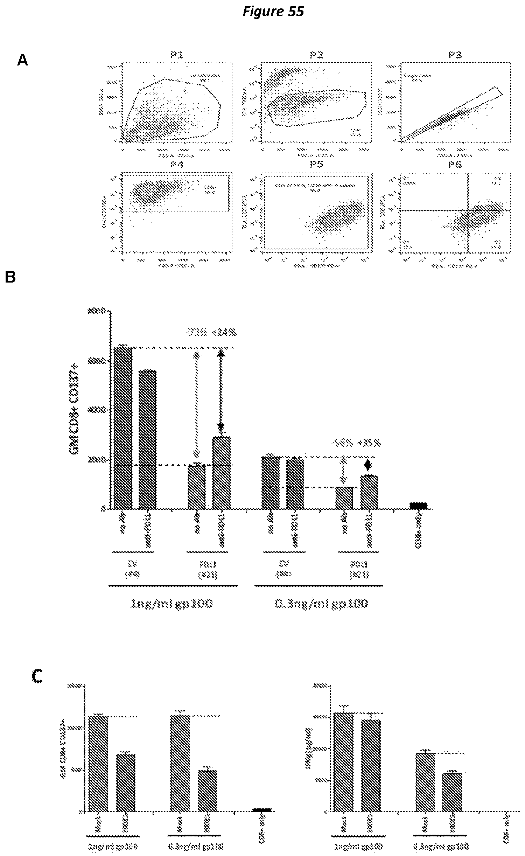

**Figure 55****:** The effect of HIDE1 and PDL1 on pmel-1 CD8+ T cells activation upon co-culture with EL4 cells pulsed with gp-100. Pre-activated pmel-1 splenocytes were cultured alone (5x10⁴/well) or together with gp-100 (0.3 or 1ng/ml) pulsed EL4 cells (25x10⁴/well) at 2:1 E:T ratio. (A) Gating strategy for flow cytometry analysis of resting pmel-1 CD8+ cells following 4d activation of pmel-1 splenocytes with gp-100 peptide and IL2 as described in materials and methods. (B) Values of histograms represent CD137 expression (gMFI) levels of activated CD8+ pmel-1 cells co-cultured with PDL1- transduced EL4 as positive control treated with or without blocking anti-PDLl. (C) Values of histograms represent CD137 expression (gMFI) (left) or IFNγ (right) levels of activated CD8+ pmel-1 cells co-cultured with HIDE1- transduced EL4 cells. Each bar is the mean ± SEM of triplicate samples. One representative experiment out of three independent experiments performed is shown.

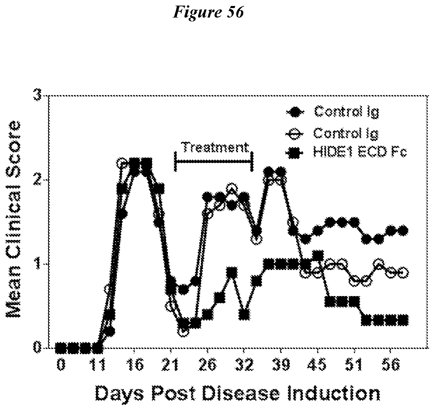

**Figure 56****:** Therapeutic effect of HIDE1-ECD-Ig (SEQ ID: 18) in the PLP139-151-induced R-EAE model in SJL mice. HIDE1-ECD-Ig (SEQ ID: 18) was administered in a therapeutic mode from the onset of disease remission, at 100 microg/mouse i.p. 3 times per week for two weeks. Therapeutic effects on clinical symptoms are demonstrated as reduction in Mean Clinical Score.

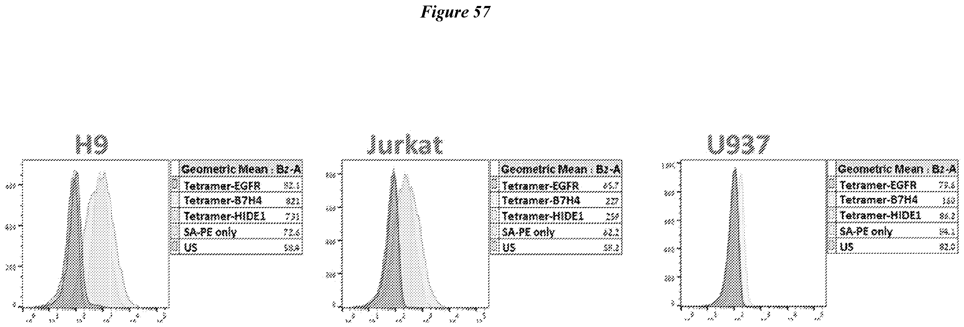

**Figure 57****:** Presents HIDE1 tetramer binding to H9, Jurkat and U937 human cell lines. Cells were stained with viability dye, then incubated with HIDE1 tetramer (orange) and control EGFR and B7H4 tetramers (dark green and light green, respectively) at 3µg/well (60µg/ml) and evaluated by flow cytometry. The binding of HIDE1 is shown compared to control EGFR gated on live cells following singlet gating. Values of histograms represent the geometric mean fluorescent intensity (Geo Mean) of gated cells. Shown is one representative experiment out of three independent experiments performed.

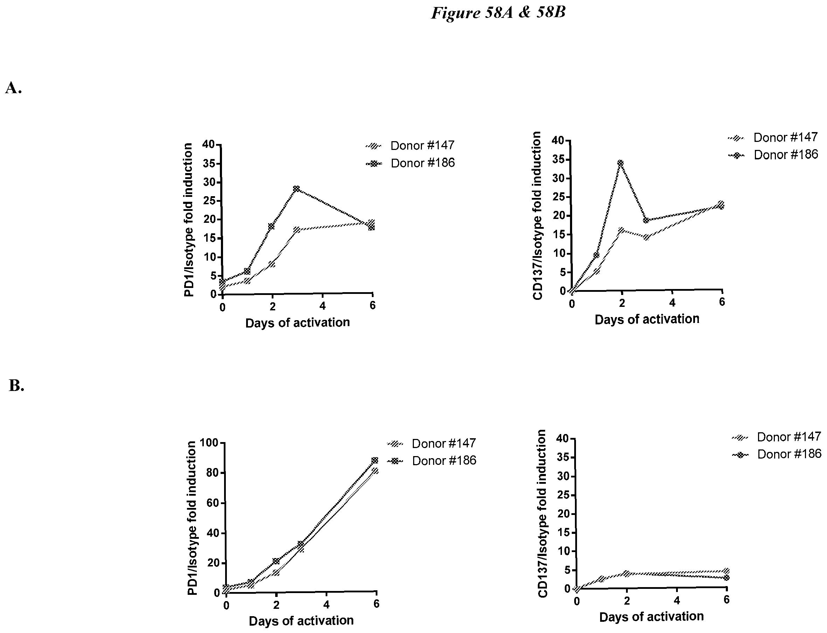

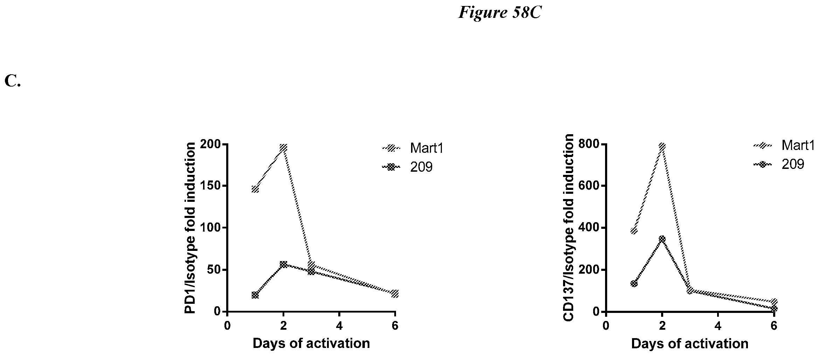

**Figure 58A, 58B****, &** **58C****:** Shows detection of CD137 and PD-1 surface expression. CD8+ T cells, CD4+ T cells and TILs were activated and monitored over time at 4 time-points as described in M&M. Resting or activated cells were first gated for lymphocytes (FSC-A vs. SSC-A), followed by live cells gating, further gated for singlets (FSC-H vs. FSC-A), CD4/CD8 positive cells and further gated for CD137 and PD1. Shown is surface expression of PD-1 (left) and CD137 (right) on (A) CD8+ T cells (B) CD4+ T cells and (C) TILs at different time-points normalized to isotype control over the time course of activation.

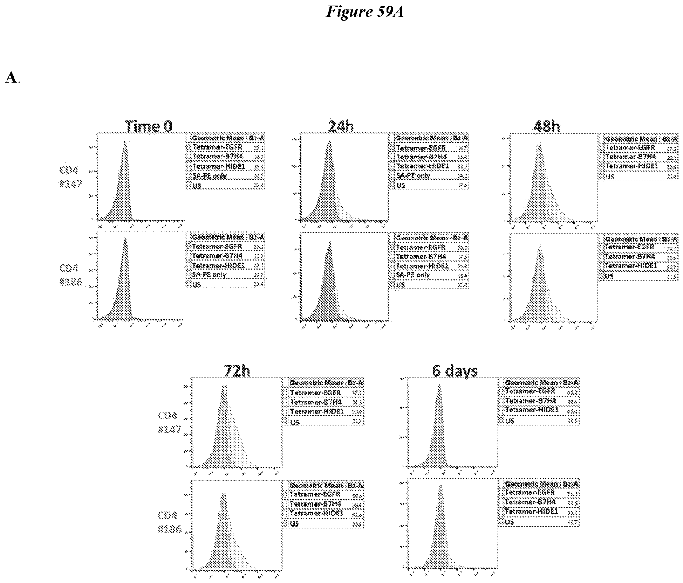

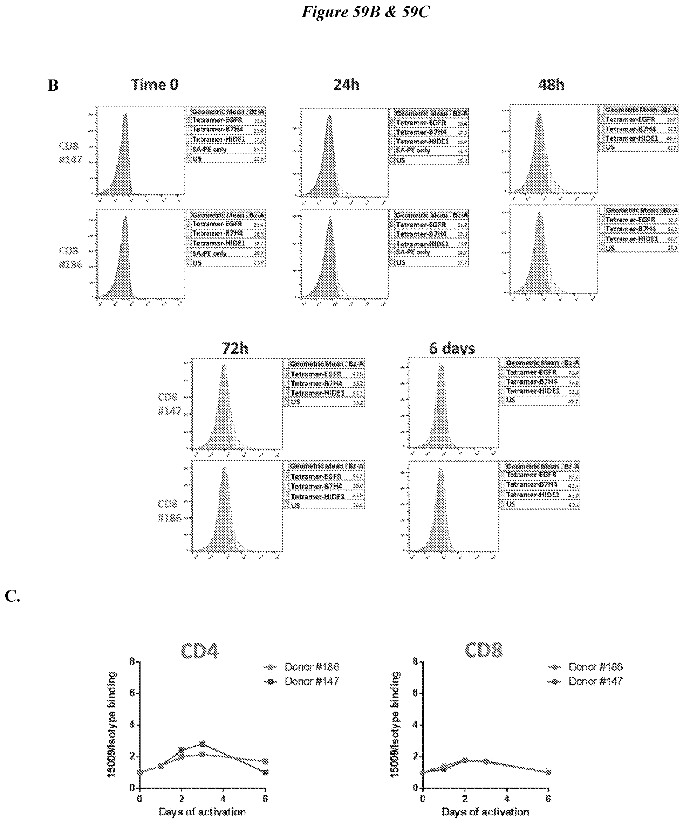

**Figure 59A****,** **59B, & 59C****:** Shows HIDE1 binding to resting and activated CD4+ T and CD8+ T cells. CD4+ and CD8+ T cells were activated and monitored over time at 4 time-points as described in M&M. Cells were stained with viability dye, then incubated with HIDE1 tetramer and controls (3µg/well), and evaluated by flow cytometry. (A) Binding of HIDE1 to CD4+ T cells. Binding of HIDE1 to live resting (time 0) and activated CD4+ cells following singlet gating for 24, 48, 72h and 144h compared to EGFR control. (B) Binding of HIDE1 to CD8+ T cells. Binding of HIDE1 to live resting (time 0) and activated CD8+ cells following singlet gating for 24, 48, 72h and 144h compared to EGFR control. Shown are the Geometric Mean of the fluorescent intensity values obtained. (C) Fold change of HIDE1 tetramer binding normalized to EGFR tetramer control over the time course of activation.

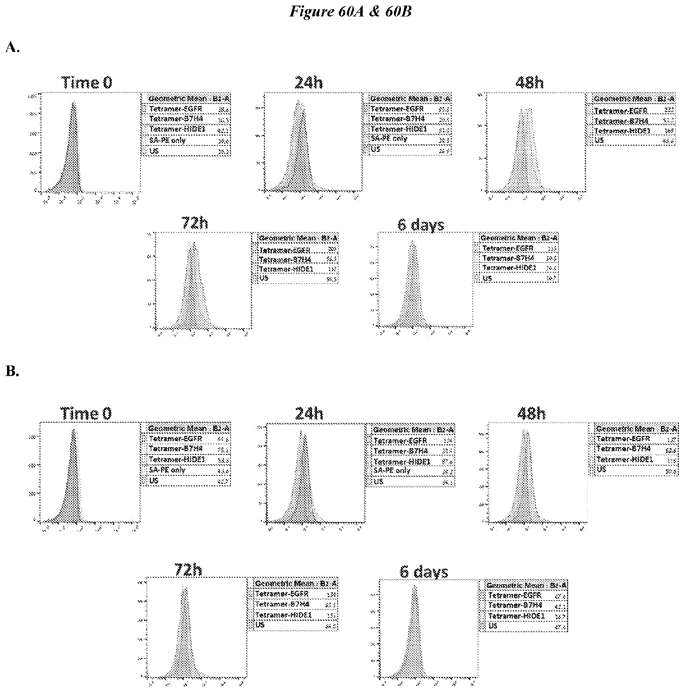

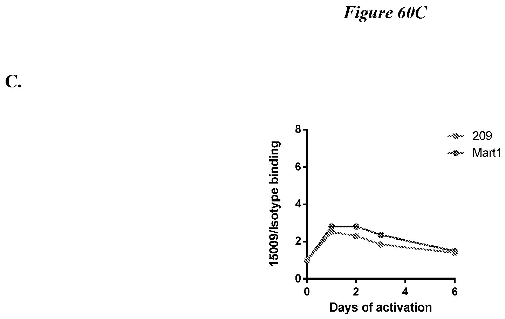

**Figure 60A, 60B****, &** **60C****:** Shows HIDE1 binding to resting and activated TILs. TIL Mart1 and 209 were activated and monitored over time at 4 time-points as described in M&M. Cells were stained with viability dye, then incubated with HIDE1 tetramer and controls (3µg/well), and evaluated by flow cytometry. **(A)** Binding of HIDE1 to TIL Mart1. Binding of HIDE1 to live resting (time 0) and activated TIL following singlet gating for 24, 48, 72h and 144h compared to EGFR control. **(B)** Binding of HIDE1 to TIL 209. Binding of HIDE1 to live resting (time 0) and activated TIL following singlet gating for 24, 48, 72h and 144h compared to EGFR control. Shown are the Geometric Mean of the fluorescent intensity values obtained. **(C)** Fold change of HIDE1 tetramer binding normalized to EGFR tetramer control over the time course of activation.

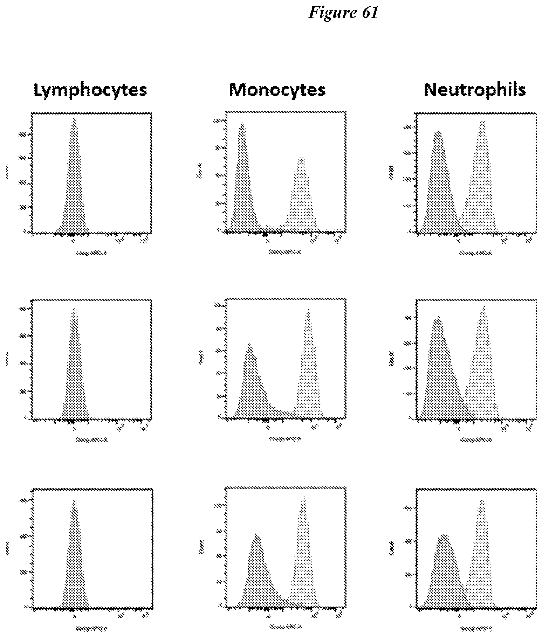

**Figure 61****:** Surface expression of HIDE1 by healthy PBLs was evaluated using whole blood taken from 3 different healthy donors using anti Hide1 mAbs 33B4-2F7 as compared to staining with isotype control. Isotype: Red line. Anti HIDE1 mAb: Blue line

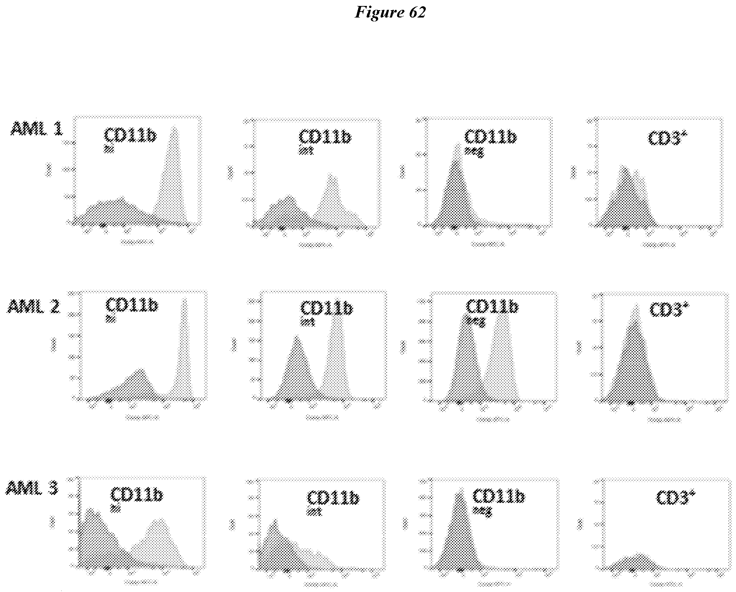

**Figure 62****:** Surface expression of HIDE1 by whole blood taken from 3 different AML patients using anti Hide1 mAbs 33B4-2F7 as compared to staining with isotype control. Isotype: Red line. Anti HIDE1 mAb: Blue line.

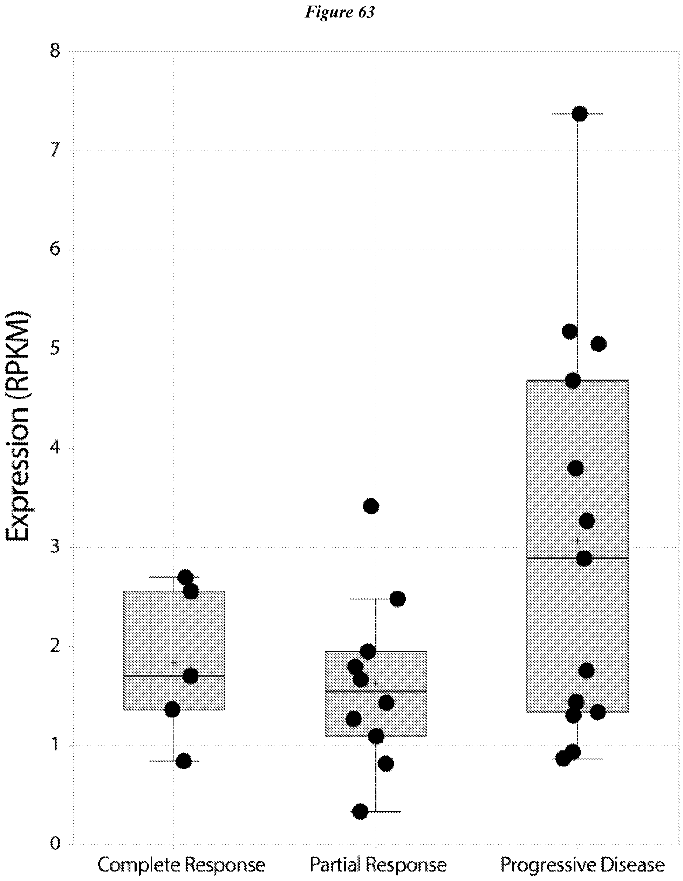

**Figure 63****:** Shows levels of HIDE1 RNA expression in patients treated with PD-1 inhibitor

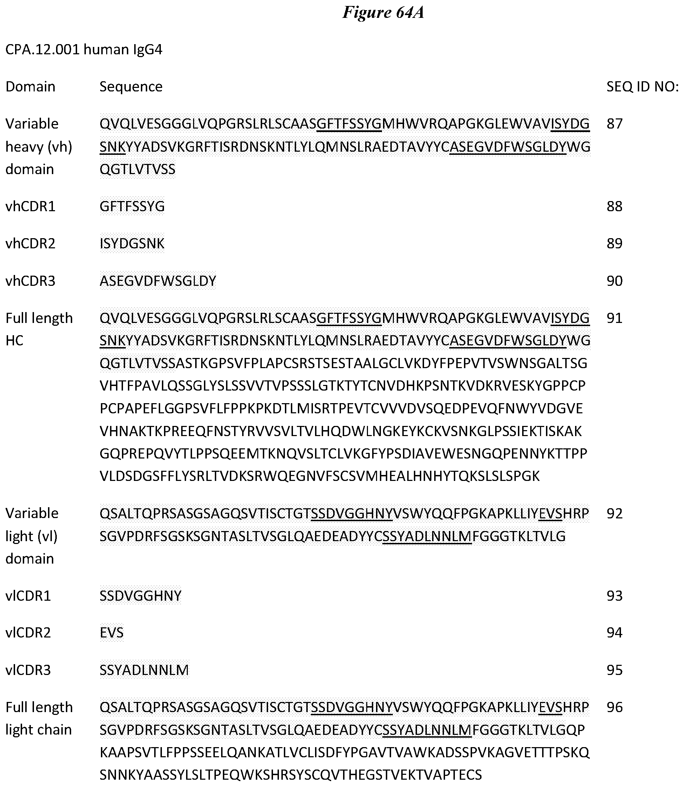

**Figure 64A****-N:** Anti-HIDE1 antibody sequences CPA. 12.001 human IgG4, CPA. 12.002 human IgG4, CPA. 12.003 human IgG4, CPA.12.004 human IgG4, CPA.12.005 human IgG4, CPA. 12.006 human IgG4, CPA.12.007 human IgG4, CPA.12.008 human IgG4, CPA. 12.009 human IgG4, CPA. 12.011 human IgG4, CPA. 12.012 human IgG4, CPA. 12.013 human IgG4, CPA. 12.014 human IgG4, and CPA. 12.015 human IgG4

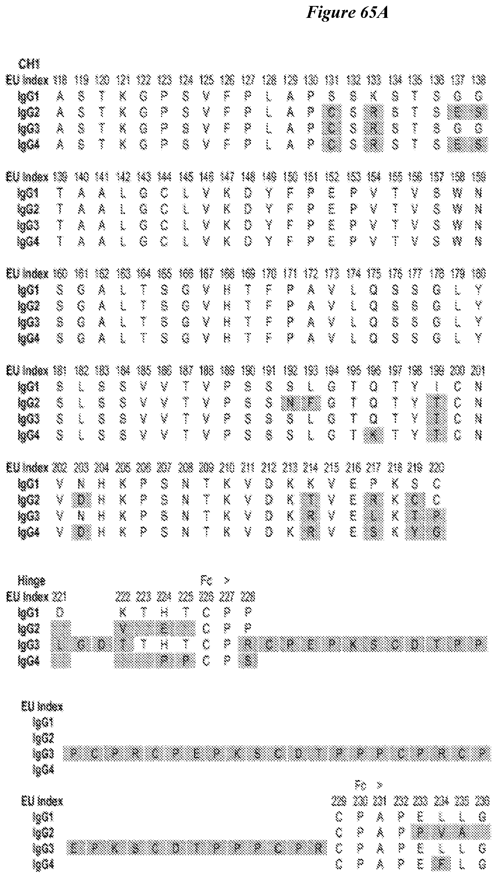

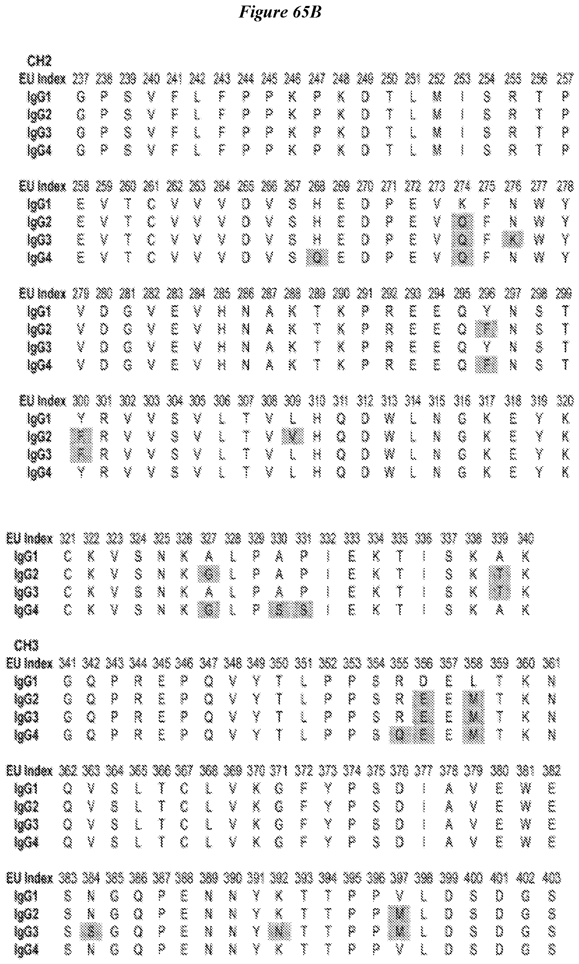

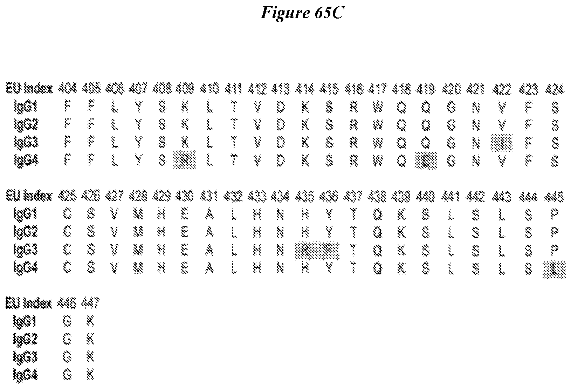

**Figure 65****:** IgG1, IgG2, IgG3, and IgG4 sequences.
**Figure 66****:** HIDE1 ECD and peptide seqeunces.

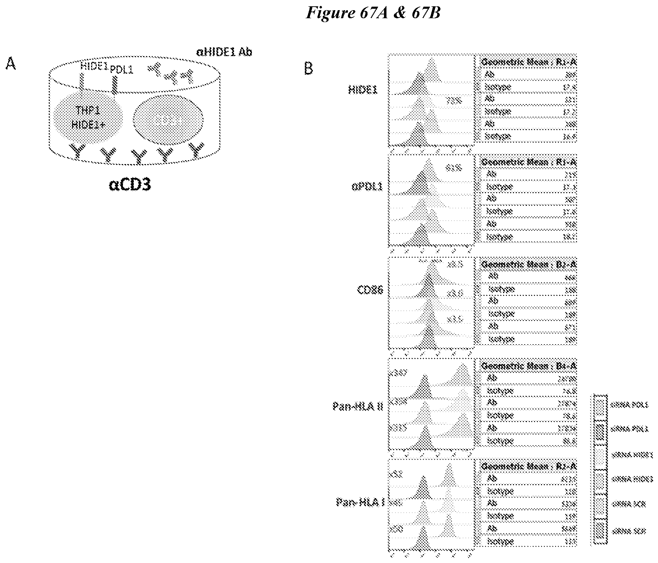

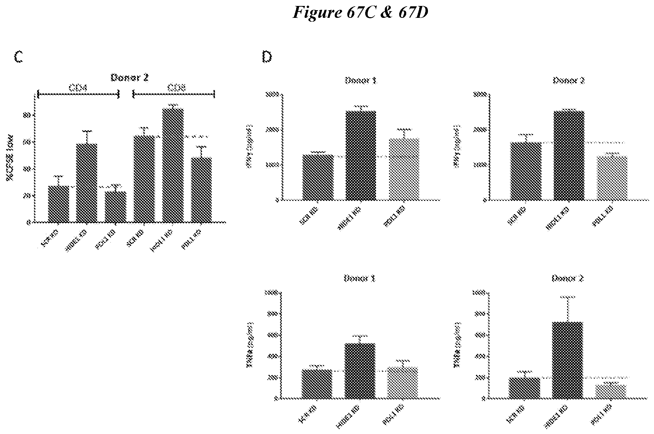

**Figure 67****:** Enhanced proliferation and cytokine secretion observed for HTDE1 siRNA knockdown **(A)** schematic representation of THP1-KD cells co-cultured with CD3 T cells polyclonal activated at 1ug/ml immobilized anti-CD3. **(B)** Histogram depicting levels of HIDE1, PDL1, CD86, HLA-I & HLA-II expression compared to isotype control. HTDE1 and PDL1 knockdown levels calculated were 71% and 61% respectively, as noted in histograms. Fold change of CD86, HLAI & HLA-II noted in histograms calculated showed no variations between HIDE1, PDL1 and SCR KD cells. (C) Levels of CD4 and CD8 T cell proliferation was determined by dilution of CFSE and quantified for triplicates D. Levels of IFNγ and TNFα was determined from the supernatant using TH-1/2/17 CBA kit (BD biosciences) and quantified for triplicates.

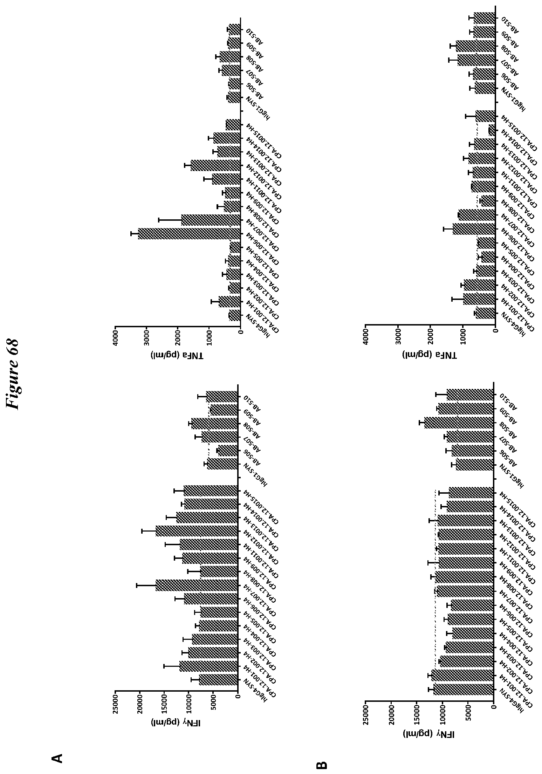

**Figure 68****:** Effect of anti-HIDE1 antibodies on IFNγ and TNFα secretion in THP-1 Treated T cell poly activation assay. Graph depicts normalization of IFNy & TNFα levels, where each bar represents the effect of a specific αHIDE-1 hIgG1 or hIgG4 Ab on IFNγ or TNFα secretion from A. Donor 18 B. Donor 19.

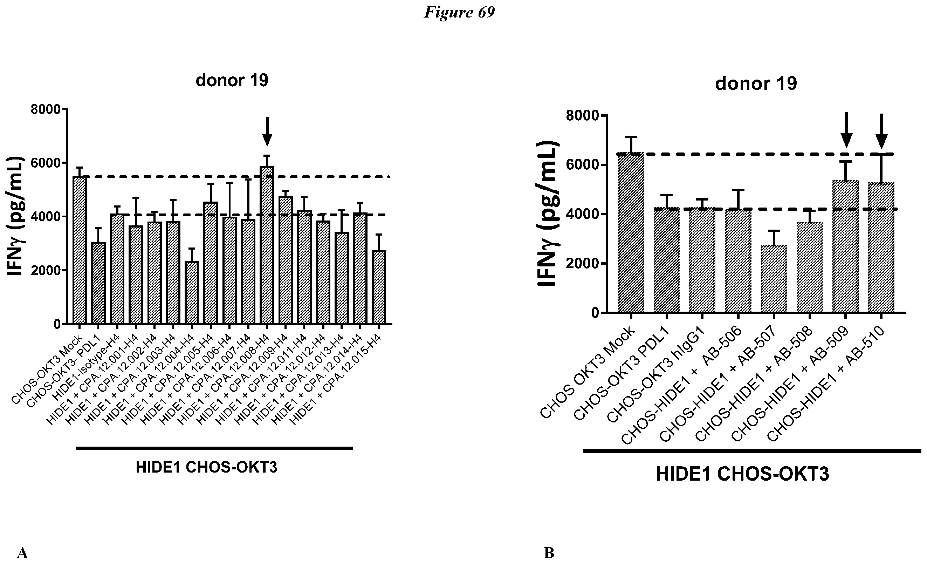

**Figure 69****:** Effect of anti-HIDE1 antibodies on IFNγ secretion in HIDE1 overexpression CHOS-OKT3 assay Graph depicts normalization of IFNγ levels, where each bar represents the effect of a specific αHIDE-1 hIgG1 Ab on IFNγ secretion from Donor 19.

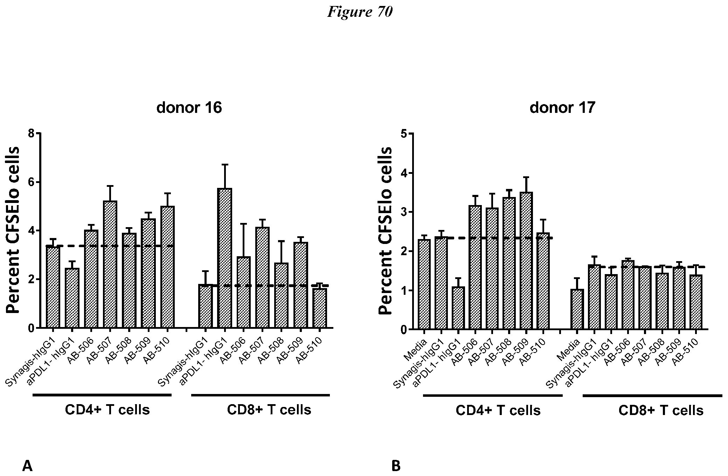

**Figure 70****:** Effect of anti-HIDE1 antibodies on T cell proliferation in immature DC-MLR. Graph depicts normalization of proliferation, where each bar represents the effect of a specific αHIDE-1 hIgG1 Ab on CD4 or CD8 T cell proliferation from Donor 16 (A) and Donor 17 (B).

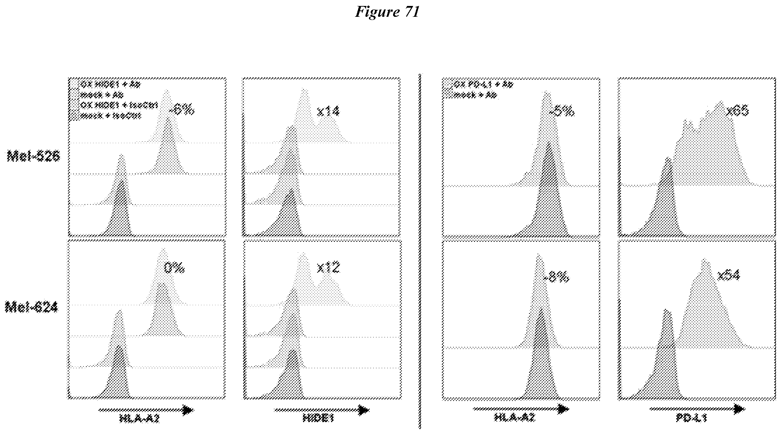

**Figure 71****:** hHIDE1/ hPD-L1 and HLA-A2 surface expression on Mel-526 and Mel-624 cells. Mock and hPDL1/HIDE1 transduced Mel-526 and Mel-624 cells (0.5x10⁵ per sample) were stained with APC-anti-PDL1 (5µg/ml) or AF647 anti HIDE1 (1ug/mL) and PE-anti-HLA-A2 (5µg/ml) antibodies. gMFI levels of PE, APC and AF647 were compared between over expressing cells to mock transduced cells. HLA-A2 levels comparison is indicated by percentage. HIDE1 or PD-L1 expression is indicated by fold of expression.

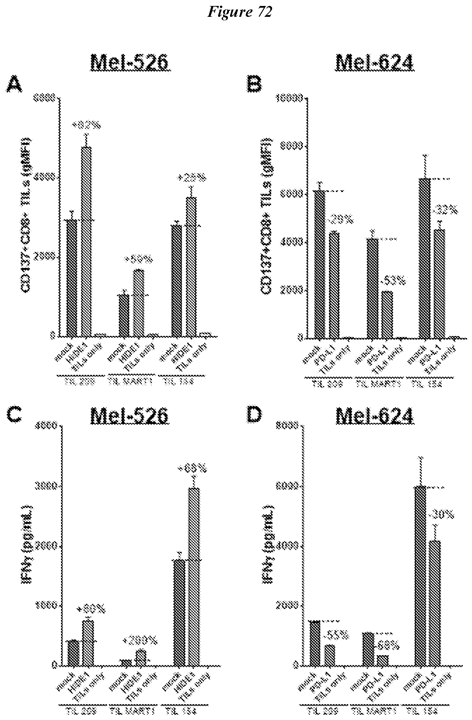

**Figure 72****:** Effect of hPDL1 and hHIDE1 on TIL activation upon co-culture with Mel-526 and Mel-624 cells. **(A-B)** gp100 or MART-1 reactive TILs were co-cultured with hPDL1 **(A,C)** or hHIDE1 **(B,D)** overexpressing Mel-526 and Mel-624 cells at 1:1 effector to target ratio as described in material and methods. Values of histograms represent CD137 expression (gMFI) or IFNγ secretion from TIL-209, TIL-154 and TIL-MART1. Each bar is the mean ± SEM of triplicate samples.

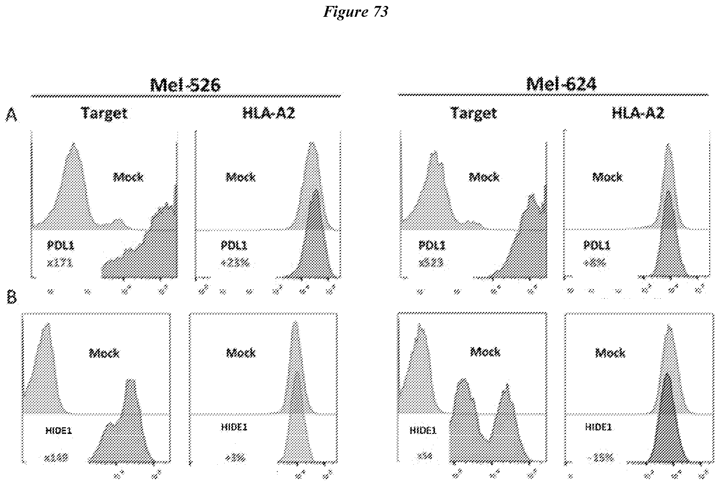

**Figure 73****:** HIDE1 expression and HLA-A2 expression on Mel-526 and Mel-624 cells. **(A)** Mock and hPDL1 transduced Mel-526 and Mel-624 cells (0.5x10⁵ per sample) were stained with APC-anti-PDL1 (5µg/ml) and PE-anti-HLA-A2 (5µg/ml) antibodies. **(B)** Mock cells and hHIDE1 transuded Mel-526 and Mel-624 cells were stained with AF647 anti-HIDE1 (In-house, 1µg/ml) and analyzed by flow cytometry.

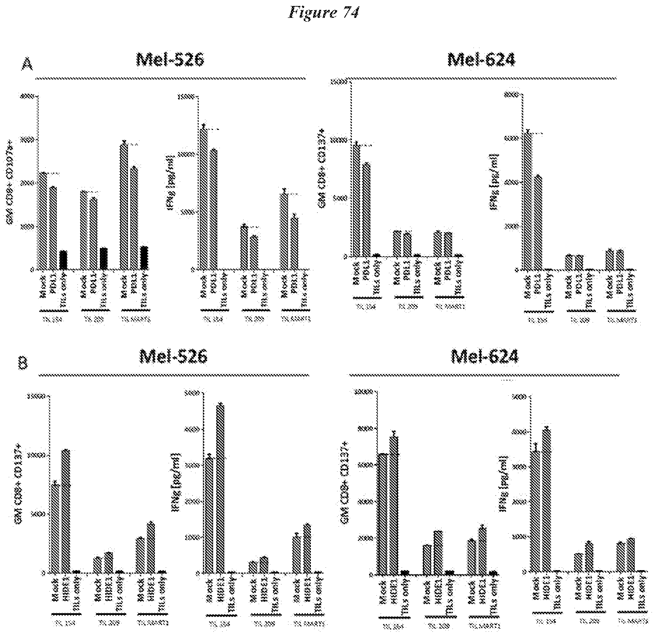

**Figure 74****:** Effect of hPDL1 and hHIDE1 on TIL activation upon co-culture with Mel-526 and Mel-624 cells. **(A-B)** gp100 or MART-1 reactive TILs were co-cultured with hPDL1 **(A)** or hHIDE1 **(B)** overexpressing Mel-526 and Mel-624 cells at 1:1 effector to target ratio as described in material and methods. Values of histograms represent CD137 expression (gMFI) or IFNγ secretion from TIL-209, TIL-154 and TIL-MART-1. Each bar is the mean ± SEM of triplicate samples.

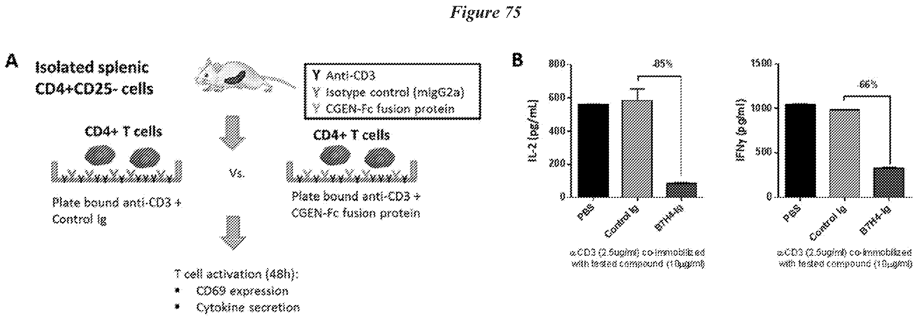

**Figure 75****:** Experimental system and B7H4-Ig effect on T cell activity. **(A)** Schematic illustration of the experimental system. Plates were coated with anti-CD3 mAb (2ug/mL) in the presence of tested compound, as described in material and methods. The effect of the tested protein on T cell activation, manifested by activation markers and cytokine secretion, was analyzed. **(B)** Culture supernatants were collected 48 h post-stimulation and mouse IL-2 and IFNγ levels were analyzed by ELISA. Results are shown as Mean ± Standard errors of duplicate samples.

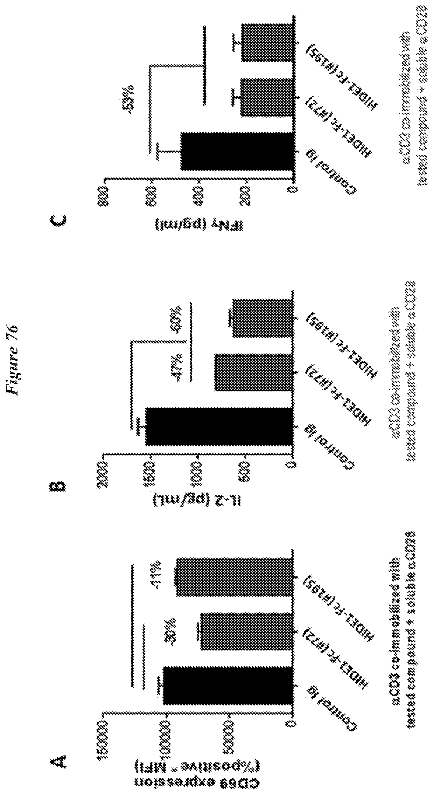

**Figure 76****:** Effect of HIDE1-Fc on mouse CD4 T cell activation. Plates were coated with anti-CD3 mAb (2µg/mL) in the presence of 10µg/ml HIDE1-Fc or control mIgG2a as described in materials and methods. Wells were plated with 1x10⁵ CD4+CD25- mouse T cells per well in the presence of 2ug/ml of soluble anti-CD28. **(A)** The expression of CD69 was analyzed by flow cytometry at 48h post-stimulation. **(B-C)** Culture supernatants were collected at 48 h post-stimulation and mouse IFNγ or IL-2 levels were analyzed by ELISA. Results are shown as Mean ± Standard errors of duplicate samples.

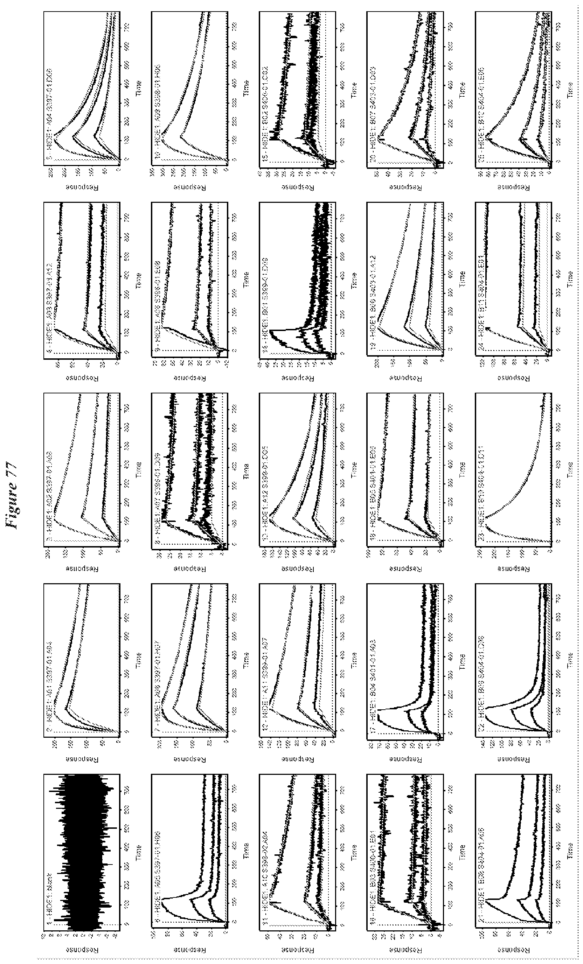

**Figure 77****:** Anti-HIDE1 Fabs injected over HIDE1- HH-1 captured to a GLC chip (black lines). A 1:1 kinetic binding model (red line) provided rough estimates of the binding parameters. Panels where no fitting lines are shown indicate complex kinetics.

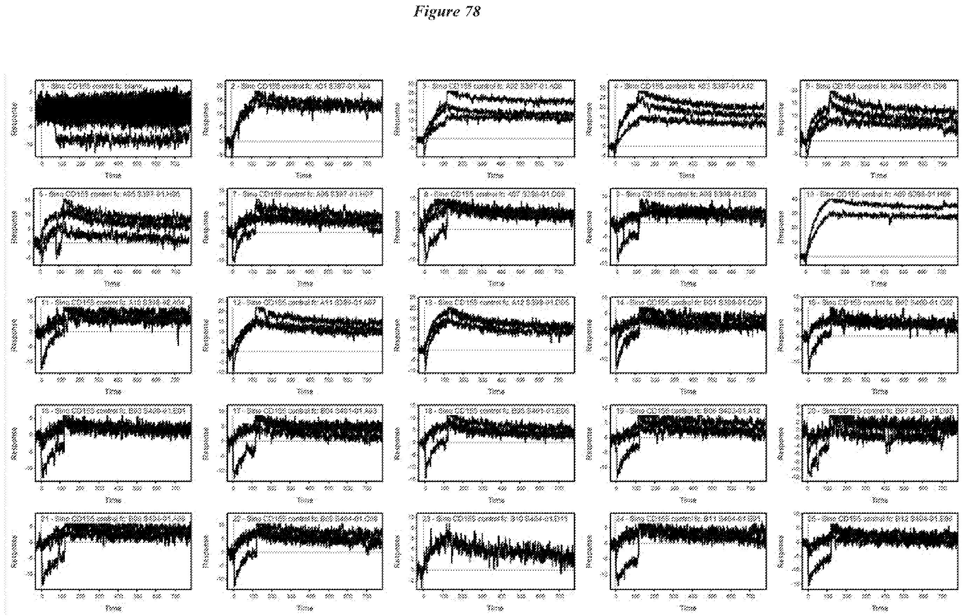

**Figure 78****:** Anti-HIDE1 Fabs injected over CD155 control fusion protein captured to a GLC chip (black lines). There were either no binding responses or the binding responses were minimal compared to the same HIDE1 Fabs injected over HIDE1- fusion protein **(****Figure 77****).**

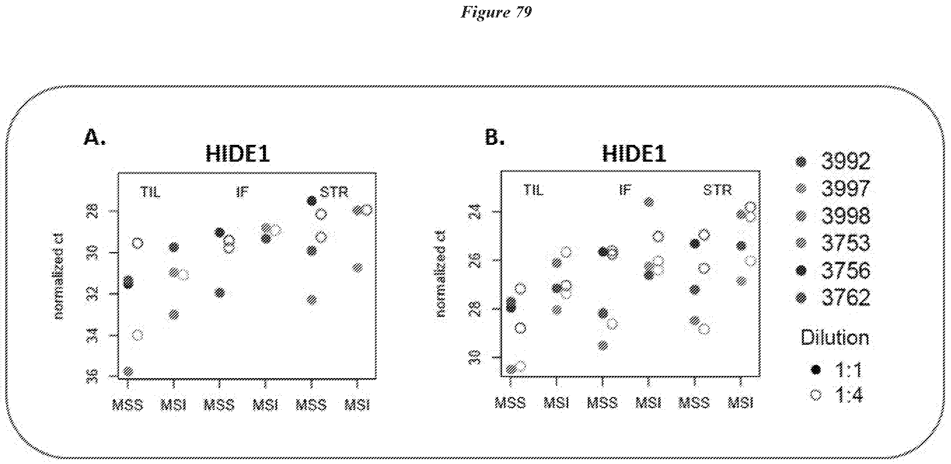

**Figure 79****:** Transcript expression of human HIDE1 in cells derived from three different areas (TILs, Invasive Front and Stroma) from MSI and MSS colorectal cancer patients. Expression of human transcript of HIDE1 was analyzed by qRT-PCR using 2 specific TaqMan probes Hs01128131_m1 (B) and Hs01128129_m1 (A). Analysis indicating higher transcript expression in TIL, Invasive front and Stroma areas in MSI patients (3/3) compared to MSS patients and higher expression in stroma and IF areas compared to TIL area.

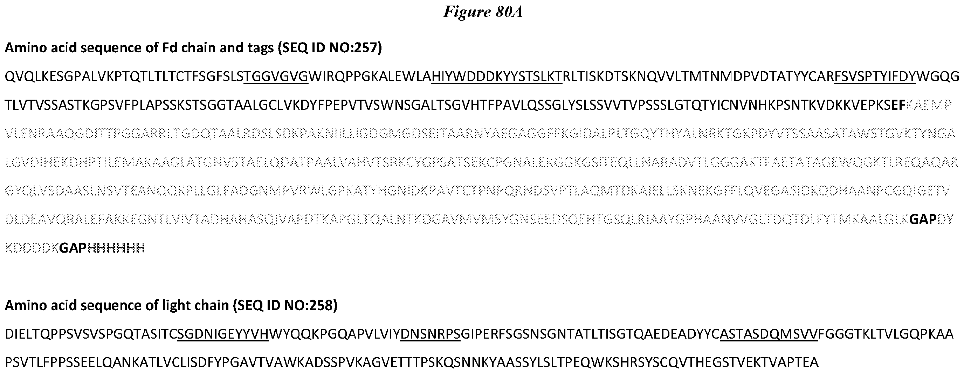

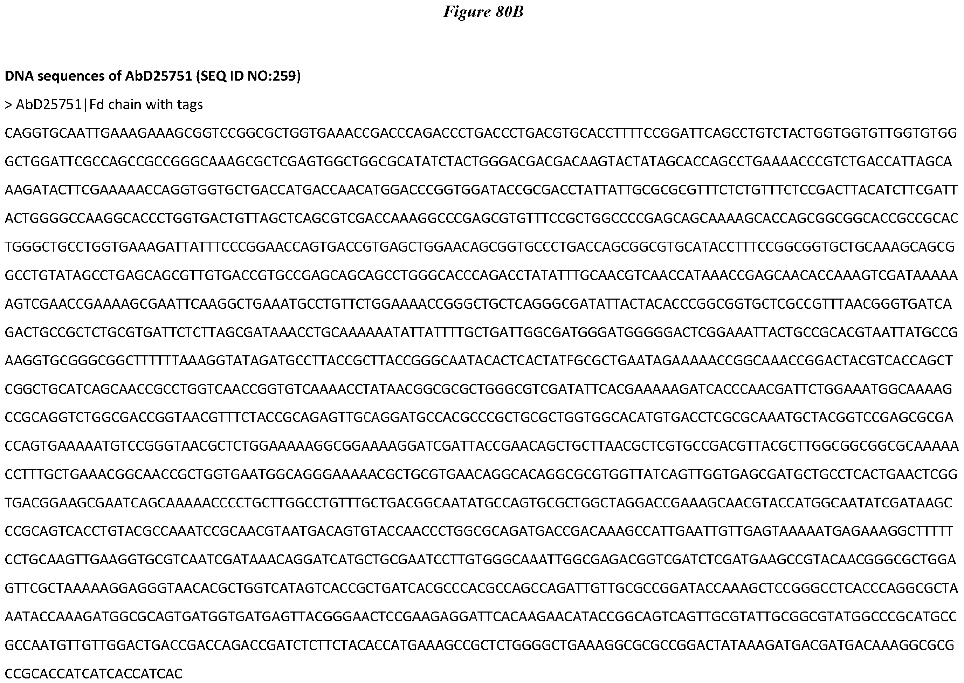

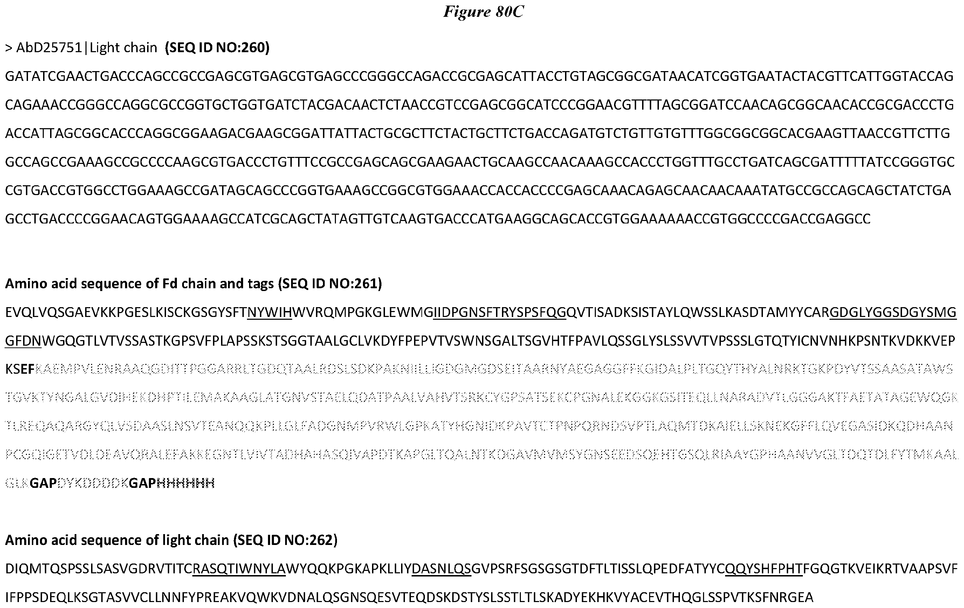

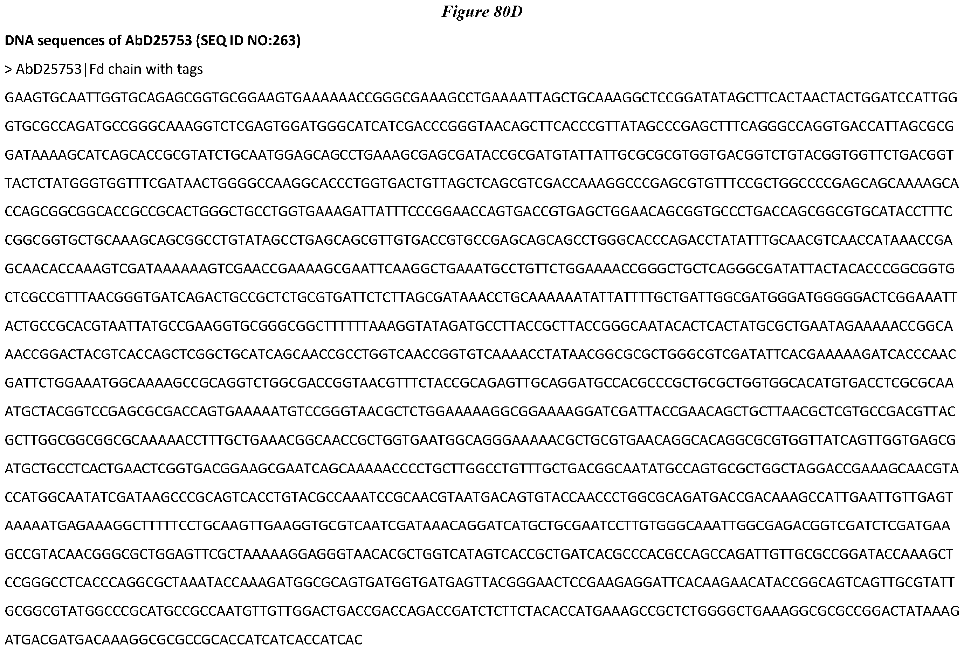

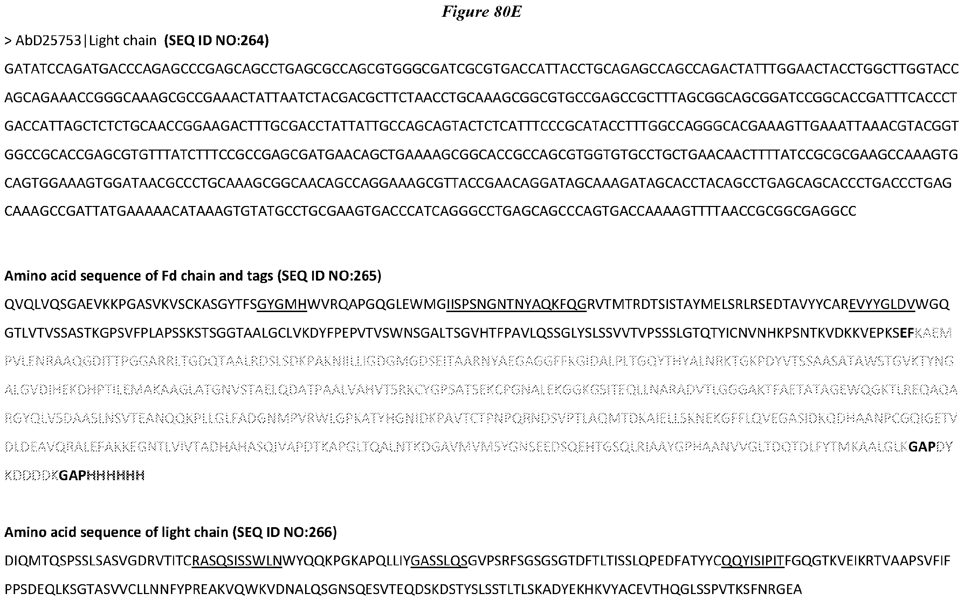

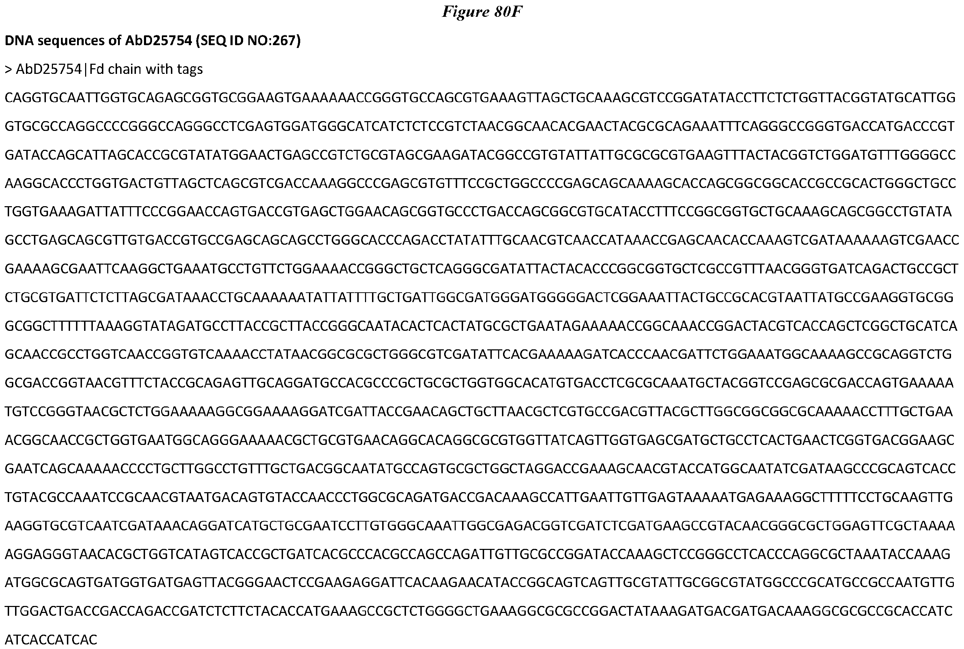

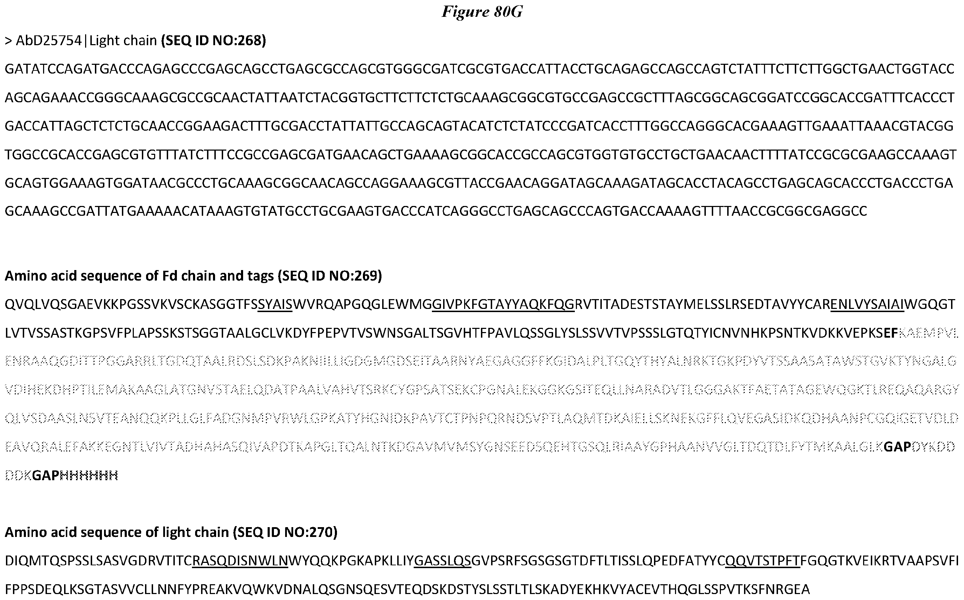

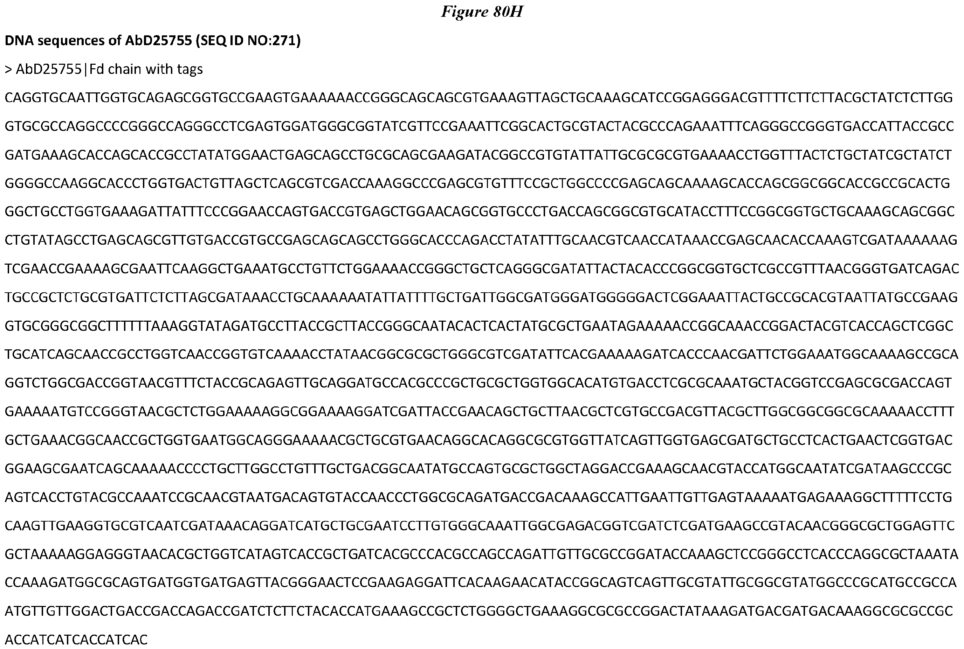

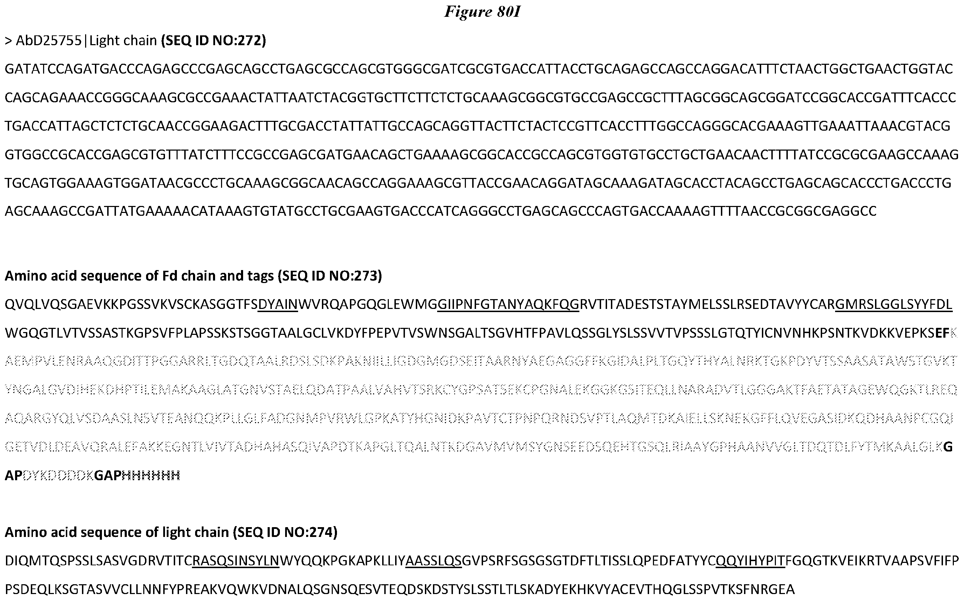

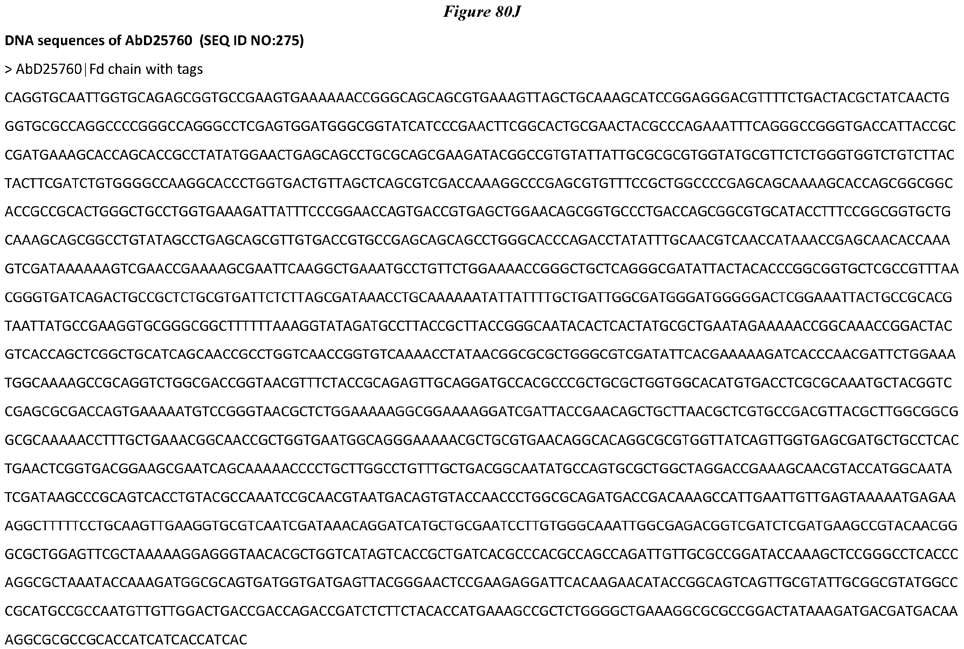

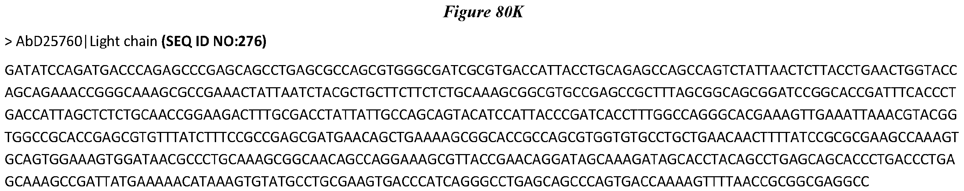

**Figure 80****:** Serotec antibody sequences. Complementarity determining regions (CDRs) are underlined. CDR definition is according to standard definitions (Krebs, B, et al., J Immunol Methods 2001, 254:67-84). Constant domains CH1 and CL sequence are in italics, dimerization domain sequence (AP) is in green (ref. 4), linker sequences are in bold, FLAG® tag (ref. 5) is in pink and His6 tag (ref. 6) is in blue.

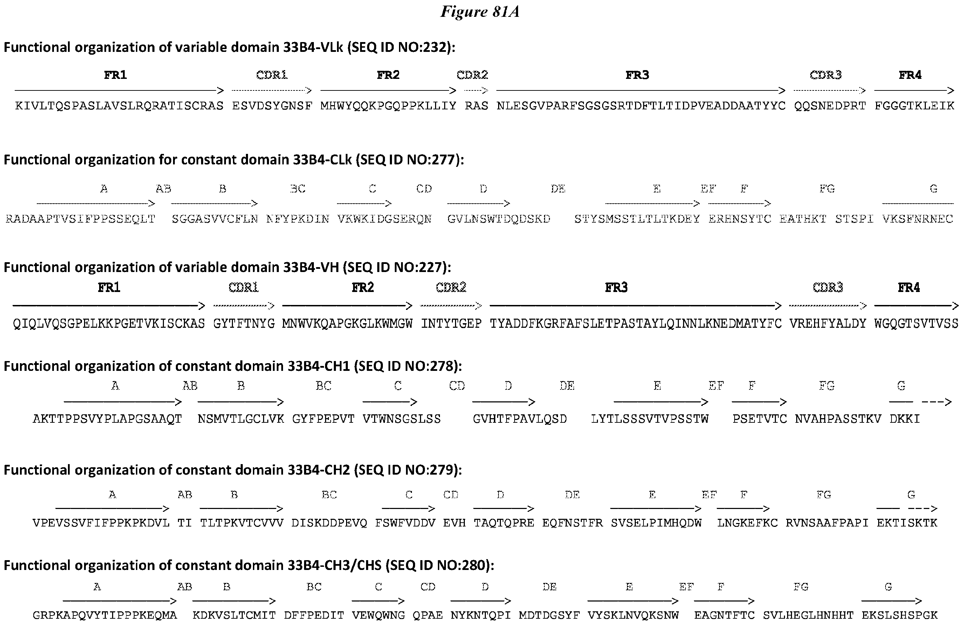

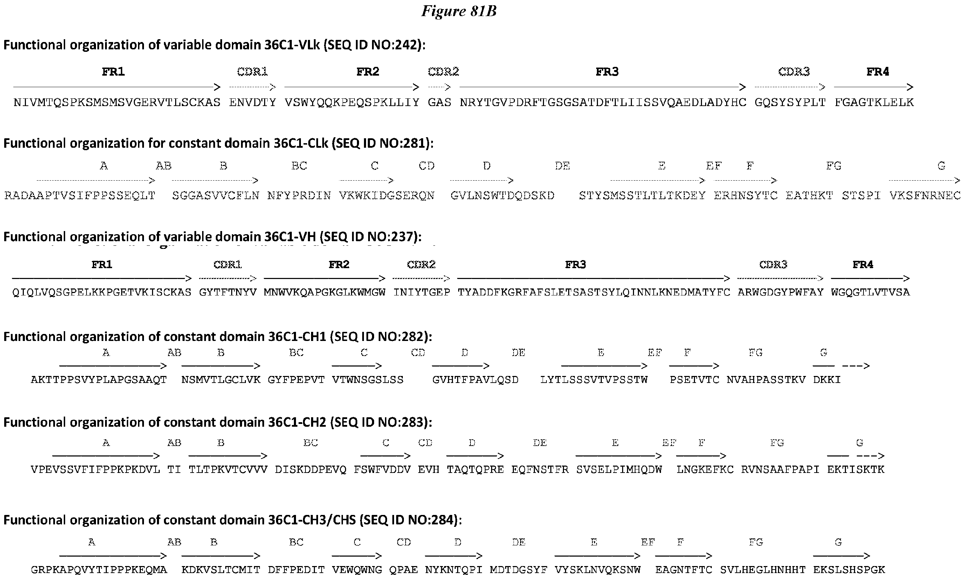

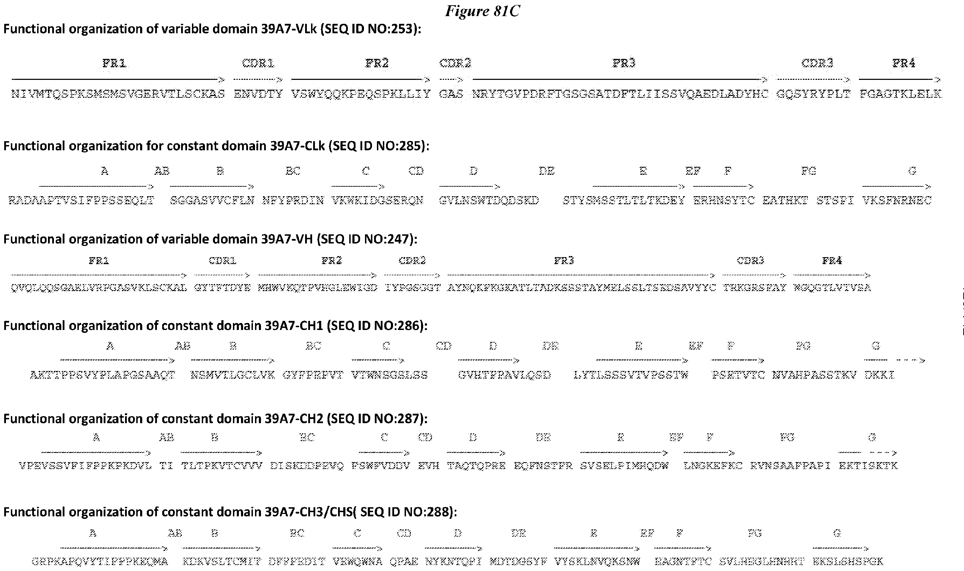

**Figure 81****:** Anti-HIDE1 antibody seqeunces 33B4, 36C1, and 39A7.

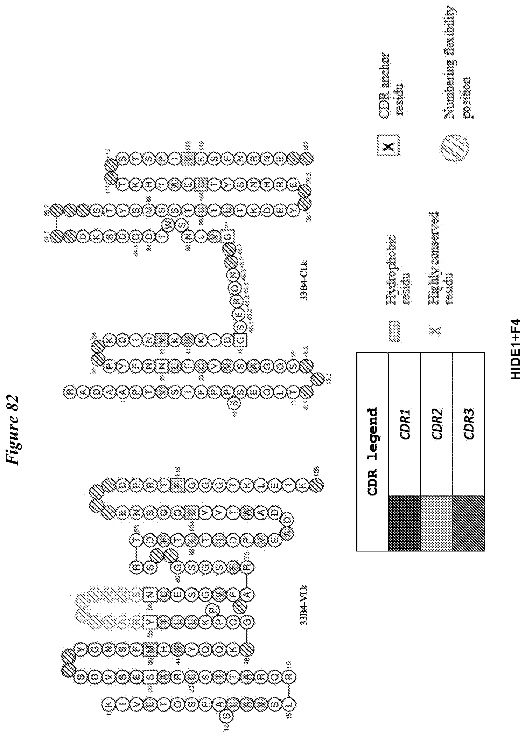

**Figure 82****:** Schematic representation of 33B4-Vlk and 33B4-Clk.

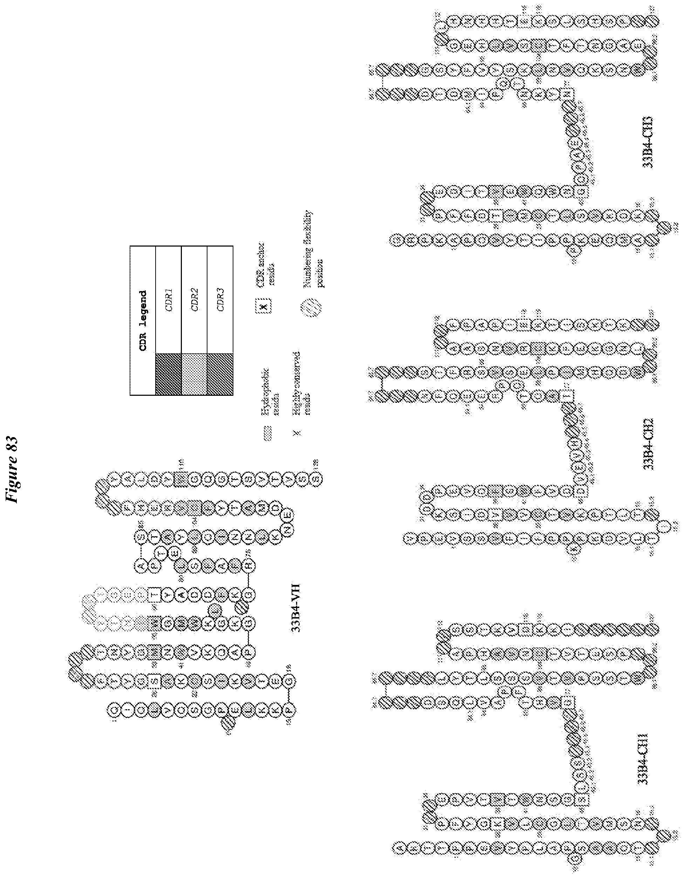

**Figure 83****:** Schematic representation of 33B4-VH and 33B4-CH.

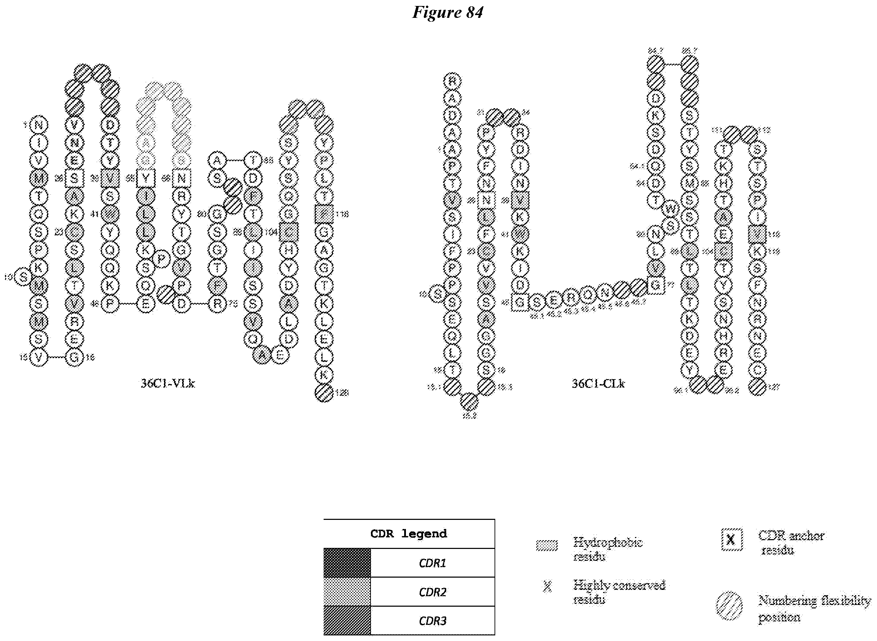

**Figure 84****:** Schematic representation of 36C1-Vlk and 36C1-Clk.

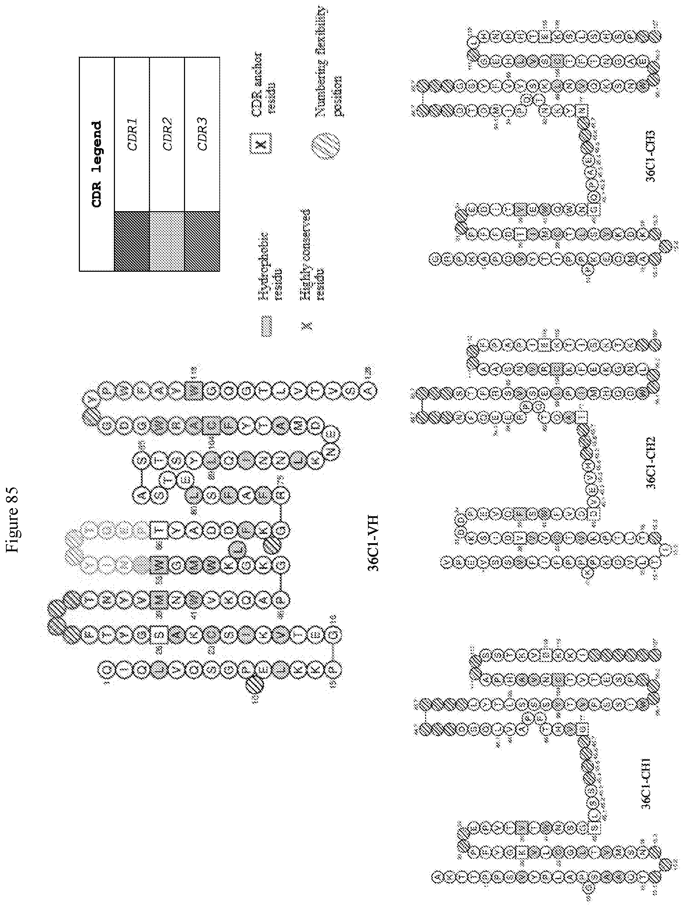

**Figure 85****:** Schematic representation of 36C1-VH and 36C1-CH.

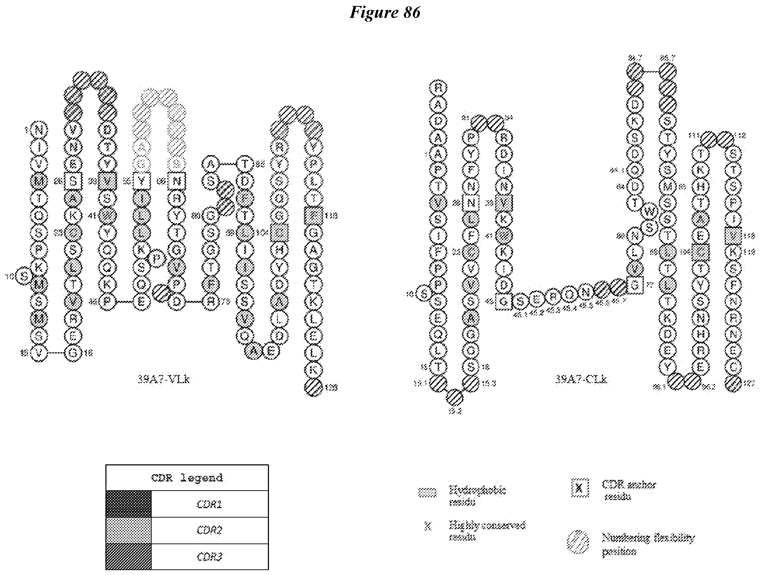

**Figure 86****:** Schematic representation of 39A7-Vlk and 39A7-Clk.

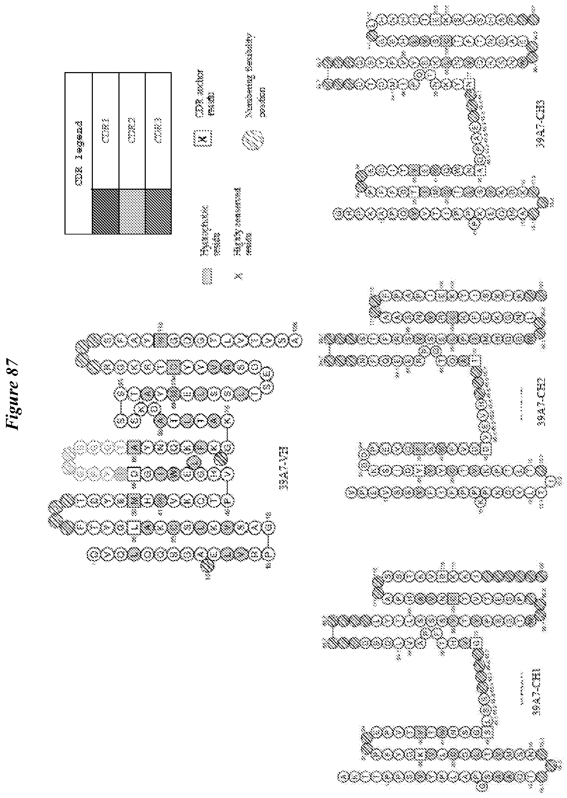

**Figure 87****:** Schematic representation of 39A7-VH and 39A7-CH.

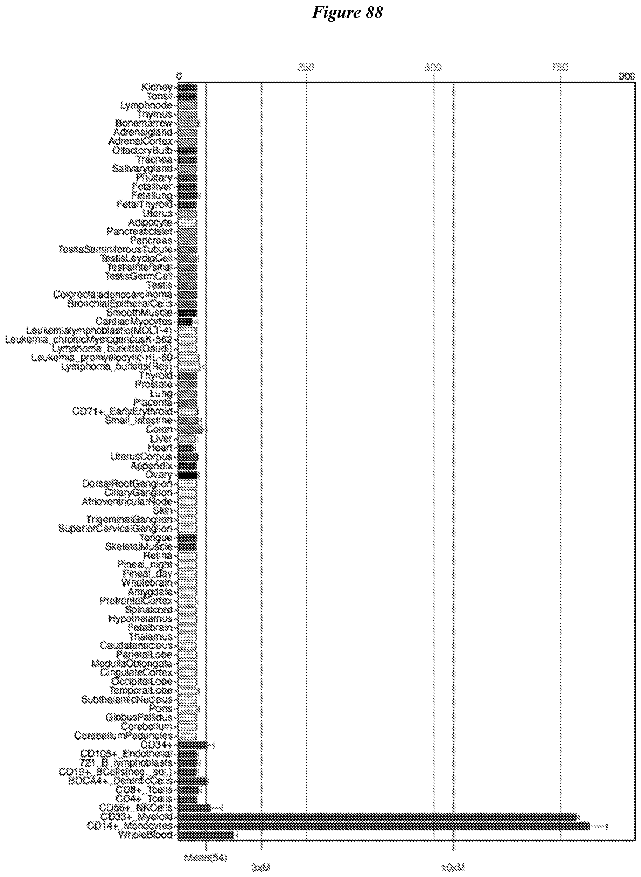

**Figure 88****:** HIDE1 expression in blood cells and tissues with enriched blood cells (GTEx data).

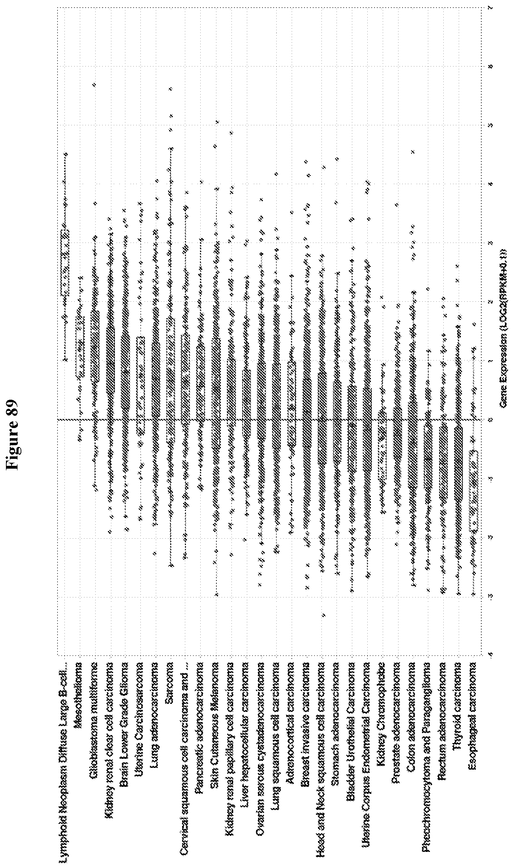

**Figure 89****:** HIDE1 expression pattern in BioGPS.

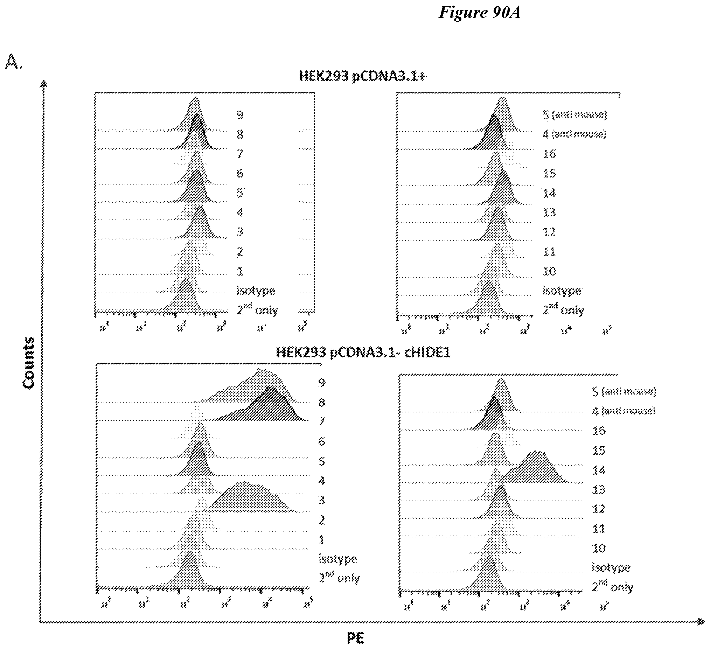

**Figure 90 A-B****:** FACS analysis using anti-human HIDE1 and anti-mouse HIDE1 Fab's on HEK293 cells over-expressing cyno HIDE1 Flag protein. (A)HEK293 cells over-expressing the cyno HIDE1 (lower panel) or HEK293 transduced with empty vector (upper panel) were analyzed by FACS using Serotec anti-human HIDE1 Fab's (1-16) and anti-mouse HIDE1 Fab's (4,5). Detection was carried out using Goat Anti Human IgG F(ab')2-PE secondary Ab. (B) Summary table of Serotec anti-human HIDE1 Fab's (1-16) and anti-mouse HIDE1 Fab's (4,5) FACS analysis, the Geo mean ratio of HEK293 cells overexpressing the cyno HIDE1 Flag/ HEK293 transduced with empty vector.

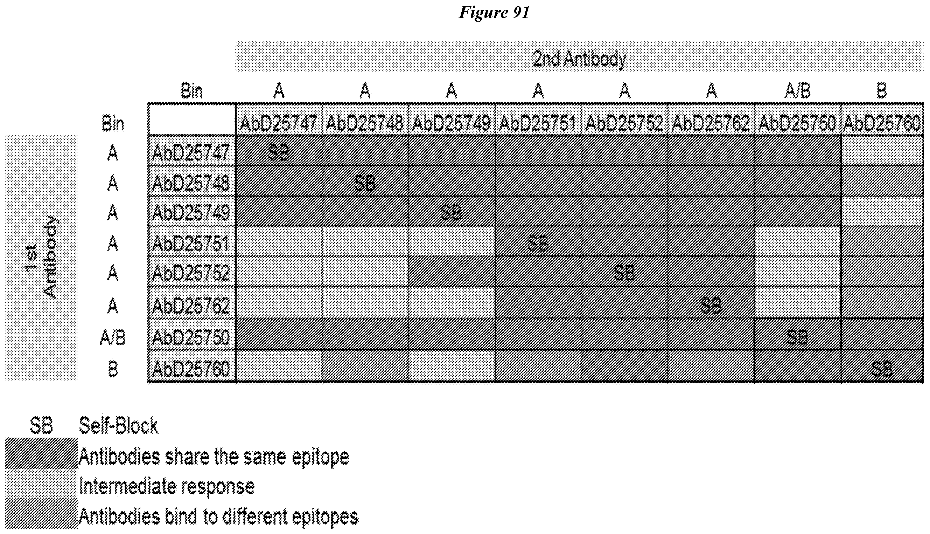

**Figure 91****:** Serotec summary for the epitope binning data for anti human HIDE1 antibodies, group 1.

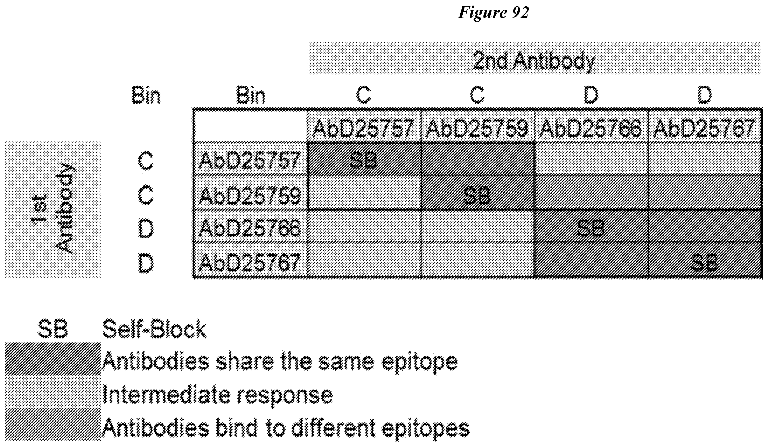

**Figure 92****:** Serotec summary for the epitope binning data for anti human HIDE1 antibodies, group 2.

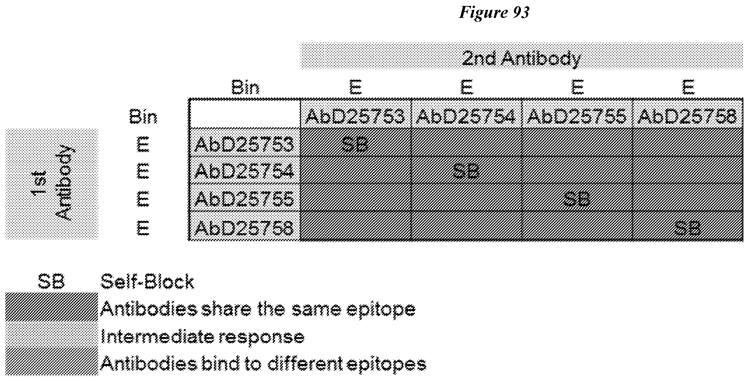

**Figure 93****:** Serotec summary for the epitope binning data for anti human HIDE1 antibodies, group 3.

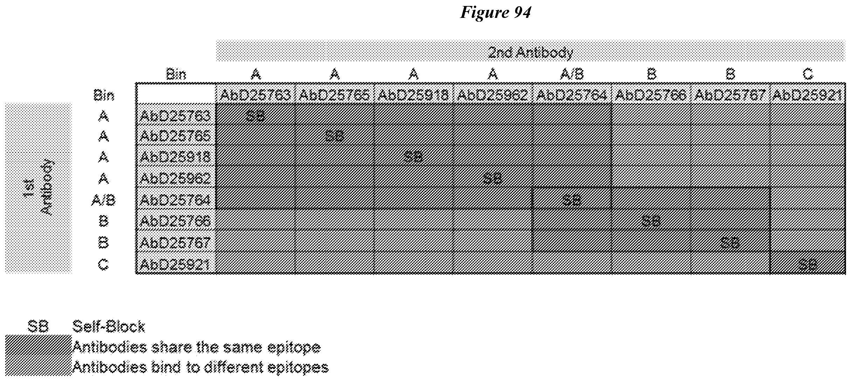

**Figure 94****:** Serotec summary for the epitope binning data for anti mouse HIDE1 antibodies

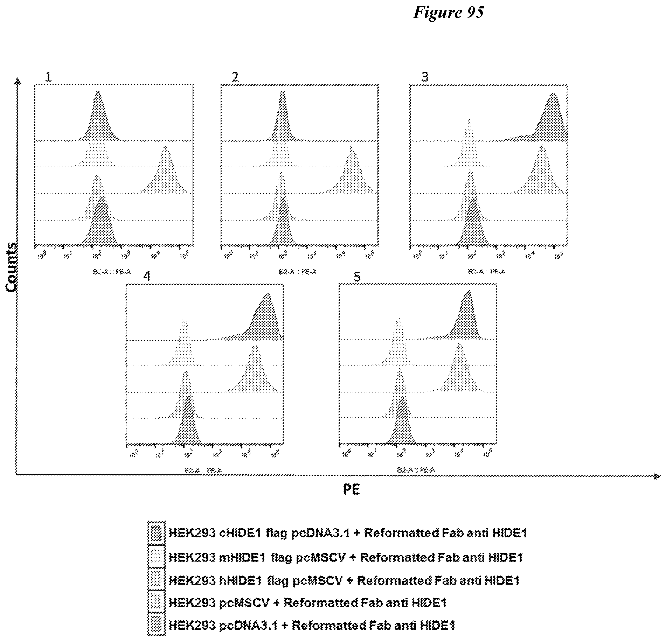

**Figure 95****:** FACS analysis using anti human HIDE1 Reformatted Fab's on HEK293 cells over-expressing human/mouse/cyno HIDE1 Flag protein. (A)HEK293 cells over-expressing the human HIDE1 Flag (orange line) or HEK293 cells over-expressing the mouse HIDE1 Flag (light green line) or HEK293 cells over-expressing the cyno HIDE1 (dark green line) or HEK293 transduced/transfected with empty vector (blue and red line respectively) were analyzed by FACS using Serotec anti-Human HIDE1 reformatted Fab's (1-5). Detection was carried out using Goat Anti Human IgG-PE secondary Ab.

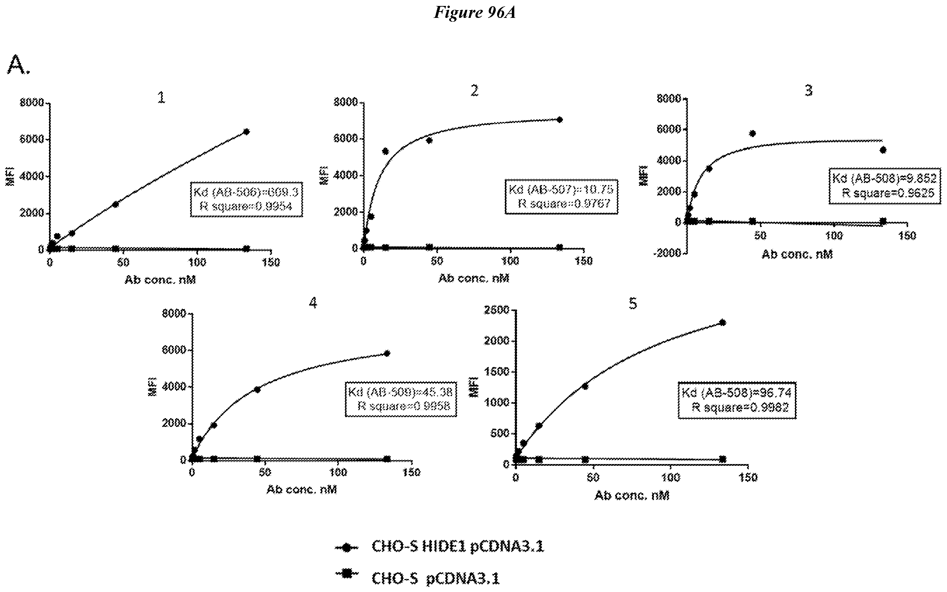

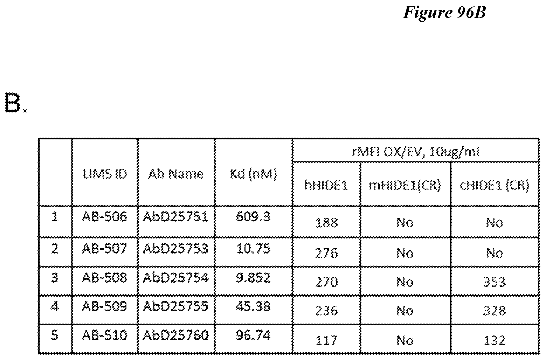

**Figure 96****:** Affinity measurements using FACS application for the anti-human HIDE1 reformatted Fab's on CHO-S cells over-expressing human HIDE1 Flag protein. (A)CHO-S cells over-expressing the human HIDE1 Flag (circle dots) or HEK293 transduced with empty vector (square dots) were analyzed by FACS using Serotec anti-Human HIDE1 reformatted antibodies (1-5) in 7 concentrations- series dilution 1:3, 10-0.01µg/ml . Detection was carried out using Goat Anti Human-PE secondary Ab. (B) Summary table of Serotec anti-human HIDE1 reformatted antibodies 1-5 FACS analysis, the Geo mean ratio of HEK293 cells over-expressing the human HIDE1 Flag/ HEK293 transduced with empty vector and the cross reactive validation, the Geo-mean ratio of HEK293 cells over-expressing the mouse/cyno HIDE1 Flag/ HEK293 transduced/transfected with empty vector. And Kd (nM) for each reformatted Fab.

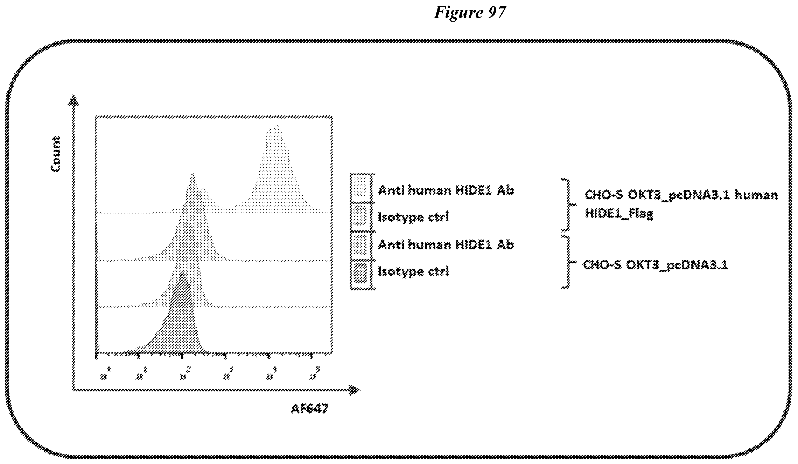

**Figure 97****:** FACS analysis of CHO-S OKT3 cells ectopically expressing human HIDE1 Flag pcDNA3.1 vector. CHO-S OKT3 cells over expressing human HIDE1 Flag or CHO-S OKT3 cells transfected with an empty vector were analyzed by FACS using mouse monoclonal anti human HIDE1 (BIOTEM, 33B4-2F7- Alexa 647). Mouse IgG (Biotem, F1150528d-2695- Alexa 647) was used as an isotype control

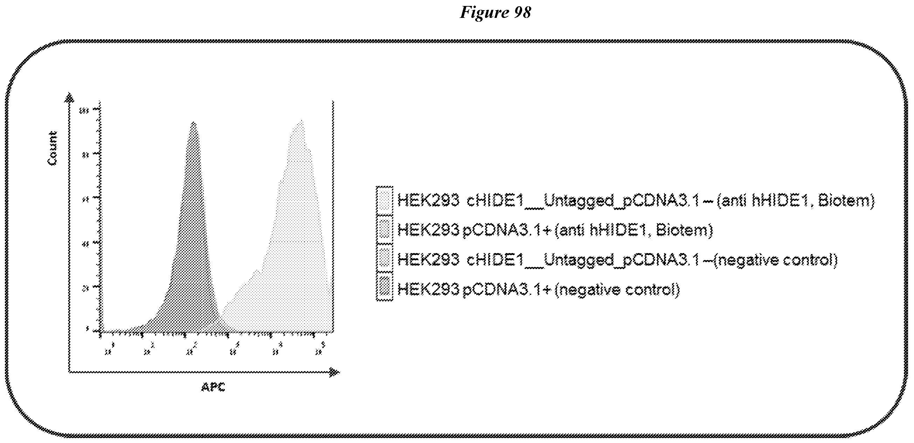

**Figure 98****:** FACS analysis of hek293 cells ectopically expressing cyno HIDE1 pcDNA3.1 vector. Hek293 cells over expressing cyno HIDE1 or HEK293 cells transfected with an empty vector were analyzed by FACS using mouse monoclonal anti human HIDE1 (BIOTEM, 33B4-2F7- Alexa 647). Mouse IgG (Biotem, F1150528d-2695- Alexa 647) was used as an isotype control.

### DETAILED DESCRIPTION OF THE INVENTION

### I. Introduction

Cancer can be considered as an inability of the patient to recognize and eliminate cancerous cells. In many instances, these transformed (e.g. cancerous) cells counteract immunosurveillance. There are natural control mechanisms that limit T-cell activation in the body to prevent unrestrained T-cell activity, which can be exploited by cancerous cells to evade or suppress the immune response. Restoring the capacity of immune effector cells-especially T cells-to recognize and eliminate cancer is the goal of immunotherapy. The field of immuno-oncology, sometimes referred to as "immunotherapy" is rapidly evolving, with several recent approvals of T cell checkpoint inhibitory antibodies such as Yervoy, Keytruda and Opdivo. These antibodies are generally referred to as "checkpoint inhibitors" because they block normally negative regulators of T cell immunity. It is generally understood that a variety of immunomodulatory signals, both costimulatory and coinhibitory, can be used to orchestrate an optimal antigen-specific immune response. Generally, these antibodies bind to checkpoint inhibitor proteins such as CTLA-4 and PD-1, which under normal circumstances prevent or suppress activation of cytotoxic T cells (CTLs). By inhibiting the checkpoint protein, for example through the use of antibodies that bind these proteins, an increased T cell response against tumors can be achieved. That is, these cancer checkpoint proteins suppress the immune response; when the proteins are blocked, for example using antibodies to the checkpoint protein, the immune system is activated, leading to immune stimulation, resulting in treatment of conditions such as cancer and infectious disease.

The present invention is directed to antibodies to HIDE1 for use in treating cancer, wherein the antibodies stimulate the immune system by inhibiting the action of HIDE1. HIDE1 is expressed on the cell surface of myeloid cells including but not limited to monocytes, dendritic cells, macrophages, M1/M2 tumor associated macrophages, neutrophils, Myeloid-derive suppressor cells (MDSC), and shares several similarities to other known immune checkpoints.

Functional effects of HIDE1 blocking antibodies on myeloid cells including, for example, but not limited to monocytes, dendritic cells, macrophages, M1/M2 tumor associated macrophages, neutrophils, Myeloid-derive suppressor cells (MDSC), and/or on NK and T-cells can be assessed *in vitro* (and in some cases *in vivo,* as described more fully below) by measuring changes in the following parameters: proliferation, cytokine release and cell-surface makers. For NK cells, increases in cell proliferation, cytotoxicity (ability to kill target cells as measured by increases in CD107a, granzyme, and perforin expression, or by directly measuring target cells killing), cytokine production (*e.g.* IFN-*γ* and TNF), and cell surface receptor expression *(e.g.,* CD25) is indicative of immune modulation, *e.g.* enhanced killing of cancer cells. For T-cells, increases in proliferation, increases in expression of cell surface markers of activation (*e.g*.,CD25, CD69, CD137, and PD1), cytotoxicity (ability to kill target cells), and cytokine production (*e.g*., IL-2, IL-4, IL-6, IFNγ, TNF-a, IL-10, IL-17A) are indicative of immune modulation, e.g. enhanced killing of cancer cells. For myeloid cells: effect on myeloid cells polarization, such as M2 to M1 shift, improvement of antigen presentation by myeloid cells including more efficient cross-presntation by professional as well as non-professional antigen-presenting cells, enhanced antigen uptake and processing by antigen-presenting cells. Additional effects can include relief of T cell suppression (*i.e.* indirect effect on T cell activation), effect on cell recruitment (*i.e.* influx of immune cells and shift to more "inflamed tumors").

In some embodiments, the anti-HIDE1 antibody is a depleting HIDE1 antibody. In some embodiments, a depleting anti-HIDE1 antibody binds to cell surface HIDE1. In some embodiments, the anti HIDE1 depleting antibody preferably is able to deplete HIDE1 expressing cells including but not limited to monocytes, dendritic cells, macrophages, M1/M2 tumor associated macrophages, neutrophils, Myeloid-derive suppressor cells (MDSC) and as a result reduce the number of HIDE1 expressing cells in a patient treated with the anti HIDE1 depleting antibody. Such depletion may be achieved via various mechanisms such as antibody-dependent cell mediated cytotoxicity (ADCC) and/or complement dependent cytotoxicity (CDC), inhibition of HIDE1 expressing cells proliferation and/or induction of HIDE1+ cell death (*e.g.* via apoptosis).

HIDE1 expressing cell depleting anti HIDE1 antibody might optionally be conjugated with or fused to a cytotoxic agent.

"Complement dependent cytotoxicity" or "CDC" refers to the lysis of a target cell in the presence of complement. Activation of the classical complement pathway is initiated by the binding of the first component of the complement system to antibodies which are bound to their cognate antigen. To assess complement activation, a CDC assay, e.g. as described in Gazzano-Santoro et al. (1997), or any other CDC assay known in the art, can be performed or employed with the anti-HIDE1 antibodies of the invention.

"Antibody-dependent cell-mediated cytotoxicity" or "ADCC" refers to a form of cytotoxicity in which secreted antibodies bound onto Fc receptors (FcRs) present on certain cytotoxic cells *(e.g.,* Natural Killer (NK) cells, neutrophils, monocytes and macrophages) allow the cytotoxic effector cells to bind specifically to an antigen-bearing target cell and subsequently kill the target cell.

Accordingly, in at least some embodiments the present invention provides antibodies, including antigen binding domains, that bind to human HIDE1 and activate T cells and/or NK cells and/or activate myeloid cells which induce activatory, migratory, secretory effect enhancing T/NK cell function and migration to treat cancer. According to at least some embodiments of the present invention also provides antibodies, including antigen binding domains,that bind to human HIDE1 for use in methods of depleting myeloid cells, or other circulating tumor cells, in order to treat cancer.

Furthermore, without wishing to be limited by a single hypothesis, HIDE1 shows potentiating effects on the following immune functions: induction or differentiation and proliferation of inducible T regulatory or suppressor cells (iTregs). These cells are known to be involved in eliciting tolerance to self-antigens and to suppress anti-tumor immunity. Again without wishing to be limited by a single hypothesis, HIDE1 contributes to a non-functional phenotype of CD8 T cell from the tumor environment, also known as T cell exhaustion.

The flip side of immuno-oncology is the suppression of T cell activation in conditions where the immune system is too active, or is launching an immune response to an auto-antigen, etc. Thus, by providing HIDE1 proteins (for example as fusion proteins, as discussed below), treatment of immune conditions such as auto-immune disease, inflammation and allergic diseases can be treated. That is, as HIDE1 has an inhibitory effect on specific immune cells such as CD4⁺ T cells, CD8⁺ T cells or CTLs, and NK cells, which cells are known to be involved in the pathology of certain immune conditions such as autoimmune and inflammatory disorders, as well as eliciting a potentiating effect on Tregs or immuno-supressive myeloid cells, HIDE1 polypeptides which potentiate or agonize the effects of HIDE1 on immunity may optionally be used for treating conditions wherein the suppression of T cell or NK mediated immunity and/or the induction of immune tolerance or prolonged suppression of antigen-specific immunity is therapeutically desirable, e.g., the treatment of autoimmune, inflammatory or allergic conditions, and/or the suppression of undesired immune responses such as to cell or gene therapy, adverse immune responses during pregnancy, and adverse immune responses to transplanted heterologous, allogeneic or xenogeneic cells, organs and tissues and for inhibiting or preventing the onset of graft versus host disease (GVHD) after transplant.

Therefore, in one embodiment the present invention broadly relates to the development of novel "immunomodulatory proteins" that antagonize or block the effects of HIDE1 on immunity and particularly the effects of HIDE1, on specific types of immune cells and cytokine production (*i. e.* immunostimulatory HIDE1 antibodies).

### II. HIDE1 Mechanism of Action

Accordingly, as discussed herein, HIDE1 is an immune checkpoint protein, sometimes referred to as "an immuno-oncology protein". As has been shown for PD-1 and CTLA-4, among others, immune checkpoint proteins can be exploited in several ways, to either immunopotentiate the immune system to increase immune activity, such as through the activation of T cells for treatments of diseases such as cancer and infectious disease, or through immunoinhibition, where immunosuppression is desired, for example in allergic reactions, autoimmune diseases and inflammation. HIDE1 as shown herein exhibits negative signaling on the immune system, by suppressing T cell activation and other pathways as outlined herein. Thus, by reducing the activity of HIDE1, for example by inhibiting its binding ability to its ligand (*i.e.,* inhibiting binding to its binding partner or signaling partner), the suppression is decreased and the immune system can be activated or stimulated to treat cancer, for example. Conversely, by increasing the activity of HIDE1 ("stimulating" the activity with a "stimulator" and/or with an enhancer of HIDE1 associated immune suppression), for example by adding recombinant HIDE1 ECD that mimics HIDE1 immune suppressive activitiy through binding to HIDE1 counterpart (a "stimulator of HIDE1" or an enhancer of HIDE1 associated immune suppression; *i.e.,* HIDE1 binding and/or signaling partner) to a host in the form of a soluble ECD (and optionally a fusion protein), the suppression is increased and the immune system is suppressed, allowing for treatment of diseases associated with increased immune function such as autoimmune diseases and others outlined herein. As shown in the Example section, HIDE1 tetramers have been shown to bind to activated T cells and TILs, activation status of T cells has been manifested by increased PD-1 and CD137 expression. Additionally, introduction of HIDE1 (for example, as an ECD Fc-fusion protein) was shown to inhibit the activation of T cells, as shown in the Examples. Accordingly, anti-HIDE1 antibodies can be used to treat conditions for which T cell or NK cell activation is desired such as cancer.

Accordingly, in some embodiments the present invention is directed to compounds that suppress the signaling pathway triggered by the binding interaction of HIDE1 and its binding and/or signaling partner (leading to increased T cell and NK cell activation, among other things, leading to treatment of cancer).

Thus, specific mechanisms of action are provided for the immunostimulatory actions of anti-HIDE1 antibodies, that are useful for increasing immune function for the treatment of cancer. These include, but are not limited to, (i) increases immune response, (ii) increases T cell activity, (iii) increases activation of αβ and/or γδ T cells, (iv) increases cytotoxic T cell activity, (v) increases NK and/or NKT cell activity, (vi) alleviates αβ and/or γδ T-cell suppression, (vii) increases pro-inflammatory cytokine secretion, (viii) increases IL-2 secretion; (ix) increases interferon-y production, (x) increases Th1 response, (xi) decrease Th2 response, (xii) decreases or eliminates cell number and/or activity of at least one of regulatory T cells (Tregs), myeloid derived suppressor cells (MDSCs), iMCs, mesenchymal stromal cells, TIE2-expressing monocytes, (xiii) reduces regulatory cell activity, and/or the activity of one or more of myeloid derived suppressor cells (MDSCs), iMCs, mesenchymal stromal cells, TIE2-expressing monocytes, (xiv) decreases or eliminates M2 macrophages, (xv) reduces M2 macrophage pro-tumorigenic activity, (xvi) decreases or eliminates N2 neutrophils, (xvii) reduces N2 neutrophils pro-tumorigenic activity, (xviii) reduces inhibition of T cell activation, (xix) reduces inhibition of CTL activation, (xx) reduces inhibition of NK and/or NKT cell activation, (xxi) reverses αβ and/or γδ T cell exhaustion, (xxii) increases αβ and/or γδ T cell response, (xxiii) increases activity of cytotoxic cells, (xxiv) stimulates antigen-specific memory responses, (xxv) elicits apoptosis or lysis of cancer cells, (xxvi) stimulates cytotoxic or cytostatic effect on cancer cells, (xxvii) induces direct killing of cancer cells, (xxviii) increases Th17 activity and/or (xxix) modulating myeloid cell polarization, (xxx) modulating myeloid cell shifting toward a pro-inflammatory response, (xxxi) shifting myeloid from M2 toward M1 phenotype, (xxxii) modulating myeloid cell in the TME to support anti-cancer immune response, (xxxiii) restricting the pro-tumorigenic effects of the myeloid cells in the TME, (xxxiv) enhancing myeloid and lymphoid infiltration into the tumor cite thereby shifting the tumor into more immunogenic, (xxxv) induces complement dependent cytotoxicity and/or antibody dependent cell-mediated cytotoxicity.

Functional effects of HIDE1 blocking antibodies on myeloid cells including but not limited to monocytes, dendritic cells, macrophages, M1/M2 tumor associated macrophages, neutrophils, Myeloid-derive suppressor cells (MDSC), and/or on NK and T-cells can be assessed *in vitro* (and in some cases *in vivo,* as described more fully below) by measuring changes in the following parameters: proliferation, cytokine release and cell-surface makers. For NK cells, increases in cell proliferation, cytotoxicity (ability to kill target cells as measured by increases in CD107a, granzyme, and perforin expression, or by directly measuring target cells killing), cytokine production (*e.g.* IFN-γ and TNF), and cell surface receptor expression (*e.g.* CD25) is indicative of immune modulation, *e.g.* enhanced killing of cancer cells. For T-cells, increases in proliferation, increases in expression of cell surface markers of activation (*e.g.* CD25, CD69, CD137, and PD1), cytotoxicity (ability to kill target cells), and cytokine production (*e.g.* IL-2, IL-4, IL-6, IFNγ, TNF-a, IL-10, IL-17A) are indicative of immune modulation, e.g. enhanced killing of cancer cells. For monocytes, increases in monocyte differentiation, M1-M2 skuwing and vise versa detected by specific differentiating markers (M1- iNOS, IL-12p35, TNFa, IL-1b; M2 - IL-10, OH-1, CCL17, CCL22, Msr2, MRC1); Monocyte activation detected by cell surface markers of activation (e.g MHC, CD80/86, PDL1) and cytokine and chemokine secretion (CCL19/21). The afforimentioned effects could be examined upon differentiating primary myeloid cells as well as cellslines towards macrophage-like cells using M1/M2 polarazing conditions. Indirect suppressive effect of HYDE1-expressing myeloid cells on effector immune cells, including but not limited to CD4+ T cells, CD8+ T cells, NK cells, NKT cells, could be reversed with HIDE1-blocking Ab upon poly-clonal or antigen-specific activation of immune effector cells in the presence of HIDE1- expressing myeloid cells.

Accordingly, in some embodiments the present invention provides antibodies, including antigen binding domains, that bind to human HIDE1 and activate myeloid cells including but not limited to monocytes, dendritic cells, macrophages, M1/M2 tumor associated macrophages, neutrophils, Myeloid-derive suppressor cells (MDSC), and/or on, T cells and/or NK cells to treat cancer . According to at least some embodiments of the present invention also provides antibodies, including antigen binding domains, that bind to human HIDE1 for use in methods of depleting myeloid cells, or other circulating tumor cells, in order to treat cancer.

Therefore, in one embodiment the present invention broadly relates to the development of novel immunomodulatory proteins that antagonize or block the effects of HIDE1 on immunity and particularly the effects of HIDE1, on specific types of immune cells and cytokine production (*i*. *e.* immunostimulatory HIDE1 antibodies).

### III. HIDE1 Proteins

As used herein, the term "HIDE1" or "HIDE1 protein" or "HIDE1 polypeptide" may optionally include any such protein, or variants, conjugates, or fragments thereof, including but not limited to known or wild type HIDE1, as described herein, including but not limited to SEQ ID NO:1, and also other HIDE1 variants, including but not limited to SEQ ID NOs: 7-11, as well as any soluble HIDE1 protein, including but not limited to any of SEQ ID NOs: 2,3,6,19-47, and/or variants thereof possessing at least 80% sequence identity, more preferably at least 90% sequence identity therewith and even more preferably at least 95, 96, 97, 98 or 99% sequence identity therewith, and/or fusions and or conjugates thereof, and/or polynucleotides encoding same. As used herein, the term "HIDE1" or "HIDE1 protein" or "HIDE1 polypeptide" may optionally include any such protein, or variants, conjugates, or fragments thereof, including but not limited to non-human HIDE1 orthologs, such as for example, mouse HIDE1 protein as set forth in SEQ ID NO: 12, and/or its corresponding exracellular domain, as set forth in any of SEQ ID NOs: 13-16.

The HIDE 1 sequence, as described herein under SEQ ID NO:1, is copied below.

MPWTILLFAAGSLAIPAPSIRLVPPYPSSQEDPIHIACMAPGNFPGANFTLYRGGQVVQLLQAPTDQRGVTFNLS
GGSSKAPGGPFHCQYGVLGELNQSQLSDLSEPVNVSFPVPTWILVLSLSLAGALFLLAGLVAVALVVRKVKLRNL
QKKRDRESCWAQINFDSTDMSFDNSLFTVSAKTMPEEDPATLDDHSGTTATPSNSRTRKRPTSTSSSPETPEFST
FRACQ

The HIDE1 ECD sequence, as described herein under SEQ ID NO:2, copied below.

IPAPSIRLVPPYPSSQEDPIHIACMAPGNFPGANFTLYRGGQVVQLLQAPTDQRGVTFNLSGGSSKAPGGPFHCQ
YGVLGELNQSQLSDLSEPVNVSFPVPTWIL

The HIDE1 ECD sequence, as described herein under SEQ ID NO:3, copied below.

IPAPSIRLVPPYPSSQEDPIHIACMAPGNFPGANFTLYRGGQVVQLLQAPTDQRGVTFNLSGGSSKAPGGPFHCQ
YGVLGELNQSQLSDLSEPVNVSFPVPTW

The term "soluble" form of HIDE1 is also used interchangeably with the terms "soluble ectodomain (ECD)" or "ectodomain" or "fragments of HIDE1 polypeptides" or "extracellular domain" , and which may refer broadly to one or more of the following optional polypeptides:

Optionally, the HIDE1 ECD proteins and fragments thereof refer to any one of the polypeptide sequences listed in any of SEQ ID NOs: 2-6, 21-47, and/or variants thereof possessing at least 80% sequence identity, more preferably at least 90% sequence identity therewith and even more preferably at least 95, 96, 97, 98 or 99% sequence identity therewith, and/or mouse ortholog thereof listed in any one of SEQ ID NOs: 13-16, and/or fusions and or conjugates thereof, and/or polynucleotides encoding same.

Optionally, the fragment is of at least about 95 and so forth amino acids of the extracellular domain of HIDE1 protein, set forth in SEQ ID NO: 2, up to 115 amino acids of the HIDE1 protein extracellular domain, optionally including any integral value between 95 and 115 amino acids in length. Preferably, the fragment is of at least about 101 and up to 109 amino acids of the HIDE1 protein extracellular domain, optionally including any integral value between 101 and 109 amino acids in length. Also preferably the fragment is of at least about 103 up to 105 amino acids of the HIDE1 protein extracellular domain, optionally including any integral value between 103 and 105 amino acids in length. More preferably, the fragment is about 105 amino acids. The HIDE1 fragment protein according to at least some embodiments of the present invention may or may not include a signal peptide sequence, and may or may not include 1, 2, 3, 4, or 5 contiguous amino acids from the HIDE1 transmembrane domain.

In particular, the fragments of the extracellular domain of HIDE1 can include any sequence corresponding to any portion of or comprising the Ig domain of the extracellular domain of HIDE1, having any sequence corresponding to residues of HIDE1 (SEQ ID NO:1) starting from any position between 17 and 21 and ending at any position between 109 and 113.

Without wishing to be limited by a single hypothesis, the HIDE1 proteins contain an immunoglobulin domain within the extracellular domain. The Ig domain may optionally be responsible for receptor binding, by analogy to the other B7 family members. The Ig domain of the extracellular domain includes one disulfide bond formed between intra domain cysteine residues, as is typical for this fold and may optionally be important for structure-function. In SEQ ID NO: 2 these cysteines are located at residues 19 and 69.

A HIDE1 polypeptide that is a fragment of full-length HIDE1 typically has at least 20 percent, 30 percent, 40 percent, 50 percent, 60 percent, 70 percent, 80 percent, 90 percent, 95 percent, 98 percent, 99 percent, 100 percent, or even more than 100 percent of the ability to bind its natural molecular partner(s) and/or of the modulation (preferably enhancing and/or agonizing of one or more of the functional effects of HTDK1 on immunity and on specific immune cells as compared to full-length HIDE1; optionally such a percentage may be any integral value between 20 and 100 percent.

Soluble HIDE1 polypeptide fragments are fragments of HIDE1 polypeptides that may optionally be shed, secreted or otherwise extracted from the producing cells. In other aspects, the soluble fragments of HIDE1 polypeptides include fragments of the HIDE1 extracellular domain that retain HIDE1 biological activity, such as fragments that retain the ability to bind to their natural receptor or receptors and/or which modulate T or NK cell activation. The extracellular domain can include 1, 2, 3, 4, or 5 contiguous amino acids from the transmembrane domain, and/or 1, 2, 3, 4, or 5 contiguous amino acids from the signal sequence. Alternatively, the extracellular domain can have 1, 2, 3, 4, 5 or more amino acids removed from the C-terminus, N-terminus, or both.

In some aspects the HIDE1 extracellular domain polypeptide comprises the amino acid sequence of the Ig domain as set forth in any one of SEQ ID NO: 6, 24-47, or fragments or variants thereof. In other aspects the HIDE1 extracellular domain polypeptide consists essentially of the amino acid sequence of the Ig domain as set forth in any one of SEQ ID NOs: 6, 24-47.

Generally, the SEQ ID NOs:6, 24-47polypeptide fragments are expressed from nucleic acids that include sequences that encode a signal sequence. The signal sequence is generally cleaved from the immature polypeptide to produce the mature polypeptide lacking the signal sequence. The signal sequence of HIDE1 can be replaced by the signal sequence of another polypeptide using standard molecule biology techniques to affect the expression levels, secretion, solubility, or other property of the polypeptide. The signal peptide sequence that is used to replace the HIDE1 signal peptide sequence can be any known in the art.

In one aspect such "soluble ectodomain (ECD)" or "ectodomain" or "soluble" form of HIDE1 will modulate (preferably enhance and/or agonize) one or more of HIDE1's effects on immunity and specific types of immune cells such as T helper, cytotoxic or effector T cells, Tregs NK cells and antigen presenting cells.

Optionally, the HIDE1 ECD fragments refer also to any one of the polypeptide sequences listed in any of SEQ ID NOs: 19-20, which are reasonably expected to comprise functional regions of the HIDE1 protein. This expectation is based on a systematic analysis of a set of protein complexes with solved 3D structures, which contained complexes of Ig proteins (for example PDB ID 1i85 which describe the complex of CTLA4 and CD86). The intermolecular contact residues from each co-structure were collected and projected on the sequence of HIDE1. Several regions with clusters of interacting residues supported by several contact maps were identified and are reasonably expected to mimic the structure of the intact full length protein and thereby modulate one or more of the effects of HIDE1 on immunity and on specific immune cell types.The HIDE1 extracellular domain polypeptides according to at least some embodiments of the present invention are expected to be useful for treatment of autoimmune diseases which have proved resistant to other drug treatments, because, without wishing to be limited by a single hypothesis, the mode of action of these HIDE1 extracellular domain polypeptides is expected to enable them to overcome such resistance.

### A. ADDITION OF GROUPS

If a protein according to some embodiments of the present invention is a linear molecule, it is possible to place various functional groups at various points on the linear molecule which are susceptible to or suitable for chemical modification. Functional groups can be added to the termini of linear forms of the protein according to at least some embodiments of the present invention. In some embodiments, the functional groups improve the activity of the protein with regard to one or more characteristics, including but not limited to, improvement in stability, penetration (through cellular membranes and/or tissue barriers), tissue localization, efficacy, decreased clearance, decreased toxicity, improved selectivity, improved resistance to expulsion by cellular pumps, and the like. For convenience sake and without wishing to be limiting, the free N-terminus of one of the sequences contained in the compositions according to at least some embodiments of the present invention will be termed as the N-terminus of the composition, and the free C-terminal of the sequence will be considered as the C-terminus of the composition. Either the C-terminus or the N-terminus of the sequences, or both, can be linked to a carboxylic acid functional groups or an amine functional group, respectively.

Non-limiting examples of suitable functional groups are described in Green and Wuts, "Protecting Groups in Organic Synthesis", John Wiley and Sons, Chapters 5 and 7, (1991). Preferred protecting groups are those that facilitate transport of the active ingredient attached thereto into a cell, for example, by reducing the hydrophilicity and increasing the lipophilicity of the active ingredient, these being an example for "a moiety for transport across cellular membranes".

These moieties can optionally and preferably be cleaved *in vivo,* either by hydrolysis or enzymatically, inside the cell. (Ditter et al., J. Pharm Sci. 57:783 (1968); Ditter et al., J. Pharm. Sci. 57:828 (1968); Ditter et al., J. Pharm. Sci. 58:557 (1969); King et al., Biochemistry 26:2294 (1987); Lindberg et al., Drug Metabolism and Disposition 17:311 (1989); and Tunek et al., Biochem. Pharm. 37:3867 (1988), Anderson et al., Arch. Biochem. Biophys. 239:538 (1985) and Singhal et al., FASEB J. 1:220 (1987)). Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups. In one embodiment, the carboxylic acid group in the side chain of one or more glutamic acid or aspartic acid residue in a composition accorind to some embodiments of the present invention is protected, preferably with a methyl, ethyl, benzyl or substituted benzyl ester, more preferably as a benzyl ester.

Non-limiting, illustrative examples of N-terminal protecting groups include acyl groups (-CO-R1) and alkoxy carbonyl or aryloxy carbonyl groups (-CO-O-R1), wherein R1 is an aliphatic, substituted aliphatic, benzyl, substituted benzyl, aromatic or a substituted aromatic group. Specific examples of acyl groups include but are not limited to acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carbonyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-CO-, phenyl-O- CO-, substituted phenyl-O-CO- and benzyl-O-CO-, (substituted benzyl)- O-CO-, Adamantan, naphtalen, myristoleyl, toluen, biphenyl, cinnamoyl, nitrobenzoy, toluoyl, furoyl, benzoyl, cyclohexane, norbornane, or Z-caproic. In order to facilitate the N-acylation, one to four glycine residues can be present in the N-terminus of the molecule.

B. The carboxyl group at the C-terminus of the compound can be protected, for example, by a group including but not limited to an amide (i. e., the hydroxyl group at the C-terminus is replaced with -NH₂, -NHR₂ and -NR₂R₃) or ester (i. e., the hydroxyl group at the C-terminus is replaced with -OR₂). R₂ and R₃ are optionally independently an aliphatic, substituted aliphatic, benzyl, substituted benzyl, aryl or a substituted aryl group. In addition, taken together with the nitrogen atom, R₂ and R₃ can optionally form a C4 to C8 heterocyclic ring with from about 0-2 additional heteroatoms such as nitrogen, oxygen or sulfur. Non-limiting suitable examples of suitable heterocyclic rings include piperidinyl, pyrrolidinyl, morpholino, thiomorpholino or piperazinyl. Examples of C-terminal protecting groups include but are not limited to -NH₂, -NHCH₃, -N(CH₃)₂, -NH(ethyl), -N(ethyl)₂, -N(methyl) (ethyl), -NH(benzyl), -N(C1-C4 alkyl)(benzyl), -NH(phenyl), -N(C1-C4 alkyl) (phenyl), -OCH₃,-O-(ethyl), -O-(n-propyl), -O-(n-butyl), -O-(iso-propyl), -O-(sec- butyl), -O-(t-butyl), - O-benzyl and -O-phenyl. **SUBSTITUTION BY PEPTIDOMIMETIC MOIETIES**

A "peptidomimetic organic moiety" can optionally be substituted for amino acid residues in the composition of this invention both as conservative and as nonconservative substitutions. These moieties are also termed "non-natural amino acids" and may optionally replace amino acid residues, amino acids or act as spacer groups within the peptides in lieu of deleted amino acids. The peptidomimetic organic moieties optionally and preferably have steric, electronic or configurational properties similar to the replaced amino acid and such peptidomimetics are used to replace amino acids in the essential positions, and are considered conservative substitutions. However such similarities are not necessarily required. According to some embodiments of the present invention, one or more peptidomimetics are selected such that the composition at least substantially retains its physiological activity as compared to the native protein according to some embodiments of the present invention.

Peptidomimetics may optionally be used to inhibit degradation of the peptides by enzymatic or other degradative processes. The peptidomimetics can optionally and preferably be produced by organic synthetic techniques. Non-limiting examples of suitable peptidomimetics include D amino acids of the corresponding L amino acids, tetrazol (Zabrocki et al., J. Am. Chem. Soc. 110:5875-5880 (1988)); isosteres of amide bonds (Jones et al., Tetrahedron Lett. 29: 3853-3856 (1988)); LL-3-amino-2-propenidone-6-carboxylic acid (LL-Acp) (Kemp et al., J. Org. Chem. 50:5834-5838 (1985)). Similar analogs are shown in Kemp et al., Tetrahedron Lett. 29:5081-5082 (1988) as well as Kemp et al., Tetrahedron Lett. 29:5057-5060 (1988), Kemp et al., Tetrahedron Lett. 29:4935-4938 (1988) and Kemp et al., J. Org. Chem. 54:109-115 (1987). Other suitable but exemplary peptidomimetics are shown in Nagai and Sato, Tetrahedron Lett. 26:647-650 (1985); Di Maio et al., J. Chem. Soc. Perkin Trans., 1687 (1985); Kahn et al., Tetrahedron Lett. 30:2317 (1989); Olson et al., J. Am. Chem. Soc. 112:323-333 (1990); and Garvey et al., J. Org. Chem. 56:436 (1990). Further suitable exemplary peptidomimetics include hydroxy- 1,2,3,4-tetrahydroisoquinoline- 3-carboxylate (Miyake et al., J. Takeda Res. Labs 43:53-76 (1989)); 1, 2,3,4-tetrahydro- isoquinoline-3-carboxylate (Kazmierski et al., J. Am. Chem. Soc. 133:2275-2283 (1991)); histidine isoquinolone carboxylic acid (HIC) (Zechel et al., Int. J. Pep. Protein Res. 43 (1991)); (2S, S)-methyl-phenylalanine, (2S, 3R)-methyl-phenylalanine, (2R, 3S)-methyl- phenylalanine and (2R, 3R)-methyl-phenylalanine (Kazmierski and Hruby, Tetrahedron Lett. (1991)).

Exemplary, illustrative but non-limiting non-natural amino acids include β-amino acids (β3 and β2), homo-amino acids, cyclic amino acids, aromatic amino acids, Pro and Pyr derivatives, 3-substituted Alanine derivatives, Glycine derivatives, ring-substituted Phe and Tyr Derivatives, linear core amino acids or diamino acids. They are available from a variety of suppliers, such as Sigma-Aldrich (USA).

### C. PROTEIN CHEMICAL MODIFICATIONS

In the present invention, according to at least some embodiments, any part of a protein according to at least some embodiments of the present invention may optionally be chemically modified, *i. e.* changed by addition of functional groups. For example the side amino acid residues appearing in the native sequence may optionally be modified, although as described below alternatively other parts of the protein may optionally be modified, in addition to or in place of the side amino acid residues. The modification may optionally be performed during synthesis of the molecule if a chemical synthetic process is followed, for example by adding a chemically modified amino acid. However, chemical modification of an amino acid when it is already present in the molecule ("*in situ*" modification) is also possible.

The amino acid of any of the sequence regions of the molecule can optionally be modified according to any one of the following exemplary types of modification (in the peptide conceptually viewed as "chemically modified"). Non-limiting exemplary types of modification include carboxymethylation, acylation, phosphorylation, glycosylation or fatty acylation. Ether bonds can optionally be used to join the serine or threonine hydroxyl to the hydroxyl of a sugar. Amide bonds can optionally be used to join the glutamate or aspartate carboxyl groups to an amino group on a sugar (Garg and Jeanloz, Advances in Carbohydrate Chemistry and Biochemistry, Vol. 43, Academic Press (1985); Kunz, Ang. Chem. Int. Ed. English 26:294-308 (1987)). Acetal and ketal bonds can also optionally be formed between amino acids and carbohydrates. Fatty acid acyl derivatives can optionally be made, for example, by acylation of a free amino group *(e.g.,* lysine) (Toth et al., Peptides: Chemistry, Structure and Biology, Rivier and Marshal, eds., ESCOM Publ., Leiden, 1078-1079 (1990)).

As used herein the term "chemical modification", when referring to a protein or peptide according to some embodiments of the present invention, refers to a protein or peptide where at least one of its amino acid residues is modified either by natural processes, such as processing or other post-translational modifications, or by chemical modification techniques which are well known in the art. Examples of the numerous known modifications typically include, but are not limited to: acetylation, acylation, amidation, ADP-ribosylation, glycosylation, GPI anchor formation, covalent attachment of a lipid or lipid derivative, methylation, myristoylation, pegylation, prenylation, phosphorylation, ubiquitination, or any similar process.

Other types of modifications optionally include the addition of a cycloalkane moiety to a biological molecule, such as a protein, as described in PCT Application No. WO 2006/050262. These moieties are designed for use with biomolecules and may optionally be used to impart various properties to proteins.

Furthermore, optionally any point on a protein may be modified. For example, pegylation of a glycosylation moiety on a protein may optionally be performed, as described in PCT Application No. WO 2006/050247. One or more polyethylene glycol (PEG) groups may optionally be added to O-linked and/or N-linked glycosylation. The PEG group may optionally be branched or linear. Optionally any type of water-soluble polymer may be attached to a glycosylation site on a protein through a glycosyl linker.

### D. ALTERED GLYCOSYLATION

Proteins according to at least some embodiments of the present invention may optionally be modified to have an altered glycosylation pattern (i. e., altered from the original or native glycosylation pattern). As used herein, "altered" means having one or more carbohydrate moieties deleted, and/or having at least one glycosylation site added to the original protein.

Glycosylation of proteins is typically either N-linked or O-linked. N-linked refers to the attachment of the carbohydrate moiety to the side chain of an asparagine residue. The tripeptide sequences, asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may optionally also be used.

Addition of glycosylation sites to proteins according to at least some embodiments of the present invention is conveniently accomplished by altering the amino acid sequence of the protein such that it contains one or more of the above-described tripeptide sequences (for N-linked glycosylation sites). The alteration may optionally also be made by the addition of, or substitution by, one or more serine or threonine residues in the sequence of the original protein (for O-linked glycosylation sites). The protein's amino acid sequence may optionally also be altered by introducing changes at the DNA level.

Another means of increasing the number of carbohydrate moieties on proteins is by chemical or enzymatic coupling of glycosides to the amino acid residues of the protein. Depending on the coupling mode used, the sugars may optionally be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, CRC Crit. Rev. Biochem., 22: 259-306 (1981).

Removal of any carbohydrate moieties present on proteins according to at least some embodiments of the present invention may optionally be accomplished chemically or enzymatically. Chemical deglycosylation requires exposure of the protein to trifluoromethanesulfonic acid, or an equivalent compound. This treatment results in the cleavage of most or all sugars except the linking sugar (N-acetylglucosamine or N-acetylgalactosamine), leaving the amino acid sequence intact.

Chemical deglycosylation is described by Hakimuddin et al., Arch. Biochem. Biophys., 259: 52 (1987); and Edge et al., Anal. Biochem., 118: 131 (1981). Enzymatic cleavage of carbohydrate moieties on proteins can be achieved by the use of a variety of endo- and exo-glycosidases as described by Thotakura et al., Meth. Enzymol., 138: 350 (1987).

### IV. Particular Constructs

The disclosed HIDE1 fusion proteins optionally contain a peptide or polypeptide linker domain that separates the HIDE1 polypeptide from the second polypeptide (*see,* **Figure 66****).** Various non-limiting examples of such linker domains are described herein. In one aspect, the linker domain contains the hinge region of an immunoglobulin. In a further aspect, the hinge region is derived from a human immunoglobulin. Suitable human immunoglobulins that the hinge can be derived from include IgG, IgD and IgA. In a further aspect, the hinge region is derived from human IgG. Amino acid sequences of immunoglobulin hinge regions and other domains are well known in the art. In one aspect, HIDE1 fusion polypeptides contain the hinge, C_{H2} and C_{H3} regions of a human immunoglobulin Cγ1 chain, optionally with the Cys at position 220 (according to full length human IgG1, position 5 in SEQ ID NO:48) replaced with a Ser having at least 85%, 90%, 95%, 99% or 100% sequence homology to amino acid sequence set forth in SEQ ID NO:49:

SEQ ID NO:48

EPKSCDKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQKS
LSLSPGK

SEQ ID NO:49
EPKSCDKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQKS
LSLSPGK

SEQ ID NO:50
EPKSSDKTHTCPPCPAPELLGGPSVFLFPPKPKDTLMISRTPEVTCVVVDVSHEDPEVKFNWYVDGVEVHNAKTK
PREEQYASTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQKS
LSLSPGK

The hinge can be further shortened to remove amino acids 1, 2, 3, 4, 5, or combinations thereof of any one of SEQ ID NOs: 48-50. In one aspect, amino acids 1-5 of any one of SEQ ID NOs:48-50 are deleted. Exemplary HIDE1 fusion polypeptides comprised of the hinge, CH2 and CH3 regions of a human immunoglobulin Cγ1 chain with the Cys at position 220 replaced with a Ser are set forth in SEQ ID NO: 17.

SEQ ID NO: 17
IPAPSIRLVPPYPSSQEDPIHIACMAPGNFPGANFTLYRGGQVVQLLQAPTDQRGVTFNLSGGSSKAPGGPFHCQ
YGVLGELNQSQLSDLSEPVNVSFPVPTWEPKSSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

In another aspect, the HIDE1 fusion polypeptides contain the CH2 and CH3 regions of human immunoglobulin Cγ1 chain having N297A mutation (SEQ ID NO:50) or the human Fc carrying the C220S, C226 and C229S mutations (SEQ ID NO:86).

In another aspect, HIDE1 fusion polypeptides contain the C_{H2} and C_{H3} regions of a human immunoglobulin Cγ1 chain having at least 85%, 90%, 95%, 99% or 100% sequence homology to amino acid sequence set forth in SEQ ID NO:51:

SEQ ID NO:51

APELLGGPSVFLFPPKPKDTLMISRTPEVTCVVVDVSHEDPEVKFNWYVDGVEVHNAKTKPREEQYNSTYRVVSV

LTVLHQDWLNGKEYKCKVSNKALPAPIEKTISKAKGQPREPQVYTLPPSRDELTKNQVSLTCLVKGFYPSDIAVE

WESNGQPENNYKTTPPVLDSDGSFFLYSKLTVDKSRWQQGNVFSCSVMHEALHNHYTQKSLSLSPGK

In another aspect, the HIDE1 fusion polypeptides contain the hinge, CH2 and CH3 regions of a murine immunoglobulin Cy2a chain at least 85%, 90%, 95%, 99% or 100% sequence homology to amino acid sequence set forth in SEQ ID NO: 52:

SEQ ID NO:52

EPRGPTIKPCPPCKCPAPNLLGGPSVFIFPPKIKDVLMISLSPIVTCVVVDVSEDDPDVQISWFVNNVEVHTAQT

QTHREDYNSTLRVVSALPIQHQDWMSGKEFKCKVNNKDLPAPIERTISKPKGSVRAPQVYVLPPPEEEMTKKQVT

LTCMVTDFMPEDIYVEWTNNGKTELNYKNTEPVLDSDGSYFMYSKLRVEKKNWVERNSYSCSVVHEGLHNHHTTK

SFSRTPGK

In another aspect, the HIDE1 fusion polypeptides contain the CH2 and CH3 regions of a murine immunoglobulin Cy2a chain having N297A mutation (SEQ ID NO:53) or the murine Fc without the Hinge (SEQ ID NO:54).

SEQ ID NO:53

EPRGPTIKPCPPCKCPAPNLLGGPSVFIFPPKIKDVLMISLSPIVTCVVVDVSEDDPDVQISWFVNNVEVHTAQT

QTHREDYASTLRVVSALPIQHQDWMSGKEFKCKVNNKDLPAPIERTISKPKGSVRAPQVYVLPPPEEEMTKKQVT

LTCMVTDFMPEDIYVEWTNNGKTELNYKNTEPVLDSDGSYFMYSKLRVEKKNWVERNSYSCSVVHEGLHNHHTTK

SFSRTPGK

SEQ ID NO:54

APNLLGGPSVFIFPPKIKDVLMISLSPIVTCVVVDVSEDDPDVQISWFVNNVEVHTAQTQTHREDYNSTLRVVSA

LPIQHQDWMSGKEFKCKVNNKDLPAPIERTISKPKGSVRAPQVYVLPPPEEEMTKKQVTLTCMVTDFMPEDIYVE

WTNNGKTELNYKNTEPVLDSDGSYFMYSKLRVEKKNWVERNSYSCSVVHEGLHNHHTTKSFSRTPGK

In another aspect, the linker domain optionally contains a hinge region of an immunoglobulin as described above, and further includes one or more additional immunoglobulin domains.

### A. Peptide or polypeptide linker domain

The disclosed HIDE1 fusion proteins optionally contain a peptide or polypeptide linker domain that separates the HIDE1 polypeptide from the second polypeptide. Various non-limiting examples of such linker domains are described herein. In one aspect, the linker domain contains the hinge region of an immunoglobulin. In a further aspect, the hinge region is derived from a human immunoglobulin. Suitable human immunoglobulins that the hinge can be derived from include IgG, IgD and IgA. In a further aspect, the hinge region is derived from human IgG. Amino acid sequences of immunoglobulin hinge regions and other domains are well known in the art. In one aspect, HIDE1 fusion polypeptides contain the hinge, CH2 and CH3 regions of a human immunoglobulin Cγ1 chain, optionally with the Cys at position 220 (according to full length human IgG1, position 5 in SEQ ID NO:48) replaced with a Ser having at least 85%, 90%, 95%, 99% or 100% sequence homology to amino acid sequence set forth in SEQ ID NO:49:

EPKSCDKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQKS
LSLSPGK

The hinge can be further shortened to remove amino acids 1, 2, 3, 4, 5, or combinations thereof of any one of SEQ ID NOs: 48-50. In one aspect, amino acids 1-5 of any one of SEQ ID NOs: 48-50 are deleted. Exemplary HIDE1 fusion polypeptides comprised of the hinge, CH2 and CH3 regions of a human immunoglobulin Cγ1 chain with the Cys at position 220 replaced with a Ser are set forth in SEQ ID NO: 17.

SEQ ID NO:17
IPAPSIRLVPPYPSSQEDPIHIACMAPGNFPGANFTLYRGGQVVQLLQAPTDQRGVTFNLSGGSSKAPGGPFHCQ
YGVLGELNQSQLSDLSEPVNVSFPVPTWEPKSSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

In another aspect, the HIDE1 fusion polypeptides contain the CH2 and CH3 regions of human immunoglobulin Cγ1 chain having N297A mutation (SEQ ID NO:50) or the human Fc carrying the C220S, C226 and C229S mutations (SEQ ID NO:86).

SEQ ID NO:86

EPKSSDKTHTSPPSPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEALHNHYTQKS
LSLSPGK

In another aspect, HIDE1 fusion polypeptides contain the CH2 and CH3 regions of a human immunoglobulin Cγ1 chain having at least 85%, 90%, 95%, 99% or 100% sequence homology to amino acid sequence set forth in SEQ ID NO:51:

APELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLSPGK

In another aspect, the HIDE1 fusion polypeptides contain the hinge, CH2 and CH3 regions of a murine immunoglobulin Cy2a chain at least 85%, 90%, 95%, 99% or 100% sequence homology to amino acid sequence set forth in SEQ ID NO: 52:

EPRGPTIKPCPPCKCPAPNLLGGPSVFIFPPKIKDVLMISLSPIVTCVVVDVSEDDPDVQISWFVNNVEVHTAQT
QTHREDYNSTLRVVSALPIQHQDWMSGKEFKCKVNNKDLPAPIERTISKPKGSVRAPQVYVLPPPEEEMTKKQVT
LTCMVTDFMPEDIYVEWTNNGKTELNYKNTEPVLDSDGSYFMYSKLRVEKKNWVERNSYSCSVVHEGLHNHHTTK
SFSRTPGK

In another aspect, the HIDE1 fusion polypeptides contain the CH2 and CH3 regions of a murine immunoglobulin Cy2a chain having N297A mutation (SEQ ID NO:53) or the murine Fc without the Hinge (SEQ ID NO:54).

SEQ ID NO:53

EPRGPTIKPCPPCKCPAPNLLGGPSVFIFPPKIKDVLMISLSPIVTCVVVDVSEDDPDVQISWFVNNVEVHTAQT

QTHREDYASTLRVVSALPIQHQDWMSGKEFKCKVNNKDLPAPIERTISKPKGSVRAPQVYVLPPPEEEMTKKQVT

LTCMVTDFMPEDIYVEWTNNGKTELNYKNTEPVLDSDGSYFMYSKLRVEKKNWVERNSYSCSVVHEGLHNHHTTK

SFSRTPGK

SEQ ID NO:54

APNLLGGPSVFIFPPKIKDVLMISLSPIVTCVVVDVSEDDPDVQISWFVNNVEVHTAQTQTHREDYNSTLRVVSA

LPIQHQDWMSGKEFKCKVNNKDLPAPIERTISKPKGSVRAPQVYVLPPPEEEMTKKQVTLTCMVTDFMPEDIYVE

WTNNGKTELNYKNTEPVLDSDGSYFMYSKLRVEKKNWVERNSYSCSVVHEGLHNHHTTKSFSRTPGK

In another aspect, the linker domain optionally contains a hinge region of an immunoglobulin as described above, and further includes one or more additional immunoglobulin domains.

### V. Formulations

The therapeutic compositions used in the practice of the foregoing methods can be formulated into pharmaceutical compositions comprising a carrier suitable for the desired delivery method. Suitable carriers include any material that when combined with the therapeutic composition retains the anti-tumor function of the therapeutic composition and is generally non-reactive with the patient's immune system. Examples include, but are not limited to, any of a number of standard pharmaceutical carriers such as sterile phosphate buffered saline solutions, bacteriostatic water, and the like (see, generally, Remington's Pharmaceutical Sciences 16th Edition, A. Osal., Ed., 1980). Acceptable carriers, excipients, or stabilizers are nontoxic to recipients at the dosages and concentrations employed, and include buffers such as phosphate, citrate, acetate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; sweeteners and other flavoring agents; fillers such as microcrystalline cellulose, lactose, corn and other starches; binding agents; additives; coloring agents; salt-forming counter-ions such as sodium; metal complexes (e.g. Zn-protein complexes); and/or non-ionic surfactants such as TWEEN™, PLURONICS™, or polyethylene glycol (PEG).

A pharmaceutical composition that comprises the antibodies of the invention may be in a water-soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The formulations to be used for *in vivo* administration are preferrably sterile. This is readily accomplished by filtration through sterile filtration membranes or other methods.

Administration of the pharmaceutical composition comprising antibodies of the present invention, preferably in the form of a sterile aqueous solution, may be done in a variety of ways, including, but not limited to subcutaneously and intravenously. Subcutaneous administration may be preferable in some circumstances because the patient may self-administer the pharmaceutical composition. Many protein therapeutics are not sufficiently potent to allow for formulation of a therapeutically effective dose in the maximum acceptable volume for subcutaneous administration. This problem may be addressed in part by the use of protein formulations comprising arginine-HCl, histidine, and polysorbate (see WO 04091658). Fc polypeptides of some embodiments of the present invention may be more amenable to subcutaneous administration due to, for example, increased potency, improved serum half-life, or enhanced solubility.

As is known in the art, protein therapeutics are often delivered by IV infusion or bolus. The antibodies of the present invention may also be delivered using such methods. For example, administration may venious be by intravenous infusion with 0.9% sodium chloride as an infusion vehicle.

In addition, any of a number of delivery systems are known in the art and may be used to administer the Fc variants of the present invention in various embodiments. Examples include, but are not limited to, encapsulation in liposomes, microparticles, microspheres (eg. PLA/PGA microspheres), and the like. Alternatively, an implant of a porous, non-porous, or gelatinous material, including membranes or fibers, may be used. Sustained release systems may comprise a polymeric material or matrix such as polyesters, hydrogels, poly(vinylalcohol), polylactides, copolymers of L-glutamic acid and ethyl-L-gutamate, ethylene-vinyl acetate, lactic acid-glycolic acid copolymers such as the LUPRON DEPOT^{RTM}, and poly-D-(-)-3-hydroxyburyric acid. The antibodies disclosed herein may also be formulated as immunoliposomes. A liposome is a small vesicle comprising various types of lipids, phospholipids and/or surfactant that is useful for delivery of a therapeutic agent to a mammal. Liposomes containing the antibody are prepared by methods known in the art, such as described in Epstein et al., 1985, Proc Natl Acad Sci USA, 82:3688; Hwang et al., 1980, Proc Natl Acad Sci USA, 77:4030; U.S. Pat. No. 4,485,045; U.S. Pat. No. 4,544,545; and PCT WO 97/38731. Liposomes with enhanced circulation time are disclosed in U.S. Pat. No. 5,013,556. The components of the liposome are commonly arranged in a bilayer formation, similar to the lipid arrangement of biological membranes. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. A chemotherapeutic agent or other therapeutically active agent is optionally contained within the liposome (Gabizon et al., 1989, J National Cancer Inst 81:1484).

The antibodies may also be entrapped in microcapsules prepared by methods including but not limited to coacervation techniques, interfacial polymerization (for example using hydroxymethylcellulose or gelatin-microcapsules, or poly-(methylmethacylate) microcapsules), colloidal drug delivery systems (for example, liposomes, albumin microspheres, microemulsions, nano-particles and nanocapsules), and macroemulsions. Such techniques are disclosed in Remington's Pharmaceutical Sciences 16th edition, Osol, A. Ed., 1980. Sustained-release preparations may be prepared. Suitable examples of sustained-release preparations include semipermeable matrices of solid hydrophobic polymer, which matrices are in the form of shaped articles, *e.g*. films, or microcapsules. Examples of sustained-release matrices include polyesters, hydrogels (for example poly(2-hydroxyethyl-methacrylate), or poly(vinylalcohol)), polylactides (U.S. Pat. No. 3,773,919), copolymers of L-glutamic acid and gamma ethyl-L-glutamate, non-degradable ethylene-vinyl acetate, degradable lactic acid-glycolic acid copolymers such as the LUPRON DEPOT.RTM. (which are injectable microspheres composed of lactic acid-glycolic acid copolymer and leuprolide acetate), poly-D-(-)-3-hydroxybutyric acid, and ProLease^{RTM} (commercially available from Alkermes), which is a microsphere-based delivery system composed of the desired bioactive molecule incorporated into a matrix of poly-DL-lactide-co-glycolide (PLG).

The dosing amounts and frequencies of administration are, in a preferred embodiment, selected to be therapeutically or prophylactically effective. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art.

The concentration of the antibody in the formulation may vary from about 0.1 to 100 weight %. In a some embodiments, the concentration of the Fc variant is in the range of 0.003 to 1.0 molar. In order to treat a patient, a therapeutically effective dose of the Fc variant of the present invention may be administered. By "therapeutically effective dose" herein is meant a dose that produces the effects for which it is administered. The exact dose will depend on the purpose of the treatment, and will be ascertainable by one skilled in the art using known techniques. Dosages may range from 0.0001 to 100 mg/kg of body weight or greater, for example 0.1, 1, 10, or 50 mg/kg of body weight, with 1 to 10 mg/kg being preferred.

A composition is said to be a "pharmaceutically acceptable carrier" if its administration can be tolerated by a recipient patient. As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion).

Such compositions include sterile water, buffered saline (e.g., Tris-HCl, acetate, phosphate), pH and ionic strength and optionally additives such as detergents and solubilizing agents (e.g., Polysorbate 20®, Polysorbate 80®), antioxidants (e. g, ascorbic acid, sodium metabisulfite), preservatives (e. g, Thimersol®, benzyl alcohol) and bulking substances (e.g., lactose, mannitol). Non-aqueous solvents or vehicles may optionally also be used as detailed below.

Examples of suitable aqueous and nonaqueous carriers that may optionally be employed in the pharmaceutical compositions according to at least some embodiments of the present invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. Depending on the route of administration, the active compound, i. e., soluble polypeptide, fusion protein or conjugate containing the ectodomain of the HIDE1 antigen, may optionally be coated in a material to protect the compound from the action of acids and other natural conditions that may optionally inactivate the compound. The pharmaceutical compounds according to at least some embodiments of the present invention may optionally include one or more pharmaceutically acceptable salts. A "pharmaceutically acceptable salt" refers to a salt that retains the desired biological activity of the parent compound and does not impart any undesired toxicological effects (see *e.g*., Berge, S. M., et al. J. Pharm. Sci. 66: 1-19. (1977)). Examples of such salts include acid addition salts and base addition salts. Acid addition salts include those derived from nontoxic inorganic acids, such as hydrochloric, nitric, phosphoric, sulfuric, hydrobromic, hydriodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition also may optionally include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, α-tocopherol, and the like; and (3) metal chelating agents, such as citric acid, ethylenediamine tetraacetic acid (EDTA), sorbitol, tartaric acid, phosphoric acid, and the like.

These compositions may optionally also contain adjuvants such as preservatives, wetting agents, emulsifying agents and dispersing agents. Prevention of presence of microorganisms may optionally be ensured both by sterilization procedures, *supra*, and by the inclusion of various antibacterial and antifungal agents, for example, paraben, chlorobutanol, phenol sorbic acid, and the like. It may optionally also be desirable to include isotonic agents, such as sugars, sodium chloride, and the like into the compositions. In addition, prolonged absorption of the injectable pharmaceutical form may optionally be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions according to at least some embodiments of the present invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin. Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0. 01 per cent to about ninety-nine percent of active ingredient, preferably from about 0. 1 per cent to about 70 per cent, most preferably from about I per cent to about 30 per cent of active ingredient in combination with a pharmaceutically acceptable carrier.

Dosage regimens are adjusted to provide the optimum desired response (e.g., a therapeutic response). For example, a single bolus may optionally be administered, several divided doses may optionally be administered over time or the dose may optionally be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms according to at least some embodiments of the present invention are dictated by and directly dependent on (a) the unique characteristics of the active compound and the particular therapeutic effect to be achieved, and (b) the limitations inherent in the art of compounding such an active compound for the treatment of sensitivity in individuals.

Alternatively, therapeutic agent can be administered as a sustained release formulation, in which case less frequent administration is required. Dosage and frequency vary depending on the half-life of the therapeutic agent in the patient. The half-life for fusion proteins may vary widely. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

The actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. In some aspects, the selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A composition can be administered via one or more routes of administration using one or more of a variety of methods known in the art. As will be appreciated by the skilled artisan, the route and/or mode of administration will vary depending upon the desired results. Routes of administration for therapeutic agents according to at least some embodiments of the present invention include intravascular delivery (e.g., injection or infusion), intravenous, intramuscular, intradermal, intraperitoneal, subcutaneous, spinal, oral, enteral, rectal, pulmonary (e.g., inhalation), nasal, topical (including transdermal, buccal and sublingual), intravesical, intravitreal, intraperitoneal, vaginal, brain delivery (*e.g*. intra-cerebroventricular, intra-cerebral, and convection enhanced diffusion), CNS delivery (e.g., intrathecal, perispinal, and intra-spinal) or parenteral (including subcutaneous, intramuscular, intravenous and intradermal), transmucosal (e.g., sublingual administration), administration or administration via an implant, or other parenteral routes of administration, for example by injection or infusion, or other delivery routes and/or forms of administration known in the art. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In a specific embodiment, a protein, a therapeutic agent or a pharmaceutical composition according to at least some embodiments of the present invention can be administered intraperitoneally or intravenously. Alternatively, a HIDE1 therapeutic agent can be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g*., Sustained and Controlled Release Drug Delivery Systems, J. R. Robinson, ed., Marcel Dekker, Inc., New York, 1978.

Therapeutic compositions can be administered with medical devices known in the art. For example, in a preferred embodiment, a therapeutic composition according to at least some embodiments of the present invention can be administered with a needles hypodermic injection device, such as the devices disclosed in U. S. Pat. Nos. 5,399,163; 5,383,851; 5,312,335; 5,064,413; 4,941,880; 4,790,824; or 4,596,556. Examples of well-known implants and modules useful in some embodiments of the present invention include: U. S. Pat. No. 4,487,603, which discloses an implantable micro-infusion pump for dispensing medication at a controlled rate; U. S. Pat. No. 4,486,194, which discloses a therapeutic device for administering medicaments through the skin; U. S. Pat. No. 4,447,233, which discloses a medication infusion pump for delivering medication at a precise infusion rate; U. S. Pat. No. 4,447,224, which discloses a variable flow implantable infusion apparatus for continuous drug delivery; U. S. Pat. No. 4,439,196, which discloses an osmotic drug delivery system having multi-chamber compartments; and U. S. Pat. No. 4,475,196, which discloses an osmotic drug delivery system. Many other such implants, delivery systems, and modules are known to those skilled in the art.

### VI. Antibodies

Accordingly, the invention provides anti-HIDE1 antibodies for use in treating cancer in a patient, wherein the antibodies stimulate the immune system by inhibiting the action of HIDE1. The antibodies of the invention are specific for the HIDE1 extracellular domain as more fully outlined herein.

As is discussed below, the term "antibody" is used generally. Antibodies that find use in some embodiments of the present invention can take on a number of formats as described herein, including traditional antibodies as well as antibody derivatives, fragments and mimetics, described below. In general, the term "antibody" includes any polypeptide that includes at least one antigen binding domain, as more fully described below. Antibodies may be polyclonal, monoclonal, xenogeneic, allogeneic, syngeneic, or modified forms thereof, as described herein, with monoclonal antibodies finding particular use in many embodiments. In some embodiments, antibodies of the invention bind specifically or substantially specifically to HIDE1 molecules. The terms "monoclonal antibodies" and "monoclonal antibody composition", as used herein, refer to a population of antibody molecules that contain only one species of an antigen-binding site capable of immunoreacting with a particular epitope of an antigen, whereas the term "polyclonal antibodies" and "polyclonal antibody composition" refer to a population of antibody molecules that contain multiple species of antigen-binding sites capable of interacting with a particular antigen. A monoclonal antibody composition, typically displays a single binding affinity for a particular antigen with which it immunoreacts.

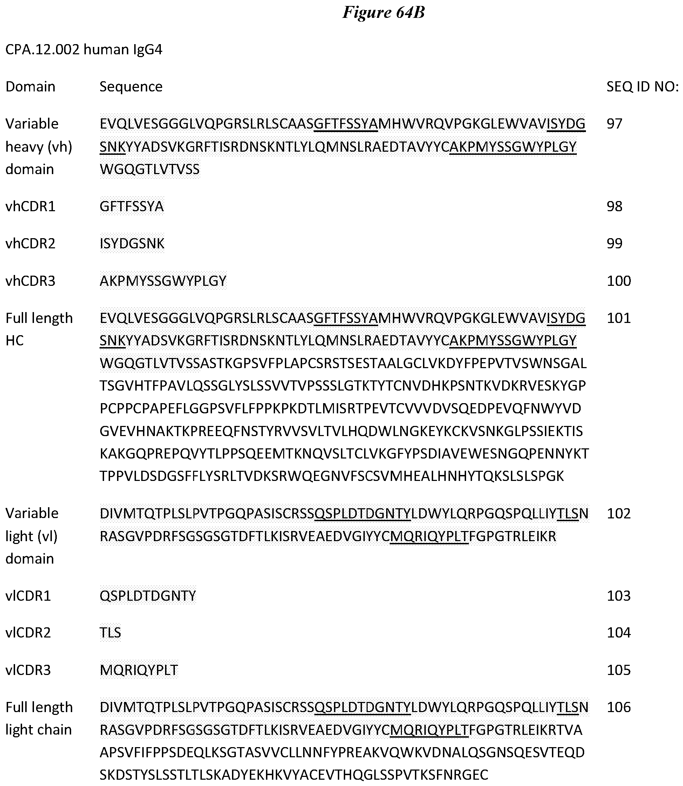

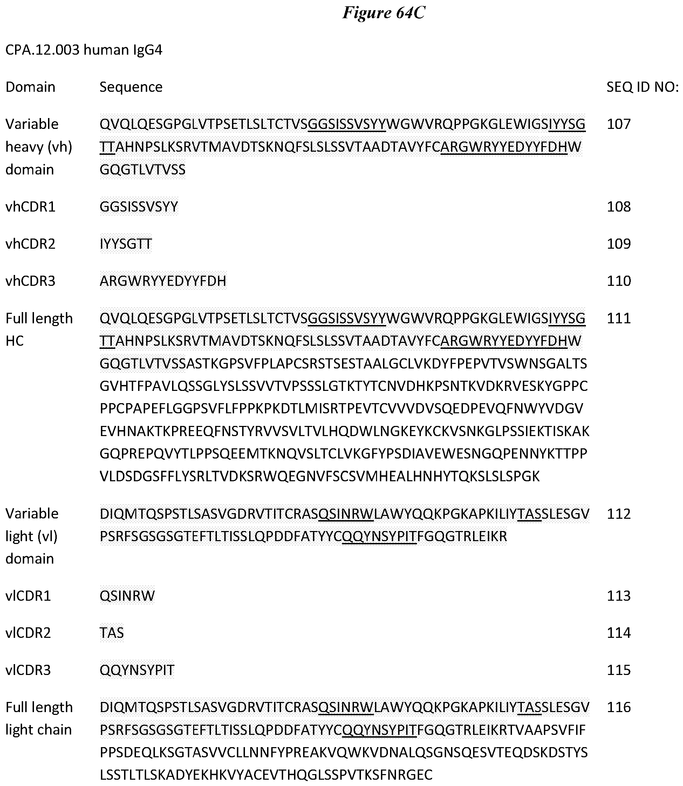

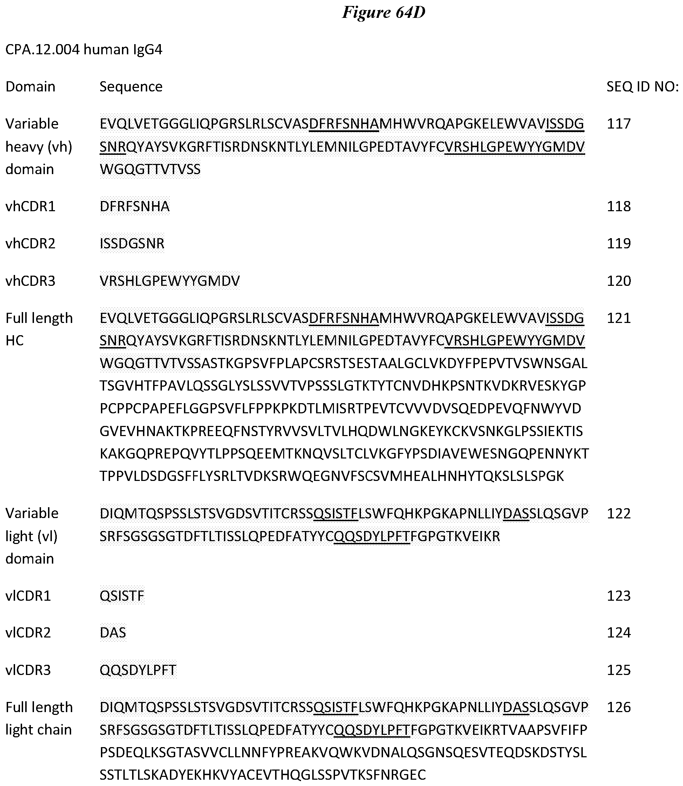

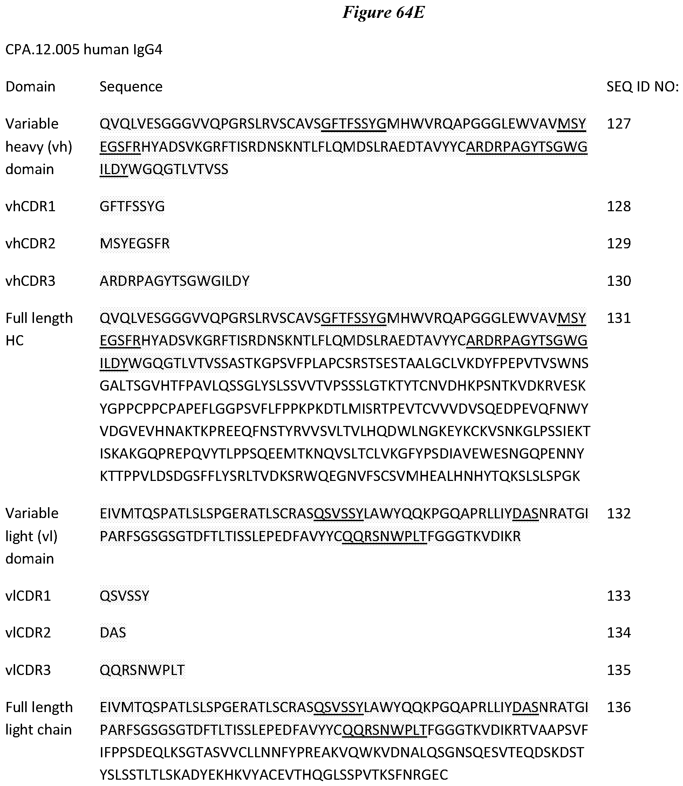

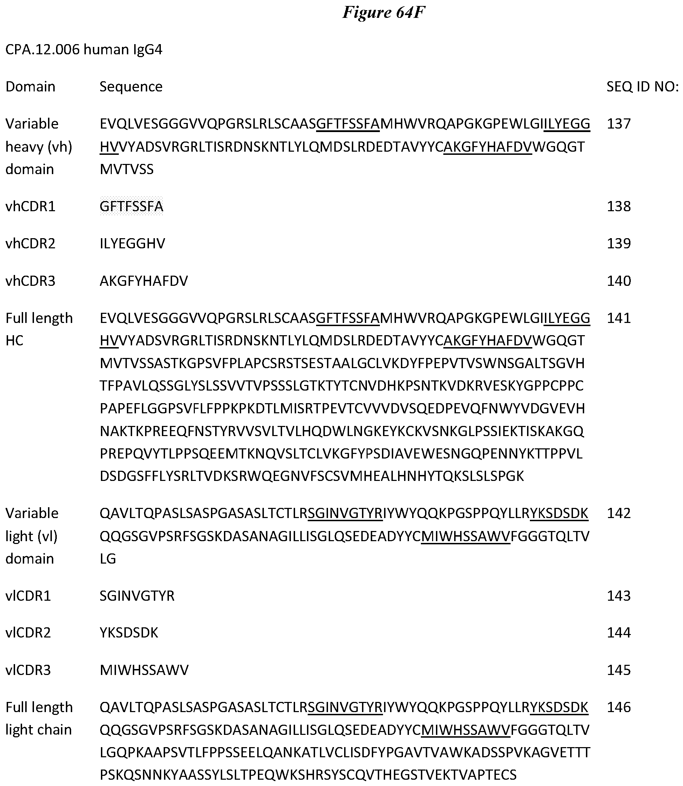

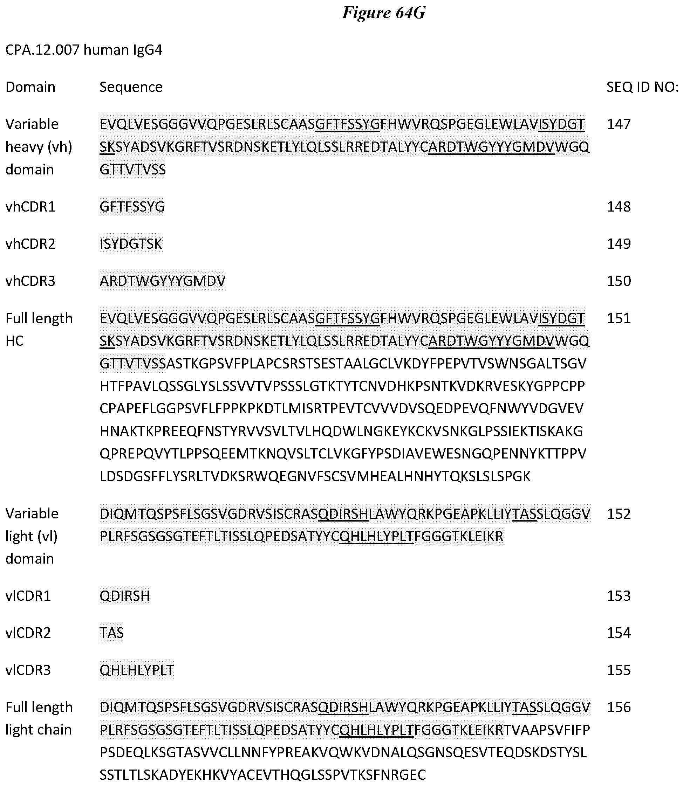

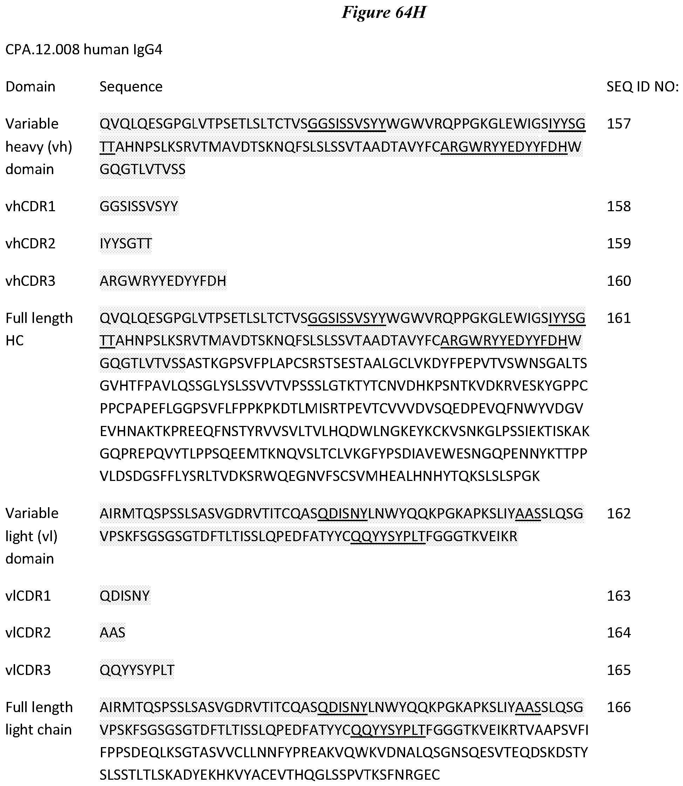

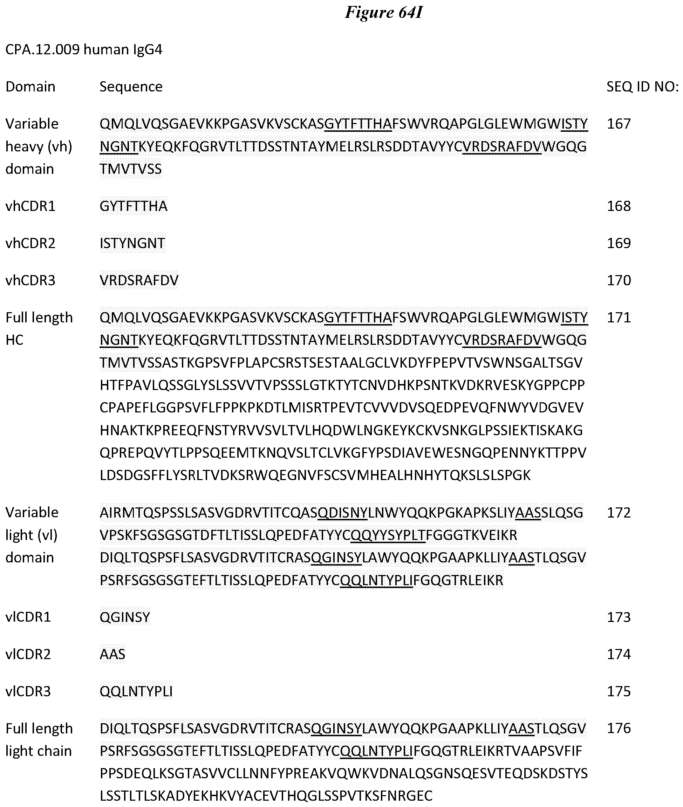

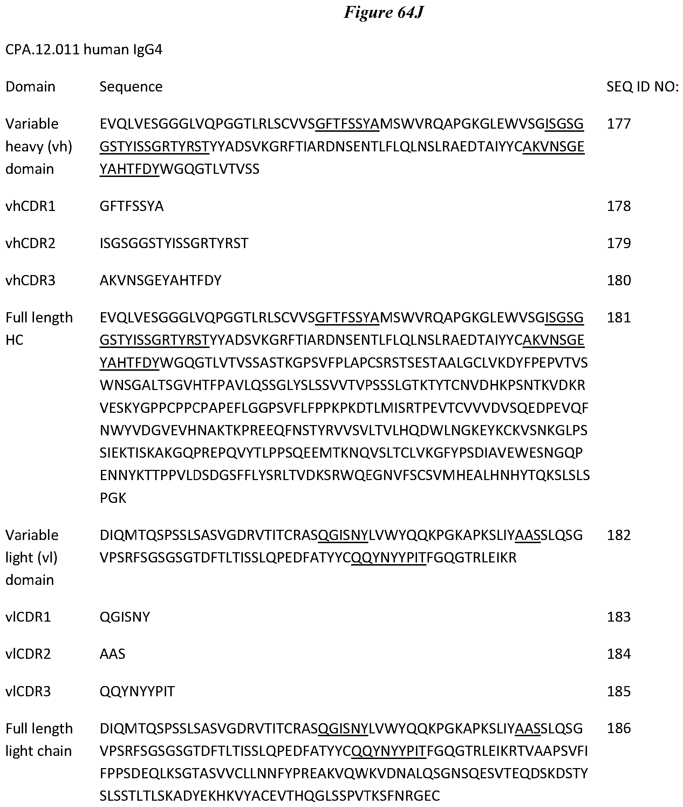

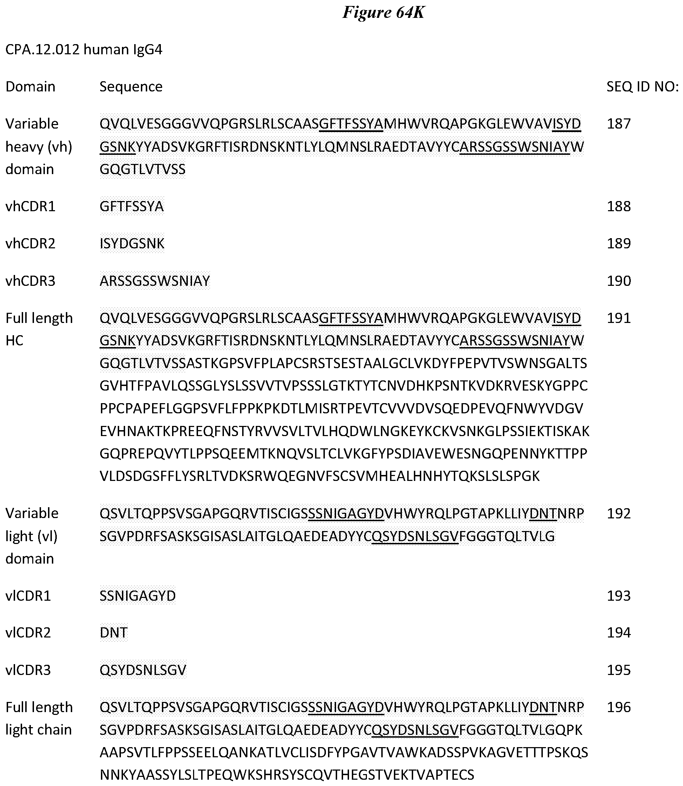

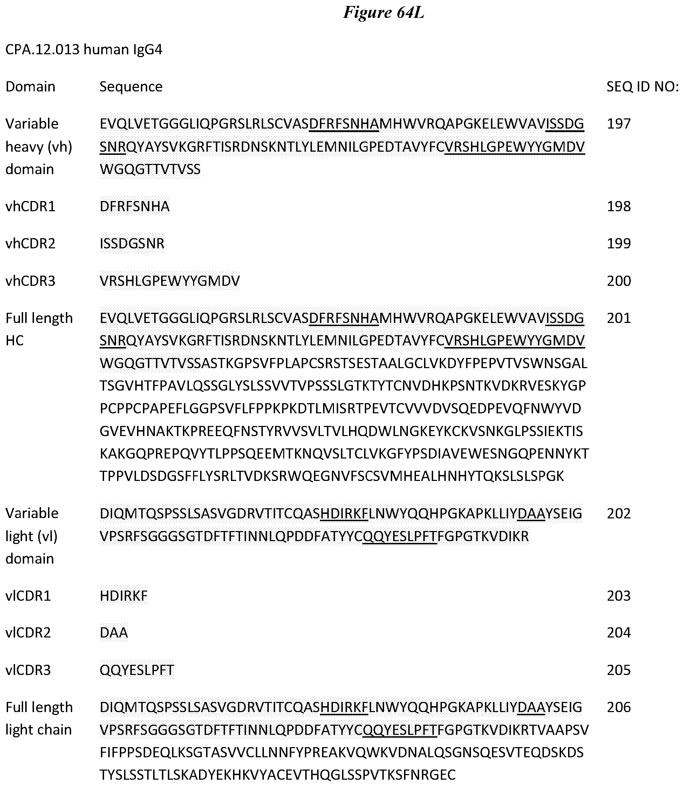

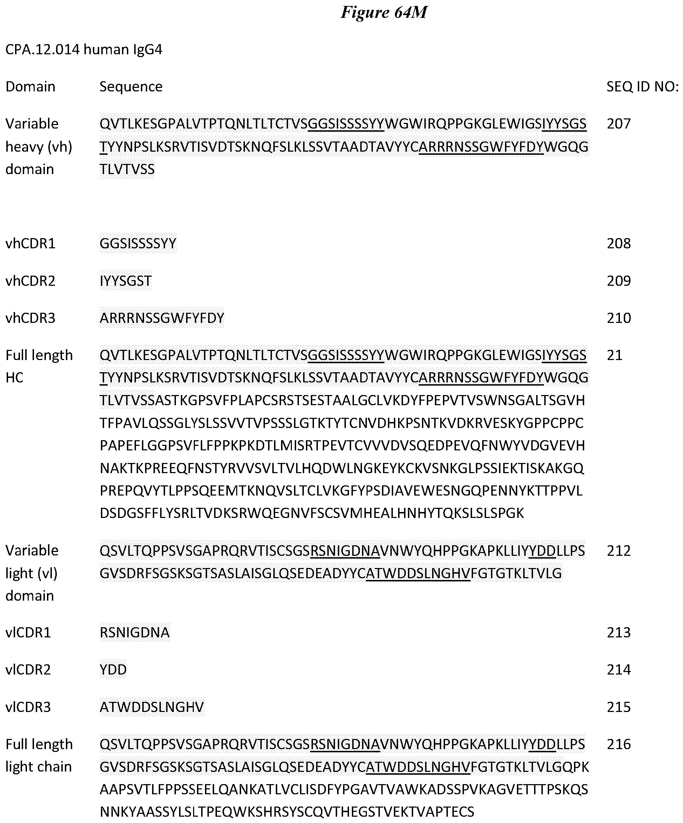

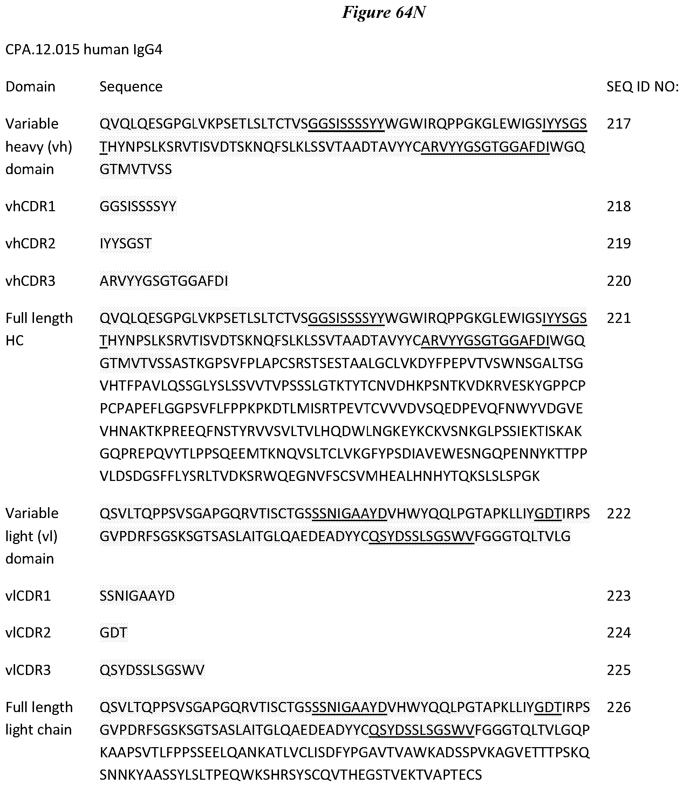

Traditional full length antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. According to at least some embodiments of the present invention is directed to the IgG class, which has several subclasses, including, but not limited to IgG1, IgG2, IgG3, and IgG4. Thus, "isotype" as used herein is meant any of the subclasses of immunoglobulins defined by the chemical and antigenic characteristics of their constant regions. While the exemplary antibodies herein designated below and in **Figure 64** are based on IgG4 and and in **Figure 80** **and** **81** are based on IgG1 heavy constant regions, as shown in the figures, the anti-HIDE1 antibodies of the invention include those using IgG1, IgG2, IgG3 and IgG4 sequences, or combinations thereof. In some embodiments, the anti-HIDE1 antibodies of the invention include IgG4 sequences. For example, as is known in the art, different IgG isotypes have different effector functions which may or may not be desirable. Accordingly, the anti-HIDE1 antibodies of the invention can also swap out the IgG4 constant domains for IgG1, IgG2 or IgG3 constant domains or can swap out the IgG1 for IgG2, IgG3, or IgG4, with IgG2 and IgG4 finding particular use in a number of situations, for example for ease of manufacture or when reduced effector function is desired, the latter being desired in some situations.

**Table 1: CPA.12.001 human IgG4**

| Domain | Sequence | SEQ ID NO: |
|---|---|---|
| Variable heavy (vh) domain | QVQLVESGGGLVQPGRSLRLSCAASGFTFSSYGMHWVRQAP GKGLEWVAVISYDGSNKYYADSVKGRFTISRDNSKNTLYLQ MNSLRAEDTAVYYCASEGVDFWSGLDYWGQGTLVTVSS | 87 |
| vhCDR1 | GFTFSSYG | 88 |
| vhCDR2 | ISYDGSNK | 89 |
| vhCDR3 | ASEGVDFWSGLDY | 90 |
| Full length HC | QVQLVESGGGLVQPGRSLRLSCAASGFTFSSYGMHWVRQAP GKGLEWVAVISYDGSNKYYADSVKGRFTISRDNSKNTLYLQ MNSLRAEDTAVYYCASEGVDFWSGLDYWGQGTLVTVSSAST KGPSVFPLAPCSRSTSESTAALGCLVKDYFPEPVTVSWNSGAL TSGVHTFPAVLQSSGLYSLSSVVTVPSSSLGTKTYTCNVDHKP SNTKVDKRVESKYGPPCPPCPAPEFLGGPSVFLFPPKPKDTLMI SRTPEVTCVVVDVSQEDPEVQFNWYVDGVEVHNAKTKPREE QFNSTYRVVSVLTVLHQDWLNGKEYKCKVSNKGLPSSIEKTIS KAKGQPREPQVYTLPPSQEEMTKNQVSLTCLVKGFYPSDIAV EWESNGQPENNYKTTPPVLDSDGSFFLYSRLTVDKSRWQEGN VFSCSVMHEALHNHYTQKSLSLSPGK | 91 |
| Variable light (vl) domain | QSALTQPRSASGSAGQSVTISCTGTSSDVGGHNYVSWYQQFP GKAPKLLIYEVSHRPSGVPDRFSGSKSGNTASLTVSGLQAEDE ADYYCSSYADLNNLMFGGGTKLTVLG | 92 |
| vlCDR1 | SSDVGGHNY | 93 |
| vlCDR2 | EVS | 94 |
| vlCDR3 | SSYADLNNLM | 95 |
| Full length light chain | QSALTQPRSASGSAGQSVTISCTGTSSDVGGHNYVSWYQQFP GKAPKLLIYEVSHRPSGVPDRFSGSKSGNTASLTVSGLQAEDE ADYYCSSYADLNNLMFGGGTKLTVLGQPKAAPSVTLFPPSSE ELQANKATLVCLISDFYPGAVTVAWKADSSPVKAGVETTTPS KQSNNKYAASSYLSLTPEQWKSHRSYSCQVTHEGSTVEKTVA PTECS | 96 |

**Table 2: CPA.12.002 human IgG4**

| Domain | Sequence | SEQ ID NO: |
|---|---|---|
| Variable heavy (vh) domain | EVQLVESGGGLVQPGRSLRLSCAASGFTFSSYAMHWVRQVPG KGLEWVAVISYDGSNKYYADSVKGRFTISRDNSKNTLYLQM NSLRAEDTAVYYCAKPMYSSGWYPLGYWGQGTLVTVSS | 97 |
| vhCDR1 | GFTFSSYA | 98 |
| vhCDR2 | ISYDGSNK | 99 |
| vhCDR3 | AKPMYSSGWYPLGY | 100 |
| Full length HC | EVQLVESGGGLVQPGRSLRLSCAASGFTFSSYAMHWVRQVPG KGLEWVAVISYDGSNKYYADSVKGRFTISRDNSKNTLYLQM NSLRAEDTAVYYCAKPMYSSGWYPLGYWGQGTLVTVSSAST KGPSVFPLAPCSRSTSESTAALGCLVKDYFPEPVTVSWNSGAL TSGVHTFPAVLQSSGLYSLSSVVTVPSSSLGTKTYTCNVDHKP SNTKVDKRVESKYGPPCPPCPAPEFLGGPSVFLFPPKPKDTLMI SRTPEVTCVVVDVSQEDPEVQFNWYVDGVEVHNAKTKPREE QFNSTYRVVSVLTVLHQDWLNGKEYKCKVSNKGLPSSIEKTIS KAKGQPREPQVYTLPPSQEEMTKNQVSLTCLVKGFYPSDIAV EWESNGQPENNYKTTPPVLDSDGSFFLYSRLTVDKSRWQEGN VFSCSVMHEALHNHYTQKSLSLSPGK | 101 |
| Variable light (vl) domain | DIVMTQTPLSLPVTPGQPASISCRSSQSPLDTDGNTYLDWYLQ RPGQSPQLLIYTLSNRASGVPDRFSGSGSGTDFTLKISRVEAED VGIYYCMQRIQYPLTFGPGTRLEIKR | 102 |
| vlCDR1 | QSPLDTDGNTY | 103 |
| vlCDR2 | TLS | 104 |
| vlCDR3 | MQRIQYPLT | 105 |
| Full length light chain | DIVMTQTPLSLPVTPGQPASISCRSSQSPLDTDGNTYLDWYLQ RPGQSPQLLIYTLSNRASGVPDRFSGSGSGTDFTLKISRVEAED VGIYYCMQRIQYPLTFGPGTRLEIKRTVAAPSVFIFPPSDEQLK SGTASVVCLLNNFYPREAKVQWKVDNALQSGNSQESVTEQD SKDSTYSLSSTLTLSKADYEKHKVYACEVTHQGLSSPVTKSFN RGEC | 106 |

**Table 3: CPA.12.003 human IgG4**

| Domain | Sequence | SEQ ID NO: |
|---|---|---|
| Variable heavy (vh) domain | QVQLQESGPGLVTPSETLSLTCTVSGGSISSVSYYWGWVRQPP GKGLEWIGSIYYSGTTAHNPSLKSRVTMAVDTSKNQFSLSLSS VTAADTAVYFCARGWRYYEDYYFDHWGQGTLVTVSS | 107 |
| vhCDR1 | GGSISSVSYY | 108 |
| vhCDR2 | IYYSGTT | 109 |
| vhCDR3 | ARGWRYYEDYYFDH | 110 |
| Full length HC | QVQLQESGPGLVTPSETLSLTCTVSGGSISSVSYYWGWVRQPP GKGLEWIGSIYYSGTTAHNPSLKSRVTMAVDTSKNQFSLSLSS VTAADTAVYFCARGWRYYEDYYFDHWGQGTLVTVSSASTK GPSVFPLAPCSRSTSESTAALGCLVKDYFPEPVTVSWNSGALT SGVHTFPAVLQSSGLYSLSSVVTVPSSSLGTKTYTCNVDHKPS NTKVDKRVESKYGPPCPPCPAPEFLGGPSVFLFPPKPKDTLMIS RTPEVTCVVVDVSQEDPEVQFNWYVDGVEVHNAKTKPREEQ FNSTYRVVSVLTVLHQDWLNGKEYKCKVSNKGLPSSIEKTISK AKGQPREPQVYTLPPSQEEMTKNQVSLTCLVKGFYPSDIAVE WESNGQPENNYKTTPPVLDSDGSFFLYSRLTVDKSRWQEGNV FSCSVMHEALHNHYTQKSLSLSPGK | 111 |
| Variable light (vl) domain | DIQMTQSPSTLSASVGDRVTITCRASQSINRWLAWYQQKPGK APKILIYTASSLESGVPSRFSGSGSGTEFTLTISSLQPDDFATYY CQQYNSYPITFGQGTRLEIKR | 112 |
| vlCDR1 | QSINRW | 113 |
| vlCDR2 | TAS | 114 |
| vlCDR3 | QQYNSYPIT | 115 |
| Full length light chain | DIQMTQSPSTLSASVGDRVTITCRASQSINRWLAWYQQKPGK APKILIYTASSLESGVPSRFSGSGSGTEFTLTISSLQPDDFATYY CQQYNSYPITFGQGTRLEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC | 116 |

**Table 4: CPA.12.004 human IgG4**

| Domain | Sequence | SEQ ID NO: |
|---|---|---|
| Variable heavy (vh) domain | EVQLVETGGGLIQPGRSLRLSCVASDFRFSNHAMHWVRQAPG KELEWVAVISSDGSNRQYAYSVKGRFTISRDNSKNTLYLEMNI LGPEDTAVYFCVRSHLGPEWYYGMDVWGQGTTVTVSS | 117 |
| vhCDR1 | DFRFSNHA | 118 |
| vhCDR2 | ISSDGSNR | 119 |
| vhCDR3 | VRSHLGPEWYYGMDV | 120 |
| Full length HC | EVQLVETGGGLIQPGRSLRLSCVASDFRFSNHAMHWVRQAPG KELEWVAVISSDGSNRQYAYSVKGRFTISRDNSKNTLYLEMNI LGPEDTAVYFCVRSHLGPEWYYGMDVWGQGTTVTVSSASTK GPSVFPLAPCSRSTSESTAALGCLVKDYFPEPVTVSWNSGALT SGVHTFPAVLQSSGLYSLSSVVTVPSSSLGTKTYTCNVDHKPS NTKVDKRVESKYGPPCPPCPAPEFLGGPSVFLFPPKPKDTLMIS RTPEVTCVVVDVSQEDPEVQFNWYVDGVEVHNAKTKPREEQ FNSTYRVVSVLTVLHQDWLNGKEYKCKVSNKGLPSSIEKTISK AKGQPREPQVYTLPPSQEEMTKNQVSLTCLVKGFYPSDIAVE WESNGQPENNYKTTPPVLDSDGSFFLYSRLTVDKSRWQEGNV FSCSVMHEALHNHYTQKSLSLSPGK | 121 |
| Variable light (vl) domain | DIQMTQSPSSLSTSVGDSVTITCRSSQSISTFLSWFQHKPGKAP NLLIYDASSLQSGVPSRFSGSGSGTDFTLTISSLQPEDFATYYC QQSDYLPFTFGPGTKVEIKR | 122 |
| vlCDR1 | QSISTF | 123 |
| vlCDR2 | DAS | 124 |
| vlCDR3 | QQSDYLPFT | 125 |
| Full length light chain | DIQMTQSPSSLSTSVGDSVTITCRSSQSISTFLSWFQHKPGKAP NLLIYDASSLQSGVPSRFSGSGSGTDFTLTISSLQPEDFATYYC QQSDYLPFTFGPGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 126 |

**Table 5: CPA.12.005 human IgG4**

| Domain | Sequence | SEQ ID NO: |
|---|---|---|
| Variable heavy (vh) domain | QVQLVESGGGVVQPGRSLRVSCAVSGFTFSSYGMHWVRQAP GGGLEWVAVMSYEGSFRHYADSVKGRFTISRDNSKNTLFLQ MDSLRAEDTAVYYCARDRPAGYTSGWGILDYWGQGTLVTVS S | 127 |
| vhCDR1 | GFTFSSYG | 128 |
| vhCDR2 | MSYEGSFR | 129 |
| vhCDR3 | ARDRPAGYTSGWGILDY | 130 |
| Full length HC | QVQLVESGGGVVQPGRSLRVSCAVSGFTFSSYGMHWVRQAP GGGLEWVAVMSYEGSFRHYADSVKGRFTISRDNSKNTLFLQ MDSLRAEDTAVYYCARDRPAGYTSGWGILDYWGQGTLVTVS SASTKGPSVFPLAPCSRSTSESTAALGCLVKDYFPEPVTVSWN SGALTSGVHTFPAVLQSSGLYSLSSVVTVPSSSLGTKTYTCNV DHKPSNTKVDKRVESKYGPPCPPCPAPEFLGGPSVFLFPPKPK DTLMISRTPEVTCVVVDVSQEDPEVQFNWYVDGVEVHNAKT KPREEQFNSTYRVVSVLTVLHQDWLNGKEYKCKVSNKGLPSS IEKTISKAKGQPREPQVYTLPPSQEEMTKNQVSLTCLVKGFYP SDIAVEWESNGQPENNYKTTPPVLDSDGSFFLYSRLTVDKSR WQEGNVFSCSVMHEALHNHYTQKSLSLSPGK | 131 |
| Variable light (vl) domain | EIVMTQSPATLSLSPGERATLSCRASQSVSSYLAWYQQKPGQA PRLLIYDASNRATGIPARFSGSGSGTDFTLTISSLEPEDFAVYYC QQRSNWPLTFGGGTKVDIKR | 132 |
| vlCDR1 | QSVSSY | 133 |
| vlCDR2 | DAS | 134 |
| vlCDR3 | QQRSNWPLT | 135 |
| Full length light chain | EIVMTQSPATLSLSPGERATLSCRASQSVSSYLAWYQQKPGQA PRLLIYDASNRATGIPARFSGSGSGTDFTLTISSLEPEDFAVYYC QQRSNWPLTFGGGTKVDIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC | 136 |

**Table 6: CPA.12.006 human IgG4**

| Domain | Sequence | SEQ ID NO: |
|---|---|---|
| Variable heavy (vh) domain | EVQLVESGGGVVQPGRSLRLSCAASGFTFSSFAMHWVRQAPG KGPEWLGIILYEGGHVVYADSVRGRLTISRDNSKNTLYLQMD SLRDEDTAVYYCAKGFYHAFDVWGQGTMVTVSS | 137 |
| vhCDR1 | GFTFSSFA | 138 |
| vhCDR2 | ILYEGGHV | 139 |
| vhCDR3 | AKGFYHAFDV | 140 |
| Full length HC | EVQLVESGGGVVQPGRSLRLSCAASGFTFSSFAMHWVRQAPG KGPEWLGIILYEGGHVVYADSVRGRLTISRDNSKNTLYLQMD SLRDEDTAVYYCAKGFYHAFDVWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWESN GQPENNYKTTPPVLDSDGSFFLYSRLTVDKSRWQEGNVFSCS VMHEALHNHYTQKSLSLSPGK | 141 |
| Variable light (vl) domain | QAVLTQPASLSASPGASASLTCTLRSGINVGTYRIYWYQQKPG SPPQYLLRYKSDSDK QQGSGVPSRFSGSKDASANAGILLISGLQSEDEADYYCMIWHS SAWVFGGGTQLTVLG | 142 |
| vlCDR1 | SGINVGTYR | 143 |
| vlCDR2 | YKSDSDK | 144 |
| vlCDR3 | MIWHSSAWV | 145 |
| Full length light chain | QAVLTQPASLSASPGASASLTCTLRSGINVGTYRIYWYQQKPG SPPQYLLRYKSDSDK QQGSGVPSRFSGSKDASANAGILLISGLQSEDEADYYCMIWHS SAWVFGGGTQLTVLGQPKAAPSVTLFPPSSEELQANKATLVC LISDFYPGAVTVAWKADSSPVKAGVETTTPSKQSNNKYAASS YLSLTPEQWKSHRSYSCQVTHEGSTVEKTVAPTECS | 146 |

**Table 7: CPA.12.007 human IgG4**

| Domain | Sequence | SEQ ID NO: |
|---|---|---|
| Variable heavy (vh) domain | EVQLVESGGGVVQPGESLRLSCAASGFTFSSYGFHWVRQSPG EGLEWLAVISYDGTSKSYADSVKGRFTVSRDNSKETLYLQLSS LRREDTALYYCARDTWGYYYGMDVWGQGTTVTVSS | 147 |
| vhCDR1 | GFTFSSYG | 148 |
| vhCDR2 | ISYDGTSK | 149 |
| vhCDR3 | ARDTWGYYYGMDV | 150 |
| Full length HC | EVQLVESGGGVVQPGESLRLSCAASGFTFSSYGFHWVRQSPG EGLEWLAVISYDGTSKSYADSVKGRFTVSRDNSKETLYLQLSS LRREDTALYYCARDTWGYYYGMDVWGQGTTVTVSSASTKG PSVFPLAPCSRSTSESTAALGCLVKDYFPEPVTVSWNSGALTS GVHTFPAVLQSSGLYSLSSVVTVPSSSLGTKTYTCNVDHKPSN TKVDKRVESKYGPPCPPCPAPEFLGGPSVFLFPPKPKDTLMISR TPEVTCVVVDVSQEDPEVQFNWYVDGVEVHNAKTKPREEQF NSTYRVVSVLTVLHQDWLNGKEYKCKVSNKGLPSSIEKTISK AKGQPREPQVYTLPPSQEEMTKNQVSLTCLVKGFYPSDIAVE WESNGQPENNYKTTPPVLDSDGSFFLYSRLTVDKSRWQEGNV FSCSVMHEALHNHYTQKSLSLSPGK | 151 |
| Variable light (vl) domain | DIQMTQSPSFLSGSVGDRVSISCRASQDIRSHLAWYQRKPGEA PKLLIYTASSLQGGVPLRFSGSGSGTEFTLTISSLQPEDSATYYC QHLHLYPLTFGGGTKLEIKR | 152 |
| vlCDR1 | QDIRSH | 153 |
| vlCDR2 | TAS | 154 |
| vlCDR3 | QHLHLYPLT | 155 |
| Full length light chain | DIQMTQSPSFLSGSVGDRVSISCRASQDIRSHLAWYQRKPGEA PKLLIYTASSLQGGVPLRFSGSGSGTEFTLTISSLQPEDSATYYC QHLHLYPLTFGGGTKLEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 156 |

**Table 8: CPA.12.008 human IgG4**

| Domain | Sequence | SEQ ID NO: |
|---|---|---|
| Variable heavy (vh) domain | QVQLQESGPGLVTPSETLSLTCTVSGGSISSVSYYWGWVRQPP GKGLEWIGSIYYSGTTAHNPSLKSRVTMAVDTSKNQFSLSLSS VTAADTAVYFCARGWRYYEDYYFDHWGQGTLVTVSS | 157 |
| vhCDR1 | GGSISSVSYY | 158 |
| vhCDR2 | IYYSGTT | 159 |
| vhCDR3 | ARGWRYYEDYYFDH | 160 |
| Full length HC | QVQLQESGPGLVTPSETLSLTCTVSGGSISSVSYYWGWVRQPP GKGLEWIGSIYYSGTTAHNPSLKSRVTMAVDTSKNQFSLSLSS VTAADTAVYFCARGWRYYEDYYFDHWGQGTLVTVSSASTK GPSVFPLAPCSRSTSESTAALGCLVKDYFPEPVTVSWNSGALT SGVHTFPAVLQSSGLYSLSSVVTVPSSSLGTKTYTCNVDHKPS NTKVDKRVESKYGPPCPPCPAPEFLGGPSVFLFPPKPKDTLMIS RTPEVTCVVVDVSQEDPEVQFNWYVDGVEVHNAKTKPREEQ FNSTYRVVSVLTVLHQDWLNGKEYKCKVSNKGLPSSIEKTISK AKGQPREPQVYTLPPSQEEMTKNQVSLTCLVKGFYPSDIAVE WESNGQPENNYKTTPPVLDSDGSFFLYSRLTVDKSRWQEGNV FSCSVMHEALHNHYTQKSLSLSPGK | 161 |
| Variable light (vl) domain | AIRMTQSPSSLSASVGDRVTITCQASQDISNYLNWYQQKPGKA PKSLIYAASSLQSGVPSKFSGSGSGTDFTLTISSLQPEDFATYYC QQYYSYPLTFGGGTKVEIKR | 162 |
| vlCDR1 | QDISNY | 163 |
| vlCDR2 | AAS | 164 |
| vlCDR3 | QQYYSYPLT | 165 |
| Full length light chain | AIRMTQSPSSLSASVGDRVTITCQASQDISNYLNWYQQKPGKA PKSLIYAASSLQSGVPSKFSGSGSGTDFTLTISSLQPEDFATYYC QQYYSYPLTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 166 |

**Table 9: CPA.12.009 human IgG4**

| Domain | Sequence | SEQ ID NO: |
|---|---|---|
| Variable heavy (vh) domain | QMQLVQSGAEVKKPGASVKVSCKASGYTFTTHAFSWVRQAP GLGLEWMGWISTYNGNTKYEQKFQGRVTLTTDSSTNTAYME LRSLRSDDTAVYYCVRDSRAFDVWGQGTMVTVSS | 167 |
| vhCDR1 | GYTFTTHA | 168 |
| vhCDR2 | ISTYNGNT | 169 |
| vhCDR3 | VRDSRAFDV | 170 |
| Full length HC | QMQLVQSGAEVKKPGASVKVSCKASGYTFTTHAFSWVRQAP GLGLEWMGWISTYNGNTKYEQKFQGRVTLTTDSSTNTAYME LRSLRSDDTAVYYCVRDSRAFDVWGQGTMVTVSSASTKGPS VFPLAPCSRSTSESTAALGCLVKDYFPEPVTVSWNSGALTSGV HTFPAVLQSSGLYSLSSVVTVPSSSLGTKTYTCNVDHKPSNTK VDKRVESKYGPPCPPCPAPEFLGGPSVFLFPPKPKDTLMISRTP EVTCVVVDVSQEDPEVQFNWYVDGVEVHNAKTKPREEQFNS TYRVVSVLTVLHQDWLNGKEYKCKVSNKGLPSSIEKTISKAK GQPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSPGK | 171 |
| Variable light (vl) domain | AIRMTQSPSSLSASVGDRVTITCQASQDISNYLNWYQQKPGKA PKSLIYAASSLQSGVPSKFSGSGSGTDFTLTISSLQPEDFATYYC QQYYSYPLTFGGGTKVEIKR DIQLTQSPSFLSASVGDRVTITCRASQGINSYLAWYQQKPGAA PKLLIYAASTLQSGVPSRFSGSGSGTEFTLTISSLQPEDFATYYC QQLNTYPLIFGQGTRLEIKR | 172 |
| vlCDR1 | QGINSY | 173 |
| vlCDR2 | AAS | 174 |
| vlCDR3 | QQLNTYPLI | 175 |
| Full length light chain | DIQLTQSPSFLSASVGDRVTITCRASQGINSYLAWYQQKPGAA PKLLIYAASTLQSGVPSRFSGSGSGTEFTLTISSLQPEDFATYYC QQLNTYPLIFGQGTRLEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 176 |

**Table 10: CPA.12.011 human IgG4**

| Domain | Sequence | SEQ ID NO: |
|---|---|---|
| Variable heavy (vh) domain | EVQLVESGGGLVQPGGTLRLSCVVSGFTFSSYAMSWVRQAPG KGLEWVSGISGSGGSTYISSGRTYRSTYYADSVKGRFTIARDN SENTLFLQLNSLRAEDTAIYYCAKVNSGEYAHTFDYWGQGTL VTVSS | 177 |
| vhCDR1 | GFTFSSYA | 178 |
| vhCDR2 | ISGSGGSTYISSGRTYRST | 179 |
| vhCDR3 | AKVNSGEYAHTFDY | 180 |
| Full length HC | EVQLVESGGGLVQPGGTLRLSCVVSGFTFSSYAMSWVRQAPG KGLEWVSGISGSGGSTYISSGRTYRSTYYADSVKGRFTIARDN SENTLFLQLNSLRAEDTAIYYCAKVNSGEYAHTFDYWGQGTL VTVSSASTKGPSVFPLAPCSRSTSESTAALGCLVKDYFPEPVTV SWNSGALTSGVHTFPAVLQSSGLYSLSSVVTVPSSSLGTKTYT CNVDHKPSNTKVDKRVESKYGPPCPPCPAPEFLGGPSVFLFPP KPKDTLMISRTPEVTCVVVDVSQEDPEVQFNWYVDGVEVHN AKTKPREEQFNSTYRVVSVLTVLHQDWLNGKEYKCKVSNKG LPSSIEKTISKAKGQPREPQVYTLPPSQEEMTKNQVSLTCLVKG FYPSDIAVEWESNGQPENNYKTTPPVLDSDGSFFLYSRLTVDK SRWQEGNVFSCSVMHEALHNHYTQKSLSLSPGK | 181 |
| Variable light (vl) domain | DIQMTQSPSSLSASVGDRVTITCRASQGISNYLVWYQQKPGKA PKSLIYAASSLQSGVPSRFSGSGSGTDFTLTISSLQPEDFATYYC QQYNYYPITFGQGTRLEIKR | 182 |
| vlCDR1 | QGISNY | 183 |
| vlCDR2 | AAS | 184 |
| vlCDR3 | QQYNYYPIT | 185 |
| Full length light chain | DIQMTQSPSSLSASVGDRVTITCRASQGISNYLVWYQQKPGKA PKSLIYAASSLQSGVPSRFSGSGSGTDFTLTISSLQPEDFATYYC QQYNYYPITFGQGTRLEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 186 |

**Table 11: CPA.12.012 human IgG4**

| Domain | Sequence | SEQ ID NO: |
|---|---|---|
| Variable heavy (vh) domain | QVQLVESGGGVVQPGRSLRLSCAASGFTFSSYAMHWVRQAP GKGLEWVAVISYDGSNKYYADSVKGRFTISRDNSKNTLYLQ MNSLRAEDTAVYYCARSSGSSWSNIAYWGQGTLVTVSS | 187 |
| vhCDR1 | GFTFSSYA | 188 |
| vhCDR2 | ISYDGSNK | 189 |
| vhCDR3 | ARSSGSSWSNIAY | 190 |
| Full length HC | QVQLVESGGGVVQPGRSLRLSCAASGFTFSSYAMHWVRQAP GKGLEWVAVISYDGSNKYYADSVKGRFTISRDNSKNTLYLQ MNSLRAEDTAVYYCARSSGSSWSNIAYWGQGTLVTVSSAST KGPSVFPLAPCSRSTSESTAALGCLVKDYFPEPVTVSWNSGAL TSGVHTFPAVLQSSGLYSLSSVVTVPSSSLGTKTYTCNVDHKP SNTKVDKRVESKYGPPCPPCPAPEFLGGPSVFLFPPKPKDTLMI SRTPEVTCVVVDVSQEDPEVQFNWYVDGVEVHNAKTKPREE QFNSTYRVVSVLTVLHQDWLNGKEYKCKVSNKGLPSSIEKTIS KAKGQPREPQVYTLPPSQEEMTKNQVSLTCLVKGFYPSDIAV EWESNGQPENNYKTTPPVLDSDGSFFLYSRLTVDKSRWQEGN VFSCSVMHEALHNHYTQKSLSLSPGK | 191 |
| Variable light (vl) domain | QSVLTQPPSVSGAPGQRVTISCIGSSSNIGAGYDVHWYRQLPG TAPKLLIYDNTNRPSGVPDRFSASKSGISASLAITGLQAEDEAD YYCQSYDSNLSGVFGGGTQLTVLG | 192 |
| vlCDR1 | SSNIGAGYD | 193 |
| vlCDR2 | DNT | 194 |
| vlCDR3 | QSYDSNLSGV | 195 |
| Full length light chain | QSVLTQPPSVSGAPGQRVTISCIGSSSNIGAGYDVHWYRQLPG TAPKLLIYDNTNRPSGVPDRFSASKSGISASLAITGLQAEDEAD YYCQSYDSNLSGVFGGGTQLTVLGQPKAAPSVTLFPPSSEELQ ANKATLVCLISDFYPGAVTVAWKADSSPVKAGVETTTPSKQS NNKYAASSYLSLTPEQWKSHRSYSCQVTHEGSTVEKTVAPTE CS | 196 |

**Table 12: CPA.12.013 human IgG4**

| Domain | Sequence | SEQ ID NO: |
|---|---|---|
| Variable heavy (vh) domain | EVQLVETGGGLIQPGRSLRLSCVASDFRFSNHAMHWVRQAPG KELEWVAVISSDGSNRQYAYSVKGRFTISRDNSKNTLYLEMNI LGPEDTAVYFCVRSHLGPEWYYGMDVWGQGTTVTVSS | 197 |
| vhCDR1 | DFRFSNHA | 198 |
| vhCDR2 | ISSDGSNR | 199 |
| vhCDR3 | VRSHLGPEWYYGMDV | 200 |
| Full length HC | EVQLVETGGGLIQPGRSLRLSCVASDFRFSNHAMHWVRQAPG KELEWVAVISSDGSNRQYAYSVKGRFTISRDNSKNTLYLEMNI LGPEDTAVYFCVRSHLGPEWYYGMDVWGQGTTVTVSSASTK GPSVFPLAPCSRSTSESTAALGCLVKDYFPEPVTVSWNSGALT SGVHTFPAVLQSSGLYSLSSVVTVPSSSLGTKTYTCNVDHKPS NTKVDKRVESKYGPPCPPCPAPEFLGGPSVFLFPPKPKDTLMIS RTPEVTCVVVDVSQEDPEVQFNWYVDGVEVHNAKTKPREEQ FNSTYRVVSVLTVLHQDWLNGKEYKCKVSNKGLPSSIEKTISK AKGQPREPQVYTLPPSQEEMTKNQVSLTCLVKGFYPSDIAVE WESNGQPENNYKTTPPVLDSDGSFFLYSRLTVDKSRWQEGNV FSCSVMHEALHNHYTQKSLSLSPGK | 201 |
| Variable light (vl) domain | DIQMTQSPSSLSASVGDRVTITCQASHDIRKFLNWYQQHPGKA PKLLIYDAAYSEIGVPSRFSGGGSGTDFTFTINNLQPDDFATYY CQQYESLPFTFGPGTKVDIKR | 202 |
| vlCDR1 | HDIRKF | 203 |
| vlCDR2 | DAA | 204 |
| vlCDR3 | QQYESLPFT | 205 |
| Full length light chain | DIQMTQSPSSLSASVGDRVTITCQASHDIRKFLNWYQQHPGKA PKLLIYDAAYSEIGVPSRFSGGGSGTDFTFTINNLQPDDFATYY CQQYESLPFTFGPGTKVDIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC | 206 |

**Table 13: CPA.12.014 human IgG4**

| Domain | Sequence | SEQ ID NO: |
|---|---|---|
| Variable heavy (vh) domain | QVTLKESGPALVTPTQNLTLTCTVSGGSISSSSYYWGWIRQPP GKGLEWIGSIYYSGSTYYNPSLKSRVTISVDTSKNQFSLKLSSV TAADTAVYYCARRRNSSGWFYFDYWGQGTLVTVSS | 207 |
| vhCDR1 | GGSISSSSYY | 208 |
| vhCDR2 | IYYSGST | 209 |
| vhCDR3 | ARRRNSSGWFYFDY | 210 |
| Full length HC | QVTLKESGPALVTPTQNLTLTCTVSGGSISSSSYYWGWIRQPP GKGLEWIGSIYYSGSTYYNPSLKSRVTISVDTSKNQFSLKLSSV TAADTAVYYCARRRNSSGWFYFDYWGQGTLVTVSSASTKGP SVFPLAPCSRSTSESTAALGCLVKDYFPEPVTVSWNSGALTSG VHTFPAVLQSSGLYSLSSVVTVPSSSLGTKTYTCNVDHKPSNT KVDKRVESKYGPPCPPCPAPEFLGGPSVFLFPPKPKDTLMISRT PEVTCVVVDVSQEDPEVQFNWYVDGVEVHNAKTKPREEQFN STYRVVSVLTVLHQDWLNGKEYKCKVSNKGLPSSIEKTISKA KGQPREPQVYTLPPSQEEMTKNQVSLTCLVKGFYPSDIAVEW ESNGQPENNYKTTPPVLDSDGSFFLYSRLTVDKSRWQEGNVF SCSVMHEALHNHYTQKSLSLSPGK | 211 |
| Variable light (vl) domain | QSVLTQPPSVSGAPRQRVTISCSGSRSNIGDNAVNWYQHPPGK APKLLIYYDDLLPSGVSDRFSGSKSGTSASLAISGLQSEDEADY YCATWDDSLNGHVFGTGTKLTVLG | 212 |
| vlCDR1 | RSNIGDNA | 213 |
| vlCDR2 | YDD | 214 |
| vlCDR3 | ATWDDSLNGHV | 215 |
| Full length light chain | QSVLTQPPSVSGAPRQRVTISCSGSRSNIGDNAVNWYQHPPGK APKLLIYYDDLLPSGVSDRFSGSKSGTSASLAISGLQSEDEADY YCATWDDSLNGHVFGTGTKLTVLGQPKAAPSVTLFPPSSEEL QANKATLVCLISDFYPGAVTVAWKADSSPVKAGVETTTPSKQ SNNKYAASSYLSLTPEQWKSHRSYSCQVTHEGSTVEKTVAPT ECS | 216 |

**Table 14: CPA.12.015 human IgG4**

| Domain | Sequence | SEQ ID NO: |
|---|---|---|
| Variable heavy (vh) domain | QVQLQESGPGLVKPSETLSLTCTVSGGSISSSSYYWGWIRQPPGKGLEWIGSIYYS GSTHYNPSLKSRVTISVDTSKNQFSLKLSSVTAADTAVYYCARVYYGSGTGGAFDI WGQGTMVTVSS | 217 |
| vhCDR1 | GGSISSSSYY | 218 |
| vhCDR2 | IYYSGST | 219 |
| vhCDR3 | ARVYYGSGTGGAFDI | 220 |
| Full length HC | QVQLQESGPGLVKPSETLSLTCTVSGGSISSSSYYWGWIRQPPGKGLEWIGSIYYS GSTHYNPSLKSRVTISVDTSKNQFSLKLSSVTAADTAVYYCARVYYGSGTGGAFDI WGQGTMVTVSSASTKGPSVFPLAPCSRSTSESTAALGCLVKDYFPEPVTVSWNSGA LTSGVHTFPAVLQSSGLYSLSSVVTVPSSSLGTKTYTCNVDHKPSNTKVDKRVESK YGPPCPPCPAPEFLGGPSVFLFPPKPKDTLMISRTPEVTCVVVDVSQEDPEVQFNW YVDGVEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQPEN NYKTTPPVLDSDGSFFLYSRLTVDKSRWQEGNVFSCSVMHEALHNHYTQKSLSLSP GK | 221 |
| Variable light (vl) domain | QSVLTQPPSVSGAPGQRVTISCTGSSSNIGAAYDVHWYQQLPGTAPKLLIYGDTIR PSGVPDRFSGSKSGTSASLAITGLQAEDEADYYCQSYDSSLSGSWVFGGGTQLTVL G | 222 |
| vlCDR1 | SSNIGAAYD | 223 |
| vlCDR2 | GDT | 224 |
| vlCDR3 | QSYDSSLSGSWV | 225 |
| Full length light chain | QSVLTQPPSVSGAPGQRVTISCTGSSSNIGAAYDVHWYQQLPGTAPKLLIYGDTIR PSGVPDRFSGSKSGTSASLAITGLQAEDEADYYCQSYDSSLSGSWVFGGGTQLTVL GQPKAAPSVTLFPPSSEELQANKATLVCLISDFYPGAVTVAWKADSSPVKAGVETT TPSKQSNNKYAASSYLSLTPEQWKSHRSYSCQVTHEGSTVEKTVAPTECS | 226 |

**Table 15: 33B4 Antibody**

| Domain | Sequence | SEQ ID NO: |
|---|---|---|
| Variable heavy (vh) domain | QIQLVQSGPELKKPGETVKISCKASGYTFTNYGMNWVKQAPGKGLKWMGWINTYTGEPTY ADDFKGRFAFSLETPASTAYLQINNLKNEDMATYFC VREHFYALDY WGQGTSVTVSS | 227 |
| vhCDR1 | GYTFTNYG | 228 |
| vhCDR2 | INTYTGEP | 229 |
| vhCDR3 | VREHFYALDY | 230 |
| Full length HC | QIQLVQSGPELKKPGETVKISCKASGYTFTNYGMNWVKQAPGKGLKWMGWINTYTGEPTY ADDFKGRFA FSLETPASTAYLQINNLKNEDMATYFCVREHFYALDYWGQGTSVTVSSAKTTPPSVYPLA PGSAAQTNS MVTLGCLVKGYFPEPVTVTWNSGSLSSGVHTFPAVLQSDLYTLSSSVTVPSSTWPSETVT CNVAHPASS TKVDKKIVPRDCGCKPCICTVPEVSSVFIFPPKPKDVLTITLTPKVTCVVVDISKDDPEV QFSWFVDDV EVHTAQTQPREEQFNSTFRSVSELPIMHQDWLNGKEFKCRVNSAAFPAPIEKTISKTKGR PKAPQVYTI PPPKEQMAKDKVSLTCMITDFFPEDITVEWQWNGQPAENYKNTQPIMDTDGSYFVYSKLN VQKSNWEAG NTFTCSVLHEGLHNHHTEKSLSHSPGK | 231 |
| Variable light (vl) domain | KIVLTQSPASLAVSLRQRATISCRAS ESVDSYGNSF MHWYQQKPGQPPKLLIY RAS NLESGVPARFSGSGSRTDFTLTIDPVEADDAATYYC QQSNEDPRT FGGGTKLEIK | 232 |
| vlCDR1 | ESVDSYGNSF | 233 |
| vlCDR2 | RAS | 234 |
| vlCDR3 | QQSNEDPRT | 235 |
| Full length light chain | KIVLTQSPASLAVSLRQRATISCRASESVDSYGNSFMHWYQQKPGQPPKLLIYRASNLE SGVPARFSGSGSRTDFTLTIDPVEADDAATYYCQQSNEDPRTFGGGTKLEIKRADAAPT VSIFPPSSEQLTSGGASVVCFLNNFYPKDINVKWKIDGSERQNGVLNSWTDQDSKDSTY SMSSTLTLTKDEYERHNSYTCEATHKTSTSPIVKSFNRNEC | 236 |

**Table 16: 36C1 Antibody**

| Domain | Sequence | SEQ ID NO: |
|---|---|---|
| Variable heavy (vh) domain | QIQLVQSGPELKKPGETVKISCKASGYTFTNYVMNWVKQAPGKGLKWMGWINIYTG EPTYADDFKGRFAFSLETSASTSYLQINNLKNEDMATYFC ARWGDGYPWFAY WGQGTLVTVSA | 237 |
| vhCDR1 | GYTFTNYV | 238 |
| vhCDR2 | INIYTGEP | 239 |
| vhCDR3 | ARWGDGYPWFAY | 240 |
| Full length HC | QIQLVQSGPELKKPGETVKISCKASGYTFTNYVMNWVKQAPGKGLKWMGWINIYTG EPTYADDFKGRFAFSLETSASTSYLQINNLKNEDMATYFCARWGDGYPWFAYWGQG TLVTVSAAKTTPPSVYPLAPGSAAQTNSMVTLGCLVKGYFPEPVTVTWNSGSLSSG VHTFPAVLQSDLYTLSSSVTVPSSTWPSETVTCNVAHPASSTKVDKKIVPRDCGCK PCICTVPEVSSVFIFPPKPKDVLTITLTPKVTCVVVDISKDDPEVQFSWFVDDVEV HTAQTQPREEQFNSTFRSVSELPIMHQDWLNGKEFKCRVNSAAFPAPIEKTISKTK GRPKAPQVYTIPPPKEQMAKDKVSLTCMITDFFPEDITVEWQWNGQPAENYKNTQP IMDTDGSYFVYSKLNVQKSNWEAGNTFTCSVLHEGLHNHHTEKSLSHSPGK | 241 |
| Variable light (vl) domain | NIVMTQSPKSMSMSVGERVTLSCKASENVDTYVSWYQQKPEQSPKLLIYGASNRYT GVPDRFTGSGSATDFTLIISSVQAEDLADYHC GQSYSYPLT FGAGTKLELK | 242 |
| vlCDR1 | ENVDTY | 243 |
| vlCDR2 | GAS | 244 |
| vlCDR3 | GQSYSYPLT | 245 |
| Full length light chain | NIVMTQSPKSMSMSVGERVTLSCKASENVDTYVSWYQQKPEQSPKLLIYGASNRYT GVPDRFTGSGSATDFTLIISSVQAEDLADYHCGQSYSYPLTFGAGTKLELKRADAA PTVSIFPPSSEQLTSGGASVVCFLNNFYPRDINVKWKIDGSERQNGVLNSWTDQDS KDSTYSMSSTLTLTKDEYERHNSYTCEATHKTSTSPIVKSFNRNEC | 246 |

**Table 17: 39A7Antibody**

| Domain | Sequence | SEQ ID NO: |
|---|---|---|
| Variable heavy (vh) domain | QVQLQQSGAELVRPGASVKLSCKAL GYTFTDYE MHWVKQTPVHGLEWIGD IYPGSGGT AYNQKFKGKATLTADKSSSTAYMELSSLTSEDSAVYYC TRKGRSFAY WGQGTLVTVSA | 247 |
| vhCDR1 | GYTFTDYE | 248 |
| vhCDR2 | IYPGSGGT | 249 |
| vhCDR3 | TRKGRSFAY | 250 |
| Full length HC | QVQLQQSGAELVRPGASVKLSCKALGYTFTDYEMHWVKQTP VHGLEWIGDIYPGSGGTAYNQKFKGKAT LTADKSSSTAYMELSSLTSEDSAVYYCTRKGRSFAYWGQGTL VTVSAAKTTPPSVYPLAPGSAAQTNSM VTLGCLVKGYFPEPVTVTWNSGSLSSGVHTFPAVLQSDLYTLS SSVTVPSSTWPSETVTCNVAHPASST KVDKKIVPRDCGCKPCICTVPEVSSVFIFPPKPKDVLTITLTPKV TCVVVDISKDDPEVQFSWFVDDVE VHTAQTQPREEQFNSTFRSVSELPIMHQDWLNGKEFKCRVNS AAFPAPIEKTISKTKGRPKAPQVYTIP PPKEQMAKDKVSLTCMITDFFPEDITVEWQWNAQPAENYKN TQPIMDTDGSYFVYSKLNVQKSNWEAGN TFTCSVLHEGLHNHHTEKSLSHSPGK | 251 |
| Variable light (vl) domain | NIVMTQSPKSMSMSVGERVTLSCKAS ENVDTY VSWYQQKPEQSPKLLIY GAS NRYTGVPDRFTGSGSATDFTLIISSVQAEDLADYHC GQSYRYPLT FGAGTKLELK | 252 |
| vlCDR1 | ENVDTY | 253 |
| vlCDR2 | GAS | 254 |
| vlCDR3 | GQSYRYPLT | 255 |
| Full length light chain | NIVMTQSPKSMSMSVGERVTLSCKASENVDTYVSWYQQKPE QSPKLLIYGASNRYTGVP DRFTGSGSATDFTLIISSVQAEDLADYHCGQSYRYPLTFGAGT KLELKRADAAPTVSIF PPSSEQLTSGGASVVCFLNNFYPRDINVKWKIDGSERQNGVLN SWTDQDSKDSTYSMSS TLTLTKDEYERHNSYTCEATHKTSTSPIVKSFNRNEC | 256 |

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions".

Each V_{H} and V_{L} is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region, although sometimes the numbering is shifted slightly as will be appreciated by those in the art; Kabat et al., SEQUENCES OF PROTEINS OF IMMUNOLOGICAL INTEREST, 5 th Ed. Public Health Service, National Institutes of Health, Bethesda, Md. (1991) and/or those residues forming a hypervariable loop (*e.g*. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention and/or for use in the methods of the invention are described above and shown in **Figures 64**, and **80****-81.** In some emboidments, specific CDRs of the invention include those in **Figure 64**, as well as Tables 1-14. In some embodiments, specific CDRs of the invention include those in **Figure 81**, as well as Tables 15-17.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5 th edition, NIH publication, No. 91-3242, E. A. Kabat et al.).

In the IgG subclass of immunoglobulins, there are several immunoglobulin domains in the heavy chain. By "immunoglobulin (Ig) domain" herein is meant a region of an immunoglobulin having a distinct tertiary structure. Of interest in some embodiments of the present invention are the heavy chain domains, including, the constant heavy (C_{H}) domains and the hinge domains. In the context of IgG antibodies, the IgG isotypes each have three CH regions. Accordingly, "C_{H}" domains in the context of IgG are as follows: "C_{H1}" refers to positions 118-220 according to the EU index as in Kabat. "C_{H2}" refers to positions 237-340 according to the EU index as in Kabat, and "C_{H3}" refers to positions 341-447 according to the EU index as in Kabat.

Accordingly, the invention provides variable heavy domains, variable light domains, heavy constant domains, light constant domains and Fc domains to be used as outlined herein. By "variable region" as used herein is meant the region of an immunoglobulin that comprises one or more Ig domains substantially encoded by any of the Vκ or Vλ, and/or V_{H} genes that make up the kappa, lambda, and heavy chain immunoglobulin genetic loci respectively. Accordingly, the variable heavy domain comprises vhFR1-vhCDR1-vhFR2-vhCDR2-vhFR3-vhCDR3-vhFR4, and the variable light domain comprises vlFR1-vlCDR1-vlFR2-vlCDR2-vlFR3-vlCDR3-vlFR4. By "heavy constant region" herein is meant the C_{H1}-hinge-C_{H2}-C_{H3} portion of an antibody. By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cγ2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some embodiments, as is more fully described below, amino acid modifications are made to the Fc region, for example to alter binding to one or more FcγR receptors or to the FcRn receptor.

Thus, "Fc variant" or "variant Fc" as used herein is meant a protein comprising an amino acid modification in an Fc domain. In some embodiments, the Fc variants of the present invention are defined according to the amino acid modifications that compose them. Thus, for example, N434S or 434S is an Fc variant with the substitution serine at position 434 relative to the parent Fc polypeptide, wherein the numbering is according to the EU index. Likewise, M428L/N434S defines an Fc variant with the substitutions M428L and N434S relative to the parent Fc polypeptide. The identity of the WT amino acid may be unspecified, in which case the aforementioned variant is referred to as 428L/434S. It is noted that the order in which substitutions are provided is arbitrary, that is to say that, for example, 428L/434S is the same Fc variant as M428L/N434S, and so on. For all positions discussed in the present invention that relate to antibodies, unless otherwise noted, amino acid position numbering is according to the EU index.

By "Fab" or "Fab region" as used herein is meant the polypeptide that comprises the V_{H}, C_{H1}, V_{L}, and C_{L} immunoglobulin domains. Fab may refer to this region in isolation, or this region in the context of a full length antibody, antibody fragment or Fab fusion protein. By "Fv" or "Fv fragment" or "Fv region" as used herein is meant a polypeptide that comprises the V_{L} and V_{H} domains of a single antibody. As will be appreciated by those in the art, these generally are made up of two chains.

Throughout the present specification, either the IMTG numbering system or the Kabat numbering system is generally used when referring to a residue in the variable domain (approximately, residues 1-107 of the light chain variable region and residues 1-113 of the heavy chain variable region) (e.g, Kabat et al., *supra* (1991)). EU numbering as in Kabat is generally used for constant domains and/or the Fc domains.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and non conformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning". Specific bins are described below.

Included within the definition of "antibody" is an "antigen-binding portion" of an antibody (also used interchangeably with "antigen-binding fragment", "antibody fragment" and "antibody derivative"). That is, for the purposes of the invention, an antibody of the invention has a minimum functional requirement that it bind to a HIDE1 antigen. As will be appreciated by those in the art, there are a large number of antigen fragments and derivatives that retain the ability to bind an antigen and yet have alternative structures, including, but not limited to, (i) the Fab fragment consisting of V_{L}, V_{H}, C_{L} and CH1 domains, (ii) the Fd fragment consisting of the V_{H} and C_{H1} domains, (iii) F(ab')2 fragments, a bivalent fragment comprising two linked Fab fragments (vii) single chain Fv molecules (scFv), wherein a V_{H} domain and a V_{L} domain are linked by a peptide linker which allows the two domains to associate to form an antigen binding site (Bird et al., 1988, Science 242:423-426, Huston et al., 1988, Proc. Natl. Acad. Sci. USA 85:5879-5883), (iv) "diabodies" or "triabodies", multivalent or multispecific fragments constructed by gene fusion (Tomlinson et. al., 2000, Methods Enzymol. 326:461-479; WO94/13804; Holliger et al., 1993, Proc. Natl. Acad. Sci. USA 90:6444-6448), (v) "domain antibodies" or "dAb" (sometimes referred to as an "immunoglobulin single variable domain", including single antibody variable domains from other species such as rodent (for example, as disclosed in WO 00/29004), nurse shark and Camelid V-HH dAbs, (vi) SMIPs (small molecule immunopharmaceuticals), camelbodies, nanobodies and IgNAR

Still further, an antibody or antigen-binding portion thereof (antigen-binding fragment, antibody fragment, antibody portion) may be part of a larger immunoadhesion molecules (sometimes also referred to as "fusion proteins"), formed by covalent or noncovalent association of the antibody or antibody portion with one or more other proteins or peptides. Examples of immunoadhesion molecules include use of the streptavidin core region to make a tetrameric scFv molecule and use of a cysteine residue, a marker peptide and a C-terminal polyhistidine tag to make bivalent and biotinylated scFv molecules. Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

In general, the anti-HIDE1 antibodies of the invention are recombinant. "Recombinant" as used herein, refers broadly with reference to a product, *e.g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "recombinant antibody", as used herein, includes all antibodies that are prepared, expressed, created or isolated by recombinant means, such as (a) antibodies isolated from an animal (*e.g*., a mouse) that is transgenic or transchromosomal for human immunoglobulin genes or a hybridoma prepared therefrom (described further below), (b) antibodies isolated from a host cell transformed to express the human antibody, *e.g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

### A. Optional Antibody Engineering

The antibodies of the invention can be modified, or engineered, to alter the amino acid sequences by amino acid substitutions.

By "amino acid substitution" or "substitution" herein is meant the replacement of an amino acid at a particular position in a parent polypeptide sequence with a different amino acid. In particular, in some embodiments, the substitution is to an amino acid that is not naturally occurring at the particular position, either not naturally occurring within the organism or in any organism. For example, the substitution E272Y refers to a variant polypeptide, in this case an Fc variant, in which the glutamic acid at position 272 is replaced with tyrosine. For clarity, a protein which has been engineered to change the nucleic acid coding sequence but not change the starting amino acid (for example exchanging CGG (encoding arginine) to CGA (still encoding arginine) to increase host organism expression levels) is not an "amino acid substitution"; that is, despite the creation of a new gene encoding the same protein, if the protein has the same amino acid at the particular position that it started with, it is not an amino acid substitution.

As discussed herein, amino acid substitutions can be made to alter the affinity of the CDRs for the HIDE1 protein (including both increasing and decreasing binding, as is more fully outlined below), as well as to alter additional functional properties of the antibodies. For example, the antibodies may be engineered to include modifications within the Fc region, typically to alter one or more functional properties of the antibody, such as serum half-life, complement fixation, Fc receptor binding, and/or antigen-dependent cellular cytotoxicity. Furthermore, an antibody according to at least some embodiments of the invention may be chemically modified (*e.g*., one or more chemical moieties can be attached to the antibody) or be modified to alter its glycosylation, again to alter one or more functional properties of the antibody. Such embodiments are described further below. The numbering of residues in the Fc region is that of the EU index of Kabat.

In one embodiment, the hinge region of C_{H1} is modified such that the number of cysteine residues in the hinge region is altered, *e.g*., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired *Staphylococcyl* protein A (SpA) binding relative to native Fc-hinge domain SpA binding. This approach is described in further detail in U.S. Pat. No. 6,165,745 by Ward et al.

In some embodiments, amino acid substitutions can be made in the Fc region, in general for altering binding to FcγR receptors. By "Fc gamma receptor", "FcγR" or "FcgammaR" as used herein is meant any member of the family of proteins that bind the IgG antibody Fc region and is encoded by an FcγR gene. In humans this family includes but is not limited to FcγRI (CD64), including isoforms FcyRIa, FcyRIb, and FcyRIc; FcγRII (CD32), including isoforms FcyRIIa (including allotypes H131 and R131), FcyRIIb (including FcγRIIb-1 and FcyRIIb-2), and FcyRIIc; and FcγRIII (CD16), including isoforms FcγRIIIa (including allotypes V158 and F158) and FcyRIIIb (including allotypes FcγRIIIb-NA1 and FcyRIIIb-NA2) (Jefferis et al., 2002, Immunol Lett 82:57-65), as well as any undiscovered human FcγRs or FcγR isoforms or allotypes. An FcγR may be from any organism, including but not limited to humans, mice, rats, rabbits, and monkeys. Mouse FcγRs include but are not limited to FcγRI (CD64), FcγRII (CD32), FcγRIII-1 (CD16), and FcγRIII-2 (CD16-2), as well as any undiscovered mouse FcγRs or FcγR isoforms or allotypes.

There are a number of useful Fc substitutions that can be made to alter binding to one or more of the FcγR receptors. Substitutions that result in increased binding as well as decreased binding can be useful. For example, it is known that increased binding to FcyRIIIa generally results in increased ADCC (antibody dependent cell-mediated cytotoxicity; the cell-mediated reaction wherein nonspecific cytotoxic cells that express FcγRs recognize bound antibody on a target cell and subsequently cause lysis of the target cell. Similarly, decreased binding to FcyRIIb (an inhibitory receptor) can be beneficial as well in some circumstances. Amino acid substitutions that find use in some embodiments of the present invention include those listed in U.S. Patent No. 8,188,231 (particularly FIG. 41) and U.S. Patent No. 6,737,056. Particular variants that find use include, but are not limited to, 236A, 239D, 239E, 332E, 332D, 239D/332E, 267D, 267E, 328F, 267E/328F, 236A/332E, 239D/332E/330Y, 239D, 332E/330L, 299T and 297N.

In addition, the antibodies of the invention are modified to increase its biological half-life. Various approaches are possible. For example, one or more of the following mutations can be introduced: T252L, T254S, T256F, as described in U.S. Pat. No. 6,277,375 to Ward. Alternatively, to increase the biological half-life, the antibody can be altered within the C_{H1} or C_{L} region to contain a salvage receptor binding epitope taken from two loops of a CH2 domain of an Fc region of an IgG, as described in U.S. Pat. Nos. 5,869,046 and 6,121,022 by Presta et al. Additional mutations to increase serum half life are disclosed in U.S. Patent Nos. 8,883,973, 6,737,056 and 7,371,826, and include 428L, 434A, 434S, and 428L/434S.

In yet other embodiments, the Fc region is altered by replacing at least one amino acid residue with a different amino acid residue to alter the effector functions of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcγ receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for FcγRI, FcyRII, FcyRIII and FcRn have been mapped and variants with improved binding have been described (see Shields, R. L. et al. (2001) J. Biol. Chem. 276:6591-6604). Specific mutations at positions 256, 290, 298, 333, 334 and 339 are shown to improve binding to FcγRIII. Additionally, the following combination mutants are shown to improve FcγRIII binding: T256A/S298A, S298A/E333A, S298A/K224A and S298A/E333A/K334A. Furthermore, mutations such as M252Y/S254T/T256E or M428L/N434S improve binding to FcRn and increase antibody circulation half-life (see Chan CA and Carter PJ (2010) Nature Rev Immunol 10:301-316).

In still another embodiment, the antibody can be modified to abrogate *in vivo* Fab arm exchange. Specifically, this process involves the exchange of IgG4 half-molecules (one heavy chain plus one light chain) between other IgG4 antibodies that effectively results in bispecific antibodies which are functionally monovalent. Mutations to the hinge region and constant domains of the heavy chain can abrogate this exchange (see Aalberse, RC, Schuurman J., 2002, Immunology 105:9-19).

In still another embodiment, the glycosylation of an antibody is modified. For example, an aglycosylated antibody can be made (*i.e*., the antibody lacks glycosylation). Glycosylation can be altered to, for example, increase the affinity of the antibody for antigen or reduce effector function such as ADCC. Such carbohydrate modifications can be accomplished by, for example, altering one or more sites of glycosylation within the antibody sequence, for example N297. For example, one or more amino acid substitutions can be made that result in elimination of one or more variable region framework glycosylation sites to thereby eliminate glycosylation at that site.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies according to at least some embodiments of the invention to thereby produce an antibody with altered glycosylation. For example, the cell lines Ms704, Ms705, and Ms709 lack the fucosyltransferase gene, FUT8 (α (1,6) fucosyltransferase), such that antibodies expressed in the Ms704, Ms705, and Ms709 cell lines lack fucose on their carbohydrates. The Ms704, Ms705, and Ms709 FUT8 cell lines are created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87:614-22). As another example, EP 1,176,195 by Hanai et al. describes a cell line with a functionally disrupted FUT8 gene, which encodes a fucosyl transferase, such that antibodies expressed in such a cell line exhibit hypofucosylation by reducing or eliminating the α 1,6 bond-related enzyme. Hanai et al. also describe cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al. (2002) J. Biol. Chem. 277:26733-26740). PCT Publication WO 99/54342 by Umana et al. describes cell lines engineered to express glycoprotein-modifying glycosyl transferases (e.g., β(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180). Alternatively, the fucose residues of the antibody may be cleaved off using a fucosidase enzyme. For example, the fucosidase α-L-fucosidase removes fucosyl residues from antibodies (Tarentino, A. L. et al. (1975) Biochem. 14:5516-23).

Another modification of the antibodies herein that is contemplated by the invention is pegylation or the addition of other water soluble moieties, typically polymers, *e.g*., in order to enhance half-life. An antibody can be pegylated to, for example, increase the biological (e.g., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies according to at least some embodiments of the invention. See for example, EP 0 154 316 by Nishimura et al. and EP 0 401 384 by Ishikawa et al.

In addition to substitutions made to alter binding affinity to FcγRs and/or FcRn and/or increase *in vivo* serum half life, additional antibody modifications can be made, as described in further detail below.

In some cases, affinity maturation is done. Amino acid modifications in the CDRs are sometimes referred to as "affinity maturation". An "affinity matured" antibody is one having one or more alteration(s) in one or more CDRs which results in an improvement in the affinity of the antibody for antigen, compared to a parent antibody which does not possess those alteration(s). In some cases, although rare, it may be desirable to decrease the affinity of an antibody to its antigen, but this is generally not preferred.

In some embodiments, one or more amino acid modifications are made in one or more of the CDRs of the VISG1 antibodies of the invention. In general, only 1 or 2 or 3-amino acids are substituted in any single CDR, and generally no more than from 1, 2, 3. 4, 5, 6, 7, 8 9 or 10 changes are made within a set of CDRs. However, it should be appreciated that any combination of no substitutions, 1, 2 or 3 substitutions in any CDR can be independently and optionally combined with any other substitution.

Affinity maturation can be done to increase the binding affinity of the antibody for the HIDE1 antigen by at least about 10% to 50-100-150% or more, or from 1 to 5 fold as compared to the "parent" antibody. Preferred affinity matured antibodies will have nanomolar or even picomolar affinities for the HIDE1 antigen. Affinity matured antibodies are produced by known procedures. See, for example, Marks et al., 1992, Biotechnology 10:779-783 that describes affinity maturation by variable heavy chain (VH) and variable light chain (VL) domain shuffling. Random mutagenesis of CDR and/or framework residues is described in: Barbas, et al. 1994, Proc. Nat. Acad. Sci, USA 91:3809-3813; Shier et al., 1995, Gene 169:147-155; Yelton et al., 1995, J. Immunol. 155:1994-2004; Jackson et al., 1995, J. Immunol. 154(7):3310-9; and Hawkins et al, 1992, J. Mol. Biol. 226:889-896, for example.

Alternatively, amino acid modifications can be made in one or more of the CDRs of the antibodies of the invention that are "silent", *e.g.* that do not significantly alter the affinity of the antibody for the antigen. These can be made for a number of reasons, including optimizing expression (as can be done for the nucleic acids encoding the antibodies of the invention).

Thus, included within the definition of the CDRs and antibodies of the invention are variant CDRs and antibodies; that is, the antibodies of the invention can include amino acid modifications in one or more of the CDRs of the enumerated antibodies of the invention. In addition, as outlined below, amino acid modifications can also independently and optionally be made in any region outside the CDRs, including framework and constant regions.

### B. HIDE1 Antibodies

According to at least some embodiments of the present invention provides anti-HIDE1 antibodies for use in treating cancer in a patient, wherein the antibodies stimulate the immune system by inhibiting the action of HIDE1. (For convenience, "anti-HIDE1 antibodies" and "HIDE1 antibodies" are used interchangeably). The anti-HIDE1 antibodies of the invention specifically bind to human HIDE1, and preferably the ECD of human HIDE1, as depicted in **Figure 66****.**

Specific binding for HIDE1 or a HIDE1 epitope can be exhibited, for example, by an antibody having a KD of at least about 10⁻⁴M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the HIDE1 antigen or epitope

However, as shown in the Examples, for optimal binding to HIDE1 expressed on the surface of myeloid cells, the antibodies preferably have a KD less 50 nM and most preferably less than 1 nM, with less than 0.1 nM and less than 1 pM and 0.1 pM finding use in the methods of the invention.

Also, specific binding for a particular antigen or an epitope can be exhibited, for example, by an antibody having a KA or Ka for a HIDE1 antigen or epitope of at least 20-, 50-, 100-, 500-, 1000-, 5,000-, 10,000- or more times greater for the epitope relative to a control, where KA or Ka refers to an association rate of a particular antibody-antigen interaction.

In some embodiments, the anti-HIDE1 antibodies of the invention bind to human HIDE1 with a K_{D} of 100 nM or less, 50 nM or less, 10 nM or less, or 1 nM or less (that is, higher binding affinity), or 1pM or less, wherein K_{D} is determined by known methods, *e.g.* surface plasmon resonance (SPR, *e.g.* Biacore assays), ELISA, KinExA, and most typically SPR at 25° or 37° C.

### C. Specific anti-HIDE1 antibodies

The invention provides antigen binding domains, including full length antibodies, which contain a number of specific, enumerated sets of 6 CDRs, including for use in the methods of the invention. *See*, **Figures 64** and **80****-81**. In some emboidments, the CDRs are those in **Figure 64**, as well as **Tables 1-14**. In some embodiments, the CDRs are those in **Figure 81**, as well as **Tables 15-17**.

As above, these sets of CDRs may also be amino acid variants as described above.

In addition, the framework regions of the variable heavy and variable light chains can be humanized as is known in the art (with occasional variants generated in the CDRs as needed), and thus humanized variants of the V_{H} and V_{L} chains of **Figures 64** and **80-81** can be generated (see, also, Tables 1-17). Furthermore, the humanized variable heavy and light domains can then be fused with human constant regions, such as the constant regions from IgG1, IgG2, IgG3 and IgG4.

In particular, as is known in the art, murine V_{H} and V_{L} chains can be humanized as is known in the art, for example, using the IgBLAST program of the NCBI website, as outlined in Ye et al. Nucleic Acids Res. 41:W34-W40 (2013). IgBLAST takes a murine VH and/or VL sequence and compares it to a library of known human germline sequences. As shown herein, for the humanized sequences, the databases that can be used are IMGT human V_{H} genes (F+ORF, 273 germline sequences) and IMGT human V_{L} kappa genes (F+ORF, 74 germline sequences). CDRs were and will be defined according to the AbM definition (see, the World Wide Web at bioinfo.org.uk/abs).

Specific humanized antibodies of CPA antibodies include those shown in **Figure 64**, as well as **Tables 1-14** above. As will be appreciated by those in the art, each humanized variable heavy (Humanized Heavy; HH) and variable light (Humanized Light, HL) sequence can be combined with the constant regions of human IgG1, IgG2, IgG3 and IgG4 in order to generate first, second , *etc*., humanized sequences.

In some embodiments, the anti-HIDE1 antibodies of the present invention include anti-HIDE1 antibodies wherein the V_{H} and V_{L} sequences of different anti-HIDE1 antibodies can be "mixed and matched" to create other anti-HIDE1 antibodies. HIDE1 binding of such "mixed and matched" antibodies can be tested using the binding assays described above. *e.g*., ELISAs). In some embodiments, when V_{H} and V_{L} chains are mixed and matched, a V_{H} sequence from a particular V_{H}/V_{L} pairing is replaced with a structurally similar V_{H} sequence. Likewise, in some embodiments, a V_{L} sequence from a particular V_{H}/V_{L} pairing is replaced with a structurally similar V_{L} sequence. For example, the V_{H} and V_{L} sequences of homologous antibodies are particularly amenable for mixing and matching.

Accordingly, the antibodies of the invention comprise CDR amino acid sequences selected from the group consisting of (a) sequences as listed herein; (b) sequences that differ from those CDR amino acid sequences specified in (a) by 1, 2, 3, 4, 5, 6, 7, 8, 9, 10 or more amino acid substitutions; (c) amino acid sequences having 90% or greater, 95% or greater, 98% or greater, or 99% or greater sequence identity to the sequences specified in (a) or (b); (d) a polypeptide having an amino acid sequence encoded by a polynucleotide having a nucleic acid sequence encoding the amino acids as listed herein.

Additionally included in the definition of HIDE1 antibodies are antibodies that share identity to the HIDE1 antibodies enumerated herein. That is, in certain embodiments, an anti-HIDE1 antibody according to the invention comprises heavy and light chain variable regions comprising amino acid sequences that are homologous to isolated anti-HIDE1 amino acid sequences of preferred anti-HIDE1 immune molecules, respectively, wherein the antibodies retain the desired functional properties of the parent anti-HIDE1 antibodies. The percent identity between the two sequences is a function of the number of identical positions shared by the sequences (*i.e*., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, in some embodiments the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e.g*., XBLAST and NBLAST) can be used.

In general, the percentage identity for comparison between HIDE1 antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-HIDE1 antibodies of the invention are those that share identity along the entire variable region (for example, where the identity is 95 or 98% identical along the variable regions), or along the entire constant region, or along just the Fc domain.

In addition, also included are sequences that may have the identical CDRs but changes in the variable domain (or entire heavy or light chain). For example, HIDE1 antibodies of the invention and/or for use in the invention, include those with CDRs identical to those shown in **Figure 64** and **80****-81, as well as Tables 1-17**, but whose identity along the variable region can be lower, for example 95 or 98% percent identical.

According to at least some embodiments of the present invention provides not only the enumerated antibodies but additional antibodies that compete with the enumerated antibodies (the **Figure 64** and **80****-81** numbers enumerated herein that specifically bind to HIDE1) to specifically bind to the HIDE1 molecule. As is shown above and in **Figures 64** and **80****-81, as well as Tables 1-17.**

### 1. Generation of Additional Antibodies

Additional antibodies to human HIDE1 can be done as is well known in the art, using well known methods such as those outlined in the examples. Thus, additional anti-HIDE1 antibodies can be generated by traditional methods such as immunizing mice (sometimes using DNA immunization, for example, such as is used by Aldevron), followed by screening against human HIDE1 protein and hybridoma generation, with antibody purification and recovery.

### D. Formulations of Anti-HIDEl Antibodies

The therapeutic compositions used in the practice of the foregoing methods can be formulated into pharmaceutical compositions comprising a carrier suitable for the desired delivery method. Suitable carriers include any material that when combined with the therapeutic composition retains the anti-tumor function of the therapeutic composition and is generally non-reactive with the patient's immune system. Examples include, but are not limited to, any of a number of standard pharmaceutical carriers such as sterile phosphate buffered saline solutions, bacteriostatic water, and the like (see, generally, Remington's Pharmaceutical Sciences 16th Edition, A. Osal., Ed., 1980). Acceptable carriers, excipients, or stabilizers are nontoxic to recipients at the dosages and concentrations employed, and include buffers such as phosphate, citrate, acetate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; sweeteners and other flavoring agents; fillers such as microcrystalline cellulose, lactose, corn and other starches; binding agents; additives; coloring agents; salt-forming counter-ions such as sodium; metal complexes (e.g. Zn-protein complexes); and/or non-ionic surfactants such as TWEEN™, PLURONICS™ or polyethylene glycol (PEG).

A pharmaceutical composition that comprises the antibodies of the invention may be in a water-soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The formulations to be used for *in vivo* administration are preferrably sterile. This is readily accomplished by filtration through sterile filtration membranes or other methods.

Administration of the pharmaceutical composition comprising antibodies of the present invention, preferably in the form of a sterile aqueous solution, may be done in a variety of ways, including, but not limited to subcutaneously and intravenously. Subcutaneous administration may be preferable in some circumstances because the patient may self-administer the pharmaceutical composition. Many protein therapeutics are not sufficiently potent to allow for formulation of a therapeutically effective dose in the maximum acceptable volume for subcutaneous administration. This problem may be addressed in part by the use of protein formulations comprising arginine-HCl, histidine, and polysorbate (see WO 04091658). In some embodiments, Fc polypeptides of the present invention may be more amenable to subcutaneous administration due to, for example, increased potency, improved serum half-life, or enhanced solubility.

As is known in the art, protein therapeutics are often delivered by IV infusion or bolus. In some embodiments, the antibodies of the present invention may also be delivered using such methods. For example, administration may venious be by intravenous infusion with 0.9% sodium chloride as an infusion vehicle.

In addition, any of a number of delivery systems are known in the art and may be used to administer the Fc variants of the various embodiments of the present invention. Examples include, but are not limited to, encapsulation in liposomes, microparticles, microspheres (eg. PLA/PGA microspheres), and the like. Alternatively, an implant of a porous, non-porous, or gelatinous material, including membranes or fibers, may be used. Sustained release systems may comprise a polymeric material or matrix such as polyesters, hydrogels, poly(vinylalcohol), polylactides, copolymers of L-glutamic acid and ethyl-L-gutamate, ethylene-vinyl acetate, lactic acid-glycolic acid copolymers such as the LUPRON DEPOT.RTM., and poly-D-(-)-3-hydroxyburyric acid. The antibodies disclosed herein may also be formulated as immunoliposomes. A liposome is a small vesicle comprising various types of lipids, phospholipids and/or surfactant that is useful for delivery of a therapeutic agent to a mammal. Liposomes containing the antibody are prepared by methods known in the art, such as described in Epstein et al., 1985, Proc Natl Acad Sci USA, 82:3688; Hwang et al., 1980, Proc Natl Acad Sci USA, 77:4030; U.S. Pat. No. 4,485,045; U.S. Pat. No. 4,544,545; and PCT WO 97/38731. Liposomes with enhanced circulation time are disclosed in U.S. Pat. No. 5,013,556. The components of the liposome are commonly arranged in a bilayer formation, similar to the lipid arrangement of biological membranes. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. A chemotherapeutic agent or other therapeutically active agent is optionally contained within the liposome (Gabizon et al., 1989, J National Cancer Inst 81:1484).

The antibodies may also be entrapped in microcapsules prepared by methods including but not limited to coacervation techniques, interfacial polymerization (for example using hydroxymethylcellulose or gelatin-microcapsules, or poly-(methylmethacylate) microcapsules), colloidal drug delivery systems (for example, liposomes, albumin microspheres, microemulsions, nano-particles and nanocapsules), and macroemulsions. Such techniques are disclosed in Remington's Pharmaceutical Sciences 16th edition, Osol, A. Ed., 1980. Sustained-release preparations may be prepared. Suitable examples of sustained-release preparations include semipermeable matrices of solid hydrophobic polymer, which matrices are in the form of shaped articles, *e.g*. films, or microcapsules. Examples of sustained-release matrices include polyesters, hydrogels (for example poly(2-hydroxyethyl-methacrylate), or poly(vinylalcohol)), polylactides (U.S. Pat. No. 3,773,919), copolymers of L-glutamic acid and gamma ethyl-L-glutamate, non-degradable ethylene-vinyl acetate, degradable lactic acid-glycolic acid copolymers such as the LUPRON DEPOT.RTM. (which are injectable microspheres composed of lactic acid-glycolic acid copolymer and leuprolide acetate), poly-D-(-)-3-hydroxybutyric acid, and ProLease.RTM. (commercially available from Alkermes), which is a microsphere-based delivery system composed of the desired bioactive molecule incorporated into a matrix of poly-DL-lactide-co-glycolide (PLG).

The dosing amounts and frequencies of administration are, in a preferred embodiment, selected to be therapeutically or prophylactically effective. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art.

The concentration of the antibody in the formulation may vary from about 0.1 to 100 weight %. In a preferred embodiment, the concentration of the Fc variant is in the range of 0.003 to 1.0 molar. In order to treat a patient, in some embodiments a therapeutically effective dose of the Fc variant of the present invention may be administered. By "therapeutically effective dose" herein is meant a dose that produces the effects for which it is administered. The exact dose will depend on the purpose of the treatment, and will be ascertainable by one skilled in the art using known techniques. Dosages may range from 0.0001 to 100 mg/kg of body weight or greater, for example 0.1, 1, 10, or 50 mg/kg of body weight, with 1 to 10 mg/kg being preferred.

### E. Methods of Using Anti-HIDEl Antibodies Compositions

### 1. Therapeutic Uses

A "therapeutically effective dosage" of HIDE1 soluble protein or HIDE1 ectodomain or fusion protein containing same, according to at least some embodiments of the present invention preferably results in a decrease in severity of disease symptoms, an increase in frequency and duration of disease symptom-free periods, an increase in lifespan, disease remission, or a prevention or reduction of impairment or disability due to the disease affliction. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject.

One of ordinary skill in the art would be able to determine a therapeutically effective amount based on such factors as the subject's size, the severity of the subject's symptoms, and the particular composition or route of administration selected.

The anti-HIDE1 antibodies of the invention find use in treating patients, such as human subjects, generally with a condition associated with HIDE1. The term "treatment" as used herein, refers to both therapeutic treatment and prophylactic or preventative measures, which relates to treatment of cancer. Those in need of treatment include those already with cancer as well as those in which the cancer is to be prevented. Hence, the mammal to be treated herein may have been diagnosed as having the cancer or may be predisposed or susceptible to the cancer. As used herein the term "treating" refers to preventing, delaying the onset of, curing, reversing, attenuating, alleviating, minimizing, suppressing, halting the deleterious effects or stabilizing of discernible symptoms of the above-described cancerous diseases, disorders or conditions. It also includes managing the cancer as described above. By "manage" it is meant reducing the severity of the disease, reducing the frequency of episodes of the disease, reducing the duration of such episodes, reducing the severity of such episodes, slowing/reducing cancer cell growth or proliferation, slowing progression of at least one symptom, amelioration of at least one measurable physical parameter and the like. For example, immunostimulatory anti-HIDE1 immune molecules should promote myeloid cell, T cell or NK or cytokine immunity against target cells, *i.e.* cancer cells and thereby treat cancer by depleting the cells involved in the disease condition.

The HIDE1 antibodies of the invention are provided in therapeutically effective dosages. A "therapeutically effective dosage" of an anti-HIDE1 immune molecule according to at least some embodiments of the present invention preferably results in a decrease in severity of disease symptoms, an increase in frequency and duration of disease symptom-free periods, an increase in lifespan, disease remission, or a prevention or reduction of impairment or disability due to the disease affliction. For example, for the treatment of HIDE1 positive tumors (including, for example tumors that express HIDE1 on the cellular membrane as well as tumors that express HIDE 1 in the tumor microenvironment, also referred to as the TME), a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject.

One of ordinary skill in the art would be able to determine a therapeutically effective amount based on such factors as the subject's size, the severity of the subject's symptoms, and the particular composition or route of administration selected.

### a. Cancer Treatment

The HIDE1 antibodies of the invention find use in the treatment of cancer. In general, the antibodies of the invention are immunomodulatory, in that rather than directly attack cancerous cells, the anti-HIDE1 antibodies of the invention stimulate the immune system, generally by inhibiting the action of HIDE1. Thus, unlike tumor-targeted therapies, which are aimed at inhibiting molecular pathways that are crucial for tumor growth and development, and/or depleting tumor cells, cancer immunotherapy is aimed to stimulate the patient's own immune system to eliminate cancer cells, providing long-lived tumor destruction. Various approaches can be used in cancer immunotherapy, among them are therapeutic cancer vaccines to induce tumor-specific T cell responses, and immunostimulatory antibodies (*i.e*., antagonists of inhibitory receptors = immune checkpoints) to remove immunosuppressive pathways.

Clinical responses with targeted therapy or conventional anti-cancer therapies tend to be transient as cancer cells develop resistance, and tumor recurrence takes place. However, the clinical use of cancer immunotherapy in the past few years has shown that this type of therapy can have durable clinical responses, showing dramatic impact on long term survival. However, although responses are long term, only a small number of patients respond (as opposed to conventional or targeted therapy, where a large number of patients respond, but responses are transient).

By the time a tumor is detected clinically, it has already evaded the immune-defense system by acquiring immunoresistant and immunosuppressive properties and creating an immunosuppressive tumor microenvironment through various mechanisms and a variety of immune cells.

Accordingly, the anti-HIDE1 antibodies of the invention are useful in treating cancer. Due to the nature of an immuno-oncology mechanism of action, HIDE1 does not necessarily need to be overexpressed on or correlated with a particular cancer type; that is, the goal is to have the anti-HIDE1 antibodies de-suppress myeloid, T cell and NK cell activation, such that the immune system will go after the cancers.

"Cancer," as used herein, refers broadly to any neoplastic disease (whether invasive or metastatic) characterized by abnormal and uncontrolled cell division causing malignant growth or tumor (e.g., unregulated cell growth.) The term "cancer" or "cancerous" as used herein should be understood to encompass any neoplastic disease (whether invasive, non-invasive or metastatic) which is characterized by abnormal and uncontrolled cell division causing malignant growth or tumor, non-limiting examples of which are described herein. This includes any physiological condition in mammals that is typically characterized by unregulated cell growth. Examples of cancer are exemplified in the working examples and also are described within the specification.

Non-limiting examples of cancer that can be treated using anti-HIDE1 antibodies include, but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; multiple myeloma and post-transplant lymphoproliferative disorder (PTLD).

In some embodiments, other cancers amenable for treatment by the present invention include, but are not limited to, carcinoma, lymphoma, blastoma, sarcoma, and leukemia or lymphoid malignancies. More particular examples of such cancers include colorectal, bladder, ovarian, melanoma, squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome. Preferably, the cancer is selected from the group consisting of colorectal cancer, breast cancer, rectal cancer, non-small cell lung cancer, non-Hodgkin's lymphoma (NHL), renal cell cancer, prostate cancer, liver cancer, pancreatic cancer, soft-tissue sarcoma, Kaposi's sarcoma, carcinoid carcinoma, head and neck cancer, melanoma, ovarian cancer, mesothelioma, and multiple myeloma. In an exemplary embodiment the cancer is an early or advanced (including metastatic) bladder, ovarian or melanoma. In another embodiment the cancer is colorectal cancer. In some embodiments, the cancerous conditions amenable for treatment of the present invention include cancers that express or do not express HIDE1 and further include non-metastatic or non-invasive as well as invasive or metastatic cancers wherein HIDE1 expression by immune, stromal or diseased cells suppress antitumor responses and anti-invasive immune responses. The method of the present invention is particularly suitable for the treatment of vascularized tumors.

As shown in the Examples, HIDE1 is over expressed and/or correlates with tumor leukocyte infiltration (as demonstrated by correlation to CSFR1,CD68, CD86, CD11b, IL-4, IL-10, IL-13 and IFN-γ expression) in a number of different tumors of various origins, and thus is useful in treating any cancer, including but not limited to, prostate cancer, liver cancer (HCC), colorectal cancer, ovarian cancer, endometrial cancer, breast cancer, pancreatic cancer, stomach cancer, cervical cancer, head and neck cancer, thyroid cancer, testis cancer, urothelial cancer, lung cancer, melanoma, non melanoma skin cancer (squamous and basal cell carcinoma), glioma, renal cancer (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma and esophageal cancer. In some embodiments, the tumors are myeloid or circulating tumor types. In some embodiments, the cancer includes but is not limited to Acute Myeloid Leukemia, Acute Myeloid Leukemia Induction Failure, Acute Lymphoblastic Leukemia, Diffuse Large B-cell Lymphoma, Malignant Lymphoma, Non-Hodgkin Lymphoma, Diffuse Large B-Cell Lymphoma, Glioblastoma multiforme, Mesothelioma, Thymoma, Testicular Germ Cell Tumors, Kidney renal clear cell carcinoma, Sarcoma, Brain Lower Grade Glioma, Chronic Lymphocytic Leukemia, Non-Hodgkin Lymphoma - Follicular Lymphoma, Uterine Carcinosarcoma, Pediatric Brain Tumors, Lung adenocarcinoma, Cervical squamous cell carcinoma, endocervical adenocarcinoma, Pancreatic adenocarcinoma, Skin Cutaneous Melanoma, Kidney renal papillary cell carcinoma, Liver hepatocellular carcinoma; Bladder Urothelial Carcinoma, Colon adenocarcinoma, Head and Neck squamous cell carcinoma, Lung squamous cell carcinoma, Rectum adenocarcinoma, and Stomach adenocarcinoma.

"Cancer therapy" herein refers to any method which prevents or treats cancer or ameliorates one or more of the symptoms of cancer. Typically such therapies will comprises administration of immunostimulatory anti-HIDE1 antibodies (including antigen-binding fragments) either alone or in combination with chemotherapy or radiotherapy or other biologics and for enhancing the activity thereof, *i.e.,* in individuals wherein expression of HIDE1 suppresses antitumor responses and the efficacy of chemotherapy or radiotherapy or biologic efficacy.

In some embodiments, the cancer for treatment is a cancer having high immune infiltrate of myeloid cells expressing HIDE1 wherein said cancer could be treated by administering HIDE1 antibodies. In some embodiments, said cancer is a myeloid cancer. In some embodiments, the myeloid cells include but are not limited to monocytes, dendritic cells, macrophages, M1/M2 tumor associated macrophages, neutrophils, and myeloid-derive suppressor cells (MDSC). In some embodiments, the anti-HIDE1 antibody for use in cancer treatment is a depleting HIDE1 antibody. In some embodiments, a depleting anti-HIDE1 antibody binds to cell surface HIDE1. In some embodiments, the anti HIDE1 depleting antibody preferably is able to deplete HIDE1 expressing cells including but not limited to monocytes, dendritic cells, macrophages, M1/M2 tumor associated macrophages, neutrophils, Myeloid-derive suppressor cells (MDSC) and as a result reduce the number of HIDE1 expressing cells in a patient treated with the anti HIDE1 depleting antibody. Such depletion may be achieved via various mechanisms such as antibody-dependent cell mediated cytotoxicity (ADCC) and/or complement dependent cytotoxicity (CDC), inhibition of HIDE1 expressing cells proliferation and/or induction of HIDE1+ cell death (*e.g.,* via apoptosis).

### b. Assessment of Treatment

Generally the anti-HIDE1 antibodies of the invention are administered to patients with cancer, and efficacy is assessed, in a number of ways as described herein. Thus, while standard assays of efficacy can be run, such as cancer load, size of tumor, evaluation of presence or extent of metastasis, etc., immuno-oncology treatments can be assessed on the basis of immune status evaluations as well. This can be done in a number of ways, including both *in vitro* and *in vivo* assays. For example, evaluation of changes in immune status (*e.g.* presence of ICOS+ CD4+ T cells following ipi treatment) along with "old fashioned" measurements such as tumor burden, size, invasiveness, LN involvement, metastasis, etc. can be done. Thus, any or all of the following can be evaluated: the inhibitory effects of HIDE1 on CD4⁺ T cell activation or proliferation, CD8⁺ T (CTL) cell activation or proliferation, CD8⁺ T cell-mediated cytotoxic activity and/or CTL mediated cell depletion, NK cell activity and NK mediated cell depletion, the potentiating effects of HIDE1 on Treg cell differentiation and proliferation and Treg- or myeloid derived suppressor cell (MDSC)-mediated immunosuppression or immune tolerance, and/or the effects of HIDE1 on proinflammatory cytokine production by immune cells, *e.g.,* IL-2, IFN-γ or TNF-α production by T or other immune cells.

In some aspects of the disclosure, assessment of treatment is done by evaluating immune cell proliferation, using for example, CFSE dilution method, Ki67 intracellular staining of immune effector cells, and 3^{H}-Thymidine incorporation method,

In some aspects of the disclosure, assessment of treatment is done by evaluating the increase in gene expression or increased protein levels of activation-associated markers, including one or more of: CD25, CD69, CD137, ICOS, PD1, GITR, OX40, and cell degranulation measured by surface expression of CD107A.

In general, gene expression assays are done as is known in the art. See for example Goodkind et al., Computers and Chem. Eng. 29(3):589 (2005), Han et al., Bioinform. Biol. Insights 11/15/15 9(Suppl. 1):29-46, Campo et al., Nod. Pathol. 2013 Jan; 26 suppl. 1:S97-S110.

In general, protein expression measurements are also similarly done as is known in the art, see for example, Wang et al., Recent Advances in Capillary Electrophoresis-Based Proteomic Techniques for Biomarker Discovery, Methods. Mol. Biol. 2013:984:1-12; Taylor et al., BioMed Res. Volume 2014, Article ID 361590, 8 pages, Becerk et al., Mutat. Res 2011 June 17:722(2): 171-182.

In some aspects of the disclosure, assessment of treatment is done by assessing cytotoxic activity measured by target cell viability detection via estimating numerous cell parameters such as enzyme activity (including protease activity), cell membrane permeability, cell adherence, ATP production, co-enzyme production, and nucleotide uptake activity. Specific examples of these assays include, but are not limited to, Trypan Blue or PI staining, ⁵¹Cr or ³⁵S release method, LDH activity, MTT and/or WST assays, Calcein-AM assay, Luminescent based assay, and others.

In some aspects of the disclosures, assessment of treatment is done by assessing T cell activity measured by cytokine production, measure either intracellularly in culture supernatant using cytokines including, but not limited to, IFNγ, TNFα, GM-CSF, IL2, IL6, IL4, IL5, IL10, IL13 using well known techniques.

Accordingly, assessment of treatment can be done using assays that evaluate one or more of the following: (i) increases immune response, (ii) increases T cell activity, (iii) increases activation of αβ and/or γδ T cells, (iv) increases cytotoxic T cell activity, (v) increases NK and/or NKT cell activity, (vi) alleviates αβ and/or γδ T-cell suppression, (vii) increases pro-inflammatory cytokine secretion, (viii) increases IL-2 secretion; (vix) increases interferon-y production, (x) increases Th1 response, (xi) decrease Th2 response, (xii) decreases or eliminates cell number and/or activity of at least one of regulatory T cells (Tregs), myeloid derived suppressor cells (MDSCs), iMCs, mesenchymal stromal cells, TIE2-expressing monocytes, (xiii) reduces regulatory cell activity, and/or the activity of one or more of myeloid derived suppressor cells (MDSCs), iMCs, mesenchymal stromal cells, TIE2-expressing monocytes, (xiv) decreases or eliminates M2 macrophages, (xv) reduces M2 macrophage pro-tumorigenic activity, (xvi) decreases or eliminates N2 neutrophils, (xvii) reduces N2 neutrophils pro-tumorigenic activity, (xviii) reduces inhibition of T cell activation, (xix) reduces inhibition of CTL activation, (xx) reduces inhibition of NK and/or NKT cell activation, (xxi) reverses αβ and/or γδ T cell exhaustion, (xxii) increases αβ and/or γδ T cell response, (xxiii) increases activity of cytotoxic cells, (xxiv) stimulates antigen-specific memory responses, (xxv) elicits apoptosis or lysis of cancer cells, (xxvi) stimulates cytotoxic or cytostatic effect on cancer cells, (xxvii) induces direct killing of cancer cells, (xxviii) increases Th17 activity and/or (xxix) modulating myeloid cell polarization, (xxx) modulating myeloid cell shifting toward a pro-inflammatory response, (xxxi) shifting myeloid from M2 toward M1 phenotype, (xxxii) modulating myeloid cell in the TME to support anti-cancer immune response, (xxxiii) restricting the pro-tumorigenic effects of the myeloid cells in the TME, (xxxiv) enhancing myeloid and lymphoid infiltration into the tumor cite thereby shifting the tumor into more immunogenic, (xxxv) induces complement dependent cytotoxicity and/or antibody dependent cell-mediated cytotoxicity.

### 2. Combinations

As is known in the art, combination therapies comprising a therapeutic antibody targeting an immunotherapy target and an additional therapeutic agent, specific for the disease condition, are showing great promise. For example, in the area of immunotherapy, there are a number of promising combination therapies using a chemotherapeutic agent (either a small molecule drug or an anti-tumor antibody) with immuno-oncology antibodies like anti-PD-1, and as such, the anti-HIDE1 antibodies outlined herein can be substituted in the same way. Any chemotherapeutic agent exhibiting anticancer activity can be used according to various embodiments of the present invention; various non-limiting examples are described in the specification.

The underlying scientific rationale for the dramatic increased efficacy of combination therapy claims that immune checkpoint blockade as a monotherapy will induce tumor regressions only when there is pre-existing strong anti-tumor immune response to be "unleashed" when the pathway is blocked. However, in most patients and tumor types the endogenous anti-tumor immune responses are weak, and thus the induction of anti-tumor immunity is required for the immune checkpoint blockade to be effective, as shown in the **Figure 1****.** According to at least some embodiments of the present invention, HIDE1-specific antibodies, antibody fragments, conjugates and compositions comprising same, are used for treatment of all types of cancer in cancer immunotherapy in combination therapy.

The terms "in combination with" and "co-administration" are not limited to the administration of said prophylactic or therapeutic agents at exactly the same time. Instead, it is meant that the anti-HIDE1 antibody and the other agent or agents are administered in a sequence and within a time interval such that they may act together to provide a benefit that is increased versus treatment with only either anti-HIDE1 antibody of the various embodiments of the present invention or the other agent or agents. It is preferred that the anti-HIDE1 antibody and the other agent or agents act additively, and especially preferred that they act synergistically. Such molecules are suitably present in combination in amounts that are effective for the purpose intended. The skilled medical practitioner can determine empirically, or by considering the pharmacokinetics and modes of action of the agents, the appropriate dose or doses of each therapeutic agent, as well as the appropriate timings and methods of administration.

Accordingly, in some embodiments, the antibodies of the present invention may be administered concomitantly with one or more other therapeutic regimens or agents. The additional therapeutic regimes or agents may be used to improve the efficacy or safety of the anti-HIDE1 antibody. Also, the additional therapeutic regimes or agents may be used to treat the same disease or a comorbidity rather than to alter the action of the HIDE1 antibody. For example, a HIDE1 antibody of the various embodiments of the present invention may be administered to the patient along with chemotherapy, radiation therapy, or both chemotherapy and radiation therapy.

The HIDE1 antibodies of the various embodiments of the present invention may be administered in combination with one or more other prophylactic or therapeutic agents, including but not limited to cytotoxic agents, chemotherapeutic agents, cytokines, growth inhibitory agents, anti-hormonal agents, kinase inhibitors, anti-angiogenic agents, cardioprotectants, immunostimulatory agents, immunosuppressive agents, agents that promote proliferation of hematological cells, angiogenesis inhibitors, protein tyrosine kinase (PTK) inhibitors, or other therapeutic agents.

According to at least some embodiments, the anti-HIDE1 immune molecules could be used in combination with any of the known in the art standard of care cancer treatment (as can be found, for example, on the World Wide Web at cancer. gov/cancertopics).

In some embodiments, conventional/classical anti-cancer agents suitable for use with the present invention include but are not limited to platinum based compounds, antibiotics with anti-cancer activity, Anthracyclines, Anthracenediones, alkylating agents, antimetabolites, Antimitotic agents, Taxanes, Taxoids, microtubule inhibitors, Folate antagonists and/or folic acid analogs, Topoisomerase inhibitors, Aromatase inhibitors, GnRh analogs, inhibitors of 5α-reductase, bisphosphonates; pyrimidine analogs, purine analogs and related inhibitors, vinca alkaloids, epipodophyllotoxins, antibiotics, L-Asparaginase, topoisomerase inhibitor, interferons, platinum coordination complexes, anthracenedione substituted urea, methyl hydrazine derivatives, adrenocortical suppressant, adrenocorticosteroids, progestins, estrogens, antiestrogen, androgens, antiandrogen, and gonadotropin-releasing hormone analog.

Specific but non-limiting examples of these categories of drugs are as follows: platinum based compounds such as oxaliplatin, cisplatin, carboplatin; Antibiotics with anti-cancer activity, such as dactinomycin, bleomycin, mitomycin-C, mithramycin and Anthracyclines, such as doxorubicin, daunorubicin, epirubicin, idarubicin; Anthracenediones, such as mitoxantrone; Alkylating agents, such as dacarbazine, melphalan, cyclophosphamide, temozolomide, chlorambucil, busulphan, nitrogen mustard, nitrosoureas; Antimetabolites, such as fluorouracil, raltitrexed, gemcitabine, cytosine arabinoside, hydroxyurea and Folate antagonists, such as methotrexate, trimethoprim, pyrimethamine, pemetrexed; Antimitotic agents such as polokinase inhibitors and Microtubule inhibitors, such as Taxanes and Taxoids, such as paclitaxel, docetaxel; Vinca alkaloids such as vincristine, vinblastine, vindesine, vinorelbine; Topoisomerase inhibitors, such as etoposide, teniposide, amsacrine, topotecan, irinotecan, camptothecin; Cytostatic agents including Antiestrogens such as tamoxifen, fulvestrant, toremifene, raloxifene, droloxifene, iodoxyfene, Antiandrogens such as bicalutamide, flutamide, nilutamide and cyproterone acetate, Progestogens such as megestrol acetate, Aromatase inhibitors such as anastrozole, letrozole, vorozole, exemestane; GnRH analogs, such as leuprorelin, goserelin, buserelin, degarelix; inhibitors of 5α-reductase such as finasteride.

More preferably, the chemotherapeutic agent is selected from the group consisting of 5-fluorouracil (5-FU), leucovorin (LV), irenotecan, oxaliplatin, capecitabine, paclitaxel and doxetaxel. Two or more chemotherapeutic agents can be used in a cocktail to be administered in combination with administration of the anti-VEGF antibody. One preferred combination chemotherapy is fluorouracil-based, comprising 5-FU and one or more other chemotherapeutic agent(s). Suitable dosing regimens of combination chemotherapies are known in the art and described in, for example, Saltz et al. (1999) Proc ASCO 18:233a and Douillard et al. (2000) Lancet 355:1041-7. The biologic may optionally be another immune potentiators such as antibodies to PD-L1, PD-L2, CTLA-4, or VISTA as well as PD-L1, PD-L2, CTLA-4 or VISTA fusion proteins as well as cytokines, growth factor antagonists and agonists, hormones and anti-cytokine antibodies.

According to at least some embodiments of the present invention, targeted therapies used as agents for combination with anti HIDE1 antibodies for treatment of cancer are selected from the group consisting of but not limited to: histone deacetylase (HDAC) inhibitors, such as vorinostat, romidepsin, panobinostat, belinostat, mocetinostat, abexinostat, entinostat, resminostat, givinostat, quisinostat, sodium butyrate; Proteasome inhibitors, such as bortezomib, carfilzomib, disulfiram; mTOR pathway inhibitors, such as temsirolimus, rapamycin, everolimus; PI3K inhibitors, such as perifosine, CAL101, PX-866, IPI-145, BAY 80-6946; B-raf inhibitors such as vemurafenib, sorafenib; JAK2 inhibitors, such as lestaurtinib, pacritinib; tyrosine kinase inhibitors (TKIs), such as erlotinib, imatinib, sunitinib, lapatinib, gefitinib, sorafenib, nilotinib, toceranib, bosutinib, neratinib, vatalanib, regorafenib, cabozantinib; other protein kinase inhibitors, such as crizotinib; inhibitors of serine/threonine kinases for example Ras/Raf signalling inhibitors such as farnesyl transferase inhibitors; inhibitors of serine proteases for example matriptase, hepsin, urokinase; inhibitors of intracellular signaling such as tipifarnib, perifosine; Inhibitors of cell signalling through MEK and/or AKT kinases; aurora kinase inhibitors such as AZD1152, PH739358, VX-680, MLN8054, R763, MP235, MP529, VX-528, AX39459; cyclin dependent kinase inhibitors such as CDK2 and/or CDK4 inhibitors; inhibitors of survival signaling proteins including Bcl-2, Bcl-XL, such as ABT-737; HSP90 inhibitors; therapeutic monoclonal antibodies, such as anti-EGFR mAbs cetuximab, panitumumab, nimotuzumab, anti-ERBB2 mAbs trastuzumab, pertuzumab, anti-CD20 mAbs such as rituximab, ofatumumab, veltuzumab and mAbs targeting other tumor antigens such as alemtuzumab, labetuzumab, adecatumumab, oregovomab, onartuzumab; TRAIL pathway agonists, such as dulanermin (soluble rhTRAIL), apomab, mapatumumab, lexatumumab, conatumumab, tigatuzumab; antibody fragments, bi-specific antibodies and bi-specific T-cell engagers (BiTEs), such as catumaxomab, blinatumomab; antibody drug conjugates (ADC) and other immunoconjugates, such as ibritumomab triuxetan, tositumomab, brentuximab vedotin, gemtuzumab ozogamicin, clivatuzumab tetraxetan, pemtumomab, trastuzumab emtansine; anti-angiogenic therapy such as bevacizumab, etaracizumab, volociximab, ramucirumab, aflibercept, sorafenib, sunitinib, regorafenib, axitinib, nintedanib, motesanib, pazopanib, cediranib; metalloproteinase inhibitors such as marimastat; inhibitors of urokinase plasminogen activator receptor function; inhibitors of cathepsin activity.

Other therapeutic antibodies which may optionally be used in combination with an immunostimulatory antibody according to at least some embodiments of the present invention include but are not limited to cetuximab, panitumumab, nimotuzumab, trastuzumab, pertuzumab, rituximab, ofatumumab, veltuzumab, alemtuzumab, labetuzumab, adecatumumab, oregovomab, onartuzumab; apomab, mapatumumab, lexatumumab, conatumumab, tigatuzumab, catumaxomab, blinatumomab, ibritumomab triuxetan, tositumomab, brentuximab vedotin, gemtuzumab ozogamicin, clivatuzumab tetraxetan, pemtumomab, trastuzumab emtansine, bevacizumab, etaracizumab, volociximab, ramucirumab, aflibercept.

Therapeutic agents targeting immunosuppressive cells such as Tregs and/or MDSCs which may optionally be used in combination with an immunostimulatory antibody according to at least some embodiments of the present invention include but are not limited to antimitotic drugs, cyclophosphamide, gemcitabine, mitoxantrone, fludarabine, thalidomide, thalidomide derivatives, COX-2 inhibitors, depleting or killing antibodies that directly target Tregs through recognition of Treg cell surface receptors, anti-CD25 daclizumab, basiliximab, ligand-directed toxins, denileukin diftitox (Ontak), a fusion protein of human IL-2 and diphtheria toxin, or LMB-2, a fusion between an scFv against CD25 and the pseudomonas exotoxin, antibodies targeting Treg cell surface receptors, TLR modulators, agents that interfere with the adenosinergic pathway, ectonucleotidase inhibitors, or inhibitors of the A2A adenosine receptor, TGF-β inhibitors, chemokine receptor inhibitors, retinoic acid, all-trans retinoic acid (ATRA), Vitamin D3, phosphodiesterase 5 inhibitors, sildenafil, ROS inhibitors and nitroaspirin.

Other immunostimulatory antibodies which may according to at least some embodiments optionally be used in combination with an immunostimulatory antibody according to at least some embodiments of the present invention include but are not limited to agonistic or antagonistic antibodies targeting one or more of CTLA4, PD-1, PDL-1, LAG-3, TIM-3, BTLA, B7-H4, B7-H3, VISTA, and/or agonistic or antagonistic antibodies targeting one or more of CD40, CD137, OX40, GITR, CD27, CD28 or ICOS, or fusion proteins containing any of the foregoing or fragments thereof which function as immune agonists or antagonists.

As described *infra,* without wishing to be limited by a single hypothesis, the HIDE1 protein apparently interacts with a receptor expressed by NK cells. Accordingly, the subject immunostimulatory antibody or immunostimulatory antigen-binding fragments may optionally be used on combination or coupled to an antibody or antigen-binding fragment thereof, or other moiety which specifically binds to an NK cell receptor. Such moieties which specifically bind to an NK cell receptor may optionally agonize or antagonize the effect of said NK cell receptor. Various non-limiting examples are given herein. Such NK receptors include those of unknown function, as well as those known to inhibit NK cell activity such as KIR2DL1, KIR2DL2/3, KIR2DL4, KIR2DL5A, KIR2DL5B, KIR3DL1, KIR3DL2, KIR3DL3, LILRB1, NKG2A, NKG2C, NKG2E and LILRB5 and those known to promote or activate NK cell activity such as NKp30, NKp44, NKp46, NKp46, NKG2D, KIR2DS4 CD2, CD16, CD69, DNAX accessory molecule-1 (DNAM-1), 2B4, NK1.1; a killer immunoglobulin (Ig)-like activating receptors (KAR); ILTs/LIRs; NKRP-1, CD69; CD94/NKG2C and CD94/NKG2E heterodimers, NKG2D homodimer KIR2DS and KIR3DS.

Therapeutic cancer vaccines may also optionally be used in combination with an immunostimulatory antibody or fragment thereof according to at least some embodiments of the present invention, including but not limited to exogenous cancer and infectious agent vaccines including proteins or peptides used to mount an immunogenic response to a tumor antigen or an infectious agent, recombinant virus and bacteria vectors encoding tumor antigens, DNA-based vaccines encoding tumor antigens, proteins targeted to dendritic cells, dendritic cell-based vaccines, whole tumor cell vaccines, gene modified tumor cells expressing GM-CSF, ICOS and/or Flt3-ligand, oncolytic virus vaccines.

Cytokines which according to at least some embodiments may be used in combination with an immunostimulatory antibody according to at least some embodiments of the present invention include but are not limited to one or more cytokines such as interferons, interleukins, colony stimulating factors, and tumor necrosis factors such as IL-2, IL-7, IL-12, IL-15, IL-17, IL-18, IL-21, IL-23, IL-27, GM-CSF, IFNα (interferon α), IFNα-2b, IFNβ, IFNγ, TNF-α, TNF-β and combinations thereof.

Adoptive cell transfer therapy according to at least some embodiments that may optionally be used in combination with an immunostimulatory antibody according to at least some embodiments of the present invention include but are not limited to an *ex vivo* treatment selected from expansion of the patient autologous naturally occurring tumor specific T cells or genetic modification of T cells to confer specificity for tumor antigens.

### EXAMPLES

### EXAMPLE 1: RATIONAL FOR EVALUATING THE EFFECT OF ANTI-HIDE1 ABS

The presented dry examples 2-6 below suggest a set of functional assays to evaluate the effect of **anti-HIDE1** on T cell function. In particular, the suggested assays will be used to evaluate the immuno-modulatory effect of anti-HIDE1 antibodies (agnostic or antagonistic) that can be used to target monocytes, Tumor associated macrophages (TAMs) or other myeloid cells and screen for various functional activities, including modulating the interaction between HIDE-1 and its putative receptor(s), modulation of HIDE1 levels or direct signaling and attenuation of negative signaling and/or depletion of HIDE1 positive cells. Such recombinant antibodies may be used as modulatory molecules to decrease or prevent HIDE1 from interacting with inhibitory receptor(s) on T cells or other cells in the tumor microenvironment, thereby releasing T cells or other functional cells from HIDE1 check point ("break")/suppressive signaling

### EXAMPLE 2: MIXED LYMPHOCYTE REACTION (MLR)

A mixed lymphocyte reaction will be employed to demonstrate the effect of blocking the HIDE1 pathway to lymphocyte effector cells. T cells in the assay will be tested for proliferation and cytokine secretion in the presence or absence of an anti-HIDE1 human monoclonal antibodies. Human CD4+ or CD8+ T-cells will be purified from PBMC using a CD4+ or CD8+. Alloreactivite dendritic cells will be derived from purified monocytes cultured with 1000 U/ml of IL-4 and 500 U/ml of GM-CSF (R&D Biosystems) for seven days. Monocytes will be prepared using a monocyte negative selection kit (Mitenyi Biotech). Each culture will contain 10⁵ purified T-cells and 10⁴ allogeneic dendritic cells in a total volume of 200 µl. Anti-HIDEl blocking or agonistic mAb at varying concentrations will be added to each culture at different antibody concentrations. Either no antibody or an isotype control antibody will be used as a negative control. After day 5, the effect of anti-HIDE1 antibodies on T cell proliferation (CFSE dilution) and cytokine secretion (ELISA or TH1/2/17 CBA kits) in culture supernatants will be assessed.

### EXAMPLE 3: THP-1-JURKAT OR PRIMARY T CELL CO-CULTURE

96-well flat-bottom plates will be coated with mouse anti-human CD3 antibody (1 µg/ml in PBS; Clone HIT3a; BD Pharmingen Cat 555336) overnight at 4' C. The next day, Jurkat cells (50,000) or CFSE-labeled primary human CD4+ or CD8+ T cells will be plated in the pre-coated plates. Mytomicin C treated (50µg/ml, 1hr) THP-1 cells, which express HIDE1, (50,000) will be added to the culture in the presence of HIDE1 blocking or agonistic mAb at varying concentrations. After 1-5d at 37'C and 5.0% CO2, the effect of anti-HIDE1 antibodies on T cell proliferation (CFSE dilution) and cytokine secretion (ELISA or TH1/2/17 CBA kits) in culture supernatants will be assessed.

### EXAMPLE 4: RECALL RESPONSE OF TETANUS TOXIN OR CMV SPECIFIC T CELLS

Purified human T cells will be labeled with CFSE and cultured with autologous monocyte-derived DCs in the presence of tetanus toxoid (TT) or CMV antigens. The effect of the inclusion of HIDE1 blocking or agonistic mAb on TT or CMV specific T cell response will be evaluated. After 5-10d at 37'C and 5.0% CO2, the effect of anti-HIDE1 antibodies on T cell proliferation (CFSE dilution) and cytokine secretion (ELISA or TH1/2/17 CBA kits) in culture supernatants will be assessed.

### EXAMPLE 5: PLATE BOUND ANTI-CD3 AND HIDE1-FC ASSAY

Purified human T cells will be CFSE labeled and stimulated with anti-CD3 Ab (OKT3, 1 µg/ml) together with plate-coated HIDE1-Fc or control protein (FLAG-Fc). Control or HIDE1 mAb was added during cell culture. Cells were gated on CD8+ T cells, and their division was analyzed based on the dilution of CFSE. Inclusion of a HIDE1 neutralizing mAb is expected to enhance the T cell function which will indicate that HIDE1 interacts with putative receptor(s) expressed on T cells to inhibit T cell proliferation.

### EXAMPLE 6: CHOS-OKT3 CO-CULTURE ASSAY

CFSE-labeled T cells will be stimulated with stimulator cells (CHO cells expressing expressing membrane-bound anti-CD3 mAb fragments). CHOS-stimulator cells expressing human HIDE1 and control stimulator cells (empty vector) treated with mitomycin C before co-cultured with CFSE-labeled human T cells at the ratio of 1:5. After 5d at 37°C and 5.0% CO2, the effect of anti-HIDE1 antibodies on T cell proliferation (CFSE dilution) and cytokine secretion (ELISA or TH1/2/17 CBA kits) in culture supernatants will be assessed.

### EXAMPLE 7: EXPRESSION ANALYSIS OF HIDE1 PROTEINS

The purpose of this example was to demonstrate that HIDElis correlated to CSF1R expression in cancer samples of both human and mouse. Solid tumors contain a significant population of tumor-infiltrating myeloid cells (TIM). TIMs are now recognized as important mediators of not only tumor progression and metastasis, but also therapeutic resistance, through promoting angiogenesis and suppressing antitumor immune responses. The pro-tumorigenic role of "alternatively" activated macrophages, which are part of the TIM population, has been well established. Recently, another specific subtype of TIMs, namely myeloid-derived suppressor cells (MDSC; (Myeloid-Derived Suppressor Cells - an additional population of myeloid cells that is tumor promoting Journal for ImmunoTherapy of Cancer 2013, 1:10)), is receiving great attention in cancer research. MDSCs comprise a heterogeneous population of immature myeloid cells that originate in the bone marrow and are recruited to the tumor by a diverse array of cytokine and chemokine signals. Similar to tumor-associated macrophages (TAM), MDSCs have been shown to generate an environment favorable for tumors by heightening immunosuppression, angiogenesis, and invasion. Various cell surface markers are used to identify TIM subsets: TAMs can be identified by CD11b and F4/80, and MDSCs by CD11b and Gr-1 coexpression in murine models. Macrophage colony-stimulating factor (M-CSF or CSF1) is a potent growth factor that promotes the differentiation, proliferation, and migration of monocytes/macrophages via signaling through its receptor tyrosine kinase CSF1R (cFMS). It was recently showed that TAMs and MDSCs form a spectrum of bone marrow-derived myeloid cells dependent on CSF1/CSF1R signaling for recruitment into the tumor and that they play critical roles in tumor growth. In addition, DeNardo and colleagues highlighted the importance of CSF1/CSF1R signaling in the recruitment of TAMs in various cancers and further showed that CSF1R blockade can inhibit TAMs and improve treatment outcome (Cancer Res. 2013 May 1;73(9):2782-94).

As can be seen in **Table 18,** HIDE1 was found to be highly correlated to CSF1R in various mouse tumor models. This finding was also validated in human tumors by correlating HIDE1 and CSF1R using The Cancer Genome Atlas (TCGA) (http://cancergenome.nih.gov/)data. **Figure 3** demonstrates the high correlation of HIDE1 to CSF1R in human colon cancer, as an example. Across all tumors, in TCGA, HIDE1 shows strong correlation to myeloid/TAM markers such as CD86 and CD68 **(****Figure 5****).** HIDE1 is also found directly on MDSCs derived from mouse tumor models **Figure 7****.** Specifically, as shown in **Figure 7****,** in MDSC HIDE1 was found to be induced in later stages of tumor progression. Without wishing to be limited by a single theory, these data indicate that HIDE1 is expressed and inducible on tumor associated macrophages and thus can serve as a target for immunotherapy in cancer, particularly those cancers with a strong myeloid infiltration, (such as Bladder cancer, Lower grade glioma, colon adenocarcinoma, glioblastoma multiforme, head and neck squamous cell cancer, kidney renal clear cell cancer, kidney renal papillary cancer, liver cancer, lung adenocarcinoma, lung squamous cell carcinoma, pancreatic adenocarcinoma, rectal adenocarcinoma, melanoma, stomach adenocarcinoma, testicular germ cell carcionoma and thyroid carciona; **Figure 8** - correlation of CSF1R and HIDE1 in TCGA). In addition, HIDE1 showed higher expression in melanoma patients that did not respond to anti PD-1 therapy **(****Figure 63****.** Legend for figure: HIDE1 expression in patients treated with PD-1 inhibitor (ref:GSE78220, Hugo W, Zaretsky JM, Sun L, Song C et al. Genomic and Transcriptomic Features of Response to Anti-PD-1 Therapy in Metastatic Melanoma. Cell 2016 Mar 24;165(1):35-44)). These data suggest anti HIDE1 therapy could be beneficial to patients resistant to anti PD1 therapy. Expression analysis of the Genotype Tissue Expression (GTEx) data (http://www.nature.com/ng/journal/v45/n6/full/ng.2653.html; http://www.gtexportal.org/home/) shows high expression of HIDE1 in blood cells and tissues with enriched blood cells such as the spleen **(****Figure 88****).** Analysis of HIDE1 expression in the BioGPS database indicates also blood specific expression with a prominent myeloid expression (http://biogps.org/#goto=genereport&id=255809; **Figure 89****).**

**Figure 11** presents integrated HIDE1 gene expression analysis in cancer tumors, based on the data analysis from 5 major initiatives, namely, The Cancer Genome Characterization Initiative(http://cgap.nci.nih.gov/cgci.html), TARGET: Therapeutically Applicable Research To Generate Effective Treatments (https://ocg.cancer.gov/programs/target), TCGA: The Cancer Genome Atlas (https://tcga-data.nci.nih.gov/tcga/), ICGC: International cancer genome consortium (http://icgc.org), Multiple Myeloma Genomics Portal (https://www.broadinstitute.org/mmgp/home). This data shows that in addition to blood neoplasms like Acute myeloid leukemia and Diffuse large B cell lymphoma, HIDE1 is also expressed in a variety of solid tumors like Glioblastoma, kidney lung and skin cancers **(****Figure 11****).** Furthermore analysis of differential expression between normal and cancer using both TCGA and the GTEx data showed that HIDE1 is upregulated in breast, skin, kidney and ovarian cancers **(****Figure 12****).** Meta-analysis of gene enrichment demonstrated a strong signature of immune response, regulation by interferon gamma, leukocyte chemotaxis and lymphocyte activation **(****Figure 16****).**

The analysis was performed using both human and mouse tumor expression data sets from Gene Expression Omnibus (GEO) (www.ncbi.nlm.nih.gov/GEO, the platform used were GPL570, GPL6244 for human, and GPL1261 and GPL6246 for mouse). The methodology of analysis is described in the "methodology section". HIDE1 was found to be highly correlated to CSF1R and is a type I membrane protein (http://www.uniprot.org/uniprot/A8MVS5) in murine tumors. **Table 18** shows the correlation of AB124611 (the murine orthologue of human HIDE1) in several caner models in mice.

### Methodology:

Raw data is downloaded from the GEO site in SOFT format. In case that the raw data is in MAS5 format data is taken without manipulation. If the data is Log MAS5 then the Log is powered to linear data. If the data is in RMA format CEL files are downloaded and re analyzed using MAS5. If such data is not available the RMA format is used.

Data is then normalized by multiplicative according to the 95^{th} percentile for Affy data.

Datasets analyzed: GSE49910 , GSE47855, GSE39397, GSE36765, GSE27928, GDS4343, GDS4617, GDS4371, GDS3953, GSE35398, GSE21927.

**Table 18: Correlation of HIDE1 to CSF1R in various tumor models.**

| tumor type | tumor model | study accession number | correlation of AB124611 to CSF1R |
|---|---|---|---|
| hepato cellular carcinoma | Pdgf-c Tg | GSE31431 | 0.790 |
| breast cancer | MMTV/c-MYC | GDS3953 | 0.664 |
| neuroblastoma | MYCN overexpression and loss of caspase-8 expression | GDS4617 | 0.930 |
| pancreatic cancer | KrasG12D | GDS4343 | 0.798 |

The purpose of this example is to present the involvement of HIDE1 in autoimmune disorders.

The role of myeloid cells in autoimmune disease has been elucidated in recent years. Of the myeloid cell population Myeloid-Derived Suppressor Cells (MDSCs) seem to have a major role in autoimmunity. Understanding of the origination and functions of MDSCs has come mainly from studies in tumor models and from cancer patients. MDSCs are involved in a number of different autoimmune disorders, including multiple sclerosis (MS), type 1 diabetes, rheumatoid arthritis (RA), inflammatory bowel disease (IBD) and autoimmune hepatitis. In steady state conditions, MDSCs reside primarily in the bone marrow. Under pathological conditions, MDSC populations expand and can be detected in the spleen, lymph nodes, cancerous tumors, and bloodstream (World J Immunol 2014 March 27; 4(1): 26-33). In the chronic inflammatory condition of IBD, there are complex interactions between several immune cells infiltrating into the intestinal mucosa, with epithelial cells even ignoring the effects of microbiota. Among them, myeloid cells, including neutrophils, macrophages, and MDSCs, have been a focus of study due to their divergent role in inflammation. In particular, the immunosuppressive function of MDSCs was suggested in several mouse models of IBD. It was reported that CD11b+Gr-1+ MDSCs were accumulated in a murine colitis model, and they expressed nitric oxide synthase 2 and arginase, which are known to be critical functional mediators of MDSCs. As well as in a model of IBD, CD14+HLA-DRlow MDSCs with suppressive functions were reported to be increased in the peripheral blood of IBD patients (Intest Res. 2015 Apr; 13(2): 105-111.). The expression of murine HIDE1 is induced in colon tissues derived from 2 different mouse DSS models of IBD **(****Figure 2** and **Figure 4****).** In **Figure 2** the induction is higher as disease progress (highest expression on day 6). In **Figure 4** the induction of HIDE1 occurs both in the proximal and the distal colon. The expression in autoimmune diseases is also derived from the myeloid component as can be seen in **Figure 6****,** in which the correlation between HIDE1 and CD11b (marker for myeloid and MDSCs) is plotted in multiple autoimmune samples as well as allergy derived human samples (source immunoland Omicsoft), the correlation which is near 0.9 indicates that the expression of HIDE1 in these samples is derived from the myeloid component. These results indicate that the expression of HIDE1 is inducible in IBD specifically and in autoimmune in general therefore its modulation can result is beneficial therapeutic effect in autoimmune conditions.

Expression analysis of the Genotype Tissue Expression (GTEx) data (http://www.nature.com/ng/journal/v45/n6/full/ng.2653.html; http://www.gtexportal.org/home/) showed high expression of HIDE1 in blood cells and tissues with enriched blood cells such as the spleen **(****Figure 9****).** Analysis of HIDE1 expression in the BioGPS database indicated also blood specific expression with a prominent myeloid expression (http://biogps.org/#goto=genereport&id=255809; **Figure 10****).**

### EXAMPLE 8: GENERATION AND CHARACTERIZATION OF HIDE1-EXPRESSING STABLE TRANSFECTANT CELL POOLS

Recombinant stable pools of cell lines overexpressing HIDE1 human proteins were generated, for use in determining the effects of HIDE1 on immunity, for HIDE1 characterization, anti-HIDE1 antibody discovery, and to obtain cross-species anti-HIDE1 immune molecules.

### Materials & Methods

### Human HIDE1 Reagents

### DNA expression constructs:

Human HIDE1 flag pUC57 (Genscript)
Human HIDE1 flag pMSCV (in house)

### Recombinant cells:

HEK293 Human HIDE1 flag pMSCV (in house)
SK-MEL-5 Human HIDE1 flag pMSCV (in house)

### HIDE1 Specific Antibody:

Anti c19orf38 (HIDE1), sigma cat. HPA012150
Rabbit anti human HIDE1 pAb (GenScript, # 4885364)
Mouse anti human HIDE1 mAb (Biotem #33B4-2F7)
Mouse anti human HIDE1 mAb (Biotem #36C1-2F6)
Mouse anti human HIDE1 mAb (Biotem #39A7-3A10-3G8)

### Expression constructs

Full length cloning of human HIDE1 was performed at Genscript by gene synthesis using codon optimized sequence in pUC57 vector and subcloned into a retroviral expression vector.

### Construct encoding Human HIDE1-flag

Full length cloning of human HIDE1 flag was performed by gene synthesis in pUC57 vector and subcloned into a retroviral expression vector, pMSCV, to create the expression plasmid.

### Establishment of stable cells pools ectopically expressing HIDE1 proteins

The resulting expression constructs were transduced to cells and stable pool cell lines were generated as detailed below. The protein sequences encoded by the expression constructs are detailed herein.

### Establishment of stable pool HEK293 Flag human HIDE1 expressing cells.

GP2-293 packaging cell line (Clontech cat#631458) was transfected with pMSCV-human HIDE1 Flag or with pMSCV- empty vector using Lipofectamin 2000 reagent (Invitrogen, cat No: 11668-019). 48 hours post transfection supernatants containing virions were collected, and directly used for infection of PT67 stable virus producing cells (Clontech #631510). 48 hour post infection Puromycin antibiotic was added at concentration of 2µg/ml, and resistant colonies were selected for stable pool generation. After selection of PT67, stably human HIDE1 virions producing cells, supernatant containing virions were collected and used for infection of HEK293 cell line. 48 hour post infection, Puromycin antibiotic was added at concentration of 1µg/ml, and resistant colonies were selected for stable pool generation.

### Establishment of Stable pool SK-MEL-5 Flag human HIDE1 expressing cells.

GP2-293 packaging cell line (Clontech cat#631458) was transfected with pMSCV-human HIDE1 Flag or with pMSCV- empty vector using Lipofectamin 2000 reagent (Invitrogen, cat No: 11668-019). 48 hours post transfection supernatants containing virions were collected, and directly used for infection of PT67 stable virus producing cells (Clontech #631510). 48 hour post infection Puromycin antibiotic was added at concentration of 2µg/ml, and resistant colonies were selected for stable pool generation. After selection of PT67, stably human HIDE1 virions producing cells, supernatant containing virions were collected and used for infection of SK-Mel-5 cell line. 48 hour post infection, Puromycin antibiotic was added at concentration of 0.5µg/ml, and resistant colonies were selected for stable pool generation.

### Expression validation

### Expression validation by Flow cytometry (FACS)

In order to validate the cell surface expression of the human HIDE1 protein in the recombinant stable pools, 2x10⁵ cells were stained with Fixable viability stain 450 (BD, cat #562247) diluted 1:1000 in PBS, for 15 min at R.T. followed by cells washing with PBS. Antibodies were used as following:
- pAbs anti-human HIDE1 (GenScript, #488536_4, #488536_13) or Rabbit IgG whole molecule (Jackson, cat #011-000-003) were added to cells (5µg/ml diluted in 0.5%BSA in PBS) followed by staining with Donky anti Rabbit-PE diluted 1:100 in 0.5%BSA in PBS.
- mAbs anti-human HIDE1 (Biotem, #33B4-2F7, #36C1-2F6, #39A7-3A10-3G8) or Mouse IgG1 (Life technologies, Cat #MG100) were added to cells (10µg/ml diluted in 0.5%BSA in PBS) followed by staining with Goat anti-mouse-PE (Jackson, cat #115-116-146), diluted 1:75 in 0.5%BSA in PBS.

### Results

In order to verify cell surface expression of human HIDE1 Flag protein, the cells, described above were analyzed by FACS using Rabbit anti human-HIDE1 pAb (GenScript) as described in Material and Methods. As shown in **Figure 13****,** the binding of anti-human HIDE1 pAbs to the HEK293 cells expressing human HIDE1 Flag protein (light blue line) is higher than the binding of Rabbit IgG isotype control (pink line).

**Figure 13** presennts FACS analysis of ectopically expressed HEK293 cells expressing human HIDE1 Flag pMSCV vector HEK293 cells expressing the human HIDE1 Flag were analyzed by FACS using Rabbit anti Human HIDE1 (GenScript, light blue line). Rabbit IgG (Jackson, pink line) was used as isotype control. Detection was carried out using donkey anti-rabbit PE-conjugated secondary antibody and analysis by FACS.

The surface expression of human HIDE1 Flag protein on HEK293 cells transduced with human HIDE1 Flag pMSCV vector or HEK293 cells transduced with the empty vector were further analyzed using mouse anti-human HIDE1 mAb (Biotem) as described in Material and Methods above. As shown in **Figure 14****,** the binding of all the three anti-human HIDE1 mAbs (A-C) to the HEK293 cells expressing human HIDE1 Flag protein (green line) is higher than the binding of these antibodies to the empty vector cells (orange line) or higher than the binding of the IgG1 isotype control to HEK293 cells expressing human HIDE1 Flag protein (light blue line) or to the empty vector cells (red line).

**Figure 14** presents FACS analysis of ectopically expressed HEK293 cells expressing human HIDE1 Flag pMSCV vector using anti human HIDE1 mAbs. HEK293 cells expressing the human HIDE1 Flag or HEK293 pMSCV empty vector were analyzed by FACS using mouse anti Human HIDE1 mAbs (Biotem, A-C, green line or orange line respectively) or using Mouse IgG isotype control (light blue or pink line respectively). Detection was carried out using goat anti Mouse-PE-conjugated secondary Ab.

### A. SK-MEL-5 cells overexpressing human HIDE1- flag pMSCV vector

SK-MEL-5 cells transduced with human HIDE1 Flag pMSCV vector or with the empty vector were analyzed by FACS for their cell surface expression using Rabbit anti human-HIDE1 pAb (GenScript) as described in Material and Methods. As shown in **Figure 15****,** the binding of anti-human HIDE1 pAbs to the SK-MEL-5 cells expressing human HIDE1 Flag protein (light blue line) is higher than the binding of Rabbit IgG isotype control (pink line) to the same cells.

**Figure 15** presents FACS analysis of ectopically expressed SK-MEL-5 cells expressing human HIDE1 Flag pMSCV vector SK-MEL-5 cells expressing the human HIDE1 Flag were analyzed by FACS using Rabbit anti Human HIDE1 (GenScript, light blue line). Rabbit IgG (Jackson, pink line) was used as isotype control. Detection was carried out using donkey anti-rabbit PE-conjugated secondary antibody Ab and analysis by FACS.

### B. Generation and Characterization of Stable Cell Pools Over-expressing HIDE1 Protein

### Materials & Methods

### Reagents

### DNA expression constructs:

Human HIDE1 flag pUC57 (Genscript)
Human HIDE1 flag pCDNA3.1 (Genscript)
Mouse HIDE1 flag pUC57
Mouse HIDE1 flag pCDNA3.1
Mouse HIDE1 flag pMSCV

### Recombinant cells:

HEK293 Human HIDE1 flag pCDNA3.1 (GenScript)
HEK293 Mouse HIDE1 flag pMSCV
EL4 Mouse HIDE1 flag pMSCV

### HIDE1 Specific Antibody:

Commercial pAb, rabbit anti c19orf38 (HIDE1), sigma cat. HPA012150
Custom pAb, rabbit anti mouse HIDE1 (GenScript, # 488536_13)

### Expression constructs

**Full** length cloning of human and mouse HIDE1 was performed at Genscript by gene synthesis using codon optimized sequence in pUC57 vector and subcloned into a mammalian or retroviral expression vector.

### Construct encoding human HIDE1-flag

**Full** length cloning of human HIDE1 flag was performed by gene synthesis in pUC57 vector and subcloned into a mammalian expression vector, pcDNA3.1, to create the expression plasmid (done by GenScript)

### Construct encoding mouse HIDE1-flag

The cloning of mouse HIDE1 -Flag retroviral expressing construct is described in example 8 section A.

### Construct encoding cyno HIDE1-untagged

Full length cloning of cyno HIDE1 untagged was performed by gene synthesis in pUC57 vector and subcloned into a mammalian expression vector, pcDNA3.1, to create the expression plasmid (done by GenScript)

### Establishment of stable cells pools ectopically expressing HIDE1 proteins

The resulting expression constructs were transfected or transduced to cells and stable pool cell lines were generated as detailed below. The protein sequences encoded by the expression constructs are detailed below.

### Nucleic acid Sequence of human HIDE1-flag (SEQ ID NO:289)

ATGCCCTGGA CTATTCTCCT CTTTGCCGCC GGTTCTCTCG CCATTCCCGC CCCTTCTATC
AGACTCGTGC CACCCTACCC CTCTTCCCAG GAGGACCCTA TCCACATTGC TTGCATGGCA
CCTGGCAACT TCCCAGGAGC AAATTTTACC CTGTACCGAG GAGGACAGGT GGTCCAGCTG
CTCCAGGCCC CAACCGATCA GAGGGGCGTG ACATTCAACC TGTCTGGAGG TAGCTCCAAG
GCACCAGGAG GACCATTTCA TTGTCAGTAT GGGGTGCTGG GCGAGCTCAA CCAGTCACAG
CTGAGCGACC TCTCCGAACC CGTGAATGTC AGTTTCCCCG TGCCTACATG GATCCTGGTC
CTCAGCCTGT CCCTCGCAGG AGCTCTGTTT CTGCTCGCTG GTCTGGTGGC AGTCGCCCTC
GTGGTCAGGA AGGTGAAACT GAGAAACCTC CAGAAGAAAA GGGATAGAGA AAGCTGCTGG
GCACAGATTA ACTTCGACTC TACAGATATG AGTTTCGACA ATTCACTGTT TACAGTGAGC
GCCAAGACTA TGCCCGAGGA AGATCCTGCT ACTCTGGACG ATCACTCCGG AACCACAGCT
ACTCCTTCTA ATAGTCGGAC CCGCAAACGA CCAACTAGCA CCTCTAGTTC ACCCGAGACA
CCCGAGTTTA GCACATTCAG GGCTTGCCAG GACTACAAAG ACGATGATGA CAAATAA

### Amino acid Sequence of human HIDE1-flag (SEQ ID NO:77)

MPWTILLFAAGSLAIPAPSIRLVPPYPSSQEDPIHIACMAPGNFPGANFTLYRGGQVVQLLQAPTDQRGVTFNLS
GGSSKAPGGPFHCQYGVLGELNQSQLSDLSEPVNVSFPVPTWILVLSLSLAGALFLLAGLVAVALVVRKVKLRNL
QKKRDRESCWAQINFDSTDMSFDNSLFTVSAKTMPEEDPATLDDHSGTTATPSNSRTRKRPTSTSSSPETPEFST
FRACQDYKDDDDK

### Establishment of stable pool HEK293 human HIDE1 flag expressing cells

HEK293 cells over expressing human HIDE1 flag were generated by GenScript

### Establishment of stable pool HEK293 and EL4 mouse HIDE1 Flag expressing cells

Supernatants from 48 hours culture of PT67 mouse HIDE1 virus producing cells or PT67 pMSCV empty vector were collected and used for infection of HEK293 and EL4 cell lines. Four days post infection, puromycin antibiotic was added at a concentration of 1µg/ml for HEK293 cells or 4µg/ml for EL4 cells, and resistant colonies were selected for stable pool generation.

### Establishment of stable pool CHO-S-OKT3 human HIDE1 flag expressing cells

Stable pool of CHO-S transduced with OKT3, were electroporated by AMAXA device with 8ug of pcDNA3.1_human HIDE1_Flag construct generated at GS. 24hr post electroporation, G-418 antibiotic was added at a concentration of 300µg/ml in order to generate stable pool cells. 2 weeks after selection process was finished, sorting for cells with highly expressed human HIDE1 protein was performed.

### Establishment of stable pool HEK293 cyno HIDE1 expressing cells

The resulting expression constructs were transfected to cells and stable pool cell lines were generated as detailed below. The protein sequences encoded by the expression constructs are detailed below.

### Nucleic acid Sequence of cyno HIDE1 (SEQ ID NO:290)

ATGCCCTGGACCATCTTGCTTCTTGCAGCTGGCTCCTTGGCGATCCCGCGACCGTCCATC

CGGCTGGTGCCCCCGCACCCAAGCAACCAAGAGGACCCCATCCACATCGCATGCATGGCC
CCTGGGAACTTCCTGGGGGCGAATTTCACACTGTATCGAGGGGGGCAGGTGGTCCAGATC
CTGCAGGCCCACGGAGACCAGCGCGGGGTGACATTTAACCTGAATGGCAGCAGCAGCGAG
GCTTCAGGGGAACCCTTCCACTGCCAGTATGGAGTGTTAGGCGAGCTCAGCCAGCCCCAG
CTGTCAGACCTCAGCGAGCCCGTGAACGTCTCCTTTCCAGTGCCCACTTGGATCTTGGTG
CTCTCCCTGAGCCTGGCTGGTGCCGTCTTCCTCCTCGCTGGGCTGGTGGCTGTTGTCCTG
GTGGTCAGAAGAGTTAAACTCAAAAATTTACAGAAGAAAAGAGATCGAGAATCCTGCTGG
GCCCAGATTAACTTCAACAGCCCAGACATGTCCTTCGATAACTCCCTGTTTACCGTCTCT
GGGAAAACGATGCCAGAAGAAGACCCGGCCACCTTGGATGATCACTCAGGCACCACTGCC
ACCCCCAGCAACTCCAGGACCCGGAAGAGACCCACTTCTACGTCCTCCTTGCCTGAGATC
CCGGAATTCAGCACTTTCCGGGCCTGCCAGTGA

### Amino acid Sequence of cyno HIDE1 (SEQ ID NO:291)

MPWTILLLAAGSLAIPRPSIRLVPPHPSNQEDPIHIACMAPGNFLGANFTLYRGGQVVQI
LQAHGDQRGVTFNLNGSSSEASGEPFHCQYGVLGELSQPQLSDLSEPVNVSFPVPTWILV
LSLSLAGAVFLLAGLVAVVLVVRRVKLKNLQKKRDRESCWAQINFNSPDMSFDNSLFTVS
GKTMPEEDPATLDDHSGTTATPSNSRTRKRPTSTSSLPEIPEFSTFRACQ

HEK-293 (ATCC, CRL-1573) cells were transfected with each of the constructs described above or with the empty vector (pcDNA3.1) as negative control, using polyplus JetPrime, transfection reagent (polyplus transfection, catalog number 114-15). Geneticin, G418 (Gibco, catalog number: 11811-031) resistant colonies were selected for stable pool generation.

### Expression validation

### Expression validation by Western blot (WB)

Whole cell extracts of cells transfected or transduced with human or mouse HIDE1 protein (35 µg of total protein) were analyzed for HIDE1 protein expression by WB. As a negative control, whole cell extracts of cells transfected or transduced with an empty vector were used.

Antibodies were used as following:
- mAb mouse anti-Flag HRP, Sigma, cat.A8592, diluted 1:1000 in TTBS/5% BSA
- Commercial antibody polyclonal Rabbit anti-human HIDE1, Sigma, cat #HPA012150, diluted 1:100 in TTBS/5% BSA)
- Custom polyclonal Rabbit anti-mouse HIDE1 (GenScript, #488536_13) was diluted to a concentration of 1 µg/ml in TTBS/5% BSA

The polyclonal antibodies described above were followed by a secondary antibody goat anti-Rabbit - Peroxidase (Jackson, cat no. 111-035-003) diluted 1:20,000 in 5%milk/TTBS solution.

### Expression validation by Flow cytometry (FACS)

In order to validate the cell surface expression of the human and mouse HIDE1 protein in the recombinant stable pools, 2x10⁵ cells were stained with Fixable viability stain 450 (BD, cat #562247) diluted 1:1000 in PBS, for 10 min at R.T. followed by cells washing with PBS. Antibodies were used as following:
- For mouse HIDE1 validation: Custom pAbs anti- mouse HIDE1 (GenScript, #488536_13) or Rabbit IgG whole molecule (Jackson, cat #011-000-003) were added to cells (5µg/ml diluted in 0.5%BSA in PBS) followed by staining with Donky anti Rabbit-PE diluted 1:100 in PBS/0.5%BSA.
- For human HIDE1 validation: Custom mouse monoclonal anti human HIDE1 Ab (Biotem, 33B4-2F7- Alexa 647) or mIgG1 Isotype control (Biotem, lot. F1150528d-2695- Alexa 647) were added to the cells in concentration of 5µg/ml (diluted in 0.5%BSA/PBS).

### Results

### Expression validation of HEK293 cells stable pool over expressing the human HIDE1 protein

### HEK293 cells over expressing human HIDE1- flag pCDNA3.1 vector (GenScript):

To verify expression of the HIDE1 protein in the stably transfected HEK293 pools (generated by GenScript), whole cell extracts were analyzed by WB using anti-flag antibody and commercial anti HIDE1 antibody (Sigma, cat #HPA012150), as described in Material and Methods. As shown in **Figure 20****,** using anti Flag antibody (left panel) a band at -30 kDa is observed in the extracts of HEK293 cell pools over expressing human HIDE1 vector, but not in the cells transfected with the empty vector. Using the specific anti HIDE1 antibody (Sigma cat #HPA012150, right panel), two bands are observed, one is at ∼30kDa as observed with the anti- flag Ab, and an additional higher band at∼ 40kDa. The Calculated Mw of the protein is 25kDa.

In order to verify cell surface expression of the HIDE1 Flag protein, the same cells described above were analyzed by FACS using Rabbit anti-HIDE1 pAb (GenScript, #488536_13) as described in Material and Methods. No cell surface expression was observed in these cells (data is not shown).

### Stable pool of HEK293 cells over expressing the mouse HIDE1 protein

### HEK293 cells overexpressing mouse HIDE1- flag pMSCV vector

To verify the expression of the mouse HIDE1 protein in the stably transduced HEK293 cell pools, whole cell extracts of HEK293 stable pools transduced with pMSCV mouse HIDE1 flag vector or of HEK293 stable pools transduced with pMSCV empty vector, were analyzed by WB using anti-mouse HIDE1 pAb (GenScript #488536_13) as described in Material and Methods. The results, shown in **Figure 19C****,** demonstrate a band at ∼30kDa in the extracts of HEK293 cell pools expressing mouse HIDE1, but not in the cells transfected with the empty vector or with the human HIDE1. Additional Band at higher Mw but lower intensities was observed.

These cells were further analyzed by FACS for cell surface expression using Rabbit anti mouse-HIDE1 pAb (GenScrip t#488536_13) as described in Material and Methods. As shown in **Figure 17****.** the binding of anti-mouse HIDE1 pAbs to the HEK293 cells over expressing mouse HIDE1 Flag protein (green line) is higher than the binding of this antibody to the empty vector cells (orange line) and higher than the binding of the IgG isotype control to HEK293 cells expressing mouse HIDE1 Flag protein (light blue line), or to the empty vector cells (red line).

### Stable pool of EL4 cells over expressing the mouse HIDE1 protein

### EL4 cells overexpressing mouse HIDE1- flag pMSCV vector

To verify membrane expression, the cells were analyzed by FACS using Rabbit anti mouse-HIDE1 pAb (GenScrip t#488536_13) as described in Material and Methods. As shown in **Figure 18****,** the binding of anti-mouse HIDE1 pAbs to the EL4 cells over expressing mouse HIDE1 Flag protein (green line) is higher than the binding of this antibody to the empty vector cells (orange line) and higher than the binding of the IgG isotype control to HEK293 cells expressing mouse HIDE1 Flag protein (light blue line), or to the empty vector cells (red line).

### Stable pool of CHO-S OKT3 cells over expressing human HIDE1 protein

### CHO-S OKT3 cells overexpressing human HIDE1- flag pcDNA3.1 vector

In order to verify cell surface expression of human HIDE1 Flag protein, CHO-S OKT3 cells transfected with human HIDE1 construct (described in Material & Methods) were analyzed by FACS using Mouse anti-human HIDE1 monoclonal Ab (Biotem, 33B4-2F7- Alexa 647) as described in Material and Methods. As shown in **Figure 97****.** binding of anti-human HIDE1 Ab was observed to CHO-S OKT3 over expressing human HIDE1 protein, compared to isotype control (orange histogram) and empty vector cells (blue histogram).

### Expression validation of HEK293 cells stable pool over expressing the cyno HIDE1protein

### HEK293 cells over expressing cyno HIDE1- flag pCDNA3.1 vector

To verify the expression of the cyno HIDE1 protein in the stably transfected HEK293 cell pools, the cells were analyzed by FACS for cell surface expression using mouse anti human HIDE1 monoclonal Ab (Biotem 33B4-2F7- Alexa 647) as described in Material and Methods. As shown in **Figure 98****.** the binding of mouse anti human HIDE1 monoclonal Ab to the HEK293 cells over expressing cyno HIDE1 protein (green line) is higher than the binding of this antibody to the empty vector cells (orange line) and higher than the binding of the IgG isotype control to HEK293 cells expressing mouse HIDE1 Flag protein (light blue line), or to the empty vector cells (red line).

### EXAMPLE 9: HIDE1-ECD Ig fusion protein production

HIDE1 mECD-mIg fusion protein (SEQ ID NO:18) batch #195, was generated in CHO-DG44 cells by culturing stable cell pools for 10 days, followed by Protein A mouse HIDE1 fused to the Fc of mouse IgG2a, was produced at ProBioGen (Germany purification of cell harvest and preparative SEC purification for aggregate removal. The final product was formulated in 10mM Na/K phosphate + 140mM NaCl pH 6.0 + 0.01% Tween.

SEQ ID NO: 18 HIDE1_MM-1_(Mouse_ECD_+_mouse_IgG2_Fc)_-_without_SP

IPAPSISLVPPYPSSHEDPIYISCTAPGDILGANFTLFRGGEVVQLLQAPSDRPDVTFNVTGGGSGGGGEAAGGN

FCCQYGVMGEHSQPQLSDFSQQVQVSFPVPTEPRGPTIKPCPPCKCPAPNLLGGPSVFIFPPKIKDVLMISLSPI

VTCVVVDVSEDDPDVQISWFVNNVEVHTAQTQTHREDYNSTLRVVSALPIQHQDWMSGKEFKCKVNNKDLPAPIE

RTISKPKGSVRAPQVYVLPPPEEEMTKKQVTLTCMVTDFMPEDIYVEWTNNGKTELNYKNTEPVLDSDGSYFMYS

KLRVEKKNWVERNSYSCSVVHEGLHNHHTTKSFSRTPGK

HIDE1 mECD-mIg_fusion protein (SEQ ID NO:18, batch #233), composed of the ECD of mouse HIDE1 fused to the Fc of mouse IgG2a, was produced at ProBioGen (Germany) in CHO-DG44 cells by culturing stable cell pools for 12 days, followed by Protein A purification of cell harvest and preparative SEC purification for aggregate removal. The final product was formulated in 5mM Na citrate, 5mM Na/K phosphate, 140mM NaCl, 0.01% Tween pH5.5.

Expression vector used was ProBioGen's PBG-GPEX6. HIDE1 gene was driven by CMV/EF1 hybrid promoter followed by polyadenylation signal pA-1. The vector contained puromycin N-acetyl-transferase gene that allows selection of transfected cells using puromycin, as well as dehydrofolate reductase gene that allows selection of transfected cells using methotrexate (MTX).

HIDE1 mECD-mIg_fusion protein (SEQ ID NO: 18 batch #72), composed of the ECD of mouse HIDE1 fused to the Fc of mouse IgG2a, was produced at ExcellGene (Switzeland) by transient transfection in CHO-DG44 cells using Excellgene's proprietary vector system. Cells were cultured for 10 days, followed by Protein A purification of cell harvest. The final product was formulated in 10mM Na/K phosphate + 140mM NaCl pH 6.0 + 0.02% Tween.

HIDE1 hECD-hIg fusion protein (SEQ ID NO: 17, batch #48), composed of the ECD of human HIDE1 fused to the Fc of human IgG1 bearing C220 to S mutation at the hinge, was produced at ExcellGene (Switzeland) by transient transfection in CHO-DG44 cells using Excellgene's proprietary vector system. Cells were cultured for 10 days, followed by Protein A purification of cell harvest. The final product was formulated in 0.1M Glycine pH 6.

SEQ ID NO: 17 HIDE1_HH-1
_(Human_ECD_+_human_IgG1_Fc_mutated_C220S_at_hinge)_-_without_SP
IPAPSIRLVPPYPSSQEDPIHIACMAPGNFPGANFTLYRGGQVVQLLQAPTDQRGVTFNLSGGSSKAPGGPFHCQ
YGVLGELNQSQLSDLSEPVNVSFPVPTWEPKSSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

HIDE1 hECD-hIg fusion protein (SEQ ID NO:17 batch #259), composed of the ECD of human HIDE1fused to the Fc of human IgG1 bearing C220 to S mutation at the hinge, was produced at GenScript (China) by transient transfection in CHO-3E7 cells using GenScript's vector system. Cells were cultured for 6 days, followed by Protein A purification of cell harvest. The final product was formulated in PBS pH 7.2.

HIDE1 hECD-hIg fusion protein (SEQ ID NO:17 batch #280), composed of the ECD of human HIDE1 fused to the Fc of human IgG1 bearing C220 to S mutation at the hinge, was produced at GenScript (China) by transient transfection in CHO-3E7 cells using GenScript's vector system. Cells were cultured for 6 days, followed by Protein A purification of cell harvest. The final product was formulated in 10 mM NaPhosphate, 150mM NaCl pH 7.0 +0.01% tween 20.

### EXAMPLE 10: Generation and Characterization of custom Abs against HIDE1 protein

The aim of this example was to raise polyclonal and monoclonal antibodies specific to HIDE1 protein.

### Generation of rabbit polyclonal antibodies against human and mouse HIDE1 protein

Rabbit polyclonal antibodies were raised at GeneScript using the GenScript PolyExpress™ custom polyclonal antibody production service.

### Study Design

Generation of rabbit polyclonal antibodies was performed at GenScript (USA). Antibodies against the human HIDE1 protein were raised by using recombinant FC fused protein of human HIDE1 ECD fused to FC domain of human IgG1 (batch #280). Antibodies against the mouse HIDE1 protein were raised by using the recombinant Fc fusion protein of mouse HIDE1 ECD fused to FC domain of mouse IgG2a (batch #195).

### Materials & Methods

### Reagents used in this study:

1. Stable pool of HEK293 cells over expressing human HIDE1 flag protein.
2. Stable pool of HEK293 cells transfected with the empty pMSCV vector.
3. Stable pool of SK-MEL-5 cells over expressing human HIDE1 flag protein. The generation of the cell lines 1-3 is described herein.
4. HIDE1 ECD (H:H) recombinant Fc fusion protein -Human ECD of HIDE1 fused to the Fc domain of Human IgG1 (Batch #280).
5. HIDE1 ECD (M:M) recombinant Fc fusion protein -mouse ECD of HIDE1 fused to the Fc domain of mouse IgG2a (Batch #195).
6. Rabbit anti human HIDE1 (GenScript, # 4885364).
7. Rabbit anti mouse HIDE1 (GenScript, # 488536_13).
8. Rabbit anti-human HIDE1, C190RF38 (Sigma, cat #HPA012150).
9. Mouse anti Flag HRP, Sigma, cat.A8592.
10. Goat Anti Rabbit-HRP (Jackson, Cat # 111-035-003).
11. Fixable viability stain 450 (BD Horizon cat #562247).
12. FACS buffer: 0.5% BSA in PBS.
13. Rabbit IgG whole molecule (Jackson, cat #011-000-003).
14. R-Phycoerythrin-AffiniPure F (ab')2 Fragment Donkey Anti-Rabbit IgG, (Jackson, cat#711-116-152)

### Methods:

### Anti human or mouse HIDE1 pAbs production

Production of rabbit polyclonal antibody against Fc fusion protein of mouse or human HIDE1 included immunizations of two rabbits, production bleeds and antibody purification, using GenScript's proprietary fast immunization protocol. The immunoreactivity of the test bleeds was analyzed following the third immunization by ELISA.

### Analysis of the pAbs performance in WB application

Whole cell extracts of cells (35ug of total protein) expressing human or mouse HIDE1 protein were used to analyzed the pAbs anti-human or anti-mouse HIDE1 performance in WB. As negative control, whole cell extracts of cells transfected with the empty vector were used.

Antibodies were used as following:
- Mouse anti Flag antibody (diluted 1:1000 in TTBS/5% BSA).
- Commercial antibody Rabbit anti-human HIDE1 (Sigma, diluted 1:100 in TTBS/5% BSA).
- Rabbit anti-human or anti-mouse HIDE1 antibodies (GenScript) were diluted to a concentration of 1 µg/ml in TTBS/5% BSA.
- The secondary antibody, goat anti rabbit HRP (Jackson) was diluted 1:20,000 in 5% millk/TTBS

### Analysis of the pAbs performance in FACS application

The performance of the rabbit pAbs, anti-human or anti-mouse HIDE1 in FACS application was tested using stable cells expressing the mouse or the human HIDE1. Cells (2x10⁵) were stained with Fixable viability stain diluted 1:1000 in PBS, for 10 min at R.T followed by cells washing with PBS. The pAbs were then added to cells (5µg/ml were stained with Fixable viability stain diluted 1:1000 in PBS, for 10 min at R.T followed by cells washi

### Results

The sera of the immunized rabbits were analyzed by ELISA against the respective Fc fusion proteins (human or mouse HIDE1). The negative control was pre immune serum. ELISA results indicated binding of serum to the fusion proteins (data not shown).

### Analysis of the purified rabbit polyclonal anti human or anti mouse HIDE1 by WB

The performance of the rabbit pAbs, anti-human or anti-mouse HIDE1 for WB application was tested using whole cell extracts of HEK293 cells expressing human or mouse HIDE1 protein. As negative control, whole cell extracts of cells transfected with the empty vector were used.

**Figure 19** presents the results of WB analysis using anti Human or mouse HIDE1 pAbs on HEK293 cells expressing human or mouse HIDE1 protein. Whole cells extracts of HEK293 cells expressing the human HIDE1 flag (lanes 2), HEK293 cells expressing the mouse HIDE1 flag (lanes 3) or HEK293 transfected with an empty vector (lanes 1) were analyzed using anti Flag antibody (A), a commercial antibody anti human HIDE1 (Sigma, D), pAb anti human-HIDE1 (GenScript, B) or with pAb anti mouse-HIDE1 (GenScript, C). Detection was carried out using Goat Anti Rabbit-HRP (except anti flag which is already conjugated to HRP).

As shown in **Figure 19****,** the human HIDE1 protein expressed on HEK293 cells could be detected using the two positive controls antibodies, anti-flag antibody (A lane 2) and the commercial anti HIDE1 (D lane 2). The tested antibody, rabbit pAb anti human HIDE1 detected a band (B lane 2) in size of 35 kDa (higher than the calculated 25 kDa band), a similar band as detected by the positive antibodies. This protein could not be detected using the anti-mouse HIDE1 tested antibody (C lane 2).

The mouse HIDE1 protein expressed on HEK293 cells could be detected by the tested antibody, rabbit anti mouse HIDE1 pAb (C lane 3) but not by the other anti-human HIDE1 antibodies (commercial, D lane 3 or the custom B lane 3) or by the anti-Flag antibody (A lane 3).

### |Analysis of the purified rabbit polyclonal anti human HIDE1 by FACS

The performance of the purified polyclonal antibody against human HIDE1 Fc fusion protein, anti-human HIDE1 pAb (GenScript, ID 4885364) in FACS application was tested using HEK293 cell expressing human HIDE1 Flag or SKMEL-5 cells expressing human HIDE1 Flag. Rabbit IgG (Jackson) was used an IgG control.

As shown in **Figure 21****,** the binding of anti-human HIDE1 pAbs to the HEK293 cells expressing human HIDE1 Flag protein (A, light blue line) or to SKMEL5 expressing human HIDE1 Flag (B, light blue line) is higher than the binding of Rabbit IgG isotype control to the same cells (A and B pink line).

**Figure 21** presents results of FACS analysis using anti Human HIDE1 pAbs on HEK293 or SKMEL5 cells expressing Human HIDE1 protein. HEK293 cells expressing the human HIDE1 Flag (A) or SKMEL-5 expressing the human HIDE1 Flag (B) were analyzed by FACS using Rabbit anti Human HIDE1 (GenScript, light blue line). Rabbit IgG (Jackson, pink line) was used as isotype control. Detection was carried out using donkey anti-rabbit PE-conjugated secondary antibody and analysis by FACS.

### |Analysis of the purified rabbit polyclonal anti mouse HIDE1 by FACS

The performance of the purified polyclonal antibody against mouse HIDE1 Fc fusion protein, anti-mouse HIDE1 pAb (GenScript, ID 488536_13) for FACS application was tested using HEK293 cell expressing mouse HIDE1 Flag. HEK293 cells transfected with an empty vector were used as a negative control.

As shown in **Figure 17****,** the binding of anti-mouse HIDE1 pAbs to the HEK293 cells expressing mouse HIDE1 Flag protein (green line) is higher than the binding of this antibody to the empty vector cells (orange line) or higher than the binding of the IgG isotype control to HEK293 cells expressing mouse HIDE1 Flag protein (light blue line) or to the empty vector cells (red line).

### Generation of Mouse monoclonal antibodies against human HIDE1 protein

Mouse monoclonal antibodies were raised at BIOTEM (France) using BIOTEM R.A.D® (Rapid Antibody Development) protocol which allows the breaking of tolerance towards self-antigens and the generation of more hybridomas than classical methods in a short time.

### Study Design

Generation of murine monoclonal antibodies against the extra-cellular domain of human HIDE1 were performed at BIOTEM. The Antibodies were raised using the human HIDE1 Fc fusion protein, ECD of human HIDE1 fused to human IgG1 (batch #295) for immunizations.

### Materials & Methods

### Reagents used in this study:

1. Stable pool of HEK293 cells over expressing human HIDE1 flag protein
2. Stable pool of HEK293 cells transfected with the empty pMSCV vector.
3. HIDE1 ECD (H:H) recombinant Fc fusion protein -Human ECD of HIDE1 fused to the Fc domain of Human IgG1 (Batch #259)
4. FACS buffer: 0.5% BSA in PBS.
5. Fixable viability stain 450 (BD Horizon cat #562247)
6. Mouse mAb anti HIDE1, (Biotem #33B4-2F7)
7. Mouse mAb anti HIDE1, (Biotem #36C1-2F6)
8. Mouse mAb anti HIDE1, (Biotem #39A7-3A10-3G8)
9. Mouse IgG1 (Life technologies, Cat #MG100)
10. Goat Anti Mouse-PE (Jackson, cat #115-116-146)

### Methods

### mAbs anti human HIDE1 production

Production of mouse monoclonal antibodies includes the following steps.

The first phase of the project to raise anti-HIDE1 mAbs included immunization of 2 BALB/c mice with human HIDE1 ECD fused to human IgG1.

The second phase of the protocol included fusion of the lymphocytes from the immunized mice with Sp2/0-Ag14 myeloma cells and plating out on 10 microtiter 96-well plates.

Mature clones were screened by ELISA using the HIDE1 H:H fusion proteins. FACS analysis was subsequently carried out with the culture supernatant of ELISA-positive hybridoma clones, using anti mouse-PE (Jackson, cat. 115-116-146) as secondary Ab for detection.

The third phase included hybridoma cloning by limiting dilution and stabilization.

The fourth phase included further processing for production and purification. Antibodies were produced by *in vitro* production and purification using protein A affinity chromatography purification.

### Analysis of the mAbs performance in WB application

Whole cell extracts of cells (35ug of total protein) expressing human or mouse HIDE1 protein were used to analyzed the mAbs anti-human HIDE1 performance for WB. As negative control, whole cell extracts of cells transfected with the empty vector were used.

Antibodies were used as following:
- Mouse anti-human HIDE1 antibodies (Biotem) were diluted to a concentration of 2 µg/ml in TTBS/5% BSA.
- The secondary antibody, Goat Anti Mouse-HRP, was diluted 1:20,000 in 5% millk/TTBS.

### Analysis of the mAbs performance in FACS application

The performance of the mouse mAbs, anti-human HIDE1 for FACS application was tested using stable cells expressing the human HIDE1. Cells (2x10⁵) were stained with Fixable viability stain diluted 1:1000 in PBS, for 10 min at R.T followed by cells washing with PBS. The mAbs were then added to cells (10µg/ml diluted in FACS buffer) followed by staining with Goat anti mouse-PE (diluted 1:75 in FACS buffer).

### Results

The sera of the immunized mouse were analyzed by ELISA against the respective Fc fusion proteins (human HIDE1). The negative control was pre immune serum. ELISA results indicated binding of serum to the fusion proteins (data is not shown).

### Analysis of the purified mouse mAbs anti human HIDE1 by WB

The performance the mouse mAbs, anti-human HIDE1 (Biotem) in WB application was tested using whole cell extracts of HEK293 cells expressing human HIDE1 protein. As negative control, whole cell extracts of cells transfected with the empty vector were used.

As shown in **Figure 22****,** All the Biotem mAbs: 33B4-2F7 (A), 36C1-2F6 (B), 39A7-39A10-3G8 (C) recognize the human HIDE1 protein expressed on HEK293 cells, band in size of ∼30 kDa can be observed (lanes 2). This specific band is absence in the negative control cells (empty vector, lane 1) and in the cells expressing the mouse HIDE1 (lane 3). Antibody 36C1-2F6 (B) shows the best performance.

### Analysis of the purified mouse mAbs anti human HIDE1 by FACS

The performance of the purified momoclonal antibodies against human HIDE1 Fc fusion protein, anti-human HIDE1 mAbs (Biotem) for FACS application was tested using HEK293 cell expressing human HIDE1 Flag. HEK293 cells transfected with an empty vector were used as a negative control.

As shown in **Figure 14****,** the binding of all three anti-human HIDE1 mAbs (A-C) to the HEK293 cells expressing human HIDE1 Flag protein (green line) is higher than the binding of these antibodies to the empty vector cells (orange line) or higher than the binding of the IgG1 isotype control to HEK293 cells expressing human HIDE1 Flag protein (light blue line) or to the empty vector cells (red line).

Schematic representation of Biotem's antibodies (33B4-2F7, 36C1-2F6, 39A7-39) are shown in figures 81-87.

### B. Generation and characterization of custom Abs against HIDE1 protein by Serotec

Generation of Fab's against human and mouse HIDE1 protein Fab's were raised at AbD Serotec (Bio Rad, Germany) using Human Combinatorial Antibody Library (HuCAL®) production service. The HuCAL® library is based on the human IgG1 Fab format, which consists of the first two domains of the antibody heavy chain and the complete light chain.

### Study Design

Generation of Fab's against HIDE1 was performed at AbD Serotec (Bio Rad, Germany). Antibodies against the human and mouse HIDE1 protein were raised using the HuCAL® phage library, using 3 rounds of enrichment and counter selection against non-related human IgG1 fusion protein for the depletion of unspecific antibodies. After the panning, the enriched antibody pool from the phage display vector was subcloned into expression vector to determine the final Fab format. The selected Fab format is Fab-A-FH (Bivalent Fab bacterial alkaline phosphatase fusion antibody (FLAG®- and His-6-tags)).

The Antibodies were raised using the human HIDE1 Fc fusion protein, ECD of human HIDE1 fused to human IgG1 (batch #295) and mouse HIDE1 Fc fusion protein, ECD of mouse HIDE1 fused to mouse IgG2a (batch #233).

### Materials & Methods

### Reagents used in this study:

1. Stable pool of HEK293 cells over expressing human HIDE1 flag protein.
2. Stable pool of HEK293 cells over expressing mouse HIDE1 flag protein.
3. Stable pool of HEK293 cells over expressing cyno HIDE1 protein.
4. Stable pool of HEK293 cells transduced with the empty pMSCV vector.
5. Stable pool of HEK293 cells transduced with the empty pCDNA3.1+ vector.
6. Stable pool of CHO-S cells over expressing human HIDE1 flag protein.
7. Stable pool of CHO-S cells transduced with the empty pCDNA3.1+ vector.
8. HIDE1 (H:H) recombinant Fc fusion protein -Human ECD of HIDE1 fused to the Fc domain of Human IgG1 (Batch #280).
9. HIDE1 (M:M) recombinant Fc fusion protein -mouse ECD of HIDE1 fused to the Fc domain of mouse IgG2a (Batch #233).
10. H:H_Internal recombinant Fc fusion protein control: Human ECD of Internal control fused to the Fc domain of Human IgG1 (Batch #279).
11. M:M_Internal recombinant Fc fusion protein control: mouse ECD of internal control fused to the Fc domain of mouse IgG2a (Batch #214).
12. Commercial rabbit pAb anti-human HIDE1, C19ORF38 (Sigma, cat #HPA012150)
13. Goat Anti Rabbit-HRP (Jackson, Cat # 111-035-003).
14. Anti Fab-HRP (Bio-Rad, Cat#STAR126P).
15. Goat Anti Human IgG F(ab')2-PE (Jackson, cat#109-116-097).
16. Goat anti-human-PE (Jackson, cat# 109-116-098).
17. Fixable viability stain 450 (BD Horizon cat #562247).
18. FACS buffer 0.5% BSA in PBS.

### Methods:

### Anti human or anti mouse HIDE1 Fab's generation

Fab's generation at AbD Serotec included the following steps:
1. Antigen immobilization - immobilization of the antigen on a solid support. The standard method uses covalent coupling to magnetic beads.
2. Phage display selection - panning - The HuCAL® library presented on phage particles is incubated with the immobilized antigen. Nonspecific antibodies are removed by extensive washing and specific antibody phage are eluted by adding a reducing agent.
   An E. coli culture is infected with eluted phage and helper phage to generate an enriched antibody phage library for the next panning round. Typically, three rounds of panning.
3. Subcloning into antibody expression vector - After panning, the enriched antibody DNA is isolated as a pool and subcloned into a Fab expression vector. E. coli are transformed with the ligation mixture and plated on agar plates. Each growing colony represents a monoclonal antibody at this stage.
4. Primary screening - Colonies are picked and grown in a 384-well microtiter plate. Antibody expression is induced and the culture is lysed to release the antibody molecules. Cultures are screened for specific antigen binding by ELISA.
5. Secondary screening - Flow cytometry (FACS) on transfected cells using bacterial lysates from E.coli expression cultures.
6. Sequencing - Hits from the primary screening experiment are sequenced to identify unique antibodies.
7. Expression and purification - The unique Fab's are expressed and purified using one-step affinity chromatography.
8. Antibody QC - Purified Fab's are tested by ELISA and by FACS on transfected cells.

### Analysis of the Fab's in Western blot (WB) application

Whole cell extracts of HEK293 transfected cells over expressing the human or mouse HIDE1 flag protein or whole cell extracts of HEK293 cells transdued with an empty vector were analyzed by WB using the custom Fab's (AbD Serotec) described above at a final concentration of 1-7µg/ml in 1%TTBS/BSA. The commercial pAb anti-human HIDE1 (Sigma, cat #HPA012150) was diluted 1:100 in TTBS/1% BSA.

Staining with the custom and commercial antibodies described above was followed by a secondary antibody Anti Fab-HRP (Bio Rad) diluted 1:5000 in 5%Milk/TTBS and goat anti Rabbit IgG - Peroxidase (Jackson, cat no. 111-035-003) diluted 1:10,000 in TTBS/5% milk solution.

### Analysis of the Fab's performance in Flow Cytometry (FACS) application

The performance of the Fab's, anti-human HIDE1 and anti-mouse HIDE1 for FACS application were tested using stable cells over expressing the human HIDE1 or mouse HIDE1 or on cyno HIDE1 an empty vector transduced cells. Cells (2x10⁵) were stained with Fixable viability stain diluted 1:1000 in PBS, for 10 min at R.T followed by cells washing with PBS.

The Fab's were then added to cells (10 µg/ml diluted in FACS buffer) followed by staining with Goat Anti Human IgG F(ab')2-PE (diluted 1:100 in FACS buffer).

### Affinity (Avidity) measurements of purified antibodies

Affinity measurements were done by AbD Serotec using the ForteBio Octer RED384 instrument.

Antibody concentrations: bivalent Fab antibodies in Fab-A-FH format (Mw: 198 kDa): 9.9 µg/ml (50 nM), 1:2 dilution series. The model used to fit the data: (ForteBio Data Analysis software 8.2.0.7)
- 1:1 interaction model
- 2:1 interaction model for heterogeneous binding

### Epitope Binning

Epitope Binning measurements were done by AbD Serotec using the ForteBio Octer RED384 instrument. Antibodies were grouped into diffrents bins by using the "in tandem" measurements.

### Reformatting of the Fab's into full length immunoglobulin

The conversion to human IgG1 was done to 5 Fab's by AbD Serotec (AbD25751, AbD25753, AbD25754, AbD25755, AbD25760).

### Analysis of the reformatted Fab's (full hIgG1 Abs) performance in Flow Cytometry (FACS) application

The performance of the antibodies, anti-human HIDE1, for FACS application were tested using stable cells over expressing the human HIDE1, mouse HIDE1 or cyno HIDE1 or an empty vector transduced cells. Cells (5x10⁵) were stained with Fixable viability stain diluted 1:1000 in PBS, for 10 min at R.T followed by cells washing with PBS.

The Abs were then added to cells (10µg/ml diluted in FACS buffer) followed by staining with Goat Anti Human-PE (diluted 1:100 in FACS buffer).

### Affinity measurements of the reformatted Fab's

The performance of the antibodies, anti-human HIDE1, for the affinity measurements (KD) using FACS application were tested using stable cells over expressing the

### Results

The panning was performed at AbD Serotec using the following proteins:
Panning 1: Human HIDE1 ECDhIgG1 protein batch #280 - for generation of anti-human Fab's.
Panning 2: Mouse HIDE1 ECDmIgG2A protein batch #233 - for generation of anti-mouse Fab's.
Panning 3: Mouse HIDE1 ECDmIgG2a protein batch #233 (1^{st} and 3^{rd} panning round)/ Human HIDE1 ECDhIgG1 protein batch #280 (2^{nd} panning round) - for generation of cross reactive Fab's.

Serotec screened ∼360 clones by ELISA on human HIDE1 ECDhIgG1 protein batch #280 and mouse HIDE1 ECDmIgG2A protein batch #233 and H:H_Internal ECDhIgG1 protein batch #279 as control for the human panning or mouse M:M_Internal ECDmIgG2A protein batch #214 as control for the mouse panning.

In the human HIDE1 panning 122 clones were positive, in the mouse HIDE1 panning 61 clones were positive and in the mouse/human panning 55 clones were positive.
In secondary screening 88 positive clones were screened from panning 1, 33 positive clones were screened from panning 2 and 55 positive clones were screened from panning 3 by FACS on cells overexpressing the human HIDE1 and mouse HIDE1 and on empty vector transduced cells.

The secondary screen resulted in 43 positive clones from panning 1, 15 positive clones from panning 2 and 29 positive clones from panning 3.
Sequencing was done on 20 positive clones from panning 1 which results in 16 unique Fab's, on 15 positive clones from panning 2 which results in 9 unique Fab's and on 25 positive clones from panning 3 which results in 4 unique Fab's.

The purified Fab's were analyzed by ELISA against the respective Fc fusion protein (human HIDE1 or mouse HIDE1). ELISA results indicated binding of the purified Fab's to the fusion proteins (data not shown).

The purified Fab's were also analyzed by WB. The antibodies are not applicable for WB (data is not shown).

### Analysis of the purified Fab's anti human HIDE1 and anti-mouse HIDE1 by FACS

The performance of the purified Serotec's Fab's against human HIDE1 and mouse HIDE1, in FACS application were tested using HEK293 cell over-expressing human HIDE1 Flag or mouse HIDE1. HEK293 cells transduced with an empty vector were used as a negative control.

As shown in **Figure 23** the binding of 12 out of 16 anti-human HIDE1 Fab's (1-16) to the HEK293 cells over-expressing human HIDE1 Flag protein (blue line) is higher than the binding of these Fab's to the empty vector cells (red line), the Fab's didn't bind to the HEK293 cells over-expressing mouse HIDE1 Flag protein (orange line).

As shown in **Figure 24** the binding of 8 out of 13 anti-mouse HIDE1 Fab's (1-13) to the HEK293 cells over-expressing mouse HIDE1 Flag protein (orange line) is higher than the binding of these Fab's to the empty vector cells (red line), two Fab's also bind to the HEK293 cells over-expressing human HIDE1 Flag protein (blue line).

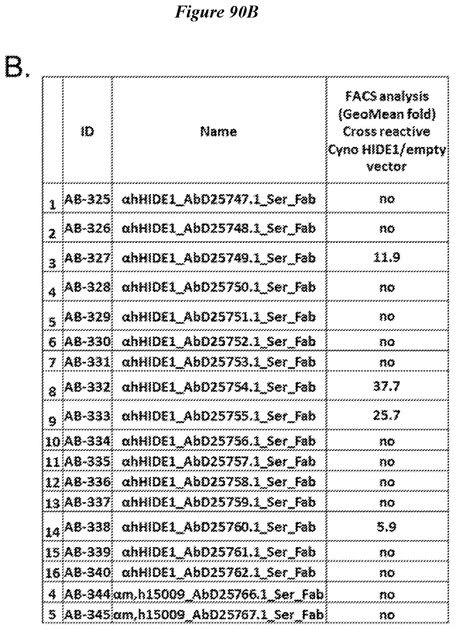

As shown in **Figure 90** the binding of 4 out of 18 anti-human HIDE1 Fab's (1-16) and anti-mouse HIDE1 Fab's (4,5) to the HEK293 cells over-expressing cyno HIDE1 protein (lower panel) is higher than the binding of these Fab's to the empty vector cells (upper panel).

### Affinity mesurements

The purified Fab's were also measured for affinity by AbD Serotec. **Table 19** and **Table 20** summarize the obtained avidity for anti human HIDE1 and anti mouse HIDE1 Fab's.

**Table 19 Serotec measurements of the kinetic rate constants and KD values for anti human HIDE1 antibodies.**

| **Alternate designation** | **Sample ID** | ***kₐ*** | ***k_{d}*** | **K_{D}** |
|---|---|---|---|---|
| | **(Antibody)** | **[1/s]** | **[1/s]** | **[nM]** |
| | AbD25747.1* | 1.77E+06 | 1.37E-02 | 7.7 |
| | AbD25748.1* | 7.32E+06 | 1.06E-02 | 1.4 |
| | AbD25749.1* | 8.61E+05 | 4.89E-03 | 5.7 |
| | AbD25750.1* | 8.19E+05 | 2.13E-03 | 2.6 |
| AB-506 | AbD25751.1 | 5.23E+04 | 5.48E-05 | 1.0 |
| | AbD25752.1* | 5.90E+05 | 3.08E-03 | 5.2 |
| AB-507 | **AbD25753.1** | **6.02E+05** | **4.45E-05** | **0.07** |
| AB-508 | AbD25754.1 | 6.09E+05 | 6.09E-05 | 0.10 |
| AB-509 | **AbD25755.1** | **1.17E+06** | **2.65E-05** | 0.02 |
| | AbD25757.1 | 1.02E+06 | 7.01E-05 | 0.07 |
| | AbD25758.1 | 1.29E+06 | 1.45E-04 | 0.11 |
| | AbD25759.1* | 1.41E+06 | 1.45E-04 | 0.10 |
| 4B-510 | **AbD25760.1** | **5.26E+05** | **1.72E-05** | **0.03** |
| | AbD25762.1 | 1.57E+05 | 1.18E-03 | 7.5 |
| | AbD25766.1 | 9.17E+05 | 5.55E-05 | 0.06 |
| | AbD25767.1 | 1.02E+06 | 5.72E-05 | 0.06 |

| | | | | |
|---|---|---|---|---|
| * heterogeneous binding | | | | |

**Table 20 Serotec measurements of the kinetic rate constants and KD values for anti mouse HIDE1 antibodies.**

| **Sample ID** | **ka** | **kd** | **KD** |
|---|---|---|---|
| **(Antibody)** | **[1/s]** | **[1/s]** | **[nM]** |
| **AbD25763.1** | **1.12E+06** | **8.78E-04** | **0.8** |
| **4bD25764.1** | **1.43E+06** | **1.81E-03** | **1.3** |
| **AbD25765.1** | **1.02E+06** | **8.71E-04** | **0.9** |
| **AbD25766.1** | **3.40E+05** | **5.89E-05** | **0.17** |
| **AbD25767.1** | **3.91E+05** | **5.11E-05** | **0.13** |
| **4bD25918.1** | **1.31E+06** | **1.34E-03** | **1.0** |
| **AbD25921.1** | **7.56E+05** | **1.43E-04** | **0.19** |
| **AbD25962.1** | **1.30E+06** | **9.50E-04** | **0.7** |

### Binning Epitope

The purified Fab's were also subjected to epitope binning by AbD Serotec. **Figure 91****,** **Figure 92****,** **Figure 93** **and** **Figure 94** summarize the binning results for anti human HIDE1 and anti mouse HIDE1 Fab's

**Figure 91** shows that antibodies from group 1 [AbD25747, AbD25748, AbD25749, AbD25751 (AB-506), AbD25752, AbD25762, AbD25750, and AbD25760 (AB-510)] share mostly the same epitope "A". AbD25760 seems to bind to another epitope B. AbD25750 share properties of both epitope A and B, and is therefore grouped into the overlapping epitope A/B. **Figure 92** shows that antibodies from group 2 [Group 2: AbD25757, AbD25759, AbD25766, and AbD25767] AbD25757 and AbD25759 can be probably grouped together in epitope C. AbD25766 and AbD25767 seem to bind to another epitope D. **Figure 93** shows that all antibodies from group 3 [AbD25753 (AB-507), AbD25754 (AB-508), AbD25755 (AB-509), and AbD25758] probably share the same epitope E.

**Figure 94** shows the binning for anti mouse HIDE1 Fab's, AbD25763, AbD25765, AbD25918 and AbD25962 share the same epitope A. AbD25764 has an overlapping epitope; parts are derived from epitope A of the above mentioned antibodies and from epitope B of AbD25766 and AbD25767. AbD25766 and AbD25767 share the same unique epitope B. AbD25921 binds to another unique epitope C.

### Analysis of the reformatted Fab's (full hIgG1 Abs) performance in Flow Cytometry (FACS)

The performance of the reformatted Serotec's antibodies against human HIDE1, in FACS application were tested using HEK293 cell over-expressing human HIDE1 Flag, mouse HIDE1 or cyno HIDE1. HEK293 cells transduced/ transfected with an empty vector were used as a negative control

As shown in **Figure 95****.** the binding of 5 out of 5 anti-human HIDE1 reformatted antibodies (1-5) to the HEK293 cells over-expressing human HIDE1 Flag protein (orange line) is higher than the binding of these reformatted antibodies to the empty vector cells (blue line), the reformatted antibodies didn't bind to the HEK293 cells over-expressing mouse HIDE1 Flag protein (light green line). Three out of five reformatted antibodies (3,4 and 5) are CR to the HEK293 cells over-expressing cyno HIDE1 protein (dark green line).

### Affinity measurements of the reformatted Fab's using Flow Cytometry (FACS)

The affinity The affinity measurements of the reformatted Serotec's Ab's (full hIgG1 Abs) against human HIDE1, using FACS application were tested using CHO-S cell over-expressing human HIDE1. CHO-S cells transduced with an empty vector were used as a negative control.

As shown in **Figure 96****.** the binding of 5 out of 5 anti-human HIDE1 reformatted Ab's (1-5) to the CHO-S cells over-expressing human HIDE1 Flag protein (circle dots) is higher than the binding of these reformatted Ab's to the empty vector cells (square dots). Two of the five antibodies (2 and 3) reaches saturation at the concentrations that were measured. The Kd value and R square are represented in the figure.

### Conclusions and summary of anti HIDE1 custom Abs

The following custom antibodies generated were validated and used for further target characterization:
Fab's anti-human HIDE1; Serotec (ID: AB-326, AB-327, AB-328, AB-329, AB-331, AB-332, AB-333, AB-335, AB-336, AB-337, AB-338, AB-340, see **Table 21)** and reformatted Fab's anti-human HIDE1; Serotec (ID: AB-506, AB-507, AB-508, AB-509, AB-510, see **Table 21)** validated for FACS application on ectopic expression, with no cross reactivity to mouse HIDE1 protein, and with cross reactivity of four Fab's (ID: AB-327, AB-332, AB-333, AB-338, see **Table 21)** and three reformatted antibodies (ID: AB-508, AB-509, AB-510, see **Table 21)** to cyno HIDE1 protein.
Fab's anti-mouse anti-HIDE1 antibody; Serotec (ID: AB-341, AB-342, AB-343, AB-344, AB-345, AB-354, AB-357, AB-359, see **Table 22)** validated for FACS application on ectopic expression, with cross reactivity of two antibodies (ID: AB-344, AB-345) to human HIDE1 protein.

**Table 21:Serotec anti-human HIDE1 Fab's and mAbs.**

| **ID** | **Name** |
|---|---|
| **AB-326** | **αhHIDE1_AbD25748.1_Ser Fab** |
| **AB-327** | **αhHIDE1_AbD2S749.1_Ser_Fab** |
| **AB-328** | **αhHIDE1_AbD25750.1_ser_Fab** |
| **AB-329** | **αhHIDE1_AbD25751.1_Ser_Fab** |
| **AB-331** | **αhHIDE1_AbD25753.1_Ser_Fab** |
| **AB-332** | **αhHIDE1_AbD25754.1_Ser_Fab** |
| **AB-333** | **αhHIDE1_AbD25755.1_Ser_Fab** |
| **AB-335** | **αhHIDE1_AbD25757.1_Ser_Fab** |
| **AB-336** | **αhNIDE1_AbD25758.1_Ser_Fab** |
| **AB-337** | **αhHIDE1_AbD25759.1_Ser_Fab** |
| **AB-338** | **αhHIDE1_AbD25760.1_Ser_Fab** |
| **AB-340** | **αhHIDE1_AbD25762.1_Ser_Fab** |
| **AB-506** | **αhHIDE1_AbD25751_hlgG1_Ser_mAb** |
| **AB-507** | **αhHIDE1_AbD25753_hlgG1_Ser_mAb** |
| **AB-508** | **αhHIDE1_AbD25754_hlgG1_Ser_mAb** |
| **AB-509** | **αhHIDE1_AbD25755_hlgG1_Ser_mAb** |
| **AB-510** | **αhHIDE1_AbD25760_hlgG1_Ser_mAb** |

**Table 22: Serotec anti-human HIDE1 Fab's.**

| **ID** | **Name** |
|---|---|
| **AB-341** | **αmHIDE1_AbD25763.1_Ser_Fab** |
| **AB-342** | **αmHIDE1_AbD25764.1_Ser_Fab** |
| **AB-343** | **αmHIDE1_AbD25765.1_Ser_Fab** |
| **AB-344** | **αmHIDE1_AbD25766.1_Ser_Fab** |
| **AB-345** | **αmHIDE1_AbD25767.1_Ser_Fab** |
| **AB-354** | **αmHIDE1_AbD25918.1_Ser_Fab** |
| **AB-357** | **αmHIDE1_AbD25921.1_Ser_Fab** |
| **AB-359** | **αmHIDE1_AbD25962.1_Ser_Fab** |

Five reformatted Fab's anti-human HIDE1; Serotec were characterized for affinity. Two of the five antibodies (AB-507 and AB-508) reached saturation at the concentrations that were measured.

### EXAMPLE 11: ENDOGENOUS EXPRESSION OF HIDE1

### Endogenous expression of HIDE1 protein in human and mouse cell lines

The aim of this study was to identify human and mouse cancer cell lines that endogenously express HIDE1.

### MATERIALS AND METHODS

### MATERIALS

• RNA extraction was performed with RNAeasy Mini Kit (Qiagen cat # 74014).
• cDNA was produced using High Capacity cDNA Reverse Transcription Kit (Applied Biosystems cat#4368814).
• Specific primers for human and mouse were designed on separate exons and detailed in **Table 27** and **Figure 25**
• Human HIDE1 TaqMan probes: Hs01128129_m1, Hs01128131_m1, Life technologies.
• Human housekeeping gene, hRPL19 TaqMan probe: Hs01577060, Life technologies.
• Mouse HIDE1 TaqMan probe: HOJEX12_CCAAZKY, custom made, Life technologies.
• Mouse housekeeping gene, mRPL19 TaqMan probe: Mm02601633_g1, Life technologies.
• Commercial cDNA panel I, Mouse, Biochain, Cat no. C8334501
• Commercial cDNA panel II, Mouse, Biochain, Cat no. C8334502
• Commercial Human and Mouse cell lines from American Type Culture Collection (ATCC) are detailed in **Table 23 and 24,** respectively.

**Table 23 Human cell lines**

| **Cell line** | **Cat. Number** | **Morphology** | **Source** |
|---|---|---|---|
| HL-60 | ATCC CCL-240 | Leukemia | Blood |
| THP1 | ATCC TIB-202 | Monocyte | Blood |
| KG-1 | ATCC CCL-246 | Macrophage | Bone marrow |
| Jurkat | ATCC TIB-152 | T Cell | Blood |
| NCI-H929 | ATCC CRL-9068 | B lymphocyte | Bone marrow |
| A-704 | ATCC HTB-45 | Epithelial | Kidney |
| RPMI8226 | ATCC CCL-155 | B lymphocyte | Blood |
| NCI-H28 | ATCC CRL-5820 | Epithelial | Lung |
| U937 | ECACC 85011440 | Monocyte | Blood |

**Table 24. Mouse cell line**

| Cell line | ATCC No. | Morphology | Source |
|---|---|---|---|
| 4T1 | CRL-2539 | Epithelial | Mammary gland |
| B16-F1 | CRL-6323 | Spindle-shaped+epithelial-like cells | Skin |
| EL4 | TIB-39 | T-lymphoblast | Blood |
| E.G7-OVA | CRL-2113 | T-lymphoblast | Blood |
| YAC-1 | TIB-160 | T-lymphoblast | Blood |
| A20 | TIB-208 | B-lymphoblast | Blood |
| P815 | TIB-64 | mast cells | Blood |
| NIH/3T3 | CRL-1658 | fibroblast | Embryo |
| Sal/N | CRL-2544 | fibroblast | Fibrosarcoma |
| J774A.1 | TIB-67 | Macrophage | Blood |
| LL/2 | CRL-1642 | Epithelial | Lung |
| B104-1-1 | CRL-1887 | Glioblastoma | Neuroblastoma |
| RAW264.7 | TIB-71 | macrophage | Blood |
| P388D1 | CCL-46 | macrophage | Blood |
| KLN205 | CRL-1453 | Epithelia | Lung |
| CT26.WT | CRL-2638 | fibroblast | Colon |

• Commercial polyclonal rabbit anti-Human HIDE1, Sigma cat HPA012150.
• Custom polyclonal rabbit anti-mouse HIDE1 Genscript, ID# 488536_13.
• Rabbit IgG whole molecule, Jackson, cat # 011-000-003.
• Goat Anti Rabbit-HRP, Jackson, cat # 111-035-003.
• Donkey anti Rabbit-PE, Jackson cat # 711-116-152.
• Custom mouse monoclonal anti-human HIDE1,(BIOTEM# 33B4-2F7), 36C1-2F6.
• Custom mouse monoclonal anti-human HIDE1, Ab-364 (BIOTEM# 33B4-2F7-AF647)
• Human IgG1 Isotype control lot. F1150528d-2695- Alexa 647, BIOTEM
• Mouse IgG1 isotype control, Life technologies cat # MG100.
• Goat Anti Mouse-PE, Jackson, cat # 115-116-146.
• AB329, anti-human HIDE1 F(ab')2 AbD25751.1, Serotec
• Ab333, anti-human HIDE1 F(ab')2 AbD25755.1, Serotec
• Ab332 anti-human HIDE1 F(ab')2 AbD25754.1, Serotec
• Ab360 anti -mouse HIDE1 F(ab')2 AbD25963.1, Serotec
• Ab359 anti -mouse HIDE1 F(ab')2 AbD25962.1, Serotec
• Ab-345 anti -mouse HIDE1 F(ab')2 AbD25767.1, Serotec
• Non Relevant F(ab')2 control: Ab 307, AbD25909.1, Serotec
• Goat Anti Human IgG F(ab')2-PE, Jackson, cat# 109-116-097
• VioBlue, Fixable viability stain 450, BD Bioscience, cat # 562247.
• Human Fc block, Trustain FcX, Biolegend, cat#422302.
• Mouse BD Fc Block, Rat Anti-Mouse CD16/CD32, BD Pharmingen, cat.553142.
• Cell line Nucleofector kit V, Lonza # VACA-1003.
• Cell line Nucleofector kit T, Lonza # VACA-1002.
• On-TARGET plus human C19orf38 siRNA SMART pool L-023981-02, Dharmacon.
• On-TARGET plus Mouse AB124611 siRNA SMART pool L-063774-01, Dharmacon.
• On-TARGET plus non targeting (scrambled) siRNA SMART pool D-001810-01-05, Dharmacon.

### Quantitate real time PCR (qRT-PCR)

RNA (1-5ug) extraction of human and mouse cell lines (detailed above in **Tables 23 and 24)** was preformed according to manufactures protocols.

cDNA was prepared according to manufactures protocols (lug RNA diluted in 20ul cDNA mix reaction).

### Two different approaches were used:

### qRT-PCR using SYBR green

cDNA, prepared as described above, diluted 1:10 (representing 25ng RNA per reaction) in DDW was used as a template for qRT-PCR reactions using the SYBR Green I assay (PE Applied Biosystem) with specific primers (listed in **Table 27).** Detection was performed using the PE Applied Biosystem SDS 7000 or QuantStudio 12k flex device.

The cycle in which the reactions reached a threshold level of fluorescence (Ct= Threshold Cycle,) was registered and was used to calculate the relative transcript quantity in the RT reactions. The relative quantity was calculated using the equation Q=Efficiency ^^{-Ct}. The efficiency of the PCR reaction was calculated from a standard curve, created by using serial dilutions of mouse commercial cDNA mixed panels (Cat no. C8334501 and C8334502, Biochain), and for human efficiency calculations, several cDNA from tissues and cell lines that were prepared in house, created for each primer set.

The resulting quantities were normalized to quantities of a housekeeping gene (hHPRT1 or mHPRT1).

### qRT-PCR using TaqMan:

cDNA, prepared as described above, diluted 1:10 (representing 25ng RNA per reaction) in DDW, was used as a template for qRT-PCR reactions, using a gene specific TaqMan probes (detailed in Materials above). Detection was performed using QuantStudio 12k flex device.

The cycle in which the reactions achieved a threshold level of fluorescence (Ct= Threshold Cycle) was registered and was used to calculate the relative transcript quantity in the RT reactions.

The absolute gene quantity was calculated by using the equation Q=2 ^^{-Ct}.

The resulting quantities were normalized to quantities of human or mouse housekeeping gene, hRPL19 or mRPL19 relatively.

### Protein expression detection by Western Blot (WB)

The expression of HIDE1 in human and mouse cell lines was analyzed by WB using whole cell extracts (50-75 µg for the cancer cell lines, and 30µg for the over expressing cell line and negative control cell line)

For human HIDE1 protein detection:
- Commercial rabbit polyclonal anti-human HIDE1 pAb, Sigma, cat # HPA012150, diluted to 2µg/ml in 5% BSA/TBST followed by secondary Ab goat anti-Rabbit - Peroxidase conjugated (Jackson, cat # 111-035-003), diluted 1:20,000 in 5% milk TBST.
- Custom mouse monoclonal anti human HIDE1, 36C1-2F6, BIOTEM, diluted to 2µg/ml in 5% BSA/TBST followed by secondary Ab Goat Anti Mouse-HRP (Jackson, cat# 115-035-146), diluted 1:20,000 in 5% milk TBST.

### For mouse HIDE1 protein detection:

- Custom rabbit polyclonal anti-mouse HIDE1 pAb (GenScript, ID# 488536_13) diluted to 2µg/ml in 5% BSA/TBST followed by a secondary Ab goat anti-rabbit -Peroxidase conjugated, diluted 1:20,000 in 5% milk TBST.

### Protein expression analysis by Flow Cytometry (FACS)

The cell surface expression of HIDE1 protein was analyzed by FACS. Human or mouse cell lines were stained with VioBlue reagent diluted 1:1000 in PBS. Cells were incubated 10 min at R.T. and then washed twice with FACS buffer (0.5% BSA in PBS). Cell lines for endogenous protein analysis were pre-incubated with the Fc receptor blocking solutions listed above in material section (2.5 µl/reaction of human blocker and 0.5mg/ml of mouse blocker were used according to the manufactures procedures). To detect the human HIDE1 protein, cells were stained with a custom mouse monoclonal anti human HIDE1 mAbs (BIOTEM, detailed in material section above) diluted to a concentration of 10µg/ml or IgG1 Isotype control at the same concentration followed by Goat anti mouse PE conjugated Ab. To detect the mouse HIDE1 protein, cells were stained with a custom rabbit polyclonal anti-mouse HIDE1 pAb (GenScript, ID# 488536_13) diluted to a concentration of 10µg/ml or rabbit IgG whole molecule as isotypes control at the same concentration followed by Donkey anti Rabbit-PE conjugated Ab diluted 1:100.

To detect the mouse HIDE1 protein, cells were stained with a custom rabbit polyclonal anti-mouse HIDE1 pAb (GenScript, ID# 488536_13) diluted to a concentration of 10µg/ml or rabbit IgG whole molecule as isotypes control at the same concentration followed by Donkey anti Rabbit-PE conjugated Ab diluted 1:100.

### HIDE1 knock down

Knock down of endogenous mouse or human HIDE1 was carried out by transient transfection of siRNA. Transfection of 50 pmol HIDE1 siRNA pool or scrambled siRNA performed by electroporation using Amaxa nucleofector device and Amaxa nucleofector kits as listed above and according to the manufacture procedure. 48 hours post transfection, cells were collected for further analysis by qRT-PCR and FACS. EL4 cell line was further tested by FACS 72 hours post transfection.

### RESULTS

### ENDOGENOUS EXPRESSION OF HIDE1 IN HUMAN CELL LINE

### Endogenous expression of HIDE1 transcripts in human cell lines detected by qRT-PCR

In order to verify the presence of the HIDE1 transcript in human cell lines (listed in Table 23), qRT-PCR was performed using a specific primers (**Table 27** and **Figure** 25.) or using TaqMan probes as describe above in Material & Methods.

As shown in **Figure 26** human HIDE1 transcript is observed using TaqMan probe Hs01128131_m1 with relatively high levels in HL-60, KG1 and U937 cell lines. Lower transcript level is observed in TFIP1 cell line, then A704 and NCI-H28 cell lines with lower transcript levels. NCI-H929 and RPMI8226 cell lines shows low to no transcript. Similar results were obtained using TaqMan probe Hs01128129_m1 and specific primers, SYBR green (data is not shown).

### Endogenous expression of HIDE1 proteins in human cell lines detected by WB

WB analysis for endogenous expression of HIDE1 protein was carried out on various human cancer cell lines lysates as detailed in **Table 23** using commercial anti human HIDE1 pAb (Sigma, HPA012150) or custom anti human HIDE1 mAb (BIOTEM, 36C1-2F6) as described in Materials & Methods above. As a positive control, whole cell extract of stable HEK293T cell pool over- expressing HIDE1 was used while cells transfected with an empty vector served as the negative control.

WB results related to positive and negative controls were as expected. WB results for endogenous expression were inconclusive as multiple bands were detected using the pAb, and no signal was observed using the mAb (data is not shown).

### Endogenous expression of HIDE1 proteins in human cell lines detected by FACS

To verify the cell-surface endogenous expression of human HIDE1, various cell lines (detailed in **Table 23**) were tested as described in Material & Methods above. The cell lines were stained with the custom monoclonal anti human-HIDEl mAbs: BIOTEM, 33B4-2F7, 36C1-2F6 (**Figure 27**, orange line, upper or lower panels respectively), or with Isotype control (**Figure 27**, light blue) followed by a secondary goat anti mouse PE Ab. As an additional negative control, cells were stained with the secondary antibody only (**Figure 27**, red line). Analysis was performed by FACS. As shown in **Figure 27** binding of the BIOTEM 33B4-2F7 antibody was observed in three human cancer cell lines U-937, THP-1 and HL-60 as compared to isotype control binding. Binding of the BIOTEM 36C1-2F6 antibody was observed only in U-937 cell line. No binding of either of the two BIOTEM antibodies was observed in NCI-H929 and Jurkat cell lines.

Further analysis for endogenous confirmation of human HIDE1 in THP1 and U937 cell lines, was done by testing various anti-human HIDE1 F(ab')2, produced by Serotec.

Both cell lines were stained with anti-human HIDE1 custom F(ab')2s (Serotec, detailed in Material & Methods)(**Figure 28**, A, B), or with Non relevant control anti-human F(ab')2, Ab307, (**Figure 28**, A, B light blue) followed by a secondary goat anti-human F(ab')2 PE Ab. As an additional negative control, cells were stained with the secondary antibody only (**Figure 28**, A, B red line). Analysis was performed by FACS. As shown in **Figure 28****, A, B**, expression of human HIDE1 in U937 human cell line was observed by Ab329, Ab333 and AbD332 Serotec F(ab')2 only as compared to isotype control expression. No expression of human HIDE1 was observed in U937 cell line by using the other F(ab')2.

No expression of human HTDE1 was observed in THP-1 human cell line by either of the various F(ab')2 (data not shown).

### Knock down of human HIDE1 by siRNA in human cancer cell lines

In order to further confirm endogenous expression of HIDE1 protein in HL-60 and U937 cell lines, human HIDE1 siRNA pool was used for knock down as described in Material & Methods.

48 hours post siRNA transfection, cells were harvested for further analysis by qRT-PCR or by FACS. As shown in **Figure 29** human HIDE1 transcript level in HL-60 (A) or U973 (B) cells transfected with human HIDE1 siRNA pool is significantly reduced as compared to cells transfected with scrambled siRNA or untreated cell lines.

Further analysis of human HIDE1 membrane expression in the same siRNA transfected cells was performed by FACS. As shown in **Figure 30** membrane expressions of human HIDE1 protein is reduced in cells transfected with HIDE1 siRNA (green) as compared to cells transfected with scrambled siRNA (orange). The fold change (anti HIDE1 vs, Isotype control) in U937 cell line is decreased from 17.7 fold to 7 fold .For HL-60 cell line the fold is changed from 5.2 fold to 3 fold.

Confirmation of HIDE1 endogenous expression was also tested in THP1 cells transfected with HIDE1 siRNA as described in M&M section. Analysis of human HIDE1 membrane expression in the siRNA transfected cells was performed by FACS. As shown in **Figure 31** membrane expressions of human HIDE1 protein is reduced in cells transfected with HIDE1 siRNA (green) as compared to cells transfected with scrambled siRNA (orange). The fold change (anti HIDE1 vs, Isotype control) in THP1 cell line is decreased from 3 fold to 1.9 fold.

Additional knock down experiment of human HIDE1 was performed in U937 cell line. Confirmation of human HIDE1 membrane expression in U937 cell line was tested by using the positive Serotec F(ab')2 (Ab329, Ab332 and Ab333 have already showed expression in U937 cell line in **Figure 28**).

As shown in **Figure 32** (A, B, C) membrane expressions of human HTDE1 protein is reduced in U937 cells transfected with HIDE1 siRNA (green) as compared to cells transfected with scrambled siRNA (orange). The fold change (anti HIDE1 vs, non-relevant F(ab')2, Ab307) in U937 cell line is decreased from 2.4 fold to 1.6 fold detected by Ab329, or from 1.9 fold to 1.3 fold detected by Ab332 or from 1.9 fold to 1.2 fold detected by Ab333.

### ENDOGENOUS EXPRESSION OF HIDE1 IN MOUSE CELL LINES

### Endogenous expression of HIDE1 transcripts in mouse cell lines detected by qRT- PCR

In order to verify the presence of the HIDE1 transcript in mouse cell lines (listed in **Table 24**), qRT-PCR experiment was performed using specific primers (Figure 25 and **Table 27**) or TaqMan probes as describe above in Material & Methods and in **Figure 25**.

As shown in **Figure 33** mouse HIDE1 transcript, detected by specific primers, SYBR green (A) was observed in J774A.1, EL4, YAC-1 and A20 cell lines, with relatively high transcript level, and lower transcript levels in the other tested cell lines. The presence of mouse HIDE1 transcript was verified in additional cell lines using TaqMan probes (B).Transcript was observed in RAW264.7 and P388D1 cell lines but not in other cell lines tested.

### Endogenous expression of HIDE1 protein in mouse cell line detected by WB

WB analysis for endogenous expression of HIDE1 protein was carried out on various mouse cancer cell lines as detailed in **Table 24**. Using GenScript anti mouse HIDE1 pAb, 488536_13 described in Materials & Methods above. As a positive control, whole cell extract of stable HEK293 cell pool ectopically expressing mouse HIDE1 was used while cells transfected with an empty vector served as the negative control. WB results related to positive and negative controls were as expected. WB results for endogenous expression were inconclusive as multiple bands were detected (data is not shown).

### Endogenous expression of HIDE1 protein in mouse cell lines detected by FACS

To verify the cell-surface endogenous expression of mouse HIDE1 protein, various cell lines (detailed in **Table 24**, were tested as described in Material & Methods above. The mouse cell lines were stained with a rabbit anti mouse HIDE1 pAb (Genescript, ID 488536_13) (Orange) or with the Isotype control (light blue) followed by Goat anti rabbit PE Ab (Jackson cat # 711-116-152). As additional negative control, cells were stained with the secondary antibody only and represented by a red line. Analysis was performed by FACS. As shown in **Figure 34** Higher binding of the anti-mouse-HIDEl antibody was observed in three mouse cell lines YAC-1, EL4 and J77A4.1 as compared to isotype control binding, with high background of the isotype control Ab on the J774A.1 cell line. No binding was observed in B16-F1 and Sal/N cell lines.

Further analysis of mouse HIDE1 endogenous expression in EL4 and J774A.2 cell lines, was done by FACS using various F(ab')2 produced by Serotec.

Both cell lines were stained with anti-mouse HIDE1 custom F(ab')2s produced by Serotec **(****Figure 35****, A, B, C**), or with anti-human Non relevant F(ab')2, , Ab307, which used as a negative control (**Figure 3**5, light blue) followed by a secondary goat anti-human F(ab')2 PE Ab. As an additional negative control, cells were stained with the secondary antibody only **(****Figure 35****,** red line). As shown in **Figure 35****, A, B** expression of mouse HIDE1 was observed in EL4 mouse cell line by the following F(ab')2: Ab345 (Figure 35A, purple line), Ab360 and Ab359 (**Figure 35B****,** yellow and blue lines respectively) as compared to isotype control expression. No expression was observed in EL4 cell line by the other F(ab')2.

Expression of mouse HIDE1 was observed also in J774A.2 mouse cell line by the following F(ab')2 as shown in **Figure 35C**: Ab360 and Ab359 (yellow and blue lines respectively) as compared to isotype control expression. No expression was observed in J774A.2 cell line by the other F(ab')2.

### Knock down of mouse HIDE1 in mouse cell lines

In order to confirm endogenous expression of mouse HIDE1 protein in EL4 cell line, mouse HIDE1 siRNA pool was used for knock down experiment as described in Material & Methods.

48 hours post siRNA transfection, cells were harvested for further analysis by qRT-PCR and FACS. As shown in **Figure 36** mouse HIDE1 transcript level in EL4 cells transfected with mouse HIDE1 siRNA pool is significantly reduced compared to cells transfected with the scrambled siRNA or untreated cell lines.

48 or 72 hours post siRNA transfection, cells were harvested and were analysed by FACS for membrane expression of mouse HIDE1. As shown in **Figure 37** membrane expressions of mouse HIDE1 protein 48 hours post transfection is slightly lower in cells transfected with HIDE1 siRNA (green) as compared to cells transfected with scrambled siRNA (orange). The fold change (anti HIDE1 vs. isotype control) is decreased from 7 fold to 4.4 fold. Expression in cells 72hr post siRNA transfection was not decreased following FACS staining.

72 hours post siRNA transfection membrane expression of mouse HIDE1 was very similar to the fold in cells transfected with the HIDE1 siRNA (data not shown).

### SUMMARY:

This example includes preliminary data on HIDE1 endogenous expression in cell lines both at the RNA level and the protein level in human and mouse cell lines.

Various human cancer cell lines were tested by qRT-PCR, WB and FACS for endogenous expression of HIDE1.

Cell surface expression was observed in HL-60, U937, and THP1 cell lines using the mouse monoclonal Ab (BIOTEM, 33B4-2F7) as shown in **Figure 27** as well as by using 3 anti-human F(ab')2 (Serotec) (*Ab329, Ab333, Ab332),* which showed cell surface expression in U937 cell line as shown in **Figure 28****.** These observations are in correlation to RNA transcript levels as shown in **Figure 26****.** Additional confirmation in HL-60 and U937 cell lines was done by knock down experiment confirming clear reduction in the RNA transcript following HIDE1 siRNA transfection, as shown in **Figure 29**, as well as some reduction observed in the protein cell surface expression as shown in **Figures 30** **and** **31****.** However, WB results were not conclusive to determine a clear band at the expected band size (data not shown).

Various mouse cell lines were tested by qRT-PCR, WB and FACS for endogenous expression of HIDE1. In the transcript level, presence of HIDE1 was observed in J774A.1, EL4, YAC-1 and A20 cell lines as well as in RAW264.7 and P388D1 cell lines as shown in **Figure 32**. Correlation between positive transcript expression and cell surface protein expression was observed in J774A.1, EL4, YAC-1 as shown in **Figures 33** **and** **34**. Additional confirmation in EL4 cell line was done by knock down experiment confirming clear reduction in the RNA transcript following HIDE1 siRNA transfection, as shown in **Figure 36**. However, the reduction in the cell surface protein as measure by FACS was low under this experimental conditions. Further knock down experiments to verify these observations are currently ongoing.

**Tables 25** and **26** below indicate the summary of the findings described in this report, highlighting the cell lines showing correlation between qPCR and FACS, confirmed by knock down. Further analyses are currently on going.

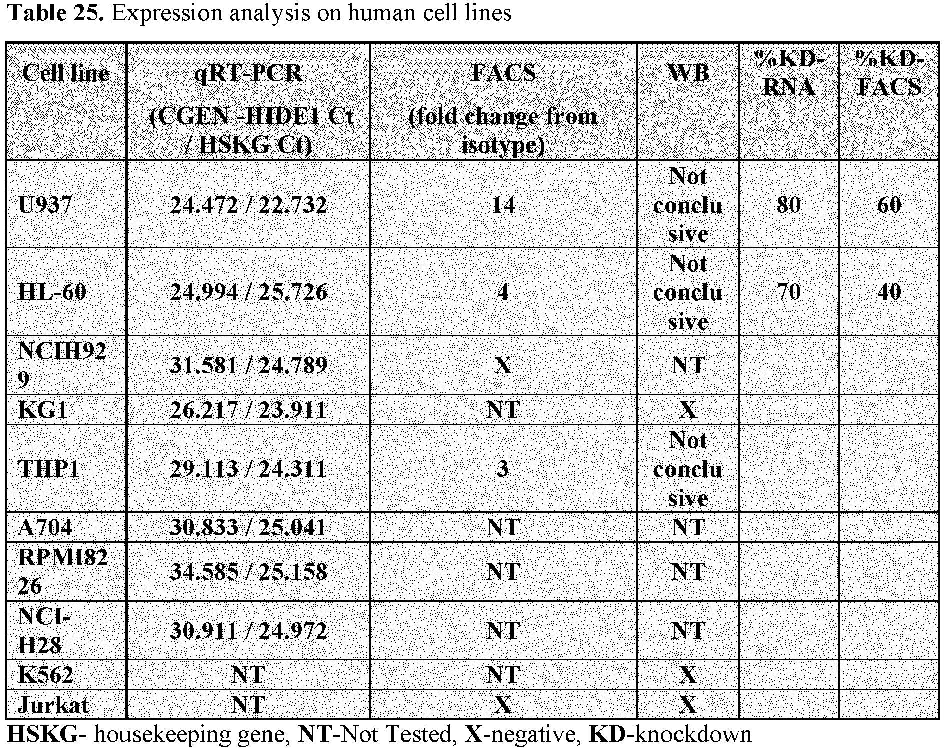

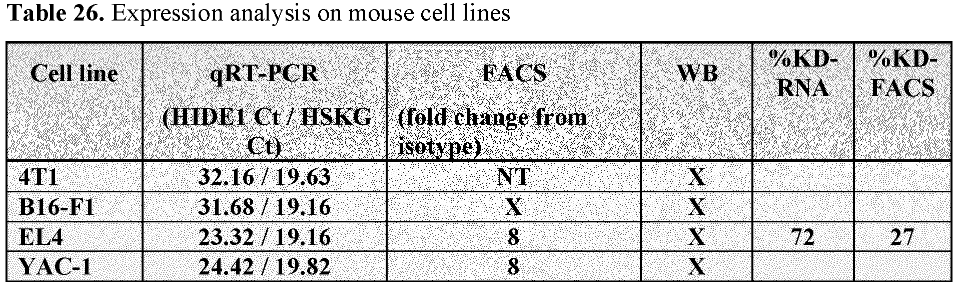

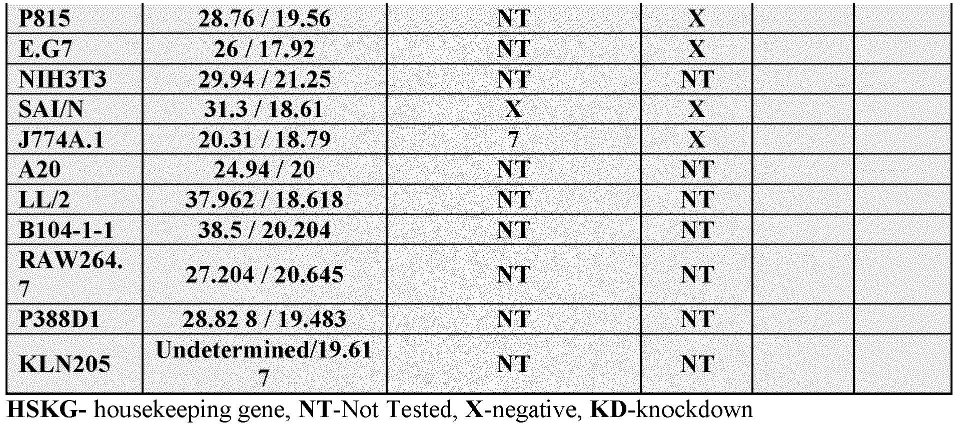

**TABLE 27. Primers Sequences Of Mouse And Human HIDE1 And The House-Keeping Gene Mouse HPRT1**

| Primer name | Primer sequence |
|---|---|
| 200-774 hHIDE1_F | GCCCAGATTAACTTCGACAG |
| 200-775 hHIDE1_R | CTGAGTGATCATCCAAGGTG |
| 200-780 mHIDE1_F | TGGGCTCAGATCAACTTCAC |
| 200-781 mHIDE1_R | CCACTGAGTCTTCCTGAGTC |
| 200-824 hHPRT1_F | TGACACTGGCAAAACAATGCA |
| 200-825 hHPRT1_R | GGTCCTTTTCACCAGCAAGCT |
| 200-719 mHPRT1_F | GCAGTACAGCCCCAAAATGG |
| 200-720 mHPRT1_R | TGCAGATTCAACTTGCGCTC |

### EXAMPLE 12: HIDE1 EXPRESSION ON HUMAN PBMCs

To evaluate the expression of human HIDE1 on human peripheral mononuclear cells (PBMCs) using 2 monoclonal antibodies for HIDE1 by flow cytometry.

### Materials and Methods

**Isolation of PBMCs:** Buffy coats were obtained from Stanford Blood Bank from healthy human donors. PBMCs were isolated by Ficoll gradient separation, washed with medium, and cryo-preserved for future experiments.

**Antibodies:** The following antibodies were used. Alexa flour 647 labeled mouse IgG1 isotype control (lot. F1150528d-2695, BIOTEM), Alexa flour 647 labeled anti-human HIDE1 antibodies (BIOTEM, 33B4-2f7 and 36-C1-2F6). Brilliant violet 510 labeled anti-CD3 (Biolegend, cat. 344828), PE-labeled anti-CD14 (Biolegend, cat. 301806) and PercP-labeled anti-CD 19 (Biolegend, cat. 302228).

### Flow cytometry analysis

PBMCs cells were thawed and stained with viability dye (BD Horizon; Cat# 562247, BD biosciences), washed and pre-blocked with Fc blocking solution (cat# 422302, Biolegend; 1:20) and/or human Ig Fc (Jackson; 200µg/ml; cat# 009-000-008) to avoid nonspecific binding via Fc receptors. Cells were then stained with anti-hHIDEl mAbs or mIgG1 isotype control in the presence or absence of lymphocytes or monocytes surface marker. All samples were run on a MACSQuant analyzer (Miltenyi) and data was analyzed using Tree Star FlowJo software. Cells were first gated for lymphocytes or monocyte (FSC-A vs. SSC-A), and further gated for live cells before analyzed for HIDE1 expression by flow cytometry.

### Results

Freshly thawed PBMCs from 2 donors were thawed and stained with viability dye, washed and pre-blocked with Fc blocking solution as described in material and methods. HIDE1 expression was observed on CD14+ cells but not on CD3+ cells (**Figure 38**) in 2 donors using 2 monoclonal antibodies against HIDE1. The staining was in the range of 3 folds over isotype control. No expression on CD19+ was observed (data not shown). To avoid non-specific binding to Fc receptor the staining was repeated in the presence of addition human Fc fragments with 4 different donors. As shown in **Figure 39** HIDE1 expression was observed in 4 different donors while no expression on lymphocytes was observed (data not shown).

### Summary

HIDE1 expression on human monocytes was observed using 2 anti-human HIDE1 mAb in 4 different donors. The expression was excluded to monocytes. No expression on T cells or B cells was observed. Further experiment are ongoing in order to analyze the expression of HIDE1 on other leukocytes population such as neutrophils and dendritic cells.

### EXAMPLE 13: HIDE1 EX-VIVO RNA EXPRESSION

### Study A:

### Material & Methods

### Reagents used in this study

1. RNA extraction was performed with RNAeasy Mini Kit (Qiagen cat # 74014).
2. cDNA was produced using High Capacity cDNA Reverse Transcription Kit (Applied Biosystems cat#4368814).
3. Mouse HTDE1 TaqMan probe: HOJEX12_CCAAZKY, custom made, Life technologies.
4. TaqMan probes for Housekeeping gene (HSKG) (Life technologies) mouse RPL19: Mm02601633_g1, mouse SDHA: Mm013526363_m1, mouse PBGD: Mm01143545_m1.
5. ABI TaqMan Fast Advanced Master mix, part no. 4444557, Applied Biosystem
6. CT26.WT cells, CRL-2638, ATCC
7. Anti PDL-1 mIgG1, Inc production (YWW243.58.870, LOT #40315,)
8. mIgG1 isotype control (MOPC-21), Biolegend.

### Methods

### Animals:

7wk old female BALB/c mice were purchased from ENVIGO laboratories and kept in specific pathogen-free conditions at the Tel Aviv University animal facility. Experiments respected institutional guidelines and were approved by the Tel Aviv University ethics committee

### Tumor model and mAb treatments:

The murine CT26, an N-nitroso-N methylurethane - induced undifferentiated colon carcinoma cell line, was obtained from American Type Culture Collection (CRL-2638). CT26 cells (5x105) were injected subcutaneously at day 0 in the right flank of BALB/c mice (female, 7wk). Tumor growth was monitored twice a week using a caliper.

Treatments were given twice and started at day 8, when tumor area reached 50mm2 and on day 12 post tumor inoculation. Treatments were injected intraperitoneal at 10mg/Kg (9 mice/treatment) of either anti PDL-1 mIgG1 (YWW243.58.870, LOT #40315,) or mIgG1 isotype control (MOPC-21).

### Tissue samples:

Whole tumor and spleens total RNA was extracted using gentleMACS™ Octo Dissociator (Miltenyi Biotec), and tissues were homogenized with M tubes (# 130-096-335).

For tumor single cell suspension, tumors were enzymatically digested with 1µg/ml Collagenase D (Worthington Biomedical Corporations) 0.5µg/ml Deoxyribonuclease I (Worthington Biomedical Corporations) in RPMI-1670 (Biological Industries) for 1hr in 370c. Remains of undigested tumor tissues were gently agitated for full a dissociation.

### Cells isolation:

Thy1.2+ T lymphocytes were purified from tumors using a CD90.2+ positive isolation kit (EasySep, #18751) and purified Thy1.2 + T lymphocytes and the negative fraction cells were lysed in RLT to extract total RNA.

CD11b+ cells were purified from tumors using a CD11b+ positive isolation kit (EasySep, #18770) and purified CD11b+ cells and the negative fraction cells were lysed in RLT to extract total RNA

### Transcript expression

### Quantitative RT-PCR (qRT-PCR)

RNA (1-5ug) extraction of mouse cells, derived from Ex-vivo experiments (346-MA-455 & 459-MA) was preformed according to manufactures protocols.

cDNA was prepared according to manufactures protocols (lug RNA diluted in 20ul cDNA mix reaction).

cDNA, prepared as described above, diluted 1:10 (representing 25ng RNA per reaction), was used as a template for qRT-PCR reactions, using a gene specific TaqMan probes (detailed in Reagents 3&4) Detection was performed using QuantStudio 12k device.

The cycle in which the reactions achieved a threshold level of fluorescence (Ct= Threshold Cycle) was registered and was used to calculate the relative transcript quantity in the RT reactions.

The absolute quantity was calculated by using the equation Q=2 ^-Ct.

The resulting relative quantities were normalized to a relative quantities of housekeeping gene, mRPL19, mSDHA and mPBGD.

### Results

### Endogenous expression of HIDE1 in mouse cells

### Endogenous expression of HIDE1 in tumor derived cells (Ex vivo# 455)

In order to verify the presence of the HIDE1 transcript in tumor derived cell, qRT-PCR was performed using a specific TaqMan probe as describe above in Material & Methods.

As shown in **Figure 40**, mouse HIDE1 transcript is observed using TaqMan probe HOJEX12-CCAAZKY with relatively high levels in CD11b+ subpopulation cells, compared to tumor derived cells and to CD11b- subpopulation.

### Endogenous expression of HIDE1 in tumor derived cells (Ex vivo# 459)

In order to verify the presence of the HIDE1 transcript in tumor derived cell, qRT-PCR was performed using a specific TaqMan probe as describe above in Material & Methods.

As shown in **Figures 41****,** **42** **and** **43** mouse HIDE1 transcript is observed using TaqMan probe HOJEX12-CCAAZKY with relatively high levels in CD11b+ subpopulation **(****Figures 41****,** **42****, and** **43****),** spleen cells (**Figure 42**) and in colon lamina propria and whole colon cells (**Figure 42**). Lower transcript level is observed in colon epithelia cells.

### Study B

### Material & Methods

### Reagents used in this study

1. RNA extraction was performed with RNAeasy Mini Kit (Qiagen cat # 74014).
2. cDNA was produced using High Capacity cDNA Reverse Transcription Kit (Applied Biosystems cat#4368814).
3. Mouse HTDE1 TaqMan probe: HOJEX12_CCAAZKY, custom made, Life technologies.
4. TaqMan probes for Housekeeping gene (HSKG) (Life technologies) mouse RPL19: Mm02601633_g1,
5. ABI TaqMan Fast Advanced Master mix, part no. 4444557, Applied Biosystem
6. CT26.WT cells, CRL-2638, ATCC
7. Anti PDL-1 mIgG1, Inc production (YWW243.58.870, LOT #40315,)
8. mIgG1 isotype control (MOPC-21), Biolegend.

### Methods

### Animals:

7wk old female BALB/c mice were purchased from ENVIGO laboratories and kept in specific pathogen-free conditions at the Tel Aviv University animal facility. Experiments respected institutional guidelines and were approved by the Tel Aviv University ethics committee

### Tumor model and mAb treatments:

The murine CT26, an N-nitroso-N methylurethane - induced undifferentiated colon carcinoma cell line, was obtained from American Type Culture Collection (CRL-2638). CT26 cells (5x105) were injected subcutaneously at day 0 in the right flank of BALB/c mice (female, 7wk). Tumor growth was monitored twice a week using a caliper.

Treatments were given twice and started at day 8, when tumor area reached 50mm2 and on day 12 post tumor inoculation. Treatments were injected intraperitoneal at 10mg/Kg (9 mice/treatment) of either anti PDL-1 mIgG1 (YWW243.58.870, LOT #40315,) or mIgG1 isotype control (MOPC-21).

### Tissue samples:

Whole tumor and spleens total RNA was extracted using gentleMACS™ Octo Dissociator (Miltenyi Biotec), and tissues were homogenized with M tubes (# 130-096-335).

For tumor single cell suspension, tumors were enzymatically digested with 1µg/ml Collagenase D (Worthington Biomedical Corporations) 0.5µg/ml Deoxyribonuclease I (Worthington Biomedical Corporations) in RPMI-1670 (Biological Industries) for 1hr in 370c. Remains of undigested tumor tissues were gently agitated for full a dissociation.

### Cells isolation:

Thy1.2+ T lymphocytes were purified from tumors using a CD90.2+ positive isolation kit (EasySep, #18751) and purified Thy1.2 + T lymphocytes and the negative fraction cells were lysed in RLT to extract total RNA.

CD11b+ cells were purified from tumors using a CD11b+ positive isolation kit (EasySep, #18770) and purified CD11b+ cells and the negative fraction cells were lysed in RLT to extract total RNA

### Transcript expression

### Quantitative RT-PCR (qRT-PCR)

RNA (1-5 µg) extraction of mouse cells, derived from Ex-vivo experiments (346-MA-455 & 459-MA) was preformed according to manufactures protocols.

cDNA was prepared according to manufactures protocols (lug RNA diluted in 20ul cDNA mix reaction).

cDNA, prepared as described above, diluted 1:10 (representing 25ng RNA per reaction), was used as a template for qRT-PCR reactions, using a gene specific TaqMan probes (detailed in Reagents 3&4) Detection was performed using QuantStudio 12k device.

The cycle in which the reactions achieved a threshold level of fluorescence (Ct= Threshold Cycle) was registered and was used to calculate the relative transcript quantity in the RT reactions.

The absolute quantity was calculated by using the equation Q=2 ^-Ct.

The resulting relative quantities were normalized to a relative quantities of housekeeping gene, mRPL19.

### Results

### Endogenous expression of HIDE1 in intestine populations derived from C57 black mouse cells

### Endogenous expression of HIDE1 in intestine populations derived from C57 black mouse cells (Ex vivo# 466)

In order to verify the presence of the HIDE1 transcript in intestine population cells derived from C57 black mouse, qRT-PCR was performed using a specific TaqMan probe as describe above in Material & Methods.

As shown in **Figure 44**, mouse HIDE1 transcript is observed using TaqMan probe HOJEX12-CCAAZKY with relatively high levels in mesenteric and peripheral lymph nodes cells, as well as in spleen and peyer patches cells compared to small intestine and colon cells.

### EXAMPLE 14: HIDE1 Tetramer Binding to Human T Cells

The aim of these experiments was to evaluate binding of HIDE1 tetramer to resting and activated human isolated primary CD4+ and CD8+ T cells, as well as TILs (Tumor Infiltrating Lymphocytes) isolated from human melanoma samples and propagated in the presence of melanoma specific antigens and IL2.

### MATERIALS AND METHODS

### BIOTINYLATED MONOMERIC PROTEINS:

Human HIDE1 was produced as a monomer, including the extracellular domain (ECD), a biotinylation signal and a His-tag (batch #502). Following enzymatic biotinylation of this protein performed by Genscript, this protein (batch #503) was used for tetramer production and binding studies. As negative and positive controls for binding studies, similar biotinylated monomeric proteins of human EGFR (EGR-H82E7, Acrobiosystems) and human B7H4 (B74-H8222, Acrobiosystems), were used for tetramer production, respectively.

### STREPTAVIDIN-PE:

Streptavidin, R-Phycoerythrin Conjugate (SA-PE) - premium grade (Molecular probes, S21388, 1 mg/ml) was used for the production of tetramers. This streptavidin-PE is a high-purity product, ensuring a uniform material with a constant molecular weight (300kDa).

### PRODUCTION OF TETRAMERS:

Similar weights of HIDE1 (batch #503), EGFR and B7H4 were used for tetramer production. Tetramers were created by mixing the above proteins with SA-PE at a 4:1 molar ratio. SA-PE was added by gradual titration into the proteins in 15 portions. After each portion was added, the mixture was incubated for 5 minutes. In the first five portions, a lower amount of SA-PE was added (1.5-fold less than the following ten portions), in order to saturate the streptavidin sites. The entire procedure was carried out at room temperature (SA-PE was kept on ice) in the dark, and tetramers were stored afterwards at 4 degrees. Similar concentration (by weight per volume) of the different tetramers was used for the binding experiments.

### PRIMARY T CELLS

In this series of experiments cells from two different donors, obtained from Astarte Biologics were used:
- CD4+ and CD8+ from donor #147
- CD4+ and CD8+ from donor #186

Human primary cells (>95% purity), were thawed 24h prior to beginning of experiment. Cells were thawed in RPMI complete medium (RPMI + 10% FBS + 1% Glutamax + 1% Na-Pyruvate + 1% Pen-Strep) supplemented with 300 U/ml of rhIL2 (Biolegend 509129). Cells were left to recover for 24 hours.

### TILS

In this series of experiments, two different TILs from resected metastases of three melanoma patients, were used:
- TIL-Mart1- HLA-A2-Mart1 specific
- TIL-209- HLA-A2-gp100 specific

Human TILs (>95% CD8+), were thawed 24h prior to beginning of experiment. Cells were thawed in 12 ml of TIL medium (IMDM + 10% human serum + 1% Glutamax + 1% Na-Pyruvate + 1% non-essential amino acids + 1% Pen-Strep) supplemented with 300 U/ml of rhIL2 (Biolegend 509129). Cells were left to recover for 24 hours.

### CELL LINES

The following human T cell lines were used: H9 (ATCC, HTB-176, clonal derivative of HUT-78), Jurkat (ATCC, TIB-152, human acute T cell leukemia) and U937 (ATCC, CRL-1593.2 monocyte).

Cells were cultured in complete media: RPMI (Biological Industries Cat# 01-100-1A) supplemented with 10% FBS (Biological Industries Cat# 04-001-1A), 1% Glutamax (Life technologies Cat# 35050-038).

### ASSAY CONDITIONS

After recovery, cells were activated using a polyclonal activation of T cells, with 1 µg/ml of plate bound anti CD3 antibody (BD-pharmingen clone Ucht-1, cat-555329), 2 µg/ml of anti CD28 ab (eBioscience clone CD28.2 cat-16-0289-85) and 300 U/ml of IL2. Activation was carried out for 24h, 48h, 72h and 144h.

### CELL STAINING

Cells were harvested and washed with PBS. Cells were stained at room temprature for 10 minutes with PBS supplemented with 1/1000 of fixable viability stain efluor 450 (BD horizon cat-562247). After staining, cells were washed twice with PBS and stained with the Abs at the concentrations listed in Table 9 for 30 minutes at room temperature (RT) in FACS buffer (PBS + 0.5% BSA + 2 mM EDTA + 0.05% Azide) and concentrations that are listed in **Table 28**.

**Table 28. Antibodies specifics and staining concentration used**

| **Antibodies** | **Isotype** | **Conjugated to** | **Manufacturer** | **Catalog number** | **concentration (µg/ul)** | **Staining concentration** |
|---|---|---|---|---|---|---|
| Anti-CD4 | mIgG1 | FITC | Biolegend | 300506 | 0.4 | 4 µg/ml |
| Anti-CD8 | mIgG1 | FITC | Biolegend | 300906 | 0.15 | 1.5 µg/ml |
| Anti-CD137 | mIgG1 | PE | Biolegend | 309804 | 0.2 | 2 µg/ml |
| Anti-PD1 | mIgG1 | APC-cy7 | Biolegend | 329922 | 0.2 | 4 µg/ml |
| isotype control | mIgG1 | PE | Biolegend | 400112 | 0.4 | 2 µg/ml |
| isotype control | mIgG1 | APC-cy7 | Biolegend | 400128 | 0.2 | 4 µg/ml |

For tetramer binding, cells were incubated for 30-40 min at RT with the tetramer proteins and controls at 3µg/well (50µl/well=60µg/ml) in FACS buffer (PBS + 0.5% BSA + 2 mM EDTA).

After binding, cells were washed three times and re-suspended in FACS buffer for analysis.

### RESULTS

The binding of HIDE1 tetramer to various human cell lines was tested, as described in Materials and Methods (M&M). HIDE1 exhibited binding to H9 and Jurkat, at comparable levels (**Figure 57**), but not to U937 myeloid cell line.

The binding of HIDE1 to activated primary human T cells and TILs was evaluated next. T cells from two different donors and TILs were left untreated (resting) or polyclonal stimulated for various timepoints as described in M&M. Cell activation state was evaluated by detection of surface expression of CD137 and PD-1 at each time point compared to isotype control (FMO), as shown for activated CD8+, CD4+ T cells and TILs **(****Figure 58A-58C** respectively). As expected, PD-1 and CD137 expression was elevated apon activation **(****Figure 58A-58C**).

Results show binding of HIDE1 to activated CD4+ and CD8+ T cells between 24 and 72 hours **(****Figure 59A-65C**). The binding of HIDE1 was more prominent to activated CD4+ T cells at 72h reaching 3-fold increase in the Geo Mean compared to EGFR negative control.

In addition, HIDE1 tetramer binding to activated Mart1 and 209 TILs was detected at 24h after activation, and sustained to 72h of activation **(****Figure 60A-60C**). On day 6 of activation, no binding of HIDE1 tetramers was detected, similarly to what was observed with resting TILs (**Figure 60**).

### SUMMARY

The study presented here demonstrates the binding of HIDE1 tetramers to human T cell lines, as well as, human CD4+ and CD8+ T cells and TILs, which were polyclonal activated. Results points to expression of the putative molecular counterpart of HIDE1 on these target cells. Binding of HIDE1 tetramer was detected only upon activation of CD4+ and CD8+ cells, reaching a peak at 72h post activation. This data suggests the expression of a HIDE1 counterpart on T cells and its up-regulation upon TCR-dependent T cell activation.

### EXAMPLE 15: IN-VITRO IMMUNOMODULATORY ACTIVITIES OF HIDE1 ECD-IG ON MOUSE T CELLS

### Experiment A:

In these experiments the immunomodulatory activities of the recombinant fused protein HIDE1-ECD-Ig was investigated on mouse T cell activation.

In order to evaluate the activity of HIDE1 protein on T cell activation, recombinant protein was produced comprising the mouse extracellular domain (ECD) of the mouse HIDE1 fused to the Fc of mouse IgG2a (designated HIDE1-ECD Ig M:M, SEQ ID NO:18). The effect of the Fc fused protein co-immobilized with anti-CD3 on mouse CD4 T cell functions, as manifested by activation markers and cytokines secretion was investigated. Mouse IgG2a (clone MOPC-173; Biolegend or C1.18.4; BioXcell) was used as isotype control.

### Materials and MethodsFc fusion protein and control Ig

The Fc fusion protein, HIDE1-ECD-Ig (batches #72 and #195, SEQ ID NO:18) were tested. Mouse IgG2a (clone MOPC-173; Biolegend or C1.18.4; BioXcell) was used as isotype control.

### Mouse CD4 T cells isolation

Untouched CD4+CD25- T cells were isolated from pools of spleens of BALB/C mice using a T cell isolation Kit (Miltenyi Cat# 130-093-227) according to the manufacturer's instructions. The purity obtained was >90%.

### Activation of mouse CD4 T cells

Anti-mouse CD3-ε mAb (clone 145-2C11; BD Biosciences) at 2µg/ml together with HIDE1-ECD-Ig protein (SEQ ID NO: 18) or control Ig at various concentration (1, 3 or 10µg/ml), were co-immobilized for 3hr at 37°C, on 96-well flat bottom tissue culture plates (Sigma, Cat. # Z707910). Control Ig was added to each well in order to complete a total protein concentration of 12µg/ml per well. Wells were washed 3 times with PBS and plated with 1x10⁵ purified CD4+CD25- T cells per well and kept in a humidified, 5% CO2, 37°C incubator. In some experiments, soluble anti-CD28 (clone: 37.51; eBioscience; 1µg/ml) was added. Culture supernatants were collected at the indicated times post stimulation and analyzed for mouse IFNγ or IL-2 secretion by ELISA kits (R&D Systems). The effect of HIDE1-ECD-Ig protein (SEQ ID NO:18) on the expression of the activation marker CD69 on mouse CD4+ T cells was analyzed by flow cytometry. Cells were stained 48h post stimulation with a cocktail of antibodies including PerCP-anti-CD4 (clone G41.5; Biolegend), FITC or PE-anti-CD69 (clone H1.2F3; Biolegend), in the presence of anti-CD16/32 (clone 2.4g2; BD Biosciences) for blocking of Fcγ-receptors. Cells were evaluated using MACSQuant analyzer 9 (Miltenyi) and data analyzed using BD CellQuest or by MACSQuantify ™ Software. Data was analyzed using Excel or Prism4 software.

### RESULTS AND SUMMARY

### Effect of HIDE1-ECD Ig M:M on mouse CD4+ T cells function

**Figure 45** shows in-vitro immunomodulatory activities of HIDE1-ECD-Ig M:M (SEQ ID NO: 18) on isolated mouse splenic T cells (CD4+, >95%purity) stimulated with microplates co-immobilized with anti-CD3 (2µg/ml) together with control Ig (mIgG2a) or HIDE1-ECD-Ig (SEQ ID NO: 18) (10 µg/ml) in the presence of soluble anti-CD28 (1µg/ml). HIDE1-ECD-Ig (SEQ ID NO:18) suppressed mouse CD4 T cell activation as manifested by reduction in TCR-induced cytokines (IL-2 and IFNγ) secretion (**Figures 45B-****45C**). The magnitude of the inhibitory effect of HIDE1-ECD-Ig (SEQ ID NO:18) was in the range of 50-60%. Under similar conditions, no effect on CD69 expression was observed (**Figure 45A**).

HIDE1-ECD-Ig (SEQ ID NO:18) inhibits T cell activation in a concentration-dependent manner when the reagent is co-immobilized with anti-CD3 on plates. Maximal inhibitory effect was observed at 10µg/ml of HIDE1-ECD-Ig (SEQ ID NO:18).

**Figure 45** presents the effect of various HIDE1-ECD-Ig (SEQ ID NO:18) on mouse CD4 T cell activation. Plates were coated with anti-CD3 mAb (2µg/mL) in the presence of 10µg/ml HIDE1-Ig (batch #195) ybar is the mean of duplicate cultures, the error bars indicating the standard deviation. (**Figures 45B-45C**) Culture supernatants were collected at 48 h post-stimulation and mouse IL-2 and IFNγ levels were analyzed by ELISA. Results are shown as Mean ± Standard errors of duplicate samples. One experiment out of two performed is presented.

The results demonstrate the inhibitory effect of HIDE1-ECD-Ig on mouse T cells activation, manifested by reduced cytokine secretion, and suppression of activation marker CD69 upregulation. This inhibition of T cell activation, supports the therapeutic potential of immunoinhibitory HIDE1-ECD polypeptides and/or fusion proteins thereof according to at least some embodiments of the present invention in treating T cell-driven autoimmune diseases, such as rheumatoid arthritis, multiple sclerosis, psoriasis and inflammatory bowel disease, as well as for other immune related diseases and/or for reducing the undesirable immune activation that follows gene therapy.

### Experiment B:

### Introduction

In order to evaluate the activity of HIDE1 protein on T cell activation, recombinant protein comprising the extracellular domain (ECD) of the mouse HIDE1 protein fused to the Fc of mouse IgG2a (designated HIDE1-Fc) was used. The effect of the HIDE1-Fc fused proteins co-immobilized with anti-CD3 on mouse CD4 T cell functions, as manifested by activation markers and cytokines secretion, was investigated.

### MATERIALS AND METHODS

**Table 29: Fc Fusion proteins and control IgG**

| **Tested proteins** | **Batch #/ Clone/Cat#** | **ECD:Fc** | **Vendor** | **Comment** |
|---|---|---|---|---|
| mIgG2A | MOPC-173 | | Biolegend | Isotype control |
| HIDE1-Fc | #195 | M:M | ProBioGen | Tested compound |
| HIDE1-Fc | #72 | M:M | Excellgene | Tested compound |
| B7H4-Ig | 4206-B7-100 | M:M | R&D Systems | Positive control |

Mouse IgG2a (clone MOPC-173; Biolegend) was used as isotype control. During assay set-up, B7H4-Ig (Cat. 4206-B7, R&D), a Fc fusion protein comprising the extracellular domain (ECD) of mouse B7H4 fused to the Fc of mouse IgG2a, was used as a positive control. **Table 29**, above.

### Mouse CD4 T cells isolation

Untouched CD4+CD25- T cells were isolated from pools of spleens of BALB/C mice using a T cell isolation Kit (Miltenyi Cat# 130-093-227) according to the manufacturer's instructions. The purity obtained was >90%. Following isolation, cells were used either fresh or after freezing (in 90% FCS, 10% DMSO) and thawing.

### Activation of mouse CD4 T cells

Anti-mouse CD3-ε mAb (clone 145-2C11; BD Biosciences) at 2µg/ml of PBS, alone or together with HIDE1-Fc protein or control Ig at various ratios, was co-immobilized for 3hr at 37°C, on 96-well flat bottom tissue culture plates (Sigma, Cat. # Z707910). Wells were washed 3 times with PBS and plated with 1x10⁵ purified CD4+CD25-T cells per well in the presence of soluble anti-CD28 (clone: 37.51; eBioscience; 2µg/ml) and kept in a humidified, 5% CO2, 37°C incubator. Culture supernatants were collected 48h post stimulation and analyzed for mouse IFNγ or IL-2 secretion by ELISA kits (R&D Systems). The effect of CGEN-Fc proteins on the expression of the activation marker CD69 on mouse CD4+ T cells was analyzed by flow cytometry. Cells were stained 48h post stimulation with a cocktail of antibodies including PerCP-anti-CD4 (clone G41.5; Biolegend), FITC or PE-anti-CD69 (clone H1.2F3; Biolegend), in the presence of anti-CD16/32 (clone 2.4g2; BD Biosciences) for blocking of Fcγ-receptors. Cells were evaluated using MACSQuant analyzer 9 (Miltenyi) and data analyzed using BD CellQuest or by MACSQuantify TM Software. Data was analyzed using Excel or Prism4 software.

### RESULTS

### Experimental system and B7H4-Ig effect on mouse T cell activity

We established a functional assay to evaluate the activity of tested protein on mouse CD4 T cells function. The assay evaluates T cell function following activation with plate-bound anti-CD3 mAb, co-immobilized with tested Fc fusion proteins, as reported before for other immune checkpoint regulators, such as B7H4 and VISTA (Ref 1, 2). B7H4-Ig was used as a positive control in the assay (inhibitory ligand) while mouse IgG2a was used as a negative control. Schematic illustration of the experimental system and the effect of B7H4-Ig on T cell activation are described in **Figure 75****.**

### Effect of HIDE1 on mouse CD4 function

In order to evaluate the immunomodulatory effect of HIDE1 on mouse CD4+ cells,
HIDE1-Fc (batch #72 and #195) was co-immobilized with anti-CD3, as described in material and methods. The effect of two batches of HIDE1-Fc on mouse CD4 T cell activation, in the presence of soluble anti-CD28, upon co-immobilization on microplates with anti-CD3 was evaluated in two separate experiments using fresh or thawed isolated CD4 T cells. **(****Figure 76** **and Table 30,** respectively). HIDE1-Fc (batch #72 and #195) inhibits freshly isolated mouse CD4+ CD25- T cell activation, as manifested by reduced CD69 expression (**Figure 76A**), and IFNγ and IL-2 secretion **(****Figure 76B-C**). The magnitude of the inhibitory effect on CD69 expression of batch #72 was reduced compare to batch #195 at 10ug/ml (-30% vs -11%, respectively) while both batches inhibit IL-2 and IFNγ secretion in the range of 50-60% compare to Control Ig (**Figure 76**). **Table 30** summarize the dose response effect of HTDE1-Fc on freshly thawed CD4+ T cell function (CD69 expression or IFNγ secretion).

No effect of HIDE1-Fc on T cells activity was observed when 1 or 3ug/ml of HIDE1-Fc were used. Nevertheless, at 10ug/ml, HIDE1-Fc (batch #72 but not #195) inhibits CD69 expression and IFNγ secretion **(Table 30**).

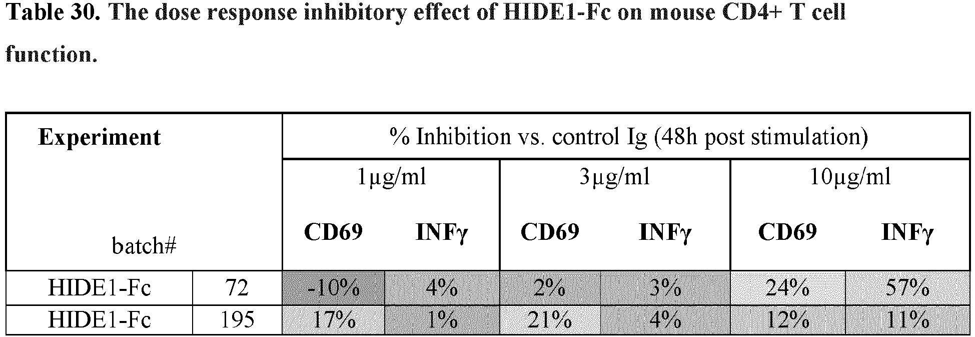

### SUMMARY

Two experiments were performed with HIDE1-Fc, co-immobilized with anti-CD3, aiming at assessment of its effect on CD4 T cells activity. In the first experiment, performed with freshly isolated CD4 T cells, both batches of HIDE1-Fc suppressed mouse CD4 T cell activation. In the second experiments, performed with thawed CD4 T cells, the two batched inhibited T cell activity for different extent. Overall, the maximal inhibitory effect of HIDE1-Fc on cytokine secretion was observed at 10ug/ml and the magnitude of the inhibition was in the range of 50-60%.

### EXAMPLE 16: EFFECT OF HIDE1-ECD FUSION PROTEIN ON HUMAN T CELLS ACTIVATED USING ANTI-CD3 AND ANTI-CD28 IN THE PRESENCE OF AUTOLOGOUS PBMCS

### MATERIALS AND METHODS

In these experiments the effects of HIDE1 on human T cells which were activated using anti-CD3 and anti-CD28 in the presence of autologous PBMCS was evaluated.

HIDE1 hECD-hIg fusion protein (SEQ ID NO:17, batch #48), composed of the ECD of human HIDE1 fused to the Fc of human IgG1 bearing C220 to S mutation at the hinge, was produced at ExcellGene (Switzeland) by transient transfection in CHO-DG44 cells using Excellgene's proprietary vector system. Cells were cultured for 10 days, followed by Protein A purification of cell harvest. The final product was formulated in 0.1M Glycine pH 6.

Expression vector used was Mammalian Expression Vector pTT5, in which HIDE1 gene is driven by CMV promoter.

CD4⁺ Human T cell Isolation Kit II is purchased from Miltenyi (Cat. #130-094-131). hIgG1 control (Synagis®) is obtained from Medimmune Inc. Anti-human CD3 Ab (OKT3, Cat# 16-0037) and anti-human CD28 Ab (clone CD28. 2; Cat# 16-0289) were purchased from eBioscience. Dynabeads M-450 Epoxy (Cat. # 140. 11) were purchased from Invitrogen. Buffy coats of human blood were obtained from LifeSource. Ficoll-Paque Plus (Cat. #17-1440-02), was purchased from GE HealthCare.

### Isolation of PBMCs from buffy coats using Ficoll separation

Total PBMCs were suspended in Ex-Vivo 20 medium, and irradiated at 3000rad. Naive CD4+ T cells were isolated from buffy coats of three healthy human donors' blood using CD4⁺ Human T cell Isolation Kit II (Miltenyi) according to manufacturer's instructions and co-cultured with irradiated autologous PBMCs at a ratio of 1:1 (1. 5x10⁵ T cells with 1. 5x10⁵ irradiated PBMCs per well). The cultures were activated with anti-CD3 (0. 5µg/ml) and anti-CD28 (0. 5 µg/ml) antibodies. HIDE1 hECD-hIg (SEQ ID NO: 17) or hIgG1 control Ig (Synagis®) were added to the culture at the indicated concentrations. After 24 hr in culture, cells were pulsed with H3-thymidine. Cells were harvested after 72 hours in culture.

### RESULTS

As shown in **Figure 46**, the addition of HIDE1 hECD-hIg_(SEQ ID NO: 17) to cultures of naive T cells activated by anti-CD3/anti-CD28 in the presence of irradiated autologous PBMCs, resulted in a dose dependent inhibition of T cell proliferation. This inhibition of T cell activation supports the therapeutic potential of immunoinhibitory HIDE1 based therapeutic agents according to at least some embodiments of the present invention, for treating T cell-driven autoimmune diseases, such as rheumatoid arthritis, multiple sclerosis, psoriasis and inflammatory bowel disease, as well as for treating other immune related diseases and/or for reducing the undesirable immune activation that follows gene or cell therapy. Essentially, HIDE1 based therapeutic agents that agonize HIDE1 should prevent or reduce the activation of T cells and the production of proinflammatory cytokines involved in the disease pathology of such conditions.

**Figure 46** demonstrates inhibition of human T cell proliferation induced by anti-CD3 and anti-CD28 in the presence of irradiated autologous PBMCs by human HIDE1 ECD-Ig (SEQ ID NO: 17). **Figure 46A** shows averages of three donors tested. **Figures 46B-48D** show the individual data of each donor. The control Ig is Synagis.

### EXAMPLE 17: IN-VITRO IMMUNOMODULATORY ACTIVITIES OF HIDE1 ON HUMAN CYTOTOXIC T CELLS (CTLS)

**The** experiments described in this example evaluated the effect of ectopic expression of human HIDE1 on different melanoma cell lines on their ability to activate CTLs (cytotoxic T lymphocytes) and serve as targets for killing by these cells.

### MATERIALS & METHODS:

**Three** human melanoma cell lines which present the MART-1 antigen in HLA-A2 context (SK-MEL-23, Mel-624 and Mel-624.38) were used as targets for CTLs. Mel-888 which does not express HLA-A2, served as a negative control.

### Ectopic expression of human HIDE1 on cytotoxic T lymphocytes (CTLs).

In order to express human HIDE1 in peripheral blood leukocyte (PBL) cultures, the cDNA encoding for HIDE1 was amplified using specific primers and cloned into an MSCV-based retroviral vector (pMSGV1). Verification of the cloning was done first using restriction enzyme digestion and subsequently by sequencing. Upon sequence confirmation, large amounts of the retroviral vector (Maxi-prep) were produced for subsequent use.

Peripheral blood leukocytes of healthy human donors were transduced with the retroviral constructs encoding HIDE1 or with the retroviral vectors encoding for NGFR1 or an empty vector, as a negative control. Transduction was carried out using a retronectin-based protocol; briefly, retroviral supernatant was produced in 293GP cells (a retroviral packaging cell line) following transfection with the retroviral vector and an amphotropic envelop gene (VSV-G). The retroviral supernatant was plated on retronectin-coated plates prior to the transduction to enable the binding of virions to the plate, and the PBLs were added to the plate for 6 hours. After that, the cells were replenished in a new culture vessel. An antibody against an intracellular FLAG sequence (cat. No 637309; Biolegend) was used in order to evaluate transduction efficiency. Expression of NGFR was detected with commercial anti-NGFR (Cat.No 345108; BioLegend). Rabbit IgG (Sigma Cat. No. 15006) was used as isotype control, and as secondary antibody we used APC-conjugated anti-rabbit IgG (Jackson, Cat. No. 711-136-152).

### Ectopic expression of the F4 T cell receptor on cytotoxic T lymphocytes (CTLs)

In order to obtain effector lymphocytes that express the MART-1-specific F4 TCR, specifically recognizing MART-1₂₆₋₃₅-/HLA-A2 peptide-MHC complex, freshly isolated human PBLs previously transduced to express either with HIDE1, NGFR or an empty vector were stimulated with PHA and cultured for 5-10 days, and subsequently transduced with *in vitro*-transcribed mRNA encoding both α and β chains from the MART-1-specific F4 TCR. The transduced lymphocytes were cultured in lymphocyte medium (Bio target medium, fetal bovine serum (10%), L Glutamine Penicillin/ Streptomicyn (100 units/ml), IL-2 300 IU), replenished every 2-3 days. F4 TCR expression levels were verified by FACS staining using a specific monoclonal antibody that recognizes the extra-cellular domain of the beta-chain from the transduced specific TCR (TCR-Vb12-PE, (Cat.No IM2291; Beckman Coulter).

### Cytokine secretion from HIDE1, NGFR or an empty vector and F4-TCR transduced lymphocytes upon co-culture with melanoma cells

PBLs expressing HIDE1 or NGFR along with F4-TCR were co-cultured with un-manipulated melanoma cells. 10⁵ transduced PBLs were co-cultured with 10⁵ melanoma target cells for 16 hours. In order to assess the response of the effector CD8 T cells to the different tumor cell lines, cytokine secretion (IFN-γ IL-2 and TNF-α) was measured by ELISA in culture supernatants (IFN-γ (Cat.No DY285E), IL-2 (Cat.No DY202E), TNF-α (Cat.No DY210E) R&D SYSTEMS), diluted to be in the linear range of the ELISA assay.

### Assessment of CTL activation

In order to analyze HIDE1 effect on T cell activation and CD137 surface expression in particular, cells were collected after 18 hr co-culture (E:T 1:1) and stained them with APC-anti-CD8a, FITC-anti-137 (Biolegend) in FACS buffer made of PBS, 0.5% BSA, and 0.02% sodium azide. All samples were analyzed by FACS.

### Results:

### General design of the experimental system

In the experimental system described herein (depicted in **Figure 47**), HIDE1 are over expressed on human PBLs which are next manipulated to express the MART1-specific and HLA-A2 restricted F4 TCR. Over expressing cells are then co-cultured with HLA-A2 positive and HLA-A2 negative melanoma cell lines. The F4 TCR was recently used in clinical trials in terminally-ill melanoma patients to specifically confer tumor recognition by autologous lymphocytes from peripheral blood by using a retrovirus encoding the TCR (Morgan et al, 2006 Science, 314:126-129). The effect of HIDE1 expression on antigen-specific activation of CD8 T cells by co-culture with cognate melanoma cells was assessed by cytokine secretion.

### Over expression of HIDE1 on human PBLs

Human PBLs were transduced with a retroviral vector encoding the HIDE1 or an empty vector as negative control, as described in Materials & Methods. The levels of HIDE1 were assessed by flow cytometry at 48hrs after transduction, and compared to cells transduced with an empty vector. Representative data showing HIDE1 expression detected by intra-cellular anti-FLAG staining is shown in **Figure 48****.**

### Over expression of F4 TCR on human PBLs

To perform functional assays with human CTLs, we used PBLs engineered to express the F4 TCR, which recognizes HLA-A2+/MART1+ melanoma cells, as described in Materials & Methods. **Figure 49** shows representative data for F4 TCR expression obtained upon TCR transduction of leukocytes previously transduced to express the different targets.

### Effect of HIDE1 expression on PBLs on IFNγ secretion

HIDE1 or Empty-vector and F4-transduced PBLs were co-cultured with melanoma cell lines. The levels of IFNγ secretion were measured at 16-hours of co-culture. The magnitude of inhibition of cytokine secretion due to over-expression is in the range of 90%. Representative data showing the effect of HIDE1 expressed on F4 expressing PBLs co-cultured with Mel-624, Mel-624.38 and SK-Mel23 on IFNγ secretion is presented in **Figure** 50. Co-culture with the HLA-A2 negative cell line Mel-888 which served as a negative control, caused only a minor activation dependent IFNγ secretion from F4-transduced lymphocytes. PBLs not expressing the F4 TCR (designated W/O) serve as an additional negative control. Altogether, two different PBL donors were transduced and three co-culture experiments were performed.

**Figure 50** presents the results of the expression of HIDE1 on the F4 expressing PBLs causes a reduction of IFNγ secretion upon co-culture with SK-MEL23, MEL-624 and MEL-624.38 in comparison to expression of an empty vector.

### Effect of HIDE1 on TNFα secretion

HIDE1 or an empty vector and F4-transduced PBLs were co-cultured with different melanoma cell lines. The levels of TNFα secretion were assessed at 16-hours of co-culture. The magnitude of inhibition of cytokine secretion due to HIDE1 expression ranged between 60 to 80%. Representative data showing the effect of HIDE1 expressed on F4 expressing PBLs co-cultured with Mel-624, Mel-624.38, SK-Mel23 and MEL-526 on TNFα secretion is presented in **Figure 51**. As expected, co-culture with the HLA-A2 negative cell line Mel-888 which served as a negative control, caused only a minor activation dependent TNFα secretion from F4-transduced lymphocytes. PBLs not expressing the F4 TCR (designated W/O) serve as an additional negative control.

**Figure 51** demonstrates that expression of HIDE1 on F4 expressing PBLs causes a reduction of secretion upon co-culture with SK-MEL23, MEL-624 and MEL-624.38.

### Effect of HIDE1 expression on PBL on expression of CD137 activation marker

**HIDE1** or an empty vector and F4-transduced PBLs were co-cultured with different melanoma cell lines. As shown in **Figure 52**, the expression levels of CD137 were assessed at 16-hours of co-culture. The magnitude of inhibition in CD137 expression due to HIDE1 expression was in the range of 80%.

**Figure 52** demonstrates that expression of HIDE1 on F4 expressing PBLs causes a reduction in the expression of CD137 (4-1BB) upon co-culture with SK-MEL23, MEL-624 and MEL-624.38.

### Summary

Without wishing to be limited by a single hypothesis, the results presented herein indicate that overexpression on primary lymphocytes results in reduced cytokine secretion by CTLs, suggesting that HIDE1 has an inhibitory effect on CTLs, further supporting the therapeutic potential of the immunoinhibitory HIDE1-ECD polypeptides and/or fusion proteins thereof for agonizing HIDE1 inhibitory effect on immunity and treatment of autoimmune diseases.

### EXAMPLE 18: EFFECT OF HIDE1 ON TCR TRANSGENIC PMEL-1 AND CD8+ T CELLS EFFECTOR FUNCTION UPON ANTIGEN-SPECIFIC ACTIVATION

In order to evaluate the immunomodulatory effect of HIDE1 on CD8+ T cell activation, the full length protein was ectopically expressed on EL4 cells which were pulsed with gp100₂₅₋₃₃ peptide and then served as specific target cells for pre-activated pmel-1 transgenic CD8+ T cells, expressing H-2Db/gp100₂₅₋₃₃. The effect of the HIDE1 on antigen-specific activity of pmel-1 CD8+ T cells was studied using several T cell activation readouts.

### Materials and Methods

### Cells.

EL4 (TIB-39, ATCC) cells were transduced to express mouse HIDE1 protein using pMSCV retroviral vector encoding for HIDE1 codon-optimized cDNA. Following selection with Puromycin (4µg/ml, SIGMA), HIDE1-expressing cells were generated. Mock-transduced EL4 cells were produced in parallel and further used as control in all experiments described below. Expression of HIDE1 in EL4 cells was confirmed applying staining with pAbs anti- mouse HIDE1 (GenScript, #488536_13), Detection was done using Donkey Anti Rabbit PE conjugated (Jackson, cat #711-116-152 (1:100)), followed by FACS analysis. To ensure comparable expression levels of H2-Db in HIDE1- and empty vector-transduced cells, staining with anti-H2Db (Cat# 111508, Biolegends) antibody and subsequent FACS analysis were used.

### pmel-1/EL4 in vitro assay.

### Primary Stimulation (day 0-4).

Spleens were harvested from pmel-1 mice (6-12 weeks male or female). Splenocytes were cultured in the presence of 1µg/ml of gp100-peptide (human gp10025-33 peptide, Sigma) and 25ng/ml of IL-2 (Cat# 589106, Biolegend) for 4 days in 6 well plate at 1.5x10⁶ cells/well (Cat# 6570160, Greiner). Culture media was RPMI+10% fetal bovine serum and the following supplements from Gibco: 2-mercaptoethanol (dilution of 1:1000), Gluta-MAX, sodium pyruvate, penicillin/streptomycin, and non-essential amino acids (all at a dilution of 1:100) (splenocytes medium). Cells were diluted 1:2 on the next day with full culture media supplemented with gp100-peptide (1µg/ml) and IL2 (25ng/ml).

### CD8+ purification and O.N resting (day 5).

Pmel-1 splenocytes were collected and CD8+ cells were isolated using CD8a (ly-2) microbead (Miltenyi, 130-049-401). CD8+ pmel-1 cells were cultured in the presence of 25ng/ml of IL-2 for O.N in 6 well plate at 1x10⁶ cells/well in splenocytes medium. The purity of CD8+ pmel-1 cells was analyzed by FACS (>88% purity).

### Secondary Stimulation: co-culture of pre-activated CD8+ pmel-1 with target cells (day 6)

Pmel-1 cultures were spun down and washed twice with fresh media. EL4 cells were pulsed with gp-100 peptide (0.3, 1 ng/ml) for 1 hour at 37°C in serum free media. Following washing , peptide-pulsed targets cells were over-night co-cultured with 50,000 cell/well pmel-1 CD8+ T cells at an effector to target (E:T) ratio of 2: 1. T cell activity was assessed based on detection of cytokines secretion (IFN-gamma, TNF-alfa, IL-2 and IL-10) in co-culture supernatants and by FACS analysis of surface expression of 41-BB (CD137) and CD25 (IL-2Ra).

### Evaluation of pmel-1 T cell activation

16-24 hours post co-culture, cells were stained with viability dye (BD Horizon; Cat# 562247, BD biosciences) followed by surface staining with FITC-anti-CD8a (Cat #100706, Biolegend), PE-anti-CD137 (Cat #106106, Biolegend) and APC-anti-CD25 (Cat # 102012, Biolegend) in the presence of anti-CD16/32 (clone 2.4g2; Cat #101302, Biolegend) for blocking of Fcγ-receptors. All samples were run on a MACSQuant analyzer (Miltenyi) and data was analyzed using Tree Star FlowJo software. Culture supernatants were collected and analyzed for mouse Th1/Th2/Th17 cytokine CBA kit (Cat# 560485, BD biosciences).

### Results

In order to evaluate the immunomodulatory effect of HIDE1 on CD8+ pmel-1 T cell function, the mouse protein was ectopically expressed on EL4 cells which served as specific target cells to pmel-1 CD8+ cells following pulsing with gp-100₂₅₋₃₃ peptide (H2-Db restricted peptide). **Figure 53A** shows the expression levels of HIDE1 and MHC class I (H2-Db) on mock or HIDE1-overexpressing cells. Similar H2-Db expression levels were observed for mock and HIDE1- transduced EL4 cells (variation of H2-Db expression of HIDE1-overexpressing cells compare to mock cells in three different experiments: (-17) - (+23) %). The folds of HIDE1 expression compare to mock cells varies between 2.1 to 3.3 (in three different experiments), the relatively low expression drives form the fact that mock cells endogenic express HIDE1. The positive control for the assay is a clone of PDL1-overexpressing cells together with a clone of mock cells. These clones were generated to overcome the obstacles of unmatched H2-Db expression between PDL1-overexpressing cells to mock cells and low fold of PDL1 expression. **Figure 53B** shows the expression levels of

PDL1 and MHC class I (H2-Db) on mock or PDL1-overexpressing clones. Similar H2-Db expression levels were observed for mock and PDL1- transduced EL4 clones cells (variation of H2-Db expression of PDL1-overexpressing cells compare to mock cells in three different experiments: (-3) - (+20) %, experiment 349-023 excluded: use of polyclonal PDL1-overexpressing cells express 43% more H2-Db compare to polyclonal mock cells. The folds of PDL1 expression compare to mock clones cells varies between 9.5 to 25 (in four different experiments).

**Figure 54** summarizes pmel-1/EL4 *in vitro* assay design. Target cells (mock, HIDE1 or PDL1-overexpressed) were pulsed with gp100 peptide and co-culture with pre-activated CD8+ pmel-1 and the effect of PDL1 and HIDE1 on mouse pmel-1 CD8+ T effector function, as manifested by activation markers and cytokines secretion, was investigated.

In order to validate this assay for both target testing and functional Abs screen for the target, the inhibitory effect of PDL1 on pmel-1 CD8+ cells activity and the reverse effect of blocking anti-PDLl (20µg/ml, YWW243.55.S70, Inc.) was assessed first upon co-culture of pre-activated pmel-1 CD8+ cells with gp100-pulsed EL4 cells with forced expression of PDL1 (2:1, E:T) - PDL1 did reduced pmel-1 CD8+ cells activity, this inhibition was also restored by anti-PDLl (CD137 expression is shown) **(****Figure 55B**). Co-culturing with gp100-pulsed EL4 cells with forced expression of HIDE1 (2:1, E:T) also resulted in reduced activity of pmel-1 CD8+ T cell **(****Figure 55C**), as manifested by reduction in activation markers (CD137 expression is shown) and by reduced cytokines secretion (IFNγ secretion is shown). **Tables 31** and **32** summarizes the inhibition percent of HIDE1 and PDL1 in all readouts checked and in four experiments under two gp-100 concentrations (0.3 and 1ng/ml)

| **Table 31.** Summary of HIDE1 inhibitory effect on pmel-1 CD8+ T cell activity in various experiments | | | | | | | | | | | | | |
|---|---|---|---|---|---|---|---|---|---|---|---|---|---|
| **HIDE1 overexpressing EL4** | | | | | | | | | | | | | |
| **Exp. #** | **GM CD137** | | **GM CD25** | | **%CD137 + CD25+** | | **IFNγ** | | **IL-10** | | **TNFα** | | **% Δ of H2D b vs. to EV** |
| **gp100 conc. (ng/ml )** | **1** | **0.3** | **1** | **0.3** | **1** | **0.3** | **1** | **0.3** | **1** | **0.3** | **1** | **0.3** | |
| **349-019** | **2%** | **43 %** | **4%** | **20 %** | **7%** | **32 %** | **13 %** | **27 %** | **16 %** | **29 %** | **23 %** | **25 %** | **- 17%** |
| **349-020** | **3%** | **17 %** | **ine rt** | **6%** | **ine rt** | **4%** | **- 9%** | **8%** | **- 3%** | **13 %** | **- 6%** | **8%** | **+12 %** |
| **349-022** | **40 %** | **57 %** | **18 %** | **31 %** | **27 %** | **42 %** | **8%** | **34 %** | **28 %** | **55 %** | **9%** | **16 %** | **+14 %** |
| **349-023** | **30 %** | **50 %** | **25 %** | **34 %** | **16 %** | **30 %** | **49 %** | **44 %** | **40 %** | **51 %** | **30 %** | **44 %** | **+23 %** |

| **Table 32.** Summary of PDL1 inhibitory effect on pmel-1 CD8+ T cell activity in various experiments | | | | | | | | | | | | | |
|---|---|---|---|---|---|---|---|---|---|---|---|---|---|
| **PDL1 overexpressing EL4** | | | | | | | | | | | | | |
| **Exp. #** | **GM CD137** | | **GM CD25** | | **%CD137 + CD25+** | | **IFNγ** | | **IL-10** | | **TNFα** | | **% Δ of H2Db vs. to EV** |
| **gp100 conc. (ng/ml )** | **1** | **0.3** | **1** | **0.3** | **1** | **0.3** | **1** | **0.3** | **1** | **0.3** | **1** | **0.3** | |
| **349-019** | **67 %** | **67 %** | **8%** | **37 %** | **20 %** | **59 %** | **- 18 %** | **46 %** | **- 11 %** | **48 %** | **- 30 %** | **25 %** | **+14%** |
| **349-020** | **- 55 %** | **28 %** | **- 14 %** | **15 %** | **- 13 %** | **11 %** | **- 40 %** | **21 %** | **- 86 %** | **37 %** | **- 35 %** | **21 %** | **-3%** |
| **349-022** | **58 %** | **74 %** | **36 %** | **46 %** | **53 %** | **66 %** | **15 %** | **39 %** | **58 %** | **71 %** | **30 %** | **35 %** | **+20%** |
| **349-023** | **ine rt** | **13 %** | **11 %** | **11 %** | **6%** | **10 %** | **ine rt** | **26 %** | **8%** | **19 %** | **ine rt** | **14 %** | **+43%** |

### Summary

Four experiments were performed to evaluate the proposed immuno-modulatory effect of ectopic expression of HIDE1 in target cells, specific to TCR-transgenic pmel-1 CD8+ T cells. The inhibitory effect of HIDE1, as well as of PDL1 as positive control, on activation of Ag-specific CD8+ T cells (pmel-1) following co-culture with target cells (EL4) pulsed with gp100₂₅₋₃₃ peptide, was observed in 4 out of 4 experiments, as manifested by reduced cytokines secretion and by suppression of the activation-induced expression of activation markers CD137 and CD25. Both, for HIDE1 and PDL1, the inhibitory effect was greater at a concentration of 0.3ng/ml gp100. In the second experiment (349-020) magnitude of inhibition was lesser than the two other experiments, but since that was also the case for PDL1 and since trends of inhibition remain, we can assume that some technical fault occurred in this experiment. In the fourth experiment (349-023) PDL1 over-expressing cells express 43% more H2-Db vs. mock, that could be the reason for the relatively low percent of inhibition by PDL1 in this experiment. This assay design is used to test the ability of HIDE1-ECD polypeptides and/or fusion proteins thereof to agonise HIDE1 mediated inhibition.

### EXAMPLE 19: EFFICACY OF HIDE1-ECD-IG IN MOUSE R-EAE MODEL OF MULTIPLE SCLEROSIS

Efficacy of HIDE1-ECD fusion protein in autoimmunity model supports immunoinhibitory function of HIDE1, which without wishing to be limited by a single theory, might allow tumor cells escape from immune surveillance. Activating immunoinhibitory HIDE1 protein with immunoinhibitory antibodies will be efficient for treatment of MS and other autoimmune disorders

To investigate further the immunoinhibitory HIDE1 properties and he therapeutic potential of immunoinhibitory HIDE1 targeting antibodies which agonize HIDE1 activity for treatment of autoimmune diseases, HIDE1-ECD-Ig is was tested in a mouse model of Multiple Sclerosis; Relapsing Remitting Experimental Autoimmune Encephalomyelitis (R-EAE).

Female SJL mice 6 weeks old were purchased from Harlan and maintained in the CCM facility for 1 week prior to beginning the experiment. Mice were randomly assigned into groups of 10 animals and primed with 50 µg PLP139-151/CFA on day 0. Mice received 6 i.p. injections of 100µg/dose of immunoinhibitory HIDE1 targeting antibody, mIgG2a isotype control, or CTLA4-Ig (mouse ECD fused to mouse IgG2a Fc) as positive control. Treatments began at the time of disease induction (preventive mode) or at onset of disease remission (therapeutic mode) and were given 3 times per week for at least 2 weeks. Mice were scored for disease symptoms on a 0-5 disease score scale: 0, no abnormality; 1, limp tail; 2, limp tail and hind limb weakness; 3, hind limb paralysis; 4, hind limb paralysis and forelimb weakness; and 5, moribund.

On day 35, (during the peak of disease relapse) 5 mice of each group are assayed for DTH (delayed type hypersensitivity) response to disease inducing epitope (PLP139-151) and to relapse-associated myelin epitope (PLP178-191) via injection of 10µg of PLP139-151 in one ear and PLP178-191 into the opposite ear. The level of ear swelling are assayed at 24 hours post challenge.

The results presented in **Figure 56** show a pronounced decrease in disease severity of R-EAE-induced mice upon treatment with HIDE1-ECD-Ig Ig (SEQ ID NO: 18), in a therapeutic mode with 100µg/dose at 3 times per week 2. The level of inhibition was similar to that of CTLA4-Ig. Similarly, HIDE1-ECD-Ig fusion proteins are anticipated to inhibit DTH responses to the disease inducing epitope (PLP139-151) and to relapse-associated epitope (PLP178-191) at day 35.

In a similar model, the dose dependency of the efficacy of HIDE1-ECD-Ig (SEQ ID NO: 18) as well as its mode of action in the PLP-induced R-EAE model is evaluated. Disease is induced as described above and mice are treated from onset of disease remission with 100, 30 or 10 µg/dose HI DE1-ECD-Ig (SEQ ID NO: 18) 3 times per week over two weeks. The level of disease severity is evaluated and is anticipated to be reduced in a dose dependent manner.

HIDE1-ECD-Ig (SEQ ID NO: 18) fusion proteins are also anticipated to inhibit DTH responses to spread epitopes PLP178-191 and MBP84-104 on days 45 and 76. Furthermore, recall responses analysis is carried out on day 45 and day 76 splenocytes and cervical lymph node cells, to PLP139-151, PLP178-191 and MBP84-104 in which proliferation as well as cytokine secretion (IFNγ, IL-4, IL-17, IL-10, TNFα, IL-6, GM-CSF, and others) are analyzed. Without being bound by a single hypothesis, modulatory effects on Th1 Th17 and/or Th2 cytokines are anticipated following treatment with HIDE1-ECD-Ig. In addition, CNS, draining (cervical) lymph nodes and spleens of mice treated with HIDE1-ECD-Ig are evaluated for infiltration of immune cells.

**Figure 56** shows the therapeutic effect of HIDE1-ECD-Ig (SEQ ID: 18) in the PLP139-151-induced R-EAE model in SJL mice. HIDE1-ECD-Ig (SEQ ID: 18) was administered in a therapeutic mode from the onset of disease remission, at 100 microg/mouse i.p. 3 times per week for two weeks. Therapeutic effects on clinical symptoms are demonstrated as reduction in Mean Clinical Score.

### EXAMPLE 20: HIDE1 EXPRESSION IN HEALTHY PBLS AND IN AML

Surface expression of HIDE1 by healthy PBLs was evaluated using whole blood taken from 3 different healthy donors using anti-Hidel mAbs 33B4-2F7 and Ab 36C1-2F6 as compared to staining with isotype control.

Prominent staining with anti HIDE1 mAb 33B4-2F7 was observed on monocytes (**Figure 61**). Neutrophils also showed positive stain, albite with a lower intensity. Essentially no stain was detected on lymphocytes. No staining was observed in any of the populations using mAb 36C1-2F.

Similarly, prominent HIDE1 expression was detected on myeloid blasts in AML while lymphocytes were essentially negative (**Figure 62**).

### EXAMPLE 21: EFFECT OF HIDE1 KNOCK DOWN (KD) AND ANTI-HIDE1 ANTIBODIES ON HUMAN T CELL FUNCTION

### Introduction

The aim of the presented functional assay was to evaluate the effect HIDE1 Knock down by siRNA or overexpression on T cell function as well as the effect of anti-HIDE1 antibodies on T cell function. In particular, to evaluate the immuno-modulatory effect of anti-HIDE1 antibodies that can be used to target monocytes, Tumor associated macrophages (TAMs), or other myeloid cells and screen for various functional activities, including modulating the interaction between HIDE-1 and its putative receptor(s), modulation of HIDE1 levels or direct signaling and attenuation of negative signaling. Such recombinant antibodies may be used as modulatory molecules to decrease or prevent HIDE1 from interacting with inhibitory receptor(s) on T cells or other cells in the tumor microenvironment, thereby releasing T cells or other functional cells from HIDE1 check point ("break")/suppressive signaling .

### MATERIALS AND METHODS

### THP1 - poly clonal co-culture assay (Ab independent)

THP1 cell line expressing HIDE1 were knocked down (KD) by specific siRNA to HIDE1 or scrambled (SCR) control or PDL1. 24h post KD, cells were treated with 500 units of IFNγ for another 24h to induce PDL1 expression and cell maturation. Prior to entering into co-culture assay, levels of HIDE1 or PDL1 KD compared to control siRNA (SCR) were evaluated amongst other markers as pan HLA-I/II or CD86 which were compared to their isotype control. THP1 cells treated with Mitomycin C (SIGMA; 50ug/ml for 1hr), were co-cultured at 1:1 ratio with CD3 T cells labeled with CFSE in 96 well flat bottom immobilized with 1000ng/ml CD3 (#555329 UCHT1) for 5 days. In order to assess the response of the effector CD3 T cells to the different THP1 KD effects, proliferation was measured by the dilution of CFSE and cytokine secretion (IFN-γ & TNF-α) was measured by TH-1/2/17 CBA kit (BD biosciences) in culture supernatants.

### Effect of anti-HIDE1 antibodies in THP1 - poly clonal co-culture assay

THP1 cell line expressing HIDE1 were pre-treated with 500 units of INFγ for 24h. 24h post INFγ treatment cells were treated with mitomycin C (SIGMA; 50ug/ml for 1hr) and co-cultured with CFSE labeled CD3+ cells at 1:1 ratio in the presence of immobilized anti-CD3 (1ug/ml; UCHT-1). Anti-HIDEl blocking or agonistic mAb at 20ug/ml were added to each culture. Either no antibody or an isotype control antibody was used as a negative control. After day 5, the effect of anti-HIDE1 antibodies on cytokine secretion was assessed from the supernatant using TH-1/2/17 CBA kit (BD biosciences).

### Effect of anti-HIDE1 antibodies in Mixed Lymphocyte Reaction (MLR)

A mixed lymphocyte reaction was employed to demonstrate the effect of blocking the HIDE1 pathway to lymphocyte effector cells. T cells in the assay were tested for proliferation and cytokine secretion in the presence or absence of an anti-HIDE1 human monoclonal antibodies (14 Abs with hIgG4 and 5 Abs with hIgG1 backbone). Human CD3 T-cells were purified from PBMC derived from Buffy coats using negative selection isolation kits (EasySep™, STEMCELLS technologies). Alloreactivite dendritic cells in a total volume of 200 µl. Anti-HIDEl blocking or agonistic mAb at 20ug/ml were added to each culture. Either no antibody or an isotype control antibody was used as a negative control. After day 5, the effect of anti-HIDE1 antibodies on T cell proliferation (CFSE dilution) and cytokine secretion (ELISA or TH1/2/17 CBA kits) in culture supernatants were assessed.

### Effect of anti-HIDE1 antibodies in CHOS-OKT3 co-culture assay

CFSE-labeled T cells were stimulated with stimulator cells (CHO cells expressing membrane-bound anti-CD3 mAb fragments). CHOS-stimulator cells expressing human HIDE1 and control stimulator cells (empty vector) treated with mitomycin C (50 µg/ml for 1h) before co-cultured with CFSE-labeled human T cells at the ratio of 1:5. After 5 days at 37°C and 5.0% CO₂, the effect of anti-HIDE1 antibodies (20ug/ml) on T cell proliferation (CFSE dilution) and cytokine secretion (ELISA or TH1/2/17 CBA kits) in culture supernatants was assessed. All samples were acquired in MACSQuant analyzer (Miltenyi) and data was analyzed using FlowJo software (v10.0.8). Culture supernatants were collected and analyzed for cytokine secretion by CBA kit (Cat #560484, BD).

### RESULTS

### Effect of HIDE1 Knock-Down in human THP-1 co-culture assay

THP1 cells were knocked down (KD) by specific siRNA to HIDE1 or scrambled (SCR) control or PDL1. 24 post KD, cells were treated with 500 units of IFNγ for another 24h which induced PDL1 expression and cell maturation. Knock down levels of HIDE1 and PDL1 were 73% and 61% respectively, sufficient for entering into assay. Moreover, no variations in levels of HLA-I, HLA-II and CD86 compared to their isotype control suggesting KD doesn't effect THP1-1 cell state **(****Figure 67B**). Proliferation and cytokine secretion was assessed for THP1 co-cultured with CD3 T cells. Enhanced proliferation was detected for HIDE1 KD when compared to SCR, with a more prominent effect in CD4 T cells compared to CD8 T cells. Moreover, robust IFNγ and TNFα secretion was observed in HIDE1 KD cells compared to SCR KD cells **(****Figure 67C-D**). In contrast, this effect was not observed for PDL1 in comparison to SCR.

### Effect of anti-HIDE1 antibodies on T cell function in THP-1 poly activation assay

IFNγ-treated THP1 cells were co-cultured with CD3+ cells at 1:1 ratio in the presence of 1ug/ml immobilized anti-CD3 and the effect of anti-HIDE1 antibodies at 20ug/ml on cytokine secretion 5d post co-culture was tested **(****Figure 68A-B**). Increase in IFNγ and TNFα secretion was observed for CPA.12.006-H4, CPA.12.007-H4, CPA.12.0012-H4, Ab-507 & AB-508 in comparison to HTDE1 isotype control.

### Effect of anti-HIDE1 antibodies on HIDE1 over-expression in CHOS-OKT3 assay

CHOS-OKT3 overexpressing HIDE1 were co-cultured with CD3+ cells at 1:1 ratio in the presence of immobilized anti-CD3 and the effect of anti-HIDE1 antibodies at 20ug/ml on cytokine secretion 5d post co-culture was tested **(****Figure 69A-B**). Increase in IFNγ secretion was observed for Ab-509, Ab-510 and CPA-12-008 in comparison to HTDE1 isotype control.

### Effect of anti-HIDE1 antibodies on T cell function in MLR assay

The effect of anti-HIDE1 blocking or agonistic mAb in MLR assay was assessed 5 days post co-culture with allogeneic imDC. Either no antibody or HIDE1 isotype control antibody was used as a negative control. After day 5, the effect of anti-HIDE1 antibodies on T cell proliferation (CFSE dilution) gating on CD4 or CD8+ cells was evaluated (**Figure 70A-B**).

### SUMMARY AND CONCLUSIONS

Several anti-HIDE1 antibodies augmented T cell function in several functional assays.

In THP-1 co-culture assay, CPA.12.006-H4, CPA.12.007-H4, CPA.12.0012-H4, Ab-507 & AB-508 enhance IFNγ & TNFα secretion levels in at least 1 donor (**Figure 68**). In CHOS-OKT3 assay, 3 anti-HIDE1 Abs (Ab-509 and Ab-510, CPA.12.008-H4) enhance IFNγ secretion in at least 1 donor (**Figure. 69**). In MLR assay 4 antibodies (Ab-506, Ab-507, Ab-508 and Ab-509) enhance T cell proliferation.

### EXAMPLE 22: EFFECT OF HIDE1 ON HUMAN TUMOR-INFILTRATING LYMPHOCYTE (TIL) FUNCTIONAL ACTIVITY

Aim: To evaluate the effect of HIDE1 on function of human Tumor-infiltrating lymphocytes (TIL) upon co-culture with HIDE1-transduced target cells.

### MATERIALS AND METHODS:

### Tumor cell lines

Mel-526 and Mel-624 cell lines are MART-1⁺, HLA-A2⁺ melanoma cell line. All cell lines were cultured in complete medium consisting of DMEM supplemented with 10% FCS, 25 mmol/L HEPES, 2 mmol/L glutamine, and combined antibiotics (all from Invitrogen Life Technologies).

### Cloning of peptide-specific tumor-infiltrating (TIL) lymphocytes

Tumor-infiltrating lymphocyte (TIL) micro-cultures were initiated and expanded from tumor specimens taken from resected metastases of two melanoma patients, as described (Uzana et al, JI 2012). Briefly, TILs were cultured in complete medium consisting of RPMI 1640 supplemented with 10% heat-inactivated human AB serum, 6000 IU/ml human rIL-2 (rhIL-2; Chiron, Amsterdam, The Netherlands), 2mM L-glutamine, 1 mM sodium pyruvate, 25 mM HEPES, 50 mM 2-ME, and combined antibiotics (Invitrogen Life Technologies). On day 14 of TIL initiation, the lymphocytes were washed with PBS, resuspended in PBS supplemented with 0.5% BSA, and stained with FITC-conjugated HLA-A*0201/MART-1₂₆₋₃₅ dextramer (Immudex, Copenhagen, Denmark) for 30 min at 4°C. The lymphocytes were then incubated with allophycocyanin-conjugated mouse anti-human CD8 (eBioscience) for an additional 30 min at 4°C. CD8+ lymphocytes, positively stained by the dextramer (CD8+/dextramer+ cells), were sorted by a BD FACSAria (BD Biosciences) and directly cloned at one or two cells/well in 96-well plates in the presence of ortho-anti-CD3 (30 ng/ml; eBioscience), rhIL-2 (6000 IU/ml), and 4 Gy-irradiated allogeneic PBMCs as feeder cells. Five days later, rhIL-2 (6000 IU/ml) was added and renewed every 2d thereafter. On day 14, the clones were assayed for IFNγ secretion in a peptide-specific manner following their co-incubation with MART-1₂₆₋₃₅-pulsed T2 cells (peptides were commercially synthesized and purified [>95%] by reverse-phase HPLC) using commercially available ELISA reagents (R&D Systems, Minneapolis, MN). The MART-1₂₆₋₃₅-reactive clones were further expanded in a second-round exposure to ortho-anti-CD3 (30 ng/ml) and (6000 IU/ml) rhIL-2 in the presence of 50-fold excess of irradiated feeder cells. A similar protocol was performed to obtain a gp100₁₅₄₋₁₆₂ and gp100₂₀₉₋₂₁₇reactive clones. MART-1₂₆₋₃₅ and gp100₁₅₄₋₁₆₂-reactive clones were derived from patient 412 while gp100₂₀₉₋₂₁₇ -reactive clones were derived from patient 209. Following TILs clones expansion the cells were stain with FITC or PE-conjugated HLA-A *0201/MART-1₂₆₋₃₅, BLA-A*0201/gp100₁₅₄₋₁₆₂ or HLA-A*0201/gp100₂₀₉₋₂₁₇ dextramer and analyzed by FACS. The study was approved by the Institutional Review Board at Sharett Institute of Oncology, Hadassah Medical Organization, Israel, and all patients gave their informed consent prior to initiation of melanoma and lymphocyte cell cultures. The TILs, which were sent to Compugen LTD under service agreement, kept in liquid nitrogen (10-20x10⁶/vial) and thawed one day before co-culture with target cells.

### Ectopic expression of hPDL1 and hHIDE1 on target cells

### Section A

Mel-526 and Mel-624 cells were transduced to express the human HIDE1 with a flag-tag in the C-terminus, using pMSGV1 retroviral vector or human PD-L1, using a pMSCV retroviral vector. Expression of hPDL1 and hHIDE1 on Mel cells was confirmed by flow cytometry following surface staining with a specific antibody to hPDL1 (Cat# 329708, Biolegend) and hHIDE1 (in-house). The expression levels of HLA-A2 in hPDL1 and hHIDE1 was compared to the levels in the mock transduced cells by using anti-HLA-A2 antibody (Cat# 343306, Biolegend).

### Section B

Mel-526 and Mel-624 cells were transduced to express the human HIDE1 with a flag-tag in the C-terminus, using pMSGV1 retroviral vector. Expression of hPDL1 and hHIDE1 was validated as indicated in section A following sorting of the cells for higher HIDE1 expression.

### Purification of HIDE1 over expressing cells (relevant for section B above)

To enlarge folds of expression of the transduced human HIDE1 Mel-526 and Mel-624 cell lines, cells were purified using anti-PE beads (Cat# 130-048-801, Milteniy,) following staining by PE anti-hHIDEl (Cat# 33B4-2F7, Biotem) followed by Goat anti-mouse PE (cat# 115-116-146, Jackson). The purified cells were used in herein assay.

### Co-culture of TILs with target cells

One day prior to co-culture, TILs were thawed and cultured with full IMDM media (cat# 01-052-1A, Biological industries ltd (BI)) supplemented with 10% human serum (Sigma, H3667), 1% glutamax (Life technologies, 35050-038), 1% MEM eagle (cat# 01-340-1B, BI), 1% Sodium pyruvate (cat# 03-042-1B, BI), 1% PenStrep (cat# 03-031-1B, BI) in the presence of 300IU/ml of rhIL-2 (Cat# 589106, Biolegend). Target cells and TILs were harvest and co-cultured at effector to target (E:T) ratios of 1:1 (1x10^5 cells from each/well) or 3:1 (1x10^5 TIL : 3x10^4 target cells/ well) in full IMDM media.

### Flow cytometry

16 hours post co-culture, cells were stained with viability dye (BD Horizon; Cat# 562247, BD biosciences) followed by surface staining with FITC-anti-CD8a (Cat # 300906, Biolegend), PE-anti-CD137 (Cat #30984, Biolegend) and APC-anti-CD107a (Cat # 328620, Biolegend) in the presence of Fc blocking solution (cat# 422302, Biolegend). All samples were run on a MACSQuant analyzer (Miltenyi) and data was analyzed using Tree Star FlowJo software. Cells were first gated for lymphocytes (FSC-A vs. SSC-A), followed by singlets gate (FSC-H vs. FSC-A), and further gated for live cells. CD137, CD107a surface expression on gated CD8+ was analyzed. Culture supernatants were collected and analyzed for mouse Th1/Th2/Th17 cytokine CBA kit (Cat# 560485, BD biosciences).

### RESULTS

### Section A

In order to investigate immune-modulatory effect of HIDE1 gp100 or MART-1 specific TILs were co-cultured with over expressing HIDE1 and HLA-A2+ target cells. The effect on T cells was assessed by analyzing activation markers surface expression and cytokine secretion. Target cells HLA-A2 and HIDE1/PD-L1 expression was validated prior to experiments (**Figure 71**). Over expressing HIDE1 cells are compared to mock transduced pMSGV1 vector cells while over expressing PD-L1 cells are compared to mock transduced pMSCV vector cells.

hPDL1 transduced Mel-526 and Mel-624 cells were used as positive control in this assay. At the first experiment two ratios of E:T were tested, 1:1 and 1:3, as well as two target cells 526 and 624. The inhibitory effect of PDL1 was observed in co-culture of TILs with both target cells and was manifested in reduced IFNg secretion and surface expression of CD137. The inhibitory effect was most pronounced in co-culture of TILs with 624 cells and so we did not proceed to test 526 cells in the second experiment. hPDL1 had an inhibitory effect on all TILs tested, gp100₁₅₄₋₁₆₂ (designated TIL-154), gp100₂₀₉₋₂₁₇ (designated TIL-209) and MART-1₂₆₋₃₅ (designated MART-1), though the inhibitory effect by hPDL1 was less pronounced with TIL 154 (**Figure 72A,72C** **and Table 33**). hHIDE1 had no inhibitory effect but a stimulatory effect on TILs activity when co-cultured with Mel-526 and 624 **Figure 72B****,D and Table 34**) with the exception of one experiment in which a 15% reduction in CD107a was observed in co-culture of TILs 209/MART1 with 526 in 3:1 E:T ratio. On the same experiment a 71% reduction in IFNg was observed in TIL MARTI:526 however the total amount of IFNg was extremely low.

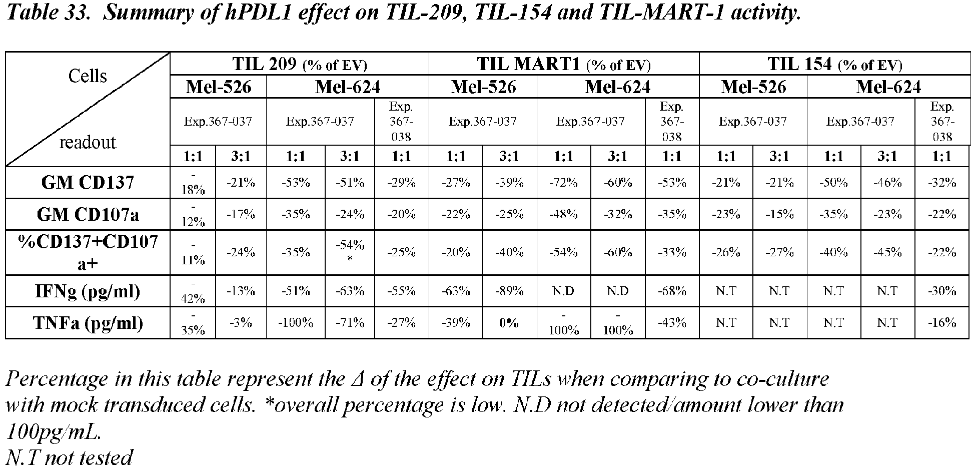

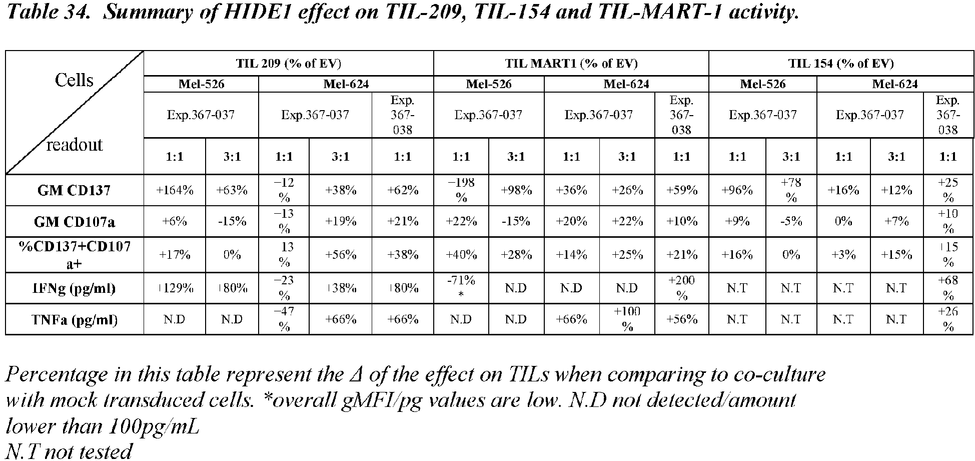

### Section B

In the experimental system described herein, gp100 or MART-1-reactive TILs were co-cultured with HLA-A2+ target cells (624 and 526 Mels), which ectopically express HIDE1 and were sorted for higher expression of HIDE1 (149 and 54 folds vs. empty vector), and the effect on TILs effector function compare to mock-transduced cells, as manifested by activation markers and cytokines secretion, was investigated. Cells were validated before each experiment for their levels of target and HLA-A2 expression and were found out to be within the tolerable limit of target expression (more than 5 folds of expression) and of HLA-A2 level (differential expression between mock and hHIDE1/hPDL1 transduced cell lines of ±30%) **(****Figure 73A-73B**). The assay was validated by using hPDL1 transduced Mel-526 and Mel-624 cells as targets. At the first repeat two ratios of E:T were tested, 1:1 and 1:3, in which the inhibitory effect of PDL1 was greater with the 1:1 ratio (data not shown), and so this ratio was used in the second repeat. hPDL1 had an inhibitory effect on all TILs tested, p100₁₅₄₋₁₆₂ (designated TIL-154), gp100₂₀₉₋₂₁₇ (designated TIL-209) and MART-1₂₆₋₃₅ (designated MART-1), though the inhibitory effect by hPDL1 was more profound with Mel-526 than with Mel-624 overexpressing cells **(****Figure 74A**). hHIDE1 had no inhibitory effect on TILs activity when co-cultured with Mel-526 and 624 **(****Figure 74B**). **Tables 35 and 36** summarizes the inhibition percent of hPDL1 and hHIDE1 in all readouts checked - for Mel-624 and TIL 154 two repeats were made, in the first repeat greater percent of inhibition by hPDL1 were observed compare to the second repeat, probably due to the low expression of HLA-A2 by hPDL1 overexpressing cells in the first repeat. For all other treatments one repeat was made.

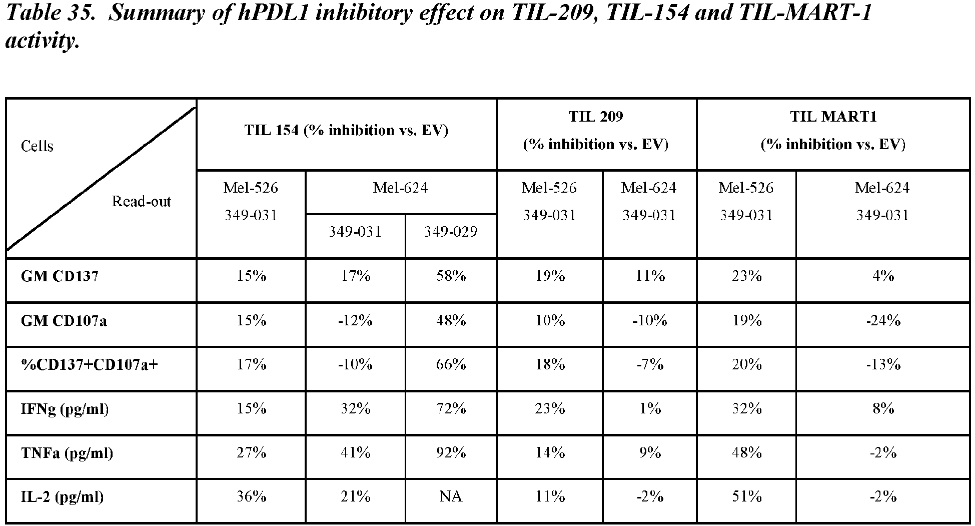

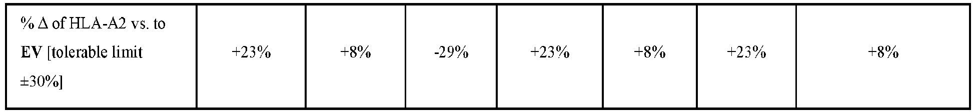

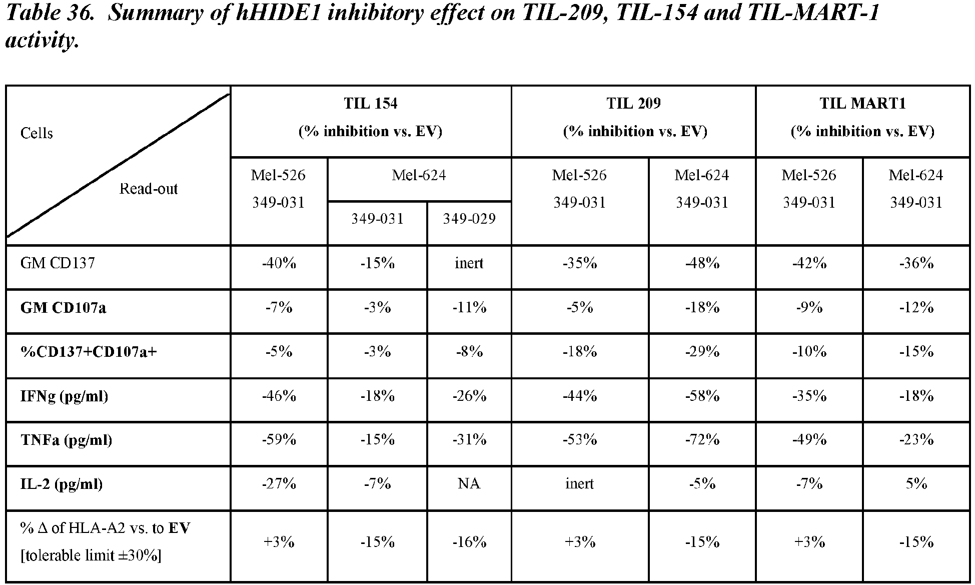

### EXAMPLE 23: HIDE1 ANTIBODY DISCOVERY

### Rationale and Objectives

This study was designed to isolate human antibodies with high affinity and specificity for the HIDE1 immuno-oncology target. This was achieved by panning a human antibody phage display library against a recombinant protein comprising the human HIDE1 extracellular domain (ECD) fused to human IgG₁ Fc (HIDE1-HH-1).

### Protocols

**Preparation of biotinylated HIDE1:** HIDE1-HH-1 (batch #280) was biotinylated using a Sulfo-NHS-LC-Biotin kit (Pierce). Free biotin was removed from the reactions by dialysis against PBS pH 7.2, 0.01% Tween 20. Synagis hIgG1 was also biotinylated to use for depletion steps in panning experiments.

**General method for ELISA:** Biotinylated proteins were captured on the wells of streptavidin-coated 96-well plates (Pierce). Antigen captured plate wells were blocked with PBS-milk (PBS pH 7.4, 5% skim milk powder). Blocking buffer was removed and a primary antibody solution was added and incubated for 1hr at room temperature (RT). Plates were washed with PBS-T (PBS pH 7.4, 0.05% Tween20) followed by PBS. HRP-conjugated secondary antibody (dependent on the assay system) was added and incubated at RT for 1hr, and plates were washed again. In some cases, a HRP-conjugated primary antibody (or other detection protein) was used directly. ELISA signals were developed using Sureblue TMB substrate (KPL Inc). Assay signals were read on 96-well plate reader at absorbance 450 nm.

Phage display and Fab screening: A single campaign was divided into four sub-campaigns with different washes **(Table 37**). Panning reactions were carried out in solution using streptavidin-coated magnetic beads to capture the biotinylated antigens. Beads were recovered using a magnetic rack (Promega).

Preparation of phage library for panning: All phage panning experiments used the XOMA031 human Fab antibody phage display library (XOMA Corporation, Berkeley, CA) blocked with 5% skim milk powder.

Antigen coupling to streptavidin beads: For each sub-campaign, biotinylated HIDE1-HH-1 was captured on Dynal streptavidin-coated magnetic beads (Life Technologies). Beads used for subtractive panning were coupled with the 'depletion' antigen (biotinylated Synagis hIgG1).

Depletion of human IgG Fc and streptavidin bead binders from the phage library: It was necessary to remove unwanted binders to streptavidin beads and the Fc region of HIDE1-HH-1 during the panning process. To achieve this, a phage aliquot was mixed with beads coupled to the Synagis hIgG1 depletion antigen and incubated at RT for 30mins. Two depletion reactions were applied to all sub-campaigns. The depletion beads were then discarded.

**Phage panning:** Four rounds of phage panning were performed for each sub-campaign. For the first round, the blocked and depleted phage library was mixed with magnetic beads coupled to biotinylated HIDE1-HH-1 and incubated at RT for 1 hr. Nonspecific phage were removed by washing with PBS-T and PBS at various stringency levels, depending on the specific panning sub-campaign (**Table 37**). After washing, bound phage were eluted by incubation with triethylamine (TEA) (EMD) and the eluate was neutralized by adding Tris-HCl pH 8.0 (Teknova). Second and later rounds were conducted the same way, except that the rescued phage supernatant from the previous round was used in place of the phage library.

**Table 37: Washing stringency used for phage panning against human HIDE1.**

| Sub-campaign | Washing Stringency | | | |
|---|---|---|---|---|
| | 1^{st} Rd | 2^{nd} Rd | 3^{rd} Rd | 4^{th} Rd |
| A | 3 PBS-T washes & 3 PBS washes | 6 PBS-T washes & 6 PBS washes | 6 PBS-T washes & 6 PBS washes | 6 PBS-T washes & 6 PBS washes |
| B | 3 PBS-T washes & 3 PBS washes | 10 PBS-T washes & 10 PBS washes | 10 PBS-T washes & 10 PBS washes | 10 PBS-T washes & 10 PBS washes |
| C | 5 PBS-T washes & 5 PBS washes | 6 PBS-T washes & 6 PBS washes | 6 PBS-T washes & 6 PBS washes | 6 PBS-T washes & 6 PBS washes |
| D | 5 PBS-T washes & 5 PBS washes | 10 PBS-T washes & 10 PBS washes | 10 PBS-T washes & 10 PBS washes | 10 PBS T washes & 10 PBS washes |

**Phage rescue:** The phage eluate was infected into TG1 *E. coli,* which transformed the cells with the XOMA031 phagemid. Transformed cells were then spread on selective agar plates (carbenicillin) and incubated overnight at 30°C.

The resulting *E. coli* lawns were scraped and re-suspended in liquid growth media. A small aliquot of re-suspended cells was inoculated into a 50 mL culture (2YT with 2% glucose and carbenicillin) and grown at 37°C until the OD at 600 nm reached 0.5. This culture was infected with M13K07 helper phage (New England Biolabs) and spun down. The cell pellet was resuspended in 50 ml media (2YT with carbenicillin and kanamycin, which is selection antibiotic for M13K07). The culture was then maintained at 25°C to allow phage packaging. An aliquot of the culture supernatant was carried over for a subsequent round of panning.

### Binding screens using Fabs prepared in periplasmic extracts (PPEs) by ELISA and FACS

**Fab PPE production:** The XOMA031 library is based on phagemid constructs that also function as IPTG inducible Fab expression vectors. Eluted phage pools from panning rounds 3 and 4 were diluted and infected into TG1 *E. coli* cells (Lucigen) so that single colonies were generated when spread on an agar plate. Individual clones were grown in 1 mL cultures (2YT with glucose and ampicillin) and protein expression was induced by adding IPTG (Teknova). Expression cultures were incubated overnight at 25°C. Fab proteins secreted into the *E. coli* periplasm were then extracted for analysis. Each plate of samples also included duplicate 'blank PPE' wells to serve as negative controls. These were created from non-inoculated cultures processed the same way as the Fab PPEs.

**ELISA binding assays:** PPEs from the sub-campaigns were tested for binding to the panning antigen (biotinylated HIDE1-HH-1) and negative control antigens, PVR-hIgG1 Fc fusion and Synagis hIgG₁. The ELISA followed the general protocol in paragraph [00668] ("**General method for ELISA**"). The primary antibody was replaced with a 50 µL aliquot of Fab or blank PPE and the secondary antibody was a HRP-conjugated anti-human F(ab')2 antibody (Jackson Immunoresearch). ELISA binding was expressed as the ratio of Fab PPE binding signal: blank PPE signal. Positive hits were identified as those giving a ratio of at least two.

**FACS screening of PPE**: FACS analyses were conducted using adherent HEK-293T cells over-expressing the human HIDE1 (293T-hHIDE1). All analyses included negative control parental HEK-293T cells.

Reagent preparation and wash steps were carried out in FACS buffer (PBS with 1% BSA). Fab and blank PPEs were mixed with an aliquot of cells, incubated for 1hr at 4°C and then washed with FACS buffer. Cells were then mixed with an anti-C-myc primary antibody (Roche). After the same incubation and wash step cells were stained with an antimouse IgG Fc AlexaFlour-647 antibody (Jackson Immunoresearch). After a final incubation and wash cells were fixed in 2% paraformaldehyde made up in FACS buffer. Samples were read on a FACS Calibur (BD Bioscience) or HTFC screening system (Intellicyt). Data were analyzed using FCS Express (De Novo Software, CA, USA) or FloJo (De Novo Software, CA, USA). Results were expressed as the ratio of mean fluorescence intensity (MFI) of 293T-hHIDE1 cells: MFI signal of 293T control cells (MFI ratio). Positive hits were identified as those giving an MFI ratio of at least five.

### SPR Kinetic Screen of 24 Anti-HIDEl Fab PPE

Preliminary Fab binding kinetics were determined using Fab PPEs produced as described in paragraph [00676] ("**Fab PPE production**")[00676]. Studies were conducted using Biacore 3000 and ProteOn XPR 36 instruments at 22°C.

### Measurement of fab concentration in PPE samples

A high density anti-human Fab antibody surface was prepared over one flow cell of a CM5 chip using a Biacore Human Fab Capture Kit (GE Healthcare) according to manufacturer's instructions. One surface of the CM5 chip was activated and blocked without coupling any anti-Fab antibody to act as a reference surface. Antibody immobilization was performed with HEPES-buffered saline, 0.005% polysorbate 20, pH 7.4 (HBS-P).

The molar concentrations of Fabs in PPE samples were quantitated using a Biacore 3000 instrument. Each Fab was diluted 10-fold and injected for 1 minute at 5µL/min over the Fab capture and reference surfaces described above. A standard human Fab at a known concentration (Bethyl Laboratories, Inc.) was then injected over the anti-Fab surface under the same conditions as the Fab supernatants. The association slopes of each sensorgram from each Fab supernatant were fit against the association slope of the standard human Fab of known concentration using CLAMP 3.40 software to estimate the molar concentrations of each Fab in supernatant (**Table39**).

### Measurement of Fab binding kinetics

A high density goat anti-human Fc antibody (Invitrogen) was immobilized by standard amine coupling over all lanes of a GLC chip using a ProteOn XPR 36 biosensor (Biorad). This surface was used to capture the recombinant HIDE1- H:H fusion protein (GenScript Lot 393383-21, batch # 280) or a control Fc fusion protein (CD155, Sino Biologicals) in the vertical flow-cell direction at a concentration of ∼0.5µg/mL for 90 seconds.

Each anti-HIDE1 Fab PPE sample was then injected at three concentrations (undiluted fab supernatant and two three-fold serial dilutions) in the horizontal direction over the captured fusion proteins. The dilutions were made using degassed PBS with 0.05% Tween 20 and 0.01% BSA. PPEs were injected for two minutes followed by 10 minutes of dissociation at a flow rate of 50 µL/min. The starting concentration range for each Fab is recorded in **Table 39**. The anti-human fc capture surfaces were regenerated with two 30-second pulses of 146 mM phosphoric acid after each cycle.

The resulting sensorgrams were processed and double-referenced using a ProteOn version of Scrubber 2.0 (BioLogic Software). Where appropriate, the sensorgrams were fit with a simple 1:1 kinetic binding model (with a term for mass transport) using ProteOn Scrubber 2.0. Sensorgrams and their respective kinetic fits are shown in **Figures 77** and **78****. Table 39** lists the resulting binding constants. Binding parameters for complex kinetics were not determined.

### Re-formatting of fab hits and production as human IgG molecules

HIDE1 binding Fabs were converted to full length human IgGs by sub-cloning the relevant V_{H} and V_{L} domains into separate heavy and light chain expression vectors that already contained the appropriate heavy or light chain constant regions: pUNO3-H4 (human IgG₄ heavy chain vector), pUNO3-HK (human kappa light chain vector) or pUNO3-HL (human lambda light chain vector). The base pUNO3 vector was sourced from Invivogen. Matched heavy and light chain constructs were co-transfected into Expi293 cells using Expifectamine and Opti-MEM (Life Technologies). Expression cultures were incubated at 37°C for a further six days and supernatants were harvested by centrifugation. IgGs were purified from the supernatants using an AKTA Pure FPLC (GE Healthcare Bio-Sciences) and HiTrap MabSelect Sure affinity columns (GE Healthcare Bio-Sciences).

FACS screening of reformatted IgG₄ antibodies: The cell lines described in paragraph [00678] ("**FACS screening of PPE**") were mixed with FACS buffer containing the purified anti-HIDE1 IgGs or isotype controls. After incubation for 1hr at 4°C, cells were washed with FACS buffer and stained with an anti-human IgG Fab AlexaFluor-647 antibody (Jackson Immunoresearch) for 45 mins at 4°C. Cells were washed again and fluorescence was detected using an Intellicyt HTFC screening system (Intellicyt). Data were analyzed using FCS Express (DeNovo) and plotted for affinity calculations (K_{D}) in GraphPad Prism (GraphPad Software, Inc.). Note that each anti-HIDE1 antibody was tested over a titration range from 0 - 80 µg/mL.

### RESULTS

### Fab PPE screening

Fab clones drawn from the four sub-campaigns were tested for binding by FACS and ELISA. Hits (defined as described in paragraphs [00677] and [00678]; "**ELISA binding assays**" and "**FACS screening of PPE**") were sequenced to eliminate redundant Fabs. General screening outcomes are presented in **Table 38.** The ELISA and FACS screening hit rates from the 3^{rd} Rd outputs ranged from 30% to 50% and 15% to 19% respectively. A sub-set of Fabs had binding activity that correlated between FACS and ELISA (35% - 57%, depending on the sub-campaign). Sequence diversity ranged from 27% to 38%, which represents unique HCDR-3 families. A HCDR-3 family contains Fabs with the same heavy chain CDR-3 sequence, but minor differences in the heavy or light chain framework regions.

The ELISA and FACS screening hit rates from the 4^{th} Rd outputs ranged from 15% to 36% and 7% to 16% respectively. A sub-set of Fabs had binding activity that correlated between FACS and ELISA (23% - 75%, depending on the sub-campaign). Sequence diversity ranged from 18% to 41%.

The HCDR-3 families selected from different sub campaigns were highly redundant for both rounds 3 and 4. However, there were some additional low-frequency clones associated with specific sub-campaigns or panning rounds. Overall, the ELISA and FACS hit rates decreased from the third round to the fourth round. This suggests that optimum phage enrichment occurred in round three. Nonetheless, positive hits from the both rounds were included for the subsequent step.

A sub-set of the ELISA and FACS positive hits were subject to SPR kinetic screening. At least one member of each HCDR-3 family was included. Most Fabs displayed 1:1 kinetic binding (**Table 39**) to HIDE1-HH-1 (**Figure 77**) and minimal binding to the negative control protein (**Figure 78**).

The overall panning and screening exercise (ELISA, FACS and SPR) yielded 14 Fabs that showed sufficient binding activity to move forward into IgG reformatting. The identified Fabs include 12 HCDR-3 families, two of which have 2 members.

**IgG reformatting and characterization:** The Fab binders described in paragraphs [00688]-[00691] ("**Fab PPE screening**") were cloned into expression vectors for production as human IgG4 molecules. All of these antibodies were successfully expressed and purified. Thirteen were shown to bind the 293T-huHIDE1 cell line (**Table 40**). None of these antibodies were cross-reactive against the mouse HIDE1 ortholog.

### SUMMARY AND CONCLUSIONS

This example provides characterization data for a panel of human antibodies raised against the HIDE1 antigen by phage display. A phage panning campaign was conducted using an ECD-Fc fusion protein. Overall, 13 IgGs binding to human HIDE1 were identified. These are recommended for further characterization of binding on cells lines that express HIDE1 endogenously, as well as *in vitro* functional assays

**Table 38: Summary of ELISA and FACS screening of Fab PPEs.** FACS results measure binding against the 293T-huHIDE1 cell line. ELISA results measure binding against recombinant HIDE1HH-1. FACS/ELISA correlation refers to the proportion of Fabs that bound in both assays (FACS *and* ELISA) compared to the total number of Fabs showing binding in either assay. Sequence diversity refers to the number of different Fabs present among the analyzed binders (*i.e.* 30% would indicate 30 different Fab sequences found within a set of 100 HIDE1 binders).

**Table 38: Summary of ELISA and FACS screening of Fab PPEs.**

| Sub-campaign | FACS hit rate | ELISA hit rate | FACS/ELISA correlation | Sequence diversity |
|---|---|---|---|---|
| 3^{rd} Rd | | | | |
| A | 15% | 31% | 47% | 38% |
| B | 18% | 50% | 35% | 27% |
| C | 17% | 30% | 57% | 41% |
| D | 19% | 38% | 50% | 33% |

| 4^{th} Rd | | | | |
|---|---|---|---|---|
| A | 7% | 30% | 23% | 19% |
| B | 11% | 36% | 32% | 18% |
| C | 11% | 15% | 75% | 41% |
| D | 16% | 25% | 65% | 38% |

**Table 39:** Preliminary Fab binding kinetics determine from PPE samples. The Fabs are listed in order of decreasing K_{D}. Asterisks indicate the values should be taken with some caution, with an explanation in the comments column. **Figure 78** shows the binding responses of the Fabs injected over the control fusion protein. Responses over the control protein showed either no binding or a minimal response compared to the responses in **Figure 77****.**

**Table 39: Preliminary Fab binding kinetics determine from PPE samples.**

| CPA ID | | **Stock conc. (M)** | **kₐ (M⁻¹sec⁻¹)** | **k_{d} (sec⁻¹)** | **Rₘₐₓ** | **K_{D} (M)** |
|---|---|---|---|---|---|---|
| CPA.12.011 | B03 S400-01.E01* | 9.41E-09 | 2.9E+05 | 1.9E-04 | 106 | 6.4E-10 |
| CPA.12.003 | A03 S397-01.A12 | 5.46E-08 | 2.5E+05 | 2.2E-04 | 110 | 9.1E-10 |
| CPA.12.009 | A10 S398-02.A04* | 7.22E-09 | 1.4E+06 | 1.4E-03 | 105 | 1.0E-09 |
| CPA.12.001 | A01 S397-01.A04 | 1.79E-07 | 6.7E+05 | 8.1E-04 | 210 | 1.2E-09 |
| CPA.12.007 | A08 S398-01.E08 | 2.48E-08 | 2.1E+05 | 3.4E-04 | 142 | 1.6E-09 |
| | B05 S401-01.E06 | 7.89E-08 | 1.6E+05 | 3.1E-04 | 114 | 1.9E-09 |
| CPA.12.004 | A04 S397-01.D06 | 2.54E-08 | 1.6E+06 | 3.3E-03 | 253 | 2.1E-09 |
| CPA.12.005 | A06 S397-01.H07 | 1.02E-07 | 4.4E+05 | 1.0E-03 | 203 | 2.3E-09 |
| | A09 S398-01.H06 | 1.68E-07 | 6.8E+05 | 1.8E-03 | 322 | 2.7E-09 |
| | B11 S404-01.E01 | 6.95E-08 | 4.1E+04 | 1.1E-04 | 441 | 2.8E-09 |
| | B10 S404-01.D11 | 2.85E-07 | 2.2E+06 | 7.4E-03 | 261 | 3.4E-09 |
| CPA.12.006 | A07 S398-01.D09 | 1.83E-07 | 6.8E+04 | 2.7E-04 | 36 | 3.9E-09 |
| | B02 S400-01.C02 | 6.82E-08 | 1.1E+05 | 4.5E-04 | 49 | 4.0E-09 |
| | B07 S403-01.D03 | 1.82E-08 | 9.1E+05 | 4.1E-03 | 87 | 4.5E-09 |
| CPA.12.002 | A02 S397-01.A08 | 1.61E-07 | 1.0E+05 | 8.3E-04 | 222 | 8.0E-09 |
| | A12 S399-01.D05 | 1.27E-07 | 2.1E+05 | 2.0E-03 | 181 | 9.6E-09 |
| | B06 S403-01.A12 | 1.41E-07 | 1.0E+05 | 1.0E-03 | 251 | 1.0E-08 |
| | A11 S399-01.A07 | 2.82E-07 | 5.9E+04 | 7.0E-04 | 163 | 1.2E-08 |
| CPA.12.014 | B12 S404-01.E06 | 1.28E-07 | 9.9E+04 | 3.7E-03 | 142 | 3.7E-08 |
| | A05 S397-01.H06 | 1.80E-08 | | | | |
| | B01 S399-01.D09 | 3.20E-07 | | | | |
| | B04 S401-01.A03 | 8.14E-08 | | | | |
| | B08 S404-01.A06 | 5.36E-08 | | | | |
| CPA.12.012 | B09 S404-01.C08 | 1.90E-07 | | | | |

**Table 40:** Overview of FACS binding results for human IgG₄ antibodies reformatted from Fab screening hits. FACS binding was tested against the 293T-huHIDE1 (human) and 293T-moHIDE1 (mouse) cell. The results are expressed as a 'Preliminary FACS K_{D}' calculated from titration curves described herein ("**SPR Kinetic Screen of 24 Anti-HIDE1 Fab PPE**"). IgGs from the same H-CDR3 family are indicated by the same color, either blue or green. CPA. 12.003 & CPA.12.008 have identical heavy chain CDR-3 regions and minor differences in framework regions or other CDRs. The same holds for CPA.12.004 & CPA.12.013. N.B. not binding.

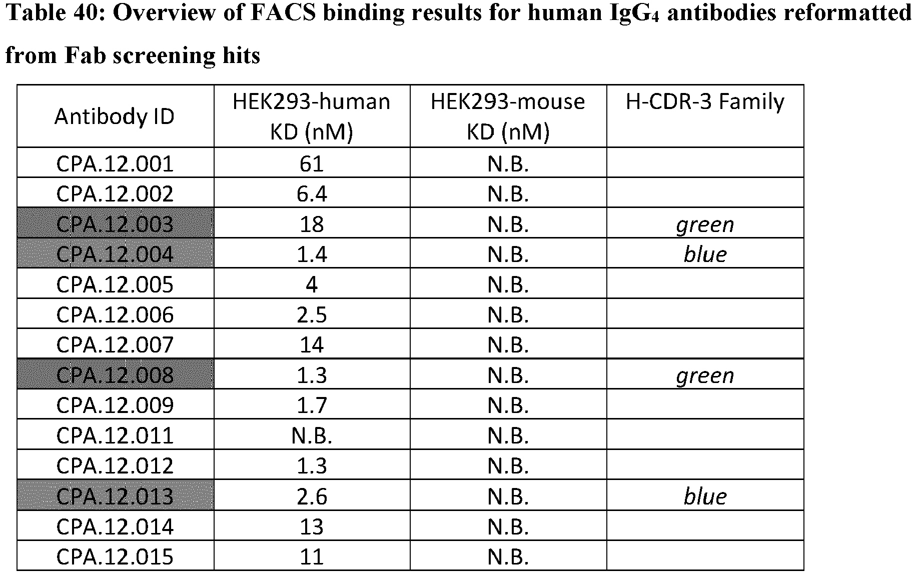

### EXAMPLE 24: HIDE1 RNA EXPRESSION IN PATIENT SAMPLES

### MATERIAL & METHODS

### Reagents

**RNA** extraction was performed with High Pure Paraffin Kit by JHU (Roche, cat# 03270289001).

cDNA was produced using High Capacity cDNA Reverse Transcription Kit by JHU (Applied Biosystems cat#4368814).

Custom Taqman PreAmp pool (Life Technologies, cat #29233051).

TaqMan® PreAmp Master Mix Kit x2 (Life Technologies, cat# 4384266).

HIDE1 TaqMan probes: Hs01128131_m1 and Hs01128129_m1, Life technologies.

TaqMan probes for Housekeeping genes (HSKG) (Life technologies) human GUSB: Hs99999908_m1, human GAPDH: Hs99999905_m1, human RPL19: Hs01577060_gH and human HPRT1: Hs02800695_m1.

TaqMan Custom Arrays, 96A TLDA, Life Technologies, cat #4342259.

### METHODS

### Tumor Samples (The Vigorous Immune Microenvironment of Microsatellite Instable Colon Cancer Is Balanced by Multiple Counter-Inhibitory Checkpoints, Cancer Discov; 5(1); 43-51.©2014 AACR)

Tumor tissues were collected at the Johns Hopkins Hospital (Baltimore, MD) from patients with primary sporadic colorectal cancer and free of prior chemotherapy. Assessment of MSI was done using the length of a panel of microsatellite markers in the tumor and a normal reference (either normal mucosa or germline) by using fragment analysis of PCR products labeled with fluorescent dyes. Fragment analysis determined the expression level of the proteins in charge of maintaining the integrity of microsatellite tracts. Differences in the length of two or more markers (the standard Bethesda panel uses five markers) were indicative of MSI status. 3 patients tested as MSI (microsatellite instable) positive and 3 patients as MSS (microsatellite stable); all patients' details are indicated in **Table 42.**

### Laser Capture Microdissection (LCM) and RNA extraction

FFPE and hematoxylin and eosin-stained tissue sections (5 µm) were used for the LCM procedure using the Leica LMD 7000 system. For each patient, tissue sections were microdissected from three defined areas (TIL, Invasive Front, and Stroma) and directly collected in tissue lysis buffer for RNA extraction. RNA was isolated following the manufacturer's instructions. RNA was converted to cDNA using the High-Capacity RNA-to-cDNA Kit. The cDNA was used as undiluted or diluted 1:4, and a step of preamplification was performed using a pool of TaqMan probes and a preamplification master mix kit, followed by TaqMan qPCR.

### Transcript expression

### Quantitative PCR (qPCR)

Preampliifed cDNA, prepared as described above, was used as a template for qPCR reactions, using a gene specific TaqMan probes (detailed in Reagents 5&6) Detection was performed using QuantStudio 12k device.

The cycle in which the reactions achieved a threshold level of fluorescence (Ct= Threshold Cycle) was registered and was used to calculate the relative transcript quantity in the PCR reactions.

The absolute quantity was calculated by using the equation Q=2 ^-Ct. Samples were normalized by the average Ct of the housekeeping genes: hGUSB, hRPL19, hGAPDH and hHPRT1, set across all samples to 25.

Undetectable value, was assigned as 40 cycles.

### RESULTS

### Endogenous expression of HIDE1 in MSS and MSI derived from colorectal cancer patients

### Endogenous expression of HIDE1 in MSI and MSS derived from colorectal cancer patients tested by qPCR

In order to verify the presence of the HIDE1 transcript in colorectal cancer derived cells, qRT-PCR was performed using a specific TaqMan probes as describe above in Material & Methods. qRT-PCR was performed by using undiluted and diluted 1:4 cDNA samples.

As shown in **Figure 79**, Analysis indicating higher transcript expression in TIL, Invasive front and Stroma areas in MSI patients (3/3) compared to MSS patients and higher expression in stroma and IF areas compared to TIL area.

**Table 41.** Represent normalized Ct values of undiluted and diluted (1:4) samples. Human HIDE1 transcript was observed using two specific TaqMan probes describes in M&M, in cell derived from three areas (TIL, IF, Stroma) from MSI and MSS colorectal cancer patients.

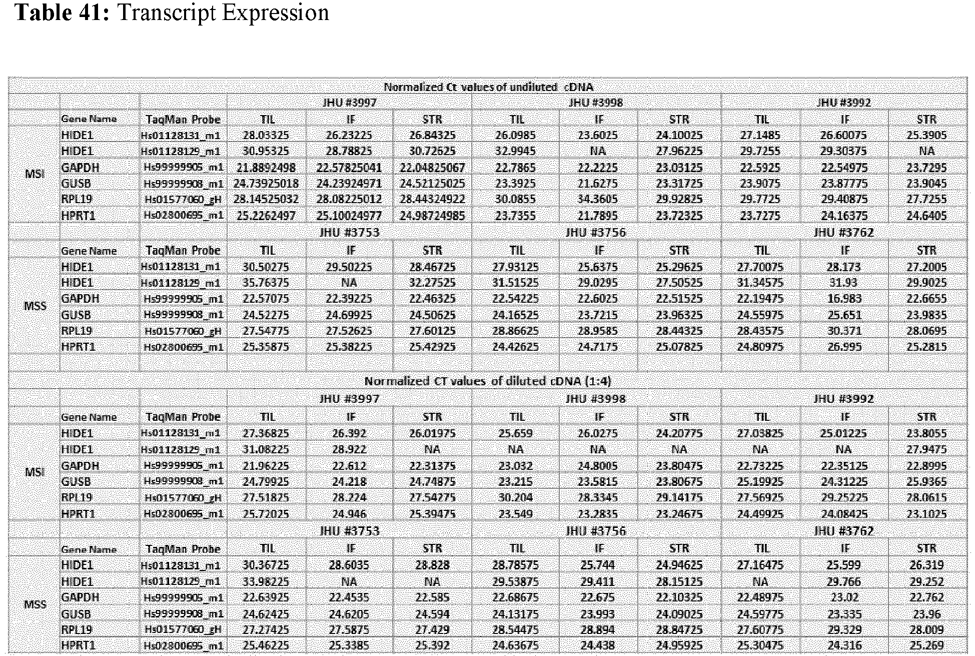

**Table 42: Patient details**

| MSI CRC | Pt ID | Age | Gender | Race | Stage | Size (mm) | Histology | Kras | BRAF |
|---|---|---|---|---|---|---|---|---|---|
| 3992 | 23949 | 92 | F | W | 2 | 45.0 | High grade Adenoca. | WT | Mutated |
| 3998 | 33706 | 79 | F | W | 2 | 72.0 | Low grade Adenoca. | Mutated | WT |
| 3997 | 30047 | 28 | F | A | 4 | 63.0 | High grade Adenoca. | WT | WT |
| | | | | | | | | | |

| MSS CRC | Pt ID | Age | Gender | Race | Stage | Size (mm) | Histology | Kras | BRAF |
|---|---|---|---|---|---|---|---|---|---|
| 3756 | 10164 | 55 | M | W | 4 | 45.0 | Low grade Adenoca. | WT | WT |
| 3762 | 20390 | 73 | M | W | 4 | 54.0 | Low grade Adenoca. | WT | WT |
| 3753 | 5555 | 49 | F | AA | 4 | 47.0 | Low grade Adenoca.w/mucinous | WT | WT |

The examples set forth above are provided to give those of ordinary skill in the art a complete disclosure and description of how to make and use the embodiments of the compositions, systems and methods of the invention, and are not intended to limit the scope of what the inventors regard as their invention. Modifications of the above-described modes for carrying out the invention that are obvious to persons of skill in the art are intended to be within the scope of the following claims. All patents and publications mentioned in the specification are indicative of the levels of skill of those skilled in the art to which the invention pertains. All headings and section designations are used for clarity and reference purposes only and are not to be considered limiting in any way. For example, those of skill in the art will appreciate the usefulness of combining various aspects from different headings and sections as appropriate according to the spirit and scope of the invention described herein.

The specific embodiments and examples described herein are offered by way of example only.

## Claims

1. An anti-HIDE1 antibody for use in treating cancer in a patient, the use comprising administering the anti-HIDE1 antibody to said patient, wherein said cancer is treated, and wherein the antibody stimulates the immune system by inhibiting the action of HIDE1.

2. An anti-HIDE1 antibody for use according to Claim 1, wherein said cancer is selected from the group consisting of Acute Myeloid Leukemia, Acute Myeloid Leukemia Induction Failure, Acute Lymphoblastic Leukemia, Diffuse Large B-cell Lymphoma, Malignant Lymphoma, Non-Hodgkin Lymphoma, Diffuse Large B-Cell Lymphoma, Glioblastoma multiforme, Mesothelioma, Thymoma, Testicular Germ Cell Tumors, Kidney renal clear cell carcinoma, Sarcoma, Brain Lower Grade Glioma, Chronic Lymphocytic Leukemia, Non-Hodgkin Lymphoma- Follicular Lymphoma, Uterine Carcinosarcoma, Pediatric Brain Tumors, Lung adenocarcinoma, Cervical squamous cell carcinoma, endocervical adenocarcinoma, Pancreatic adenocarcinoma, Skin Cutaneous Melanoma, Kidney renal papillary cell carcinoma, Liver hepatocellular carcinoma; Bladder Urothelial Carcinoma, Colon adenocarcinoma, Head and Neck squamous cell carcinoma, Lung squamous cell carcinoma, Rectum adenocarcinoma, and Stomach adenocarcinoma.

3. An anti-HIDE1 antibody for use according to Claim 1 or 2, wherein said cancer is a cancer having high immune infiltrate of myeloid cells expressing HIDE1.

4. An anti-HIDE1 antibody for use according to Claim 1, wherein the cancer is treated by restricting the pro-tumorigenic effects of the myeloid cells in the tumor microenvironment in the patient.

5. An anti-HIDE1 antibody for use according to Claim 1, wherein the cancer is treated by eliciting one or more of the following effects on immunity in the patient, wherein said effect is selected from the group consisting of: i) increases immune response, (ii) increases T cell activity, (iii) increases activation of αβ and/or γδ T cells, (iv) increases cytotoxic T cell activity, (v) increases NK and/or NKT cell activity, (vi) alleviates αβ and/or γδ T-cell suppression, (vii) increases pro-inflammatory cytokine secretion, (viii) increases IL-2 secretion; (ix) increases interferon-γ production, (x) increases Th1 response, (xi) decrease Th2 response, (xii) decreases or eliminates cell number and/or activity of at least one of regulatory T cells (Tregs), myeloid derived suppressor cells (MDSCs), iMCs, mesenchymal stromal cells, TIE2-expressing monocytes, (xiii) reduces regulatory cell activity, and/or the activity of one or more of myeloid derived suppressor cells (MDSCs), iMCs, mesenchymal stromal cells, TIE2-expressing monocytes, (xiv) decreases or eliminates M2 macrophages, (xv) reduces M2 macrophage pro-tumorigenic activity, (xvi) decreases or eliminates N2 neutrophils, (xvii) reduces N2 neutrophils pro-tumorigenic activity, (xviii) reduces inhibition of T cell activation, (xix) reduces inhibition of CTL activation, (xx) reduces inhibition of NK and/or NKT cell activation, (xxi) reverses αβ and/or γδ T cell exhaustion, (xxii) increases αβ and/or γδ T cell response, (xxiii) increases activity of cytotoxic cells, (xxiv) stimulates antigen-specific memory responses, (xxv) elicits apoptosis or lysis of cancer cells, (xxvi) stimulates cytotoxic or cytostatic effect on cancer cells, (xxvii) induces direct killing of cancer cells, (xxviii) increases Th17 activity and (xxix) modulating myeloid cell polarization, (xxx) modulating myeloid cell shifting toward a pro-inflammatory response, (xxxi) shifting myeloid from M2 toward M1 phenotype, (xxxii) modulating myeloid cell in the TME to support anticancer immune response, (xxxiii) restricting the pro-tumorigenic effects of the myeloid cells in the TME, (xxxiv) enhancing myeloid and lymphoid infiltration into the tumor cite thereby shifting the tumor into more immunogenic, (xxxv) induces complement dependent cytotoxicity and/or antibody dependent cell-mediated cytotoxicity.

6. An anti-HIDE1 antibody for use according to Claim 1, wherein the cancer is treated by depleting myeloid cells or other circulating tumor cells expressing HIDE1 from a patient or patient sample, by:
(a) contacting said patient with an anti-HIDE1 antibody, wherein said anti-HIDE1 antibody binds to HIDE1 expressing cells,
(b) identifying cells to which said anti-HIDE1 antibody has bound, and
(c) removing said cells in step (b) from said patient.

7. An anti-HIDE1 antibody for use according to Claim 1, wherein the anti-HIDE1 antibody binds to an isolated HIDE1 polypeptide consisting of a HIDE1 polypeptide ECD domain having at least 95% identity to the ECD domain of an amino acid sequence selected from the group consisting of the sequences depicted in Figure 66.

8. An anti-HIDE1 antibody for use according to Claim 7, wherein said isolated HIDE1 polypeptide has at least 99% identity to an amino acid sequence selected from the group consisting of the sequences depicted in Figure 66.

9. An anti-HIDE1 antibody for use according to Claim 7, wherein said isolated HIDE1 polypeptide is selected from the group consisting of the sequences depicted in Figure 66.

## Patentansprüche

1. Anti-HIDE1-Antikörper zur Verwendung in der Behandlung von Krebs bei einem Patienten, wobei die Verwendung Verabreichen des anti-HIDE1-Antikörpers an den Patienten umfasst, wobei der Krebs behandelt wird, und wobei der Antikörper das Immunsystem durch Inhibieren der Wirkung von HTDE1 stimuliert.

2. Anti-HIDE1-Antikörper zur Verwendung gemäß Anspruch 1, wobei der Krebs aus der Gruppe ausgewählt ist, welche aus akuter myeloischer Leukämie, Scheitern von Induktion bei akuter myeloischer Leukämie, akuter lymphoblastischer Leukämie, diffusem großzelligem B-Zell-Lymphom, malignem Lymphom, Non-Hodgkin-Lymphom, diffusem großzelligem B-Zell-Lymphom, Glioblastoma multiforme, Mesotheliom, Thymom, Hoden-Keimzelltumoren, klarzelligem Nierenkarzinom, Sarkom, Hirngliom niedrigen Grades, chronischer lymphatischer Leukämie, follikulärem Non-Hodgkin-Lymphom, Gebärmutter-Karzinosarkom, Kinder-Gehirntumoren, Lungen-Adenokarzinom, Gebärmutterhals-Plattenepithelkarzinom, endozervikalem Adenokarzinom, Bauchspeicheldrüsen-Adenokarzinom, kutanem Hautmelanom, papillärem Nierenzellkarzinom, hepatozellulärem Leberkarzinom, Blasen-Urothelkarzinom, Kolon-Adenokarzinom, Plattenepithelkarzinom von Kopf und Hals, Lungen-Plattenepithelkarzinom, Rektum-Adenokarzinom und Magen-Adenokarzinom besteht.

3. Anti-HIDE1-Antikörper zur Verwendung gemäß Anspruch 1 oder 2, wobei der Krebs ein Krebs mit viel Immuninfiltrat von myeloischen Zellen, welche HTDE1 exprimieren, ist.

4. Anti-HIDE1-Antikörper zur Verwendung gemäß Anspruch 1, wobei der Krebs durch Einschränken der tumorfördernden Wirkungen der myeloischen Zellen in der Tumormikroumgebung bei dem Patienten behandelt wird.

5. Anti-HIDE1-Antikörper zur Verwendung gemäß Anspruch 1, wobei der Krebs durch Hervorrufen von einer oder mehr der folgenden Wirkungen auf die Immunität bei dem Patienten behandelt wird, wobei die Wirkung aus der Gruppe ausgewählt ist, welche besteht aus: (i) Erhöhen von Immunantwort, (ii) Erhöhen von T-Zell-Aktivität, (iii) Erhöhen von Aktivierung von αβ- und/oder αδ-T-Zellen, (iv) Erhöhen von zytotoxischer T-Zell-Aktivität, (v) Erhöhen von NK- und/oder NKT-Zell-Aktivität, (vi) Verringern von αβ- und/oder γδ-T-Zell-Suppression, (vii) Erhöhen von proinflammatorisches Zytokin-Sekretion, (viii) Erhöhen von IL-2-Sekretion, (ix) Erhöhen von Interferon-γ-Herstellung, (x) Erhöhen von Thl-Antwort, (xi) Erniedrigen von Th2-Antwort, (xii) Erniedrigen oder Beseitigen von Zellenanzahl und/oder -aktivität von mindestens einer von regulatorischen T-Zellen (Tregs), myeloiden Suppressorzellen (MDSCs), iMCs, mesenchymalen Stromazellen, TIE2-exprimierenden Monozyten, (xiii) Verringern von regulatorischer Zellaktivität, und/oder der Aktivität von einer oder mehr von myeloiden Suppressorzellen (MDSCs), iMCs, mesenchymalen Stromazellen, TIE2-exprimierenden Monozyten, (xiv) Verringern oder Beseitigen von M2-Makrophagen, (xv) Verringern von tumorfördernder M2-Makrophagen-Aktivität, (xvi) Verringern oder Beseitigen von N2-Neutrophilen, (xvii) Verringern von tumorfördernder N2-Neutrophilen-Aktivität, (xviii) Verringern von Inhibierung von T-Zell-Aktivierung, (xix) Verringern von Inhibierung von CTL-Aktivierung, (xx) Verringern von Inhibierung von NK- und/oder NKT-Zell-Aktivierung, (xxi) Umkehren von αβ- und/oder γδ-T-Zell-Erschöpfung, (xxii) Erhöhen von αβ- und/oder γδ-T-Zell-Antwort, (xxiii) Erhöhen von Aktivität von zytotoxischen Zellen, (xxiv) Stimulieren von antigenspezifischen Gedächtnisantworten, (xxv) Hervorrufen von Apoptose oder Lyse von Krebszellen, (xxvi) Stimulieren von zytotoxischer oder zytostatischer Wirkung auf Krebszellen, (xxvii) Induzieren von direktem Abtöten von Krebszellen, (xxviii) Erhöhen von Th17-Aktivität und (xxix) Modulieren von Polarisation von myeloischen Zellen, (xxx) Modulieren von myeloischen Zellen hin zu einer proinflammatorischen Antwort, (xxxi) Verändern von Myeloid vom M2- zum M1-Phänotyp, (xxxii) Modulieren von myeloischen Zellen in der TME, wobei Antikrebs-Immunantwort unterstützt wird, (xxxiii) Einschränken der tumorfördernden Wirkungen der myeloischen Zellen in der TME, (xxxiv) Steigern von Myeloid- und Lymphoid-Infiltration in den Tumor, wobei der Tumor stärker immunogen gemacht wird, (xxxv) Induzieren von komplementabhängiger Zytotoxizität und/oder antikörperabhängiger zellvermittelter Zytotoxizität.

6. Anti-HIDE1-Antikörper zur Verwendung gemäß Anspruch 1, wobei der Krebs durch Depletion von myeloischen Zellen oder anderen zirkulierenden Tumorzellen, welche HTDE1 exprimieren, von einem Patienten oder einer Patientenprobe behandelt wird durch:
(a) Inkontaktbringen des Patienten mit einem anti-HIDE1-Antikörper, wobei der anti-HIDE1-Antikörper an HIDE1-exprimierende Zellen bindet,
(b) Identifizieren von Zellen, an welche der anti-HIDE1-Antikörper gebunden hat, und
(c) Entfernen der Zellen in Schritt (b) von dem Patienten.

7. Anti-HIDE1-Antikörper zur Verwendung gemäß Anspruch 1, wobei der anti-HIDE1-Antikörper an ein isoliertes HIDE1-Polypeptid, bestehend aus einer HIDE1-Polypeptid-ECD-Domäne mit mindestens 95 % Identität mit der ECD-Domäne einer Aminosäuresequenz, welche aus der Gruppe ausgewählt ist, die aus den in Figur 66 gezeigten Sequenzen besteht, bindet.

8. Anti-HIDE1-Antikörper zur Verwendung gemäß Anspruch 7, wobei das isolierte HIDE1-Polypeptid mindestens 99 % Identität mit einer Aminosäuresequenz, welche aus der Gruppe ausgewählt ist, die aus den in Figur 66 gezeigten Sequenzen besteht, aufweist.

9. Anti-HIDE1-Antikörper zur Verwendung gemäß Anspruch 7, wobei das isolierte HIDE1-Polypeptid aus der Gruppe ausgewählt ist, welche aus den in Figur 66 gezeigten Sequenzen besteht.

## Revendications

1. Anticorps anti-HIDE1 destiné à être utilisé dans le traitement du cancer chez un patient, l'utilisation comprenant l'administration de l'anticorps anti-HIDE1 audit patient, dans lequel ledit cancer est traité et dans lequel l'anticorps stimule le système immunitaire en inhibant l'action de HIDE1.

2. Anticorps anti-HIDE1 destiné à être utilisé selon la revendication 1, dans lequel ledit cancer est choisi dans le groupe constitué de la leucémie myéloïde aiguë, l'échec d'induction de leucémie myéloïde aiguë, la leucémie lymphoblastique aiguë, le lymphome diffus à grandes cellules B, le lymphome malin, le lymphome non hodgkinien, le lymphome diffus à grandes cellules B, le glioblastome multiforme, le mésothéliome, le thymome, les tumeurs des cellules germinales testiculaires, le carcinome à cellules claires du rein, le sarcome, le gliome cérébral de bas grade, la leucémie lymphocytaire chronique, le lymphome non hodgkinien - lymphome folliculaire, le carcinosarcome utérin, les tumeurs cérébrales pédiatriques, l'adénocarcinome pulmonaire, le carcinome malphigien cervical, l'adénocarcinome endocervical, l'adénocarcinome du pancréas, le mélanome cutané, le carcinome papillaire du rein, le carcinome hépatocellulaire ; le carcinome urothélial, l'adénocarcinome du colon, le carcinome épidermoïde de la tête et du cou, le carcinome épidermoïde du poumon, l'adénocarcinome du rectum et l'adénocarcinome de l'estomac.

3. Anticorps anti-HIDE1 destiné à être utilisé selon la revendication 1 ou 2, dans lequel ledit cancer est un cancer présentant une infiltration immunitaire élevée de cellules myéloïdes exprimant HIDE1.

4. Anticorps anti-HIDE1 destiné à être utilisé selon la revendication 1, dans lequel le cancer est traité en restreignant les effets pro-tumorigènes des cellules myéloïdes dans le microenvironnement tumoral du patient.

5. Anticorps anti-HIDE1 destiné à être utilisé selon la revendication 1, dans lequel ledit cancer est traité en provoquant un ou plusieurs des effets suivants sur l'immunité du patient, dans lequel ledit effet est choisi dans le groupe consistant en : i) l'augmentation de la réponse immunitaire, (ii) l'augmentation de l'activité des lymphocytes T, (iii) l'augmentation de l'activation des lymphocytes T αβ et/ou γδ, (iv) l'augmentation de l'activité des lymphocytes T cytotoxiques, (v) l'augmentation de l'activité des cellules NK et/ou NKT, (vi) l'allégement de la suppression des lymphocytes T αβ et/ou γδ, (vii) l'augmentation de la sécrétion de cytokine pro-inflammatoire, (viii) l'augmentation de la sécrétion d'IL-2 ; (ix) l'augmentation de la production d'interféron γ, (x) l'augmentation de la réponse Th1, (xi) la baisse de la réponse Th2, (xii) la baisse ou l'élimination du nombre et/ou de l'activité des cellules d'au moins une des lymphocytes T régulatrices (Tregs), des cellules myéloïdes suppressives (MDSC), des iMC, des cellules stromatolithiques mésenchymateuses, des monocytes exprimant TIE2, (xiii) la réduction de l'activité des cellules régulatrices et/ou de l'activité d'une ou plusieurs des cellules myéloïdes suppressives (MDSC), des iMC, des cellules stromatolithiques mésenchymateuses, des monocytes exprimant TIE2, (xiv) la baisse ou l'élimination des macrophages M2, (xv) la réduction de l'activité pro-tumorigène des macrophages M2, (xvi) la réduction ou l'élimination des neutrophiles N2, (xvii) la réduction de l'activité pro-tumorigène des neutrophiles N2, (xviii) la baisse de l'inhibition de l'activation des lymphocytes T, (xix) la réduction de l'inhibition de l'activation des CTL, (xx) la réduction de l'inhibition de l'activation des cellules NK et/ou NKT, (xxi) l'inversion de l'épuisement des lymphocytes T αβ et/ou γδ, (xxii) l'augmentation de la réponse des lymphocytes T αβ et/ou γδ, (xxiii) l'augmentation de l'activité des lymphocytes cytotoxiques, (xxiv) la stimulation des réponses de mémoire spécifiques aux antigènes, (xxv) le déclenchement de l'apoptose ou de la lyse des cellules cancérigènes, (xxvi) la stimulation de l'effet cytotoxique ou cytostatique sur les cellules cancérigènes, (xxvii) l'induction de la destruction directe des cellules cancérigènes, (xxviii) l'augmentation de l'activité de Th17 et (xxix) la modulation de la polarisation des cellules myéloïdes, (xxx) la modulation des cellules myéloïdes vers une réponse pro-inflammatoire, (xxxi) le passage des cellules myéloïdes d'un phénotype M2 à M1, (xxxii) la modulation des cellules myéloïdes dans le TME pour renforcer une réponse immunitaire anticancéreuse, (xxxiii) la restriction des effets pro-tumorigènes des cellules myéloïdes dans le TME, (xxxiv) l'augmentation de l'infiltration myéloïde et lymphoïde dans le site tumoral, portant ainsi la tumeur à un état plus immunogène, (xxxv) l'induction d'une cytotoxicité dépendante du complément et/ou d'une cytotoxicité à médiation cellulaire dépendante des anticorps.

6. Anticorps anti-HIDE1 destiné à être utilisé selon la revendication 1, dans lequel le cancer est traité par déplétion des cellules myéloïdes ou autres cellules tumorales circulant exprimant HIDE1 chez un patient ou dans un prélèvement d'un patient, en :
a) mettant en contact ledit patient avec un anticorps anti-HIDE1, dans lequel l'anticorps anti-HIDE1 se lie aux cellules exprimant HIDE1,
b) l'identification des cellules auxquelles s'est lié l'anticorps anti-HIDE1, et
c) l'élimination desdites cellules à l'étape (b) chez ledit patient.

7. Anticorps anti-HIDE1 destiné à être utilisé selon la revendication 1, dans lequel l'anticorps anti-HIDE1 se lie à un polypeptide HIDE1 isolé consistant en un domaine ECD de polypeptide HIDE1 présentant une identité d'au moins 95 % avec le domaine ECD d'une séquence d'acides aminés choisie dans le groupe consistant en les séquences présentées à la figure 66.

8. Anticorps anti-HIDE1 destiné à être utilisé selon la revendication 7, dans lequel ledit polypeptide HIDE1 isolé présente une identité d'au moins 99 % avec une séquence d'acides aminés choisie dans le groupe consistant en les séquences présentées à la figure 66.

9. Anticorps anti-HIDE1 destiné à être utilisé selon la revendication 7, dans lequel ledit polypeptide HIDE1 isolé est choisi dans le groupe consistant en les séquences présentées à la figure 66.
